# EUROPEAN PATENT APPLICATION

(11) **EP 4 831 170 A1**
(43) Date of publication of application: **28.10.2026**
(21) Application number: 24906354.6
(22) Date of filing: 17.12.2024
(51) Int. Cl.: C07D 471/04, C07D 217/00, A61K 31/395, A61K 31/4725, A61P 35/00

(54) **NITROGEN HETEROCYCLIC COMPOUND AND USE THEREOF**

(30) Priority: 18.12.2023 CN 202311754693
(71) Applicant: Nanjing Reju Therapeutics Co. Ltd., Nanjing, Jiangsu 211199 (CN)
(72) Inventor: JI, Yin, Nanjing, Jiangsu 211199 (CN); LIU, Weiguo, Nanjing, Jiangsu 211199 (CN); WANG, Dezhong, Nanjing, Jiangsu 211199 (CN); WANG, Xueping, Nanjing, Jiangsu 211199 (CN)
(74) Representative: Calysta NV
(86) International application number: PCT/CN2024/140005
(87) International publication number: WO 2025/130875

(57) **Abstract**

The present application provides a nitrogen heterocyclic compound or a pharmaceutically acceptable salt, solvate, hydrate, polymorphic substance, cocrystal, tautomer, stereoisomer or isotope compound thereof. The nitrogen heterocyclic compound has a structure represented by formula I. The nitrogen heterocyclic compound provided by the present application can be used for removing aging cells.

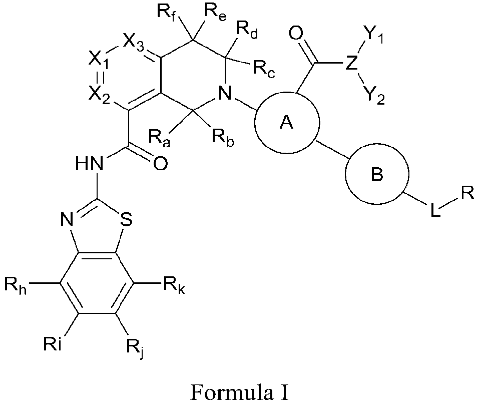

## Description

### TECHNICAL FIELD

The present invention relates to the field of medicine, and in particular to a nitrogen heterocyclic compound capable of targeted killing senescent cells, and its use in preventing or treating aging-related diseases.

### BACKGROUND

With aging, senescent cells accumulate in tissues and organs of an individual. Accumulated senescent cells in the body serve as a critical driving factor for individual aging and the occurrence of aging-related diseases: on one hand, the senescence of proliferative cells or stem cells leads to the decline of the individual's regenerative capacity, impairing the recovery and normal functions of the body; more importantly, senescent cells secrete a large number of inflammatory factors, known as Senescence-Associated Secretory Phenotype (SASP), which creates a chronic inflammatory microenvironment, accelerates the aging process of the body, and promotes the development of age-related diseases.

Given the relationship between senescent cells and diseases, selective clearance of senescent cells is a promising therapeutic approach for treating aging-related diseases and improving physiological function in elderly individuals. The present disclosure addresses these needs and provides related advantages.

### SUMMARY

In order to address at least one technical problem in the prior art, the present application provides a novel class of nitrogen heterocyclic compounds that can selectively clear senescent cells.

In a first aspect, the present application provides a nitrogen heterocyclic compound, or a pharmaceutically acceptable salt, a solvate, a hydrate, a polymorph, a co-crystal, a tautomer, a stereoisomer, or an isotopic compound thereof, wherein the nitrogen heterocyclic compound has the structure shown by Formula I: 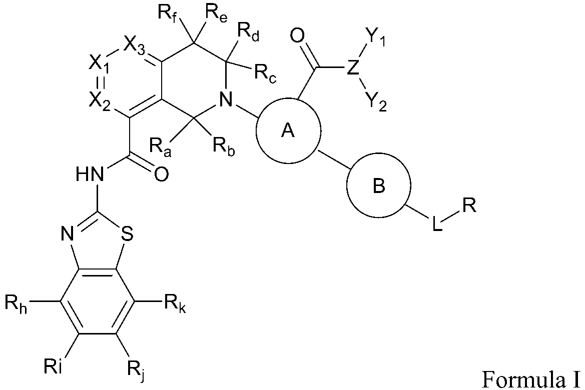
X₁, X₂ and X₃ are each independently selected from CR_{g} and N, and X₁, X₂ and X₃ are not CR_{g} at the same time;
Rₐ, R_{b}, R_{c}, R_{d}, Rₑ, R_{f}, R_{g}, Rₕ, Rᵢ, Rⱼ and Rₖ are each independently selected from hydrogen, deuterium, halogen-substituted or unsubstituted C1-C8 alkyl, halogen-substituted or unsubstituted C3-C8 cycloalkyl, halogen-substituted or unsubstituted C2-C8 alkenyl, halogen-substituted or unsubstituted C2-C8 alkynyl, halogen atom, hydroxy, amino, nitro, cyano, carboxy, acyl, and halogen-substituted or unsubstituted C2-C8 alkoxy;
Z is selected from O and N;
when Z is O, Y₂ is absent, and Y₁ is independently selected from hydrogen, deuterium, substituted or unsubstituted C1-C10 alkyl, substituted or unsubstituted C3-C10 cycloalkyl, -R₁-R₂-R₃, -R₂-R₃ and -R₁-R₃, wherein R₁ is selected from substituted or unsubstituted C1-C10 alkylene, R₂ is selected from -O-C(O)- and -O-C(O)-O-, and R₃ is selected from substituted or unsubstituted C1-C10 alkyl, substituted or unsubstituted C3-C20 cycloalkyl, substituted or unsubstituted C2-C20 alkenyl, substituted or unsubstituted C2-C20 alkynyl, substituted or unsubstituted C6-C20 aryl, substituted or unsubstituted C1-C20 heteroaryl, and substituted or unsubstituted C2-C20 heteroalicyclic group;
when Z is N, Y₁ and Y₂ are each independently selected from hydrogen, deuterium, C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl and C2-C8 alkynyl may be replaced by one or two or more groups selected from -O-, - S-, -SO₂-, -C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are optionally substituted by one or two or more substituents selected from halogen atom, cyano, nitro, C6-C20 aryl, C1-C20 heteroaryl, C1-C10 alkoxy, C6-C20 aryloxy, C2-C20 heteroalicyclic group, amino, hydroxy, mercapto, 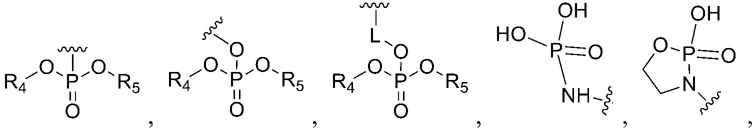 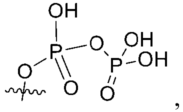 and -NR₄R₅; or
Y₁ and Y₂ together with the N atoms to which they are attached form a heteroalicyclic group, preferably a C2-C20 heteroalicyclic group, wherein the heteroalicyclic group is optionally substituted with one or two or more substituents of halogen atom, cyano, nitro, C6-C20 aryl, C1-C20 heteroaryl, C1-C10 chain alkoxy, C6-C20 aryloxy, C2-C20 heteroalicyclic group, amino, hydroxy, mercapto, 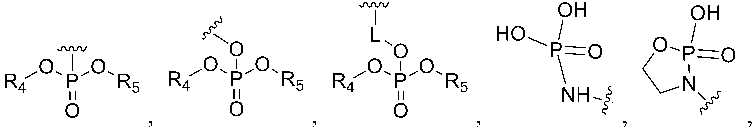 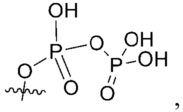 and -NR₄R₅; or
Y₁ is hydrogen or deuterium, and Y₂ is selected from C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl and C2-C8 alkynyl may be replaced by one or two or more groups selected from -O-, -S-, -SO₂-, -C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C2-C8 alkenyl, or C2-C8 alkynyl, and wherein the substituents of at least two positions together form an aliphatic ring, a heteroalicyclic ring, an aromatic ring or a heteroaromatic ring;
R₄ and R₅ are independently selected from hydrogen, deuterium, aryl, heteroaryl, C1-C8 chain alkyl, C3-C8 cycloalkyl, C2-C8 alkenyl, and C2-C8 alkynyl, wherein the aryl and heteroaryl are optionally substituted with halogen atom, hydroxy, mercapto, amino, nitro, cyano, carboxy, acyl, alkoxy, aryl, heteroaryl, heteroalicyclic group, C1-C10 alkyl, C3-C8 cycloalkyl, C2-C8 alkenyl, or C2-C8 alkynyl, and wherein optionally, the substituents of at least two positions together form an aliphatic ring, a heteroalicyclic ring, an aromatic ring or a heteroaromatic ring;
ring A is independently selected from substituted or unsubstituted aromatic ring or heteroaromatic ring;
ring B is absent or independently selected from substituted or unsubstituted aromatic ring or heteroaromatic ring, C3-C8 cycloalkyl, C3-C8 heterocycloalkyl, C6-C10 spiro-ring, C6-C10 spiro-heterocycle, C6-C10 fused cycle, and C6-C10 fused heterocycle;
L is absent or selected from C1-C6 alkylene, -C1-C6 alkylene-O-, and -C1-C3 alkylene-O-C1-C3 alkylene-, wherein the alkylene is optionally substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C3-C8 cycloalkyl, C2-C8 alkenyl or C2-C8 alkynyl;
R is independently selected from aromatic ring, heteroaromatic ring, aromatic ring-fused heteroaromatic ring, C3-C8 cycloalkyl, C3-C8 heterocycloalkyl, C6-C10 spiro-ring, and C6-C10 fused cycloalkyl; wherein the R is optionally substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C3-C8 cycloalkyl, C2-C8 alkenyl or C2-C8 alkynyl.

In some embodiments, X₃ is CR_{g}.

In some embodiments, X₁ is CR_{g}, X₂ is N.

In some embodiments, X₁ is N, X₂ is CR_{g}.

In some embodiments, X₃ is N, and X₁ and X₂ are CR_{g}.

In some embodiments, X₁ is N, and X₂ and X₃ are CR_{g}.

In some embodiments, X₂ is N, and X₁ and X₃ are CR_{g}.

In some embodiments, Rₐ, R_{b}, R_{c}, R_{d}, Rₑ, R_{f}, R_{g}, Rₕ, Rᵢ, Rⱼ and Rₖ are each independently selected from hydrogen, deuterium, halogen-substituted or unsubstituted C1-C6 chain alkyl, halogen-substituted or unsubstituted C3-C6 cycloalkyl, halogen-substituted or unsubstituted C2-C6 alkenyl, halogen-substituted or unsubstituted C2-C6 alkynyl, halogen atom, hydroxy, amino, nitro, cyano, carboxy, and halogen-substituted or unsubstituted C2-C6 alkoxy.

In some embodiments, R_{g} is hydrogen.
in some embodiments, Rₐ, R_{b}, R_{c}, R_{d}, Rₑ, R_{f}, R_{g}, Rₕ, Rᵢ, Rⱼ and Rₖ are each independently selected from hydrogen, deuterium, halogen-substituted or unsubstituted C1-C6 chain alkyl, halogen-substituted or unsubstituted C3-C6 cycloalkyl, halogen atom, and C2-C6 alkoxy.

In some embodiments, Rₐ, R_{b}, R_{c}, R_{d}, Rₑ, R_{f}, R_{g}, Rₕ, Rᵢ, Rⱼ, and Rₖ are hydrogen.

In some embodiments, Z is O, Y₂ is absent, and Y₁ is selected from hydrogen, deuterium, substituted or unsubstituted C1-C10 alkyl, substituted or unsubstituted C3-C10 cycloalkyl, -R₁-R₂-R₃, -R₂-R₃ and -R₁-R₃, wherein R₁ is selected from substituted or unsubstituted C1-C10 alkylene, R₂ is selected from -O-C(O)- and -O-C(O)-O-, and R₃ is selected from substituted or unsubstituted C1-C10 alkyl, substituted or unsubstituted C3-C20 cycloalkyl, substituted or unsubstituted C2-C20 alkenyl, substituted or unsubstituted C2-C20 alkynyl, substituted or unsubstituted C6-C20 aryl, substituted or unsubstituted C1-C20 heteroaryl, and substituted or unsubstituted C2-C20 heteroalicyclic group.

In some embodiments, Z is O, Y₂ is absent, and Y₁ is selected from hydrogen and deuterium.

In some embodiments, Z is O, Y₂ is absent, and Y₁ is selected from substituted or unsubstituted C1-C6 alkyl and substituted or unsubstituted C3-C10 cycloalkyl.

In some embodiments, Z is O, Y₂ is absent, and Y₁ is selected from -R₁-R₂-R₃, -R₂-R₃, and -R₁-R₃.

In some embodiments, R₁ is selected from substituted or unsubstituted C1-C6 alkylene, preferably substituted or unsubstituted C1-C3 alkylene, and more preferably, methylene.

In some embodiments, R₃ is selected from substituted or unsubstituted C1-C8 alkyl, substituted or unsubstituted C3-C10 cycloalkyl, substituted or unsubstituted C2-C8 alkenyl, substituted or unsubstituted C2-C8 alkynyl, substituted or unsubstituted C6-C12 aryl, substituted or unsubstituted C2-C12 heteroaryl, and substituted or unsubstituted C1-C12 heteroalicyclic group.

In some embodiments, R₃ is selected from substituted or unsubstituted C1-C6 alkyl, substituted or unsubstituted C6-C12 aryl, substituted or unsubstituted C1-C10 heteroaryl, substituted or unsubstituted C3-C8 cycloalkyl, and substituted or unsubstituted C1-C10 heteroalicyclic group.

In some embodiments, R₃ is selected from the following groups:

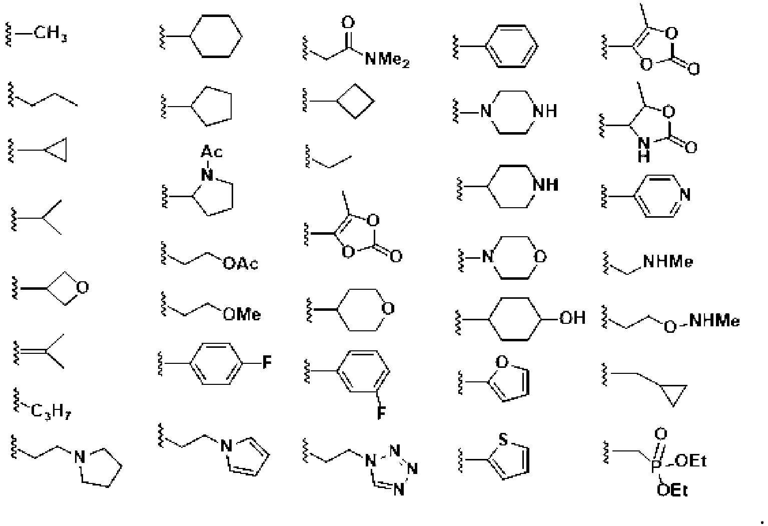

In some embodiments, Z is N.

In some embodiments, Z is N, and Y₁ and Y₂ are independently selected from C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl , C3-C8 cycloalkyl, C2-C8 alkenyl, and C2-C8 alkynyl may be replaced by one or two or more groups selected from -O-, -S-, -SO₂-, - C(O)-, and -NR₃-.

In some embodiments, the C1-C10 alkyl, C3-C8 cycloalkyl, C2-C8 alkenyl, C2-C8 alkynyl, C6-C20 aryl, C1-C20 heteroaryl, and C2-C20 heteroalicyclic group are optionally substituted with one or two or more substituents of halogen atom, cyano, nitro, C6-C20 aryl, C1-C20 heteroaryl, C1-C10 chain alkoxy, C6-C20 aryloxy, C2-C20 heteroalicyclic group, amino, hydroxy, mercapto, 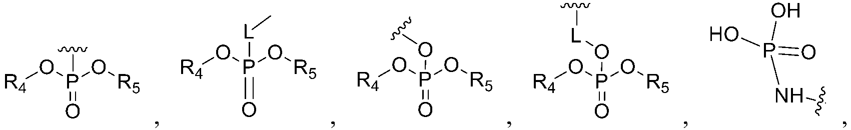 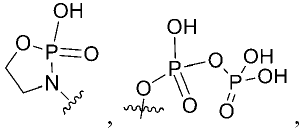 and -NR₄R₅.

In some embodiments, Y₁ and Y₂ together with the N atoms to which they are attached form a heteroalicyclic group, preferably a C2-C20 heteroalicyclic group.

In some embodiments, Y₁ is hydrogen, and Y₂ is selected from C1-C10 alkyl, C3-C8 cycloalkyl, C2-C8 alkenyl, C2-C8 alkynyl, C6-C20 aryl, C1-C20 heteroaryl, and C2-C20

heteroalicyclic group, wherein CH₂ in the C1-C10 alkyl, C3-C8 cycloalkyl, C2-C8 alkenyl and C2-C8 alkynyl may be replaced by one or two or more groups selected from -O-, -S-, -SO₂-, - C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C2-C8 alkenyl, or C2-C8 alkynyl, and wherein the substituents of at least two positions together form an aliphatic ring, a heteroalicyclic ring, an aromatic ring or a heteroaromatic ring.

In some embodiments, Y₁ and Y₂ together with the N atoms to which they are attached form a C2-C20 heteroalicyclic group such as a C2-C10 heteroalicyclic group, wherein the ring of the C2-C20 heteroalicyclic group optionally contains 1 or 2 additional heteroatoms selected from N and O.

In some embodiments, the C2-C20 heteroalicyclic group are optionally substituted with one or two or more substituents of halogen atom, cyano, nitro, C6-C10 aryl, C1-C10 heteroaryl, C1-C6 chain alkoxy, C6-C10 aryloxy, C2-C10 heteroalicyclic group, amino, hydroxy, mercapto, carbonyl, carboxy, acyl, 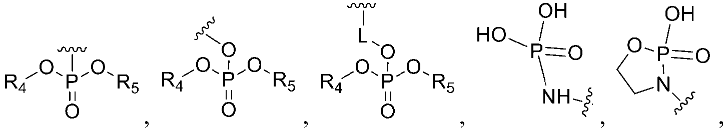 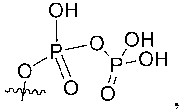 and -NR₄R₅.

In some embodiments, R₄ and R₅ are independently selected from hydrogen, C6-C10 aryl, C1-C10 heteroaryl, C1-C8 chain alkyl, C3-C8 cycloalkyl, C2-C8 alkenyl, and C2-C8 alkynyl, wherein the aryl and heteroaryl are optionally substituted with halogen atom, hydroxy, mercapto, amino, nitro, cyano, carboxy, acyl, C1-C8 alkoxy, C6-C10 aryl, C1-C10 heteroaryl, C2-C10 heteroalicyclic group, C1-C8 alkyl, C3-C8 cycloalkyl, C2-C6 alkenyl, or C2-C8 alkynyl, and optionally, wherein the substituents of at least two positions together form a C3-C10 aliphatic ring, a C2-C10 heteroalicyclic ring, a C6-C10 aromatic ring or a C1-C10 heteroaromatic ring. In some embodiments, R₄ and R₅ are independently selected from hydrogen and C1-C6 alkyl.

In some embodiments, the C2-C20 heteroalicyclic group are optionally substituted with a substituent selected from halogen atom, hydroxy, mercapto, amino, nitro, cyano, C1-C10 alkoxy, C1-C10 alkyl, C3-C8 cycloalkyl, C2-C8 alkenyl, C2-C8 alkynyl, 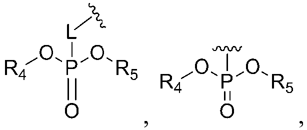 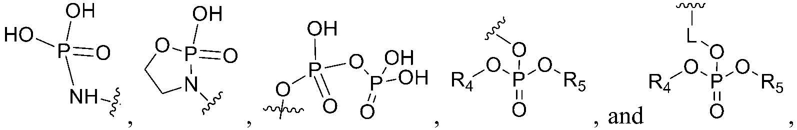 wherein R₄ and R₅ are independently selected from hydrogen and C1-C6 alkyl.

In some embodiments, the C2-C8 heteroalicyclic group is optionally substituted with a substituent selected from halogen, -NH₂, -OH, -NO₂, carbonyl, -CH₂OH, carboxy, methyl, ethyl, propyl, isopropyl, methoxy, ethoxy, propoxy, and isopropoxy.

In some embodiments, Y₁ and Y₂ together with the N atoms to which they are attached form a C4-C20 heteroalicyclic group, selected from the groups shown below: 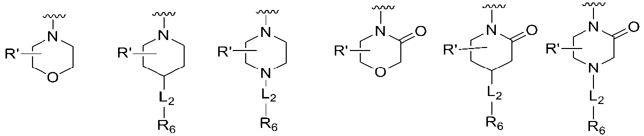 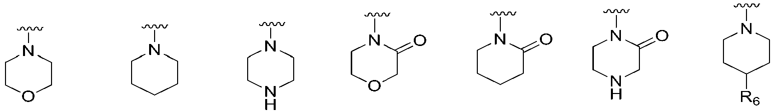 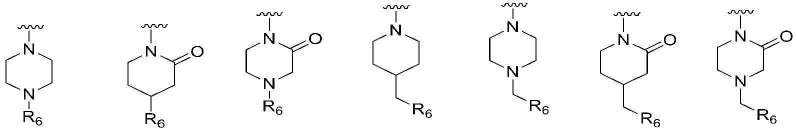 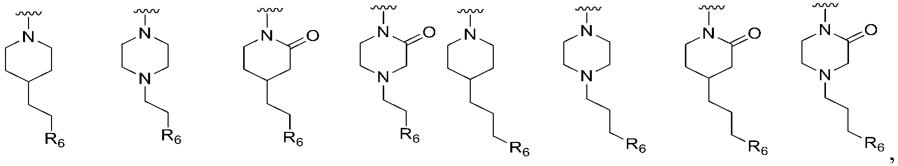
R' each independently represents no substituent, a single substituent or a plurality of substituents, each of the substituents being independently selected from deuterium, hydroxy, halogen, NH₂, carboxy (-COOH), 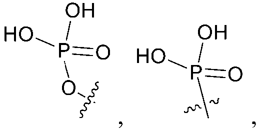 C1-C6 alkyl, halogen-substituted C1-C6 alkyl, hydroxy substituted C1-C6 chain alkyl, amino-substituted C1-C6 chain alkyl, morpholino-substituted C1-C6 chain alkyl, -COO-C1-C6 alkyl, cyano, C1-C6 alkoxy, C3-C6 cycloalkyl, halogen-substituted C3-C6 cycloalkyl, hydroxy-substituted C3-C6 cycloalkyl, phenyl, and benzyl;
L₂ is absent or C1-C6 alkylene, halogen, hydroxy, C1-C6 alkoxy-substituted C1-C6 alkylene, preferably methylene, ethylene, or propylene;
R₆ is H, deuterium, halogen, hydroxy, NH₂, carboxy (-COOH), -CONH₂, sulfo (-SO₃H), - SO₂-C1-C6 alkyl, 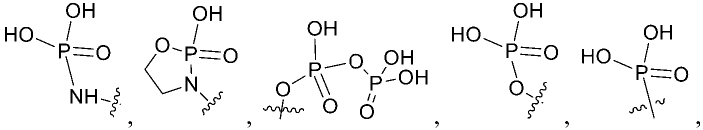 C1-C6 alkyl, halogen-substituted C1-C6 alkyl, morpholino-substituted C1-C6 alkyl, -COO-C1-C6 alkyl, cyano, C1-C6 alkoxy, hydroxy-substituted C1-C6 alkyl, amino-substituted C1-C6 alkyl, C3-C6 cycloalkyl, halogen-substituted C3-C6 cycloalkyl, hydroxy-substituted C3-C6 cycloalkyl, phenyl or benzyl.

In some embodiments, ring A is selected from pyridine, pyrimidine, pyridazine, quinoline, thiazole, imidazole, pyrrole, pyrazole, thiophene, thienofuran, thienothiazole, carbazolopyrrole, pyridopyrazole, pyridopyrrole, indole, azaindole, isoquinoline, anthracene, phenanthrene, benzofuran, benzothiophene, and indazole.

In some embodiments, ring A is a pyridine ring, a pyrimidine ring, or a pyridazine ring.

In some embodiments, ring B is selected from: pyrazole, pyrrole, imidazole, pyridine, pyrimidine, indole, indazole, tetrahydropyrrole, piperidine, azetidine, cubane, pyridazine, quinoline, thiazole, imidazole, pyrrole, pyrazole, thiophene, thienofuran, thienothiazole, carbazolopyrrole, pyridopyrazole, pyridopyrrole, indole, azaindole, isoquinoline, anthracene, phenanthrene, benzofuran, benzothiophene, indazole.

In some embodiments, ring B is absent.

In some embodiments, ring B is selected from the following groups:

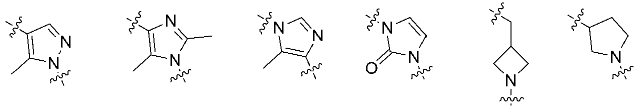

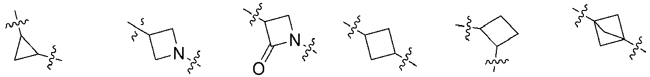

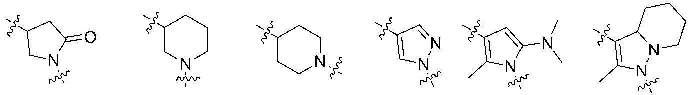

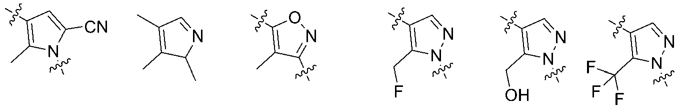

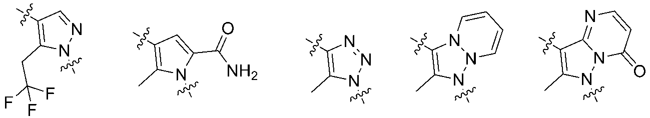

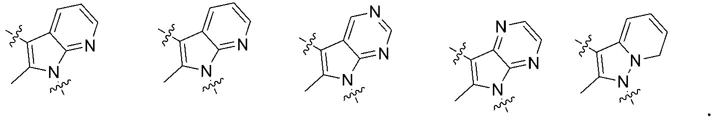

In some embodiments, ring B is selected from

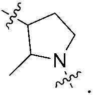

In some embodiments, R is selected from phenyl, pyridyl, pyrimidinyl, pyrazinyl, cycloalkyl, quinolinyl, isoquinolinyl, tetrahydroquinolinyl, tetrahydroisoquinolinyl, indolyl, indazolyl, cyclohexyl, cyclopentyl, cycloheptyl, oxaspiro[3.3]heptanyl, spiro[2.5]octanyl, and adamantyl.

In some embodiments, R is selected from the following groups:

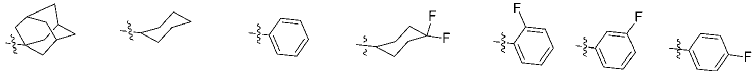

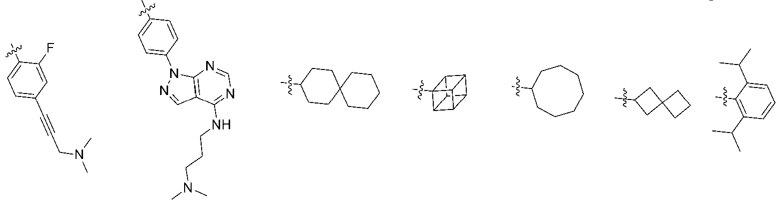

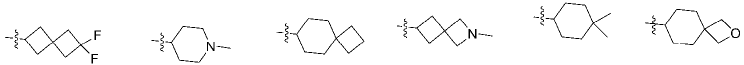

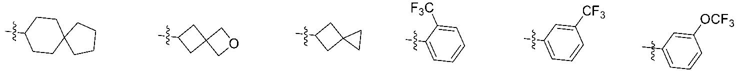

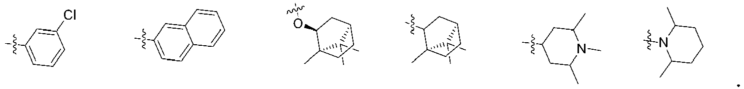

In some embodiments, R is selected from the following groups:

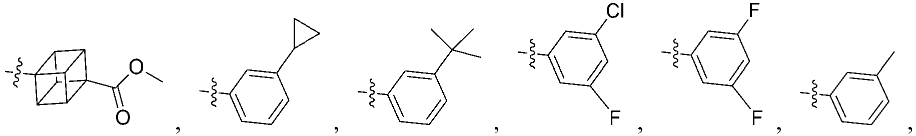

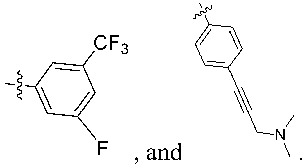

In some embodiments, the compound has the structure shown by Formula IA: 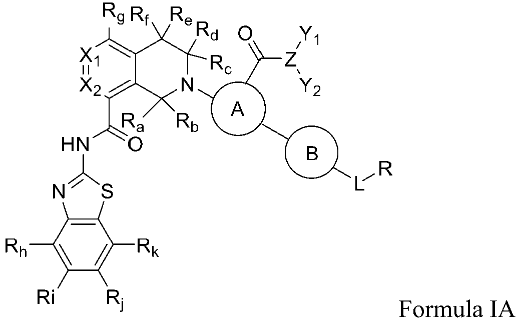 wherein each symbol is as defined in the compound of Formula I.

In some embodiments, the compound has the structure shown as any one of Formula I-1 to Formula I-6:

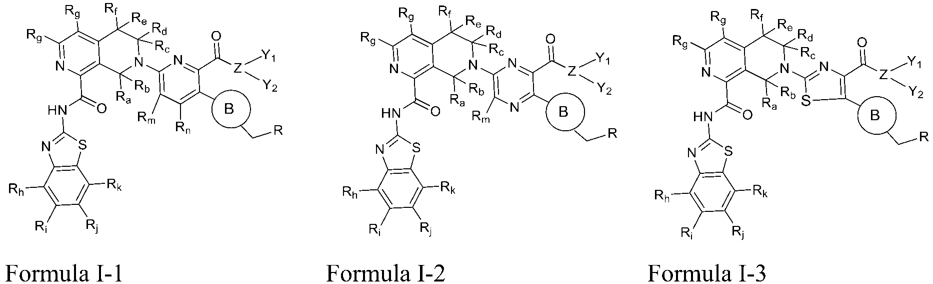

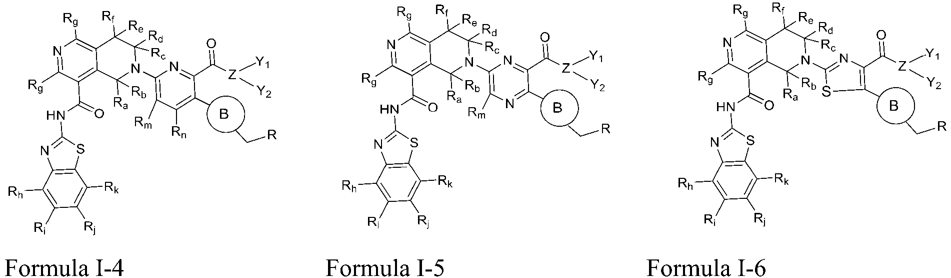

In each of above Formulas, each symbol is as defined in the compound of Formula I.

In some embodiments, the compound has the structure shown as any one of Formula I-7 to Formula I-9:

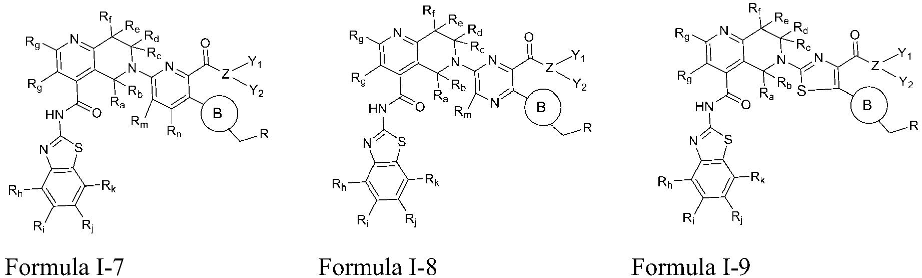

In each of above Formulas, each symbol is as defined in the compound of Formula I.

In some embodiments, the nitrogen heterocyclic compound is selected from the following groups:

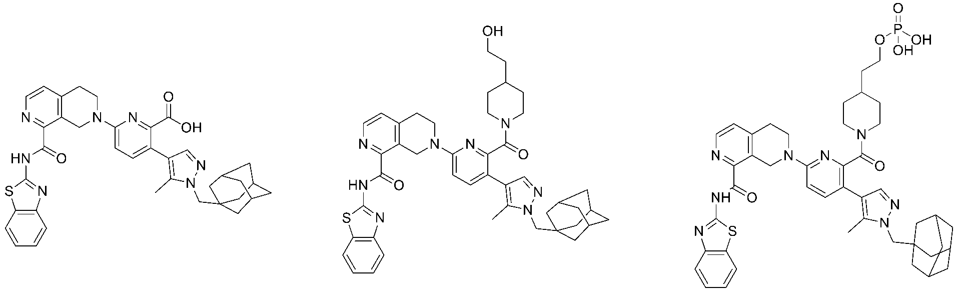

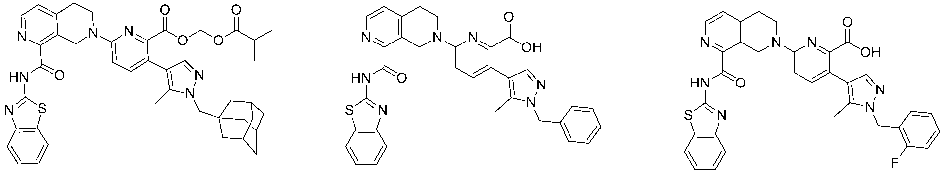

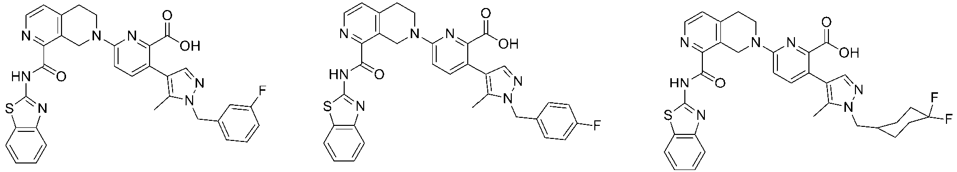

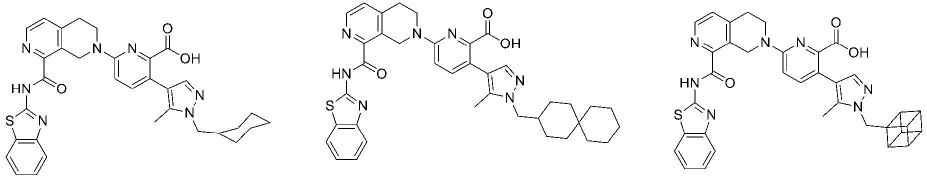

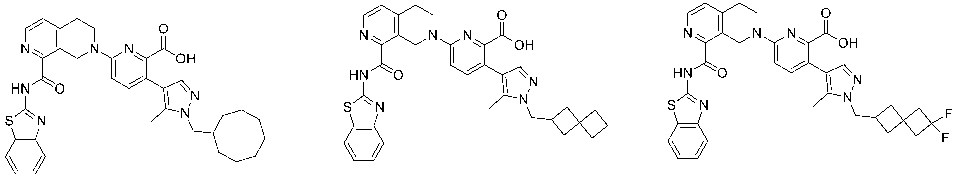

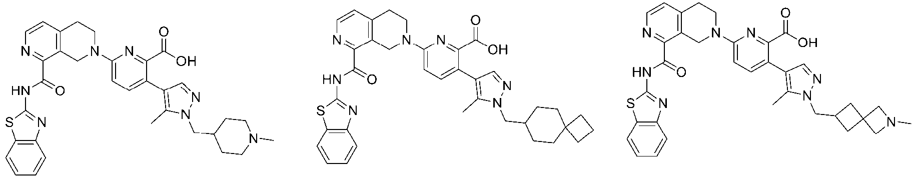

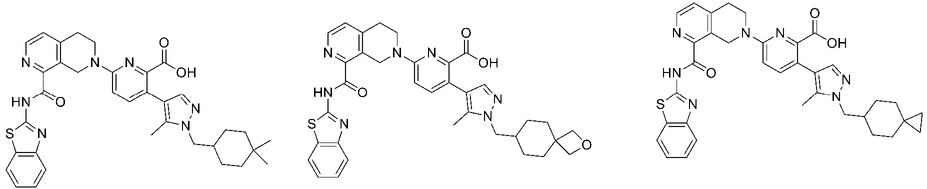

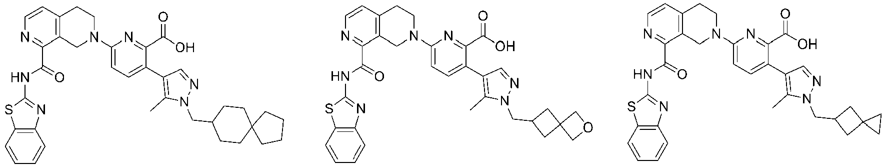

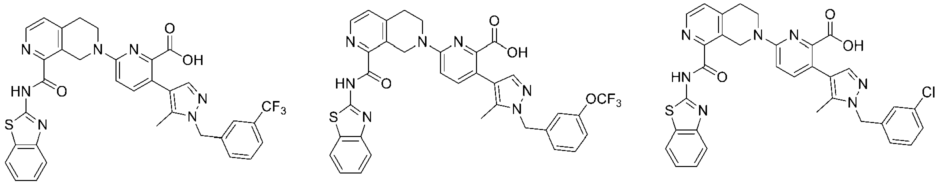

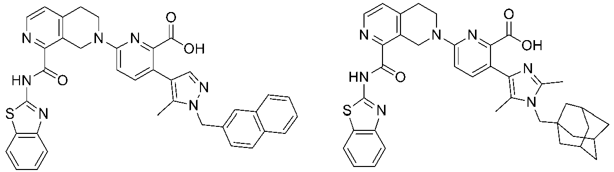

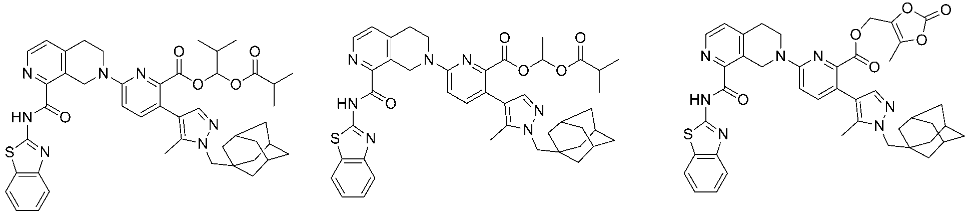

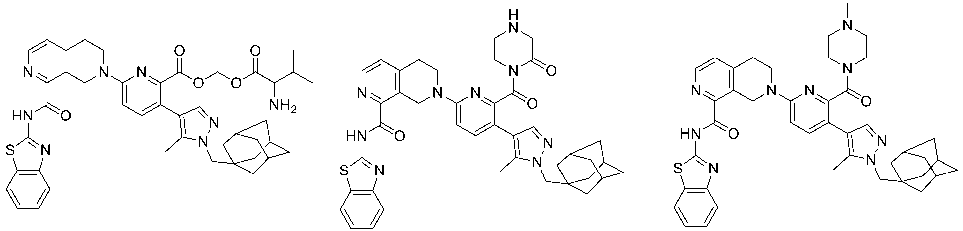

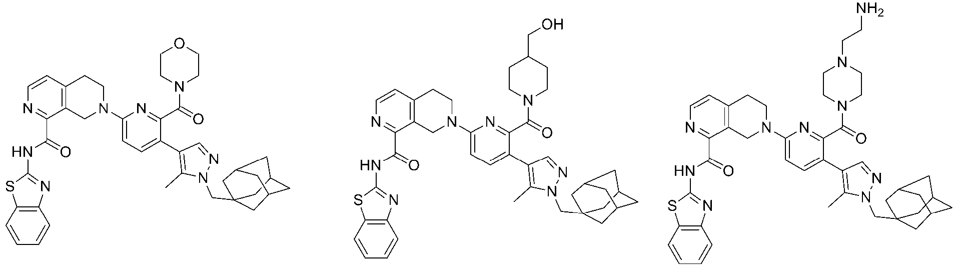

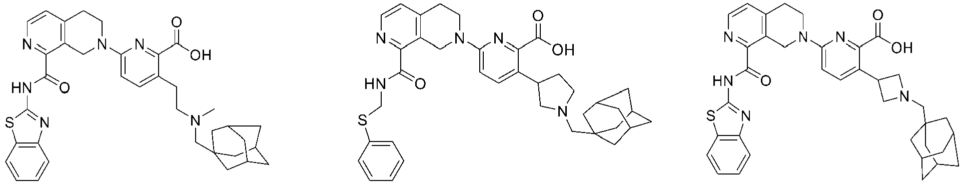

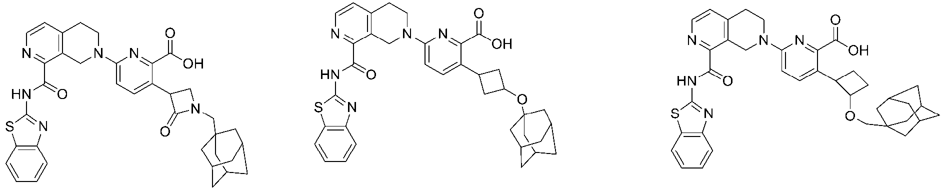

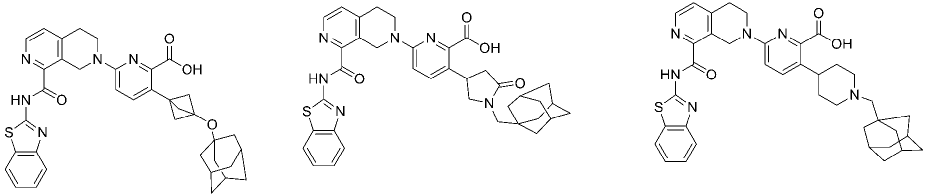

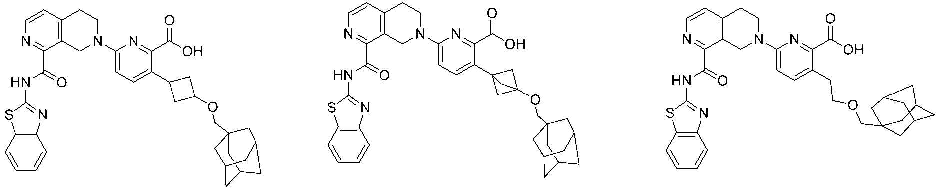

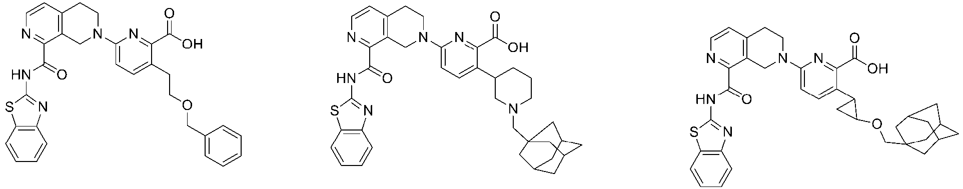

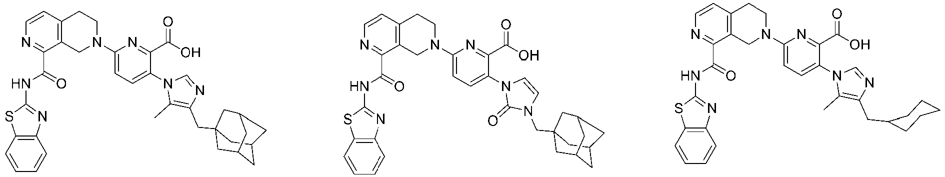

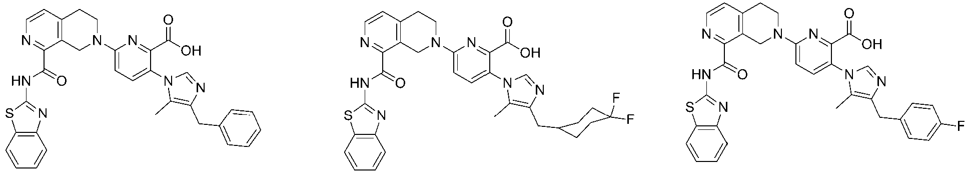

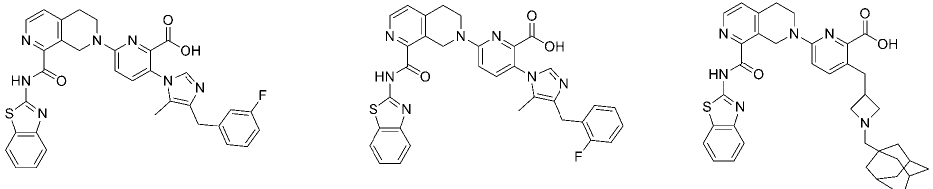

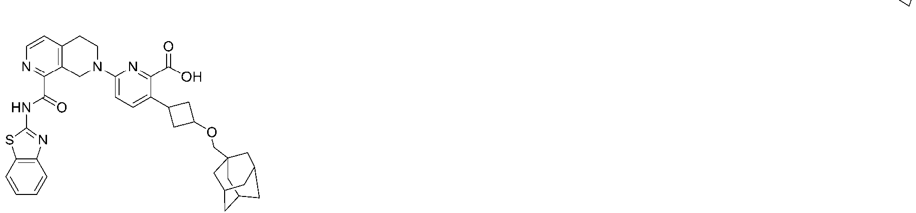

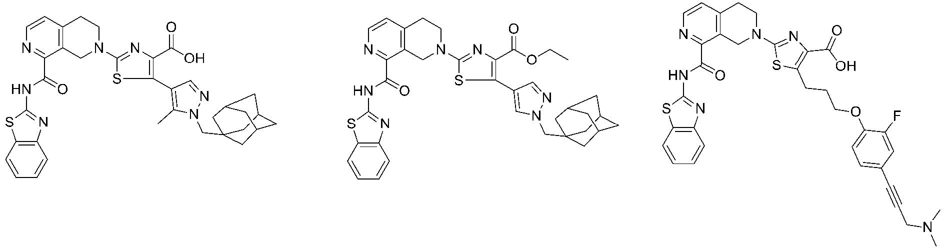

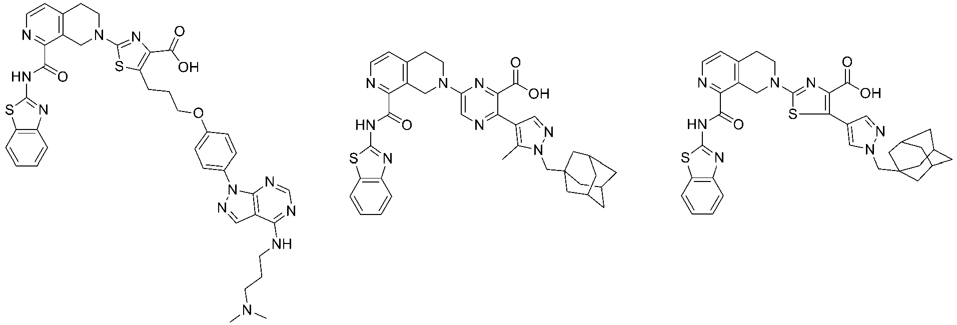

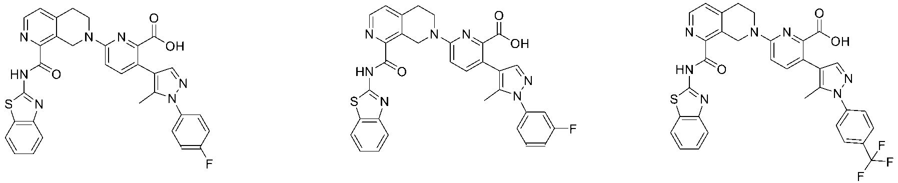

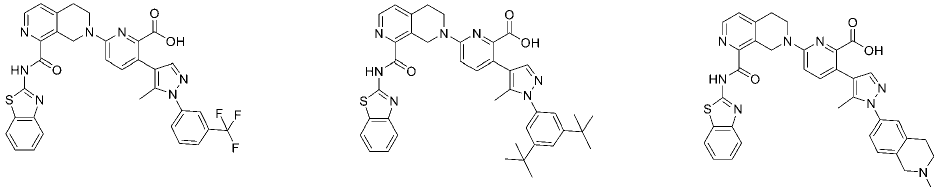

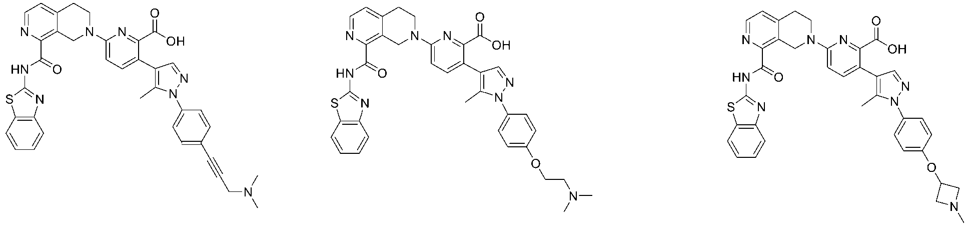

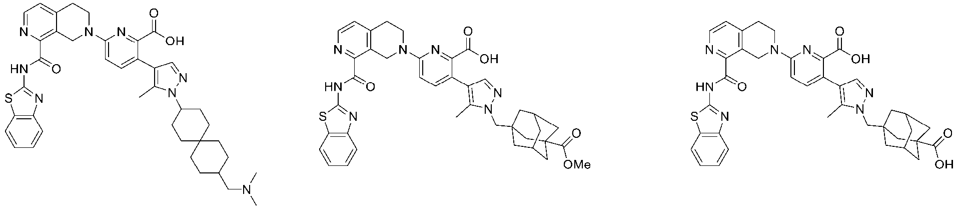

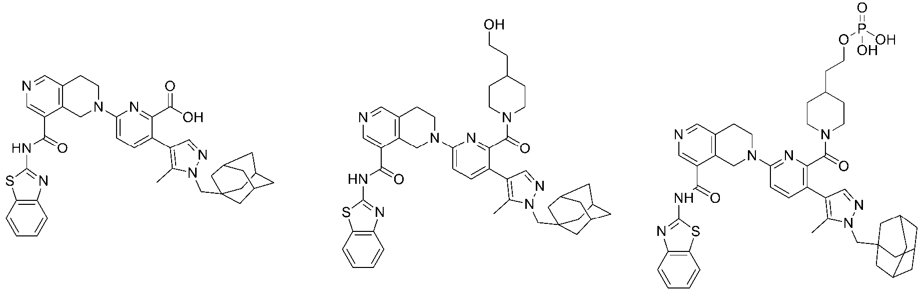

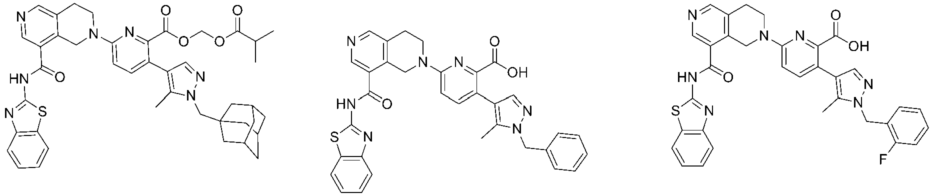

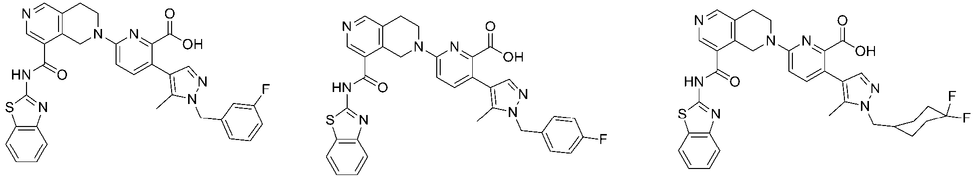

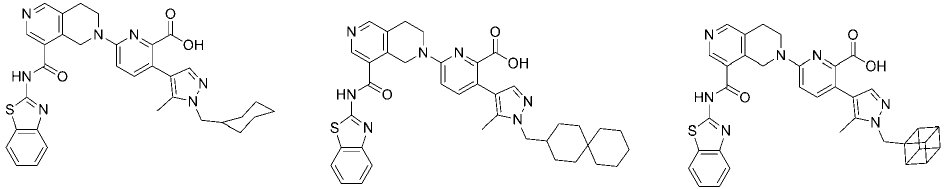

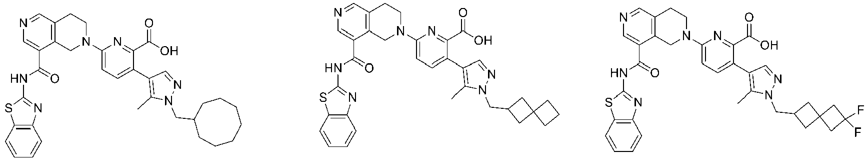

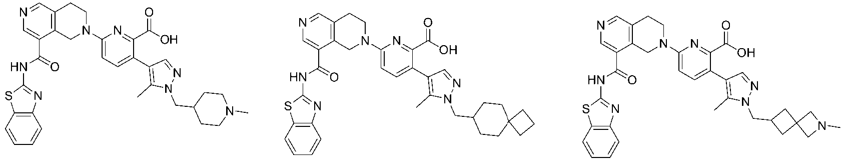

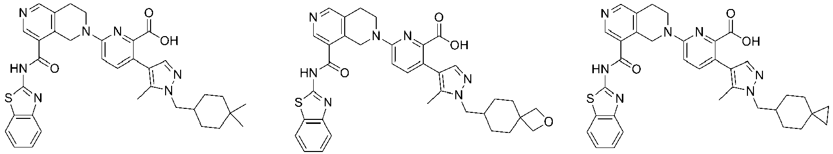

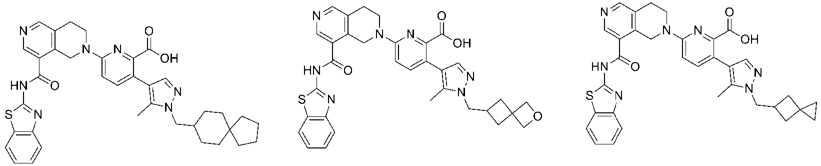

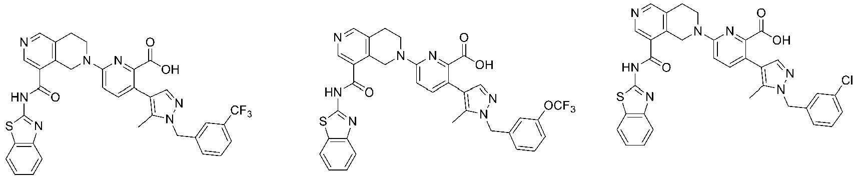

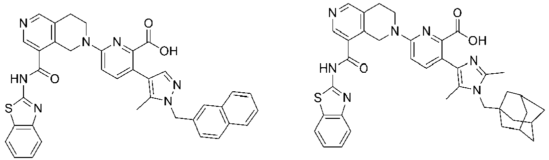

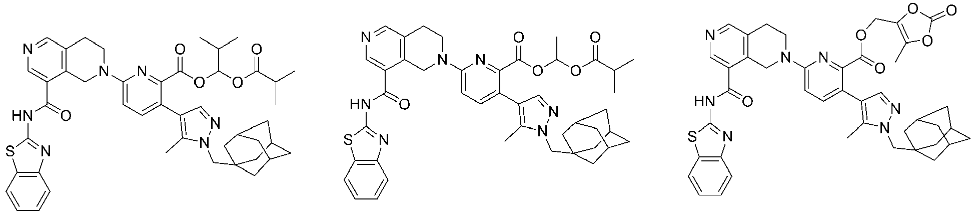

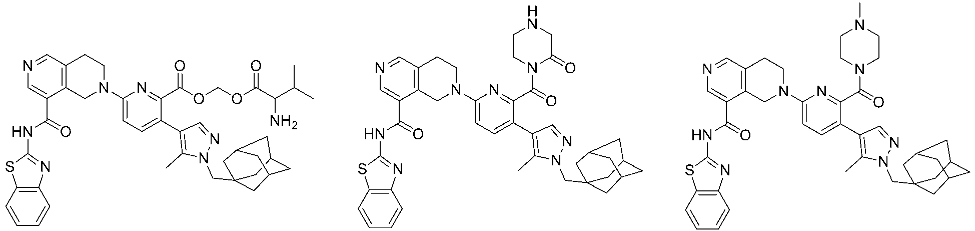

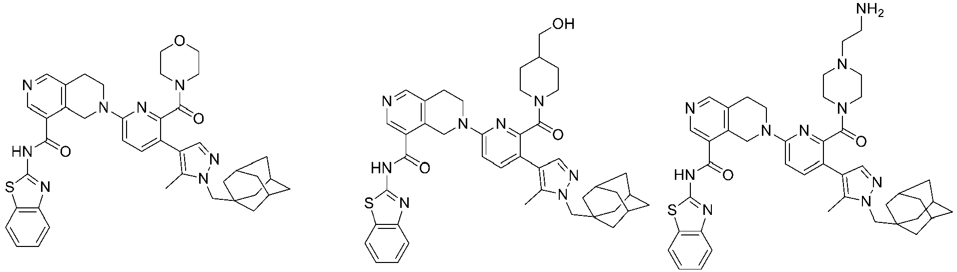

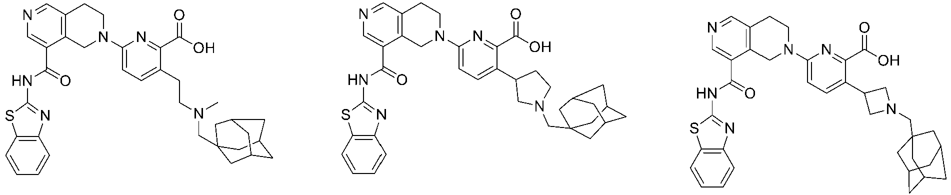

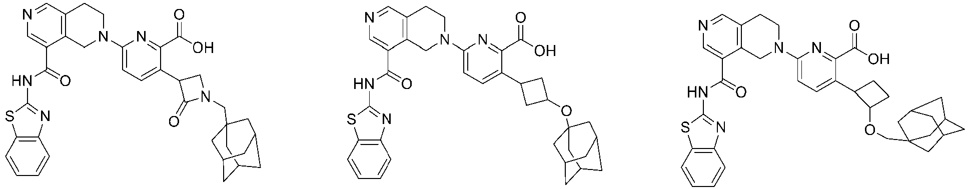

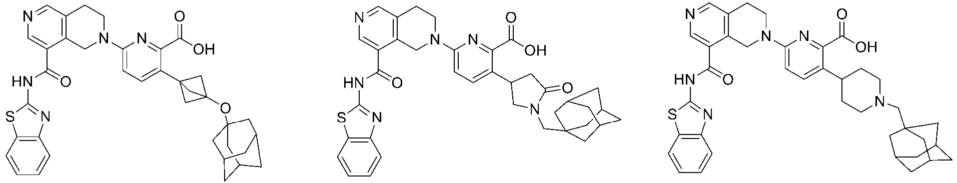

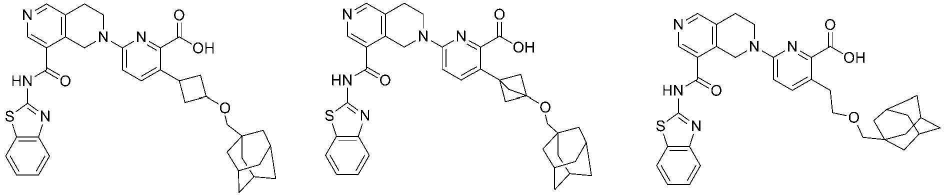

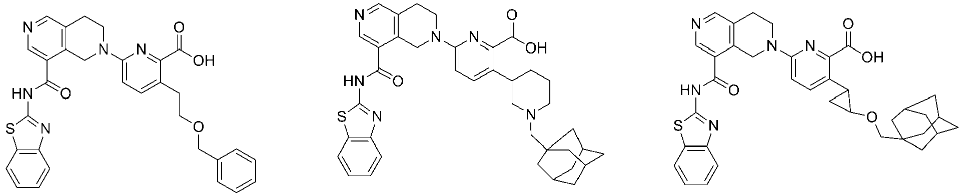

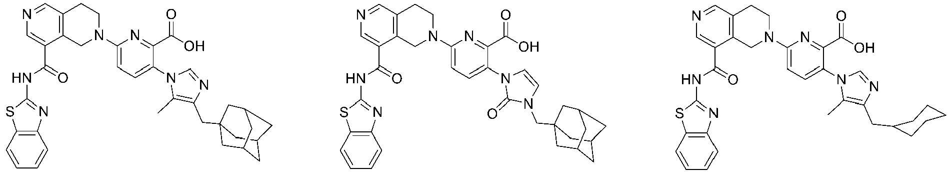

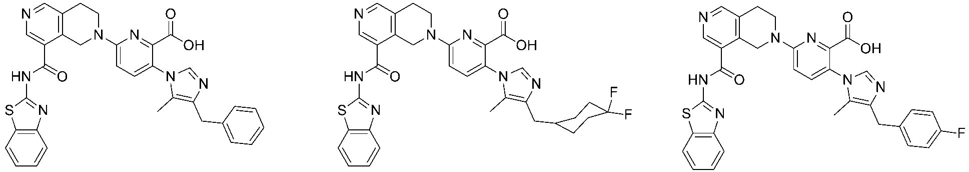

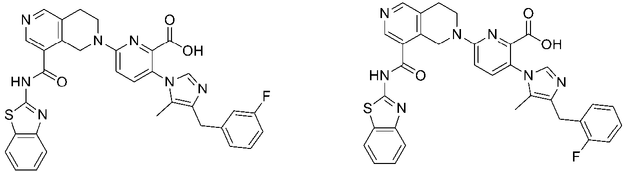

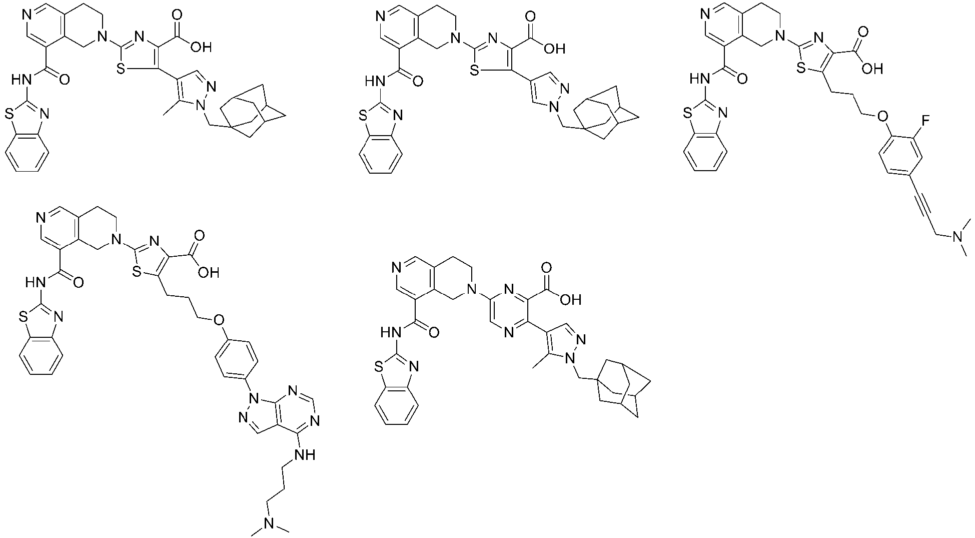

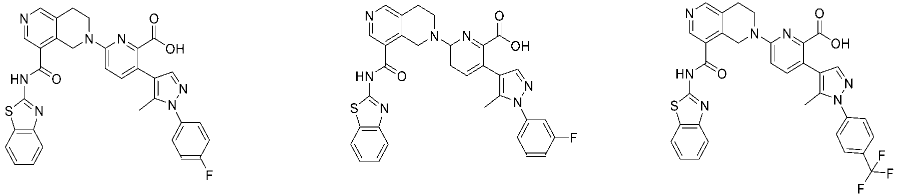

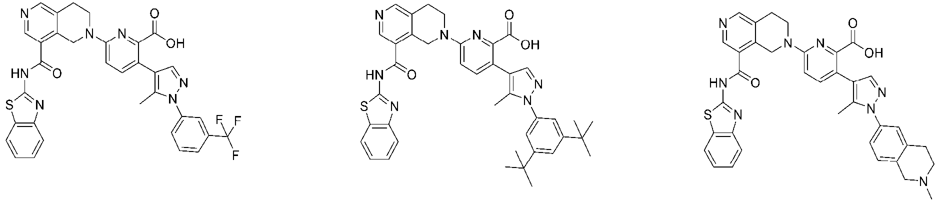

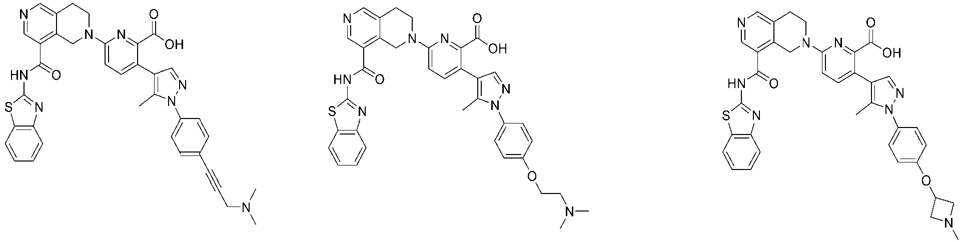

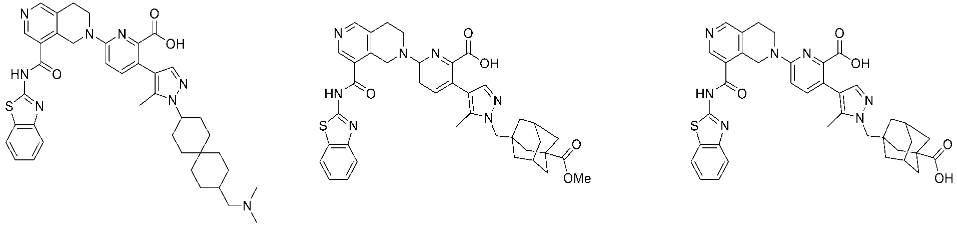

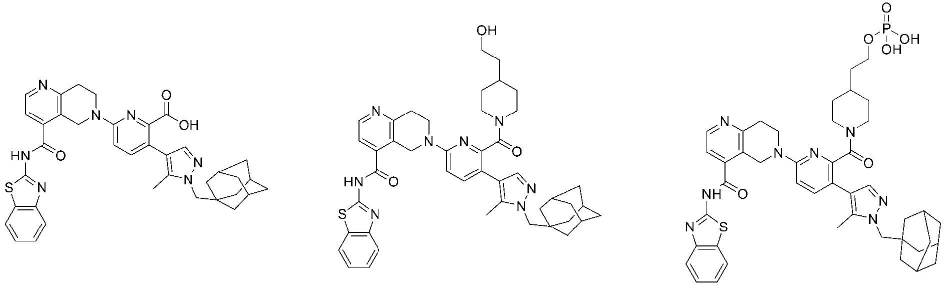

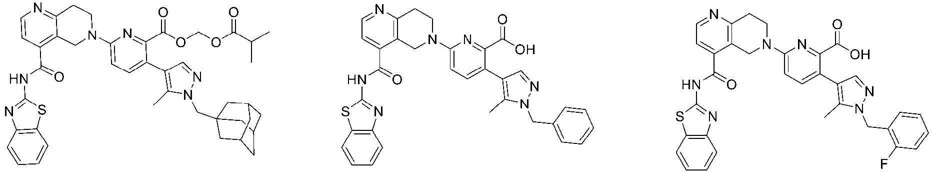

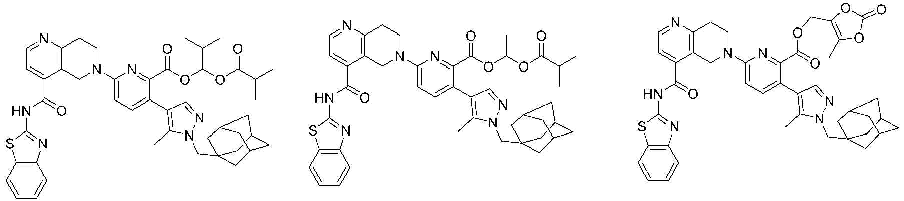

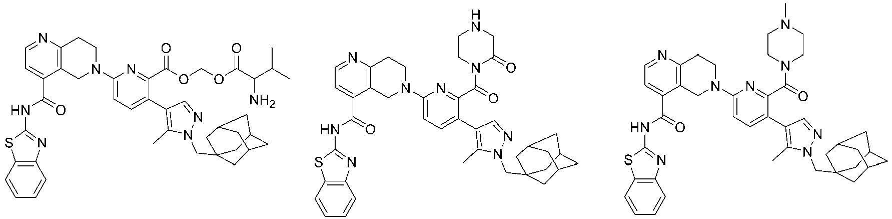

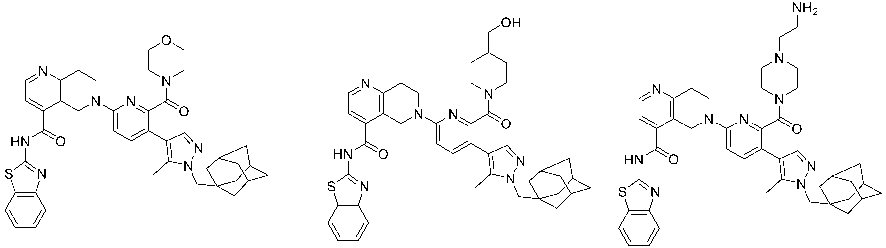

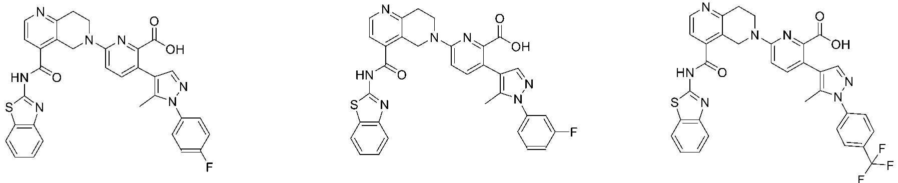

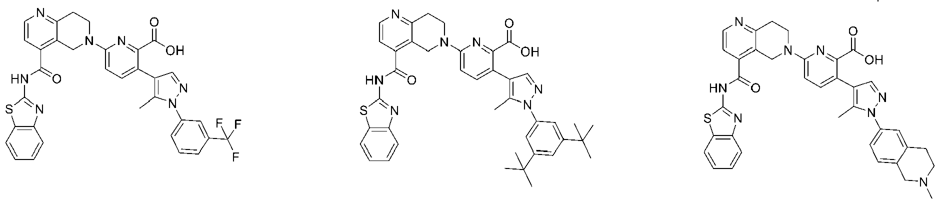

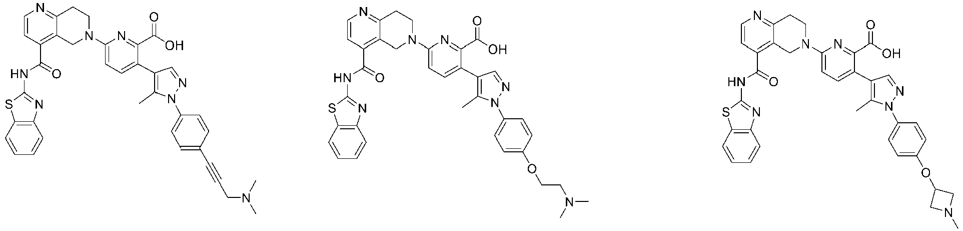

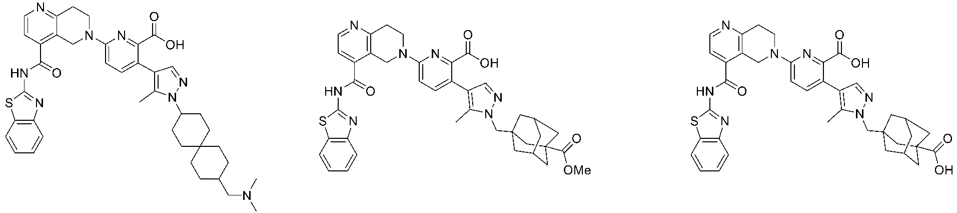

In some embodiments, the nitrogen heterocyclic compound is selected from the following groups:

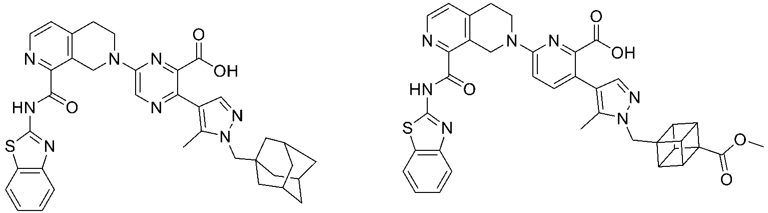

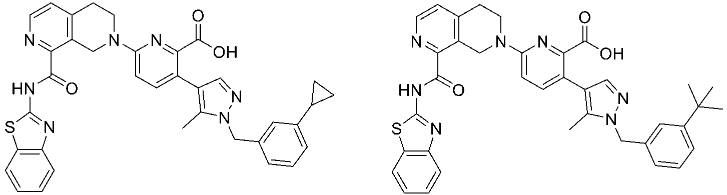

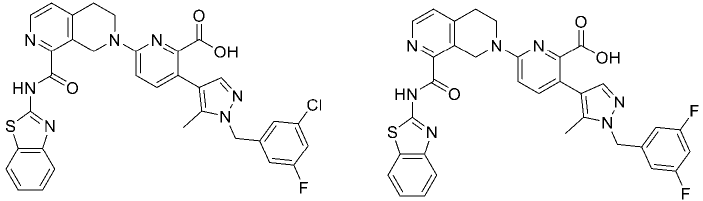

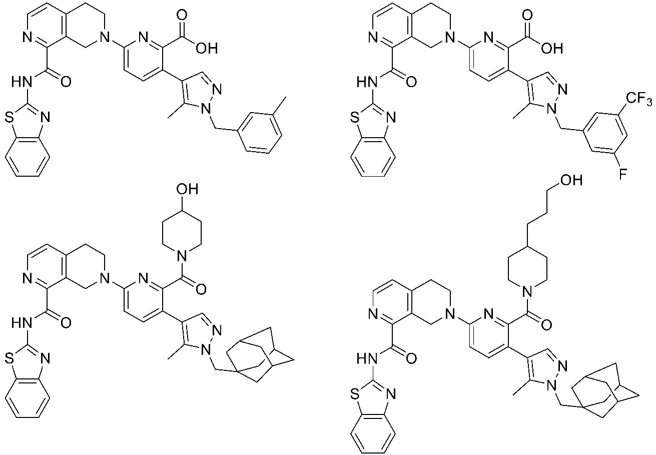

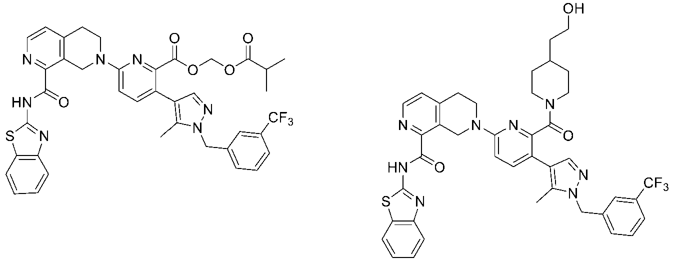

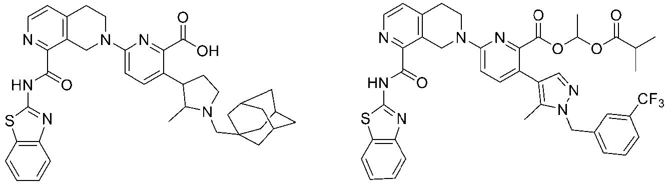

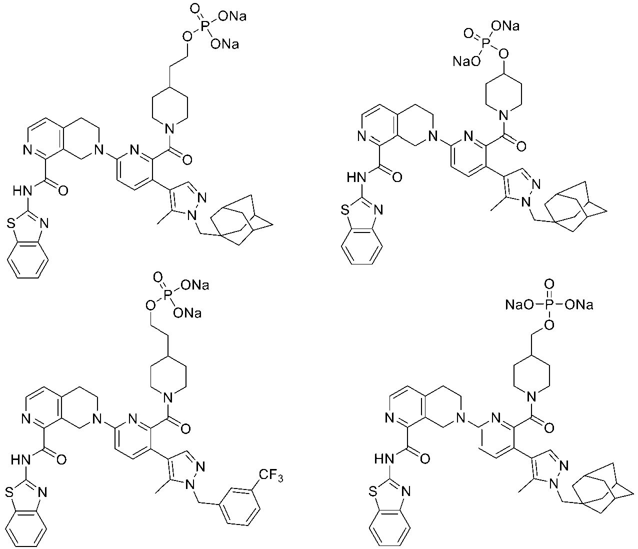

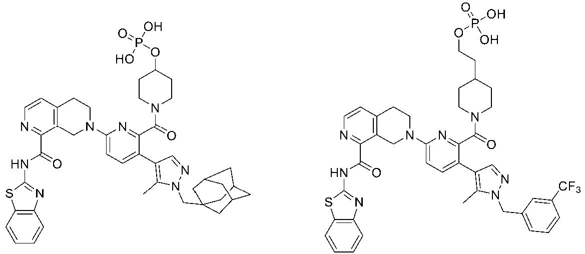

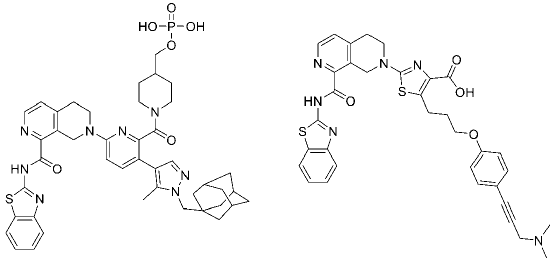

In some embodiments, the salt of the compounds is an alkali metal salt, preferably a sodium salt.

In a second aspect, the present application provides a pharmaceutical composition, comprising the compound, or a pharmaceutically acceptable salt, a solvate, a hydrate, a polymorph, a co-crystal, a tautomer, a stereoisomer, or an isotopic compound thereof of the first aspect, and a pharmaceutically acceptable excipient.

In a third aspect, the present application provides a method for preventing or treating aging-related diseases, comprising administering to a subject in need thereof a therapeutically effective amount of the compound or a pharmaceutically acceptable salt, solvate, hydrate, polymorph, co-crystal, tautomer, stereoisomer or isotopic compound thereof of the first aspect, or the pharmaceutical composition of the second aspect.

In some embodiments, the disease is selected from diseases associated with the accumulation of senescent cells, and the disease is preferably selected from one or more of idiopathic pulmonary fibrosis, pulmonary fibrosis, liver fibrosis, renal fibrosis, inflammation and tissue fibrosis and atrophy of the upper respiratory tract and lungs caused by viruses, cystic fibrosis, myelofibrosis, myocardial fibrosis, skin fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritic neuropathy, keratitis sicca, Fuchs corneal dystrophy, presbyopia, cataract, degenerative vitreous disorders including vitreomacular traction syndrome, macular hole, retinal tear, retinal detachment, proliferative vitreoretinopathy, osteoarthritis, intervertebral disc herniation, osteoporosis, Alzheimer's disease, Parkinson's disease, atherosclerosis, chronic obstructive pulmonary disease, diabetes, diabetic nephropathy, scar, superficial scar or flat scar, linear scar or contracture scar, webbed scar, depressed scar, atrophic scar, bridged scar and pedunculated scar, hypertrophic scar, keloid, scar carcinoma, scleroderma, morphea, linear scleroderma, guttate scleroderma, acroscleroderma, diffuse scleroderma, CREST syndrome, acute coronary syndrome, myocardial infarction, stroke, hypertension, obesity, adipose dysfunction, coronary artery disease, cerebrovascular disease, periodontal disease, cancer treatment-related disabilities such as atrophy and fibrosis in various tissues, myelodysplastic syndrome associated with brain and heart injury treatment, promyelocytic syndrome, ataxia-telangiectasia, Fanconi anemia, Friedreich's ataxia, dyskeratosis congenita, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

In a fourth aspect, the present application provides use of the compound or a pharmaceutically acceptable salt, solvate, hydrate, polymorph, co-crystal, tautomer, stereoisomer or isotopic compound thereof of the first aspect, or the pharmaceutical composition of the second aspect in the manufacture of a medicament for the prevention or treatment of aging-related diseases.

In some embodiments, the disease is selected from diseases associated with the accumulation of senescent cells, and the disease is preferably selected from one or more of idiopathic pulmonary fibrosis, pulmonary fibrosis, liver fibrosis, renal fibrosis, inflammation and tissue fibrosis and atrophy of the upper respiratory tract and lungs caused by viruses, cystic fibrosis, myelofibrosis, myocardial fibrosis, skin fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritic neuropathy, keratitis sicca, Fuchs corneal dystrophy, presbyopia, cataract, degenerative vitreous disorders including vitreomacular traction syndrome, macular hole, retinal tear, retinal detachment, proliferative vitreoretinopathy, osteoarthritis, intervertebral disc herniation, osteoporosis, Alzheimer's disease, Parkinson's disease, atherosclerosis, chronic obstructive pulmonary disease, diabetes, diabetic nephropathy, scar, superficial scar or flat scar, linear scar or contracture scar, webbed scar, depressed scar, atrophic scar, bridged scar and pedunculated scar, hypertrophic scar, keloid, scar carcinoma, scleroderma, morphea, linear scleroderma, guttate scleroderma, acroscleroderma, diffuse scleroderma, CREST syndrome, acute coronary syndrome, myocardial infarction, stroke, hypertension, obesity, adipose dysfunction, coronary artery disease, cerebrovascular disease, periodontal disease, cancer treatment-related disabilities such as atrophy and fibrosis in various tissues, myelodysplastic syndrome associated with brain and heart injury treatment, promyelocytic syndrome, ataxia-telangiectasia, Fanconi anemia, Friedreich's ataxia, dyskeratosis congenita, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

In a fifth aspect, the present application further provides a method of synthesizing the compound of the first aspect, comprising:
reacting the compound of Formula II with the compound of Formula III to obtain the compound of Formula I, 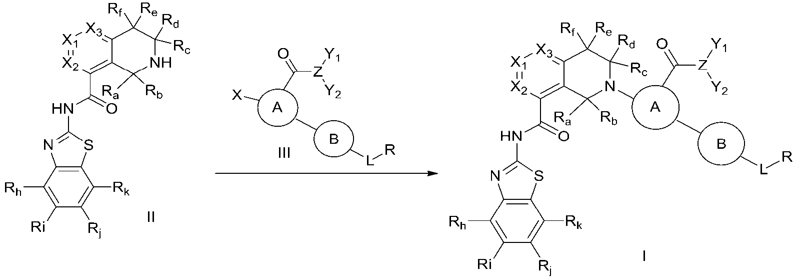
X represents halogen, preferably chlorine, bromine or iodine, and other symbols are as defined in the compound of Formula I;
or reacting the compound of Formula IV with the compound of Formula V to obtain the compound of Formula I, 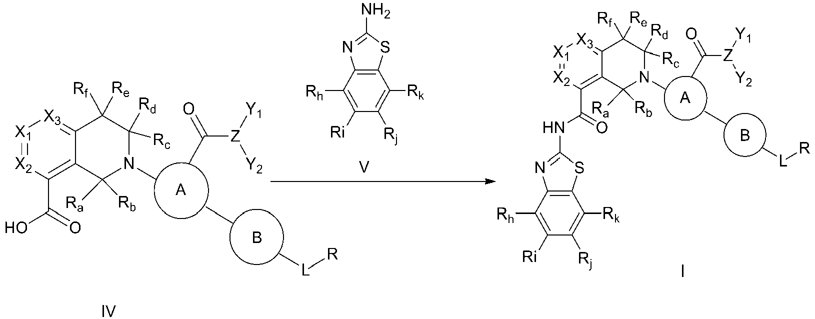
each symbol is as defined in the compound of Formula I;
or reacting the compound of Formula VI with the compound of Formula V to obtain the compound of Formula VII; the compound of Formula VII is converted to the compound of Formula I, 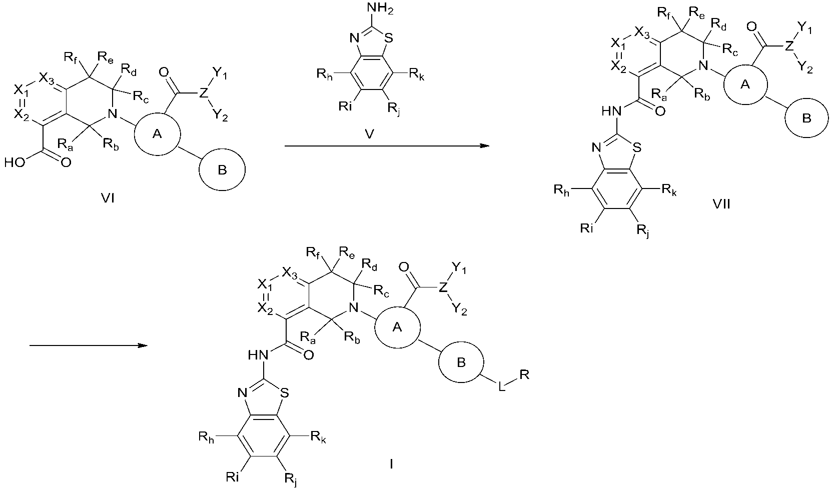
each symbol is as defined in the compound of Formula I.

In a sixth aspect, the present application further provides intermediate compounds which are selected from the compounds shown as Formula II, Formula III, Formula IV, Formula VI, or Formula VII: 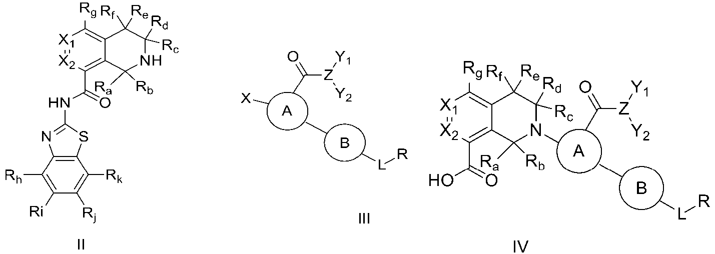 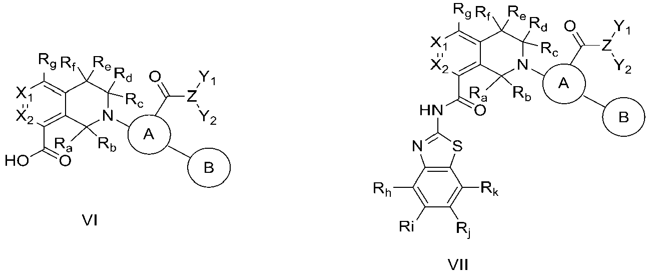 X represents halogen, preferably chlorine, bromine or iodine, and other symbols are as defined in the compound of Formula I;
In a seventh aspect, the present application further provides intermediate compounds selected from any one of the following compounds:

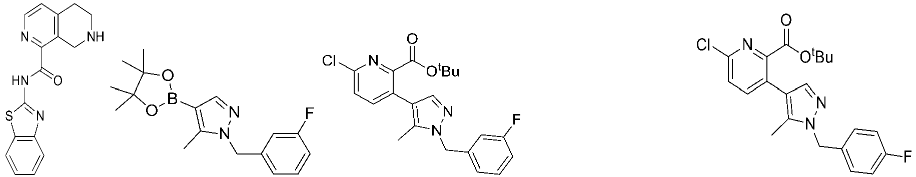

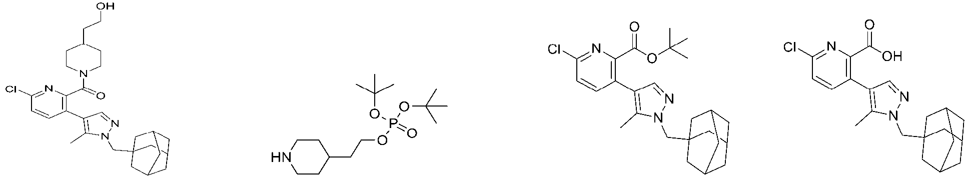

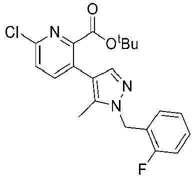

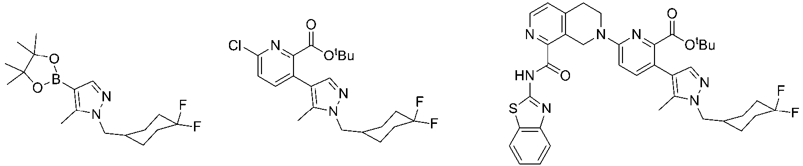

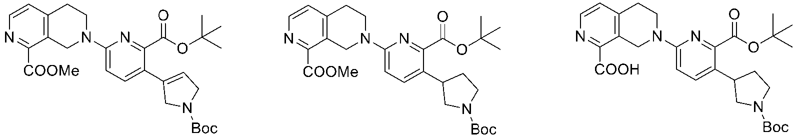

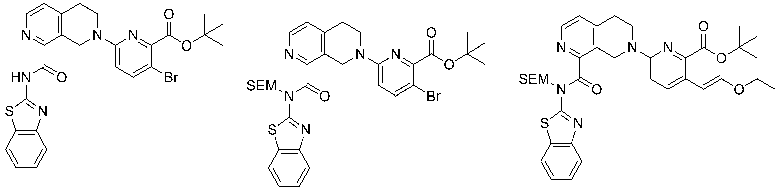

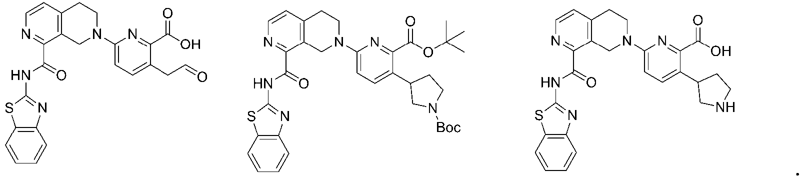

### BRIEF DESCRIPTION OF THE DRAWINGS

FIG. 1 illustrates the effect of administering compound RN001 in a mouse model of oxygen-induced retinopathy (OIR).
FIG. 2 illustrates the effect of administering compound RN001 in a mouse model of imiquimod-induced psoriasis.
FIG. 3 illustrates the effect of administering compound RN001 in a bleomycin-induced scleroderma model.
FIG. 4 illustrates the effect of administering compound RN001 in an animal model of idiopathic pulmonary fibrosis.
FIG. 5 illustrates the effect of administering compound RN001 in an animal model of osteoarthritis.
FIG. 6 illustrates the effect of administering compound RN001 in a rabbit ear hypertrophic scar animal model.

### DETAILED DESCRIPTION OF THE EMBODIMENTS

The present invention will be described in detail in combination with the following examples: the examples are carried out on the premise that the present invention is a technical solution, and detailed embodiments and processes are provided, but the embodiments provided herein are exemplary and are intended to be used for explaining the present invention, and are not to be construed as a limitation to the present invention. The conditions and methods not specified in the following examples are carried out conventionally.

### Definition

The term "alkyl" refers to an aliphatic hydrocarbon group, which may be branched or straight chain alkyl. Depending on the structure, the alkyl may be a monovalent group or a bivalent group (i.e., alkylene). In the present invention, the alkyl is preferably an alkyl having 1 to 8 carbon atoms, more preferably a "lower alkyl" having 1 to 6 carbon atoms, and even more preferably an alkyl having 1 to 4 carbon atoms. Typical alkyl includes, but is not limited to, methyl, ethyl, propyl, butyl, pentyl, hexyl, and the like. It should be understood that, the "alkyl" as mentioned herein includes all possible configurations and conformations of the alkyl. For example, the "propyl" as mentioned herein includes n-propyl and isopropyl, the "butyl" includes n-butyl, isobutyl, and tert-butyl, and the "pentyl" includes n-pentyl, isopentyl, neopentyl, tert-pentyl, and pent-3-yl, and the like.

The term "alkoxy" refers to -O-alkyl, wherein the alkyl is as defined herein. Typical alkoxy includes, but is not limited to, methoxy, ethoxy, propoxy, butoxy, pentyloxy, hexyloxy, and the like.

The term "cycloalkyl" refers to monocyclic or polycyclic group containing only carbon and hydrogen. Cycloalkyl includes a group having 3 to 12 ring atoms. Depending on the structure, the cycloalkyl may be a monovalent group or a bivalent group (i.e., cycloalkylene). In the present invention, the cycloalkyl is preferably a cycloalkyl having 3 to 8 carbon atoms, and more preferably a "lower cycloalkyl" having 3 to 6 carbon atoms. Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "aryl" refers to an aromatic ring in which each of the atoms constituting the ring is a carbon atom. An aryl ring may be composed of five, six, seven, eight, nine, or more than nine atoms. Aryl may be optionally substituted. Examples of aryl include, but are not limited to, phenyl, naphthyl, phenanthryl, anthracenyl, fluorenyl, and indenyl. Depending on the structure, aryl may be a monovalent group or a bivalent group (i.e., arylene).

The term "heteroaryl" refers to an aromatic group containing one or more ring heteroatoms selected from nitrogen, oxygen, and sulfur. N-containing "heteroaryl" moiety refers to an aromatic group in which at least one skeleton atom in the ring is nitrogen atom. Depending on the structure, heteroaryl may be a monovalent group or a bivalent group (i.e., heteroarylene). Examples of heteroaryl include, but are not limited to, pyridyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolyl, isoquinolyl, indolyl, benzimidazolyl, benzofuryl, indazolyl, indolizinyl, phthalazinyl, pyridazinyl, isoindolyl, pteridinyl, purinyl, oxadiazolyl, thiadiazolyl, furazanyl, benzofurazanyl, benzothienyl, benzothiazolyl, benzoxazolyl, quinazolinyl, naphthyridinyl, furopyridyl, and the like.

The term "alicyclic group" or "cycloalkyl" as used herein refers to a non-aromatic ring formed of three or more carbon atoms, wherein the bond between two adjacent carbon atoms in the ring may be single bond, double bond or triple bond. The number of rings may be one or more. Non-limiting examples include cyclopropyl, cyclobutyl, cyclopentyl, cyclohexyl, cycloheptyl, cyclohexenyl, cyclopentenyl, cyclohexadienyl, and the like.

The term "heterocycloalkyl" or "heteroalicyclic group" or "heteroalicyclic ring" as used herein refers to a non-aromatic ring in which one or more atoms constituting the ring are heteroatoms selected from nitrogen, oxygen and sulfur. The heterocycloalkyl may be composed of three, four, five, six, seven, eight, nine or more than nine atoms. The heterocycloalkyl may be optionally substituted. Examples of heteroalicyclic group include, but are not limited to, lactam, lactone, cyclic imine, cyclic thioimine, cyclic carbamate, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiine, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine and 1,3-oxathiolane. Depending on the structure, heteroalicyclic group may be a monovalent group or a bivalent group (i.e., heterocycloalkylene).

The term "halo" or "halogen" refers to fluorine, chlorine, bromine, and iodine.

The term "carbonyl" refers to an organic functional group (C = O) formed by connecting carbon and oxygen through a double bond.

The term "optional" refers to that one or more of subsequent events may occur or may not occur, and includes both events that occur and events that do not occur.

The salt that may be formed by the compound in the present invention are also within the scope of the present invention. Unless otherwise specified, the compound in the present invention is understood as including its salts. The term "salt" as used herein refers to an acidic or basic salt formed from inorganic or organic acid and base. Furthermore, when the compound of the present invention contains a basic fragment, it includes but is not limited to pyridine or imidazole, and when it contains an acidic fragment, it includes but is not limited to carboxylic acid, and the zwitterion ("inner salt") that may be formed is included in the scope of the term "salt". Pharmaceutically acceptable (i.e., nontoxic and physiologically acceptable) salts are preferred, although other salts are also useful, for example, in isolation or purification steps during preparation. The compounds of the present invention may form salts, for example, the compound I is reacted with a certain amount of, for example, an equivalent amount of acid or base, and the salt is obtained by salting out in a medium, or freeze-drying in an aqueous solution.

The basic fragments contained in the compound of the present invention include, but are not limited to, amines, pyridine or imidazole ring, and may form salt with organic or inorganic acid. Typical acids that can form salts include acetates (e.g., acetic acid or trihaloacetic acid, e.g. trifluoroacetic acid), adipate, alginate, ascorbate, aspartate, benzoate, benzenesulfonate, hydrosulfate, borate, butyrate, citrate, camphorate, camphorsulfonate, cyclopentanepropionate, diglycolate, dodecyl sulfate, ethanesulfonate, fumarate, glucoheptonate, glycerophosphate, hemisulfate, heptylate, hexanoate, hydrochloride, hydrobromide, hydroiodide, hydroxy-ethanesulfonate (e.g., 2-hydroxy-ethanesulfonate), lactate, maleate, methanesulfonate, naphthalenesulfonate (e.g., 2-naphthalenesulfonate), nicotinate, nitrate, oxalate, pectinate, persulfate, phenylpropionate (e.g., 3-phenylpropionate), phosphate, picrate, neopentanoate, propionate, salicylate, succinate, sulfate (e.g., formed with sulfuric acid), sulfonate, tartrate, thiocyanate, toluenesulfonate such as p-toluenesulfonate, dodecanoate, and the like.

The acidic fragments which may be contained in the compound of the present invention include, but are not limited to, carboxylic acid, and may form salt with various organic or inorganic bases. Typical salts formed with base include ammonium salt, alkali metal salts (such as sodium, lithium, and potassium salts), alkaline-earth metal salts (such as calcium and magnesium salts); and salts formed with organic base (such as organic amine), such as benzathine, dicyclohexylamine, hydrabamine (salt formed with N,N-bis(dehydroabietyl)ethylenediamine), N-methyl-D-glucamine, N-methyl-D-glucamide, tert-butylamine; and salts formed with amino acids such as arginine and lysine, and the like. Basic nitrogen-containing group can be combined with halide quaternary ammonium salts, such as small molecule alkyl halide (e.g. chloride, bromide and iodide of methyl, ethyl, propyl and butyl), dialkyl sulfate (e.g. dimethyl sulfate, diethyl sulfate, dibutyl sulfate and dipentyl sulfate), long-chain halide (e.g. chloride, bromides and iodide of decyl, dodecyl, tetradecyl and tetradecyl), arylalkyl halide (e.g. benzyl and phenyl bromides), and the like.

Prodrug and solvate of the compounds of the present invention are also within the scope of the present invention. The term "prodrug" herein refers to a compound that undergoes chemical transformation via metabolic or chemical processes to produce the compound, salt, or solvate of the present invention when treating related diseases. "Solvate" refers to a solvent addition form containing a stoichiometric or non-stoichiometric amount of solvent. Some compounds tend to trap a fixed molar ratio of solvent molecules in the crystalline solid state, thereby forming a solvate. If the solvent is water, the solvate formed is a hydrate; if the solvent is alcohol, the solvate formed is an alcoholate. A hydrate is formed by the combination of one or more water molecules with a molecule of substance, in which the water retains its molecular state H₂O. Non-limiting examples of solvate include ethanol solvate, acetone solvate, and the like.

The compound, salt or solvate of the present invention may exist in tautomeric forms (e.g. amide and imine ether). All such tautomers are parts of the present invention.

All stereoisomers of the compounds (e.g., those that may exist as asymmetric carbon atoms due to various substitutions), including their enantiomeric and diastereomeric forms, fall within the contemplated scope of the present invention. The independent stereoisomers of the compounds of the present invention may not coexist with other isomers (e.g. as a pure or substantially pure optical isomer with a special activity), or they may also be mixtures, such as racemates, or mixtures with all other stereoisomers or portions thereof. The chiral center of the present invention has S or R configuration, as defined by the recommendation of the International Union of Pure and Applied Chemistry (IUPAC) in 1974. The racemic form can be resolved by physical methods, such as stepwise crystallization, separation of crystals by derivatization into diastereomers, or separation by chiral column chromatography. Individual optical isomer can be obtained from the racemate by suitable methods, including but not limited to conventional methods, such as forming salt with an optically active acid, followed by recrystallization.

The compounds in the present invention, which are obtained by preparation, separation, and purification in sequence, have a weight content equal to or greater than 90%, for example, equal to or greater than 95%, equal to or greater than 99% ("highly pure" compound), as listed in the description. Such "very pure" compounds of the present invention are also included herein as part of the present invention.

All configurational isomers of the compounds of the present invention are included within the scope, including mixtures, pure form, or highly pure form. The definition of compounds in the present invention includes both cis (Z) and trans (E) isomers of alkenes, as well as cis and trans isomers of carbocyclic and heterocyclic rings.

Throughout the description, groups and substituents may be selected to provide stable fragments and compounds.

Definitions of specific functional groups and chemical terms are detailed below. For the present invention, the chemical elements are as defined in Periodic Table of the Elements, CAS version, Handbook of Chemistry and Physics, 75th Ed. Definitions of specific functional groups are also described therein. In addition, the basic principles of organic chemistry and specific functional groups and reactivities are also described in "Organic Chemistry", Thomas Sorrell, University Science Books, Sausalito: 1999, the entire content of which is incorporated by reference.

Certain compounds of the present invention may exist in specific geometric or stereoisomeric forms. The present invention encompasses all compounds, including their cis and trans isomers, R and S enantiomers, diastereomers, (D) isomers, (L) isomers, racemic mixtures and other mixtures. In addition, asymmetric carbon atoms can represent a substituent, such as alkyl. All isomers, as well as mixtures thereof, are included in the present invention.

According to the present invention, the ratio of isomers in a mixture of isomerides having same molecular formula can be varied. For example, a mixture with only two isomers can have the following combinations: 50: 50, 60: 40, 70: 30, 80: 20, 90: 10, 95: 5, 96: 4, 97: 3, 98: 2, 99: 1, or 100: 0. All ratios of isomers are within the scope of the present invention. Similar ratios, as well as ratios for more complex mixtures of isomers that are readily understood by those skilled in the art, are also within the scope of the present invention.

The present invention also includes isotope-labeled compounds that are equivalent to the original compounds disclosed herein. However, in practice, it often occurs that one or more atoms are replaced with atoms with a different atomic weight or mass number. Examples of isotopes that may be listed as compounds of the present invention include hydrogen, carbon, nitrogen, oxygen, phosphorus, sulfur, fluorine, and chlorine isotopes, such as ²H , ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The compound, or enantiomer, diastereomer, isomer, or pharmaceutically acceptable salt or solvate thereof in the present invention, containing the isotope or other isotopic atom of the compound specified above, are included within the scope of the present invention. Certain isotope-labeled compounds of the present invention, such as radioactive isotopes of ³H and ¹⁴C, are useful in tissue distribution experiments of drugs and substrates. Tritium, i.e.,³H and carbon-14, i.e.,¹⁴C, which are relatively easy to prepare and detect, are most preferred in isotopes. In addition, heavier isotope substitutions such as deuterium, i.e. ²H, have advantages in certain therapies due to their good metabolic stability, such as increasing half-life or reducing dosage in the body, therefore they can be considered preferentially in some cases. Isotope-labeled compounds can be prepared using general methods by replacing non-isotopic reagents with readily available isotope-labeled reagents, using the protocols disclosed in the examples.

If the synthesis of a specific enantiomer of the compound of the present invention is to be designed, it can be prepared by asymmetric synthesis, or derivatized with a chiral auxiliary, and the resulting diastereomeric mixture is separated and then the chiral auxiliary is removed to give the pure enantiomer. In addition, if the molecule contains a basic functional group, such as an amino acid, or an acidic functional group, such as carboxy, a diastereomeric salt may be formed with a corresponding suitable optically active acid or base, and then separated by conventional means such as separation crystallization or chromatography and the like, to obtain a pure enantiomer.

As described herein, the compounds of the present invention may be substituted with any number of substituents or functional groups to expand their scope. Generally, whether the term "substitution" appears before or after the term "optionally", the general formula of the formulation of the present invention including the substituents, refers to that the hydrogen radical is replaced by a specified structural substituent. When a specific structure is substituted with multiple substituents at multiple positions, the substituents may be the same or different at each position. The term "substitution" used herein includes all permitted substitutions of organic compounds. Broadly, permitted substituents include acyclic, cyclic, branched, unbranched, carbocyclic, heterocyclic, aromatic cyclic and non-aromatic cyclic organic compounds. In the present invention, heteroatom such as nitrogen may be supplemented with hydrogen substituent or any of permitted organic compounds described above to replenish its valence. Furthermore, the present invention is not intended to be limited in any way to the permitted substitution of organic compounds. The present invention considers that combinations of substituents and variable groups are good in the treatment of diseases in the form of stable compounds. The term "stable" herein refers to a compound that is stable, and maintains the structural integrity of the compound for a sufficiently long period of detection time, preferably being effective for a sufficiently long period of time, and is used herein for the above purposes.

### Example 1. Synthesis of Compound RN001

The synthetic route is as follows:

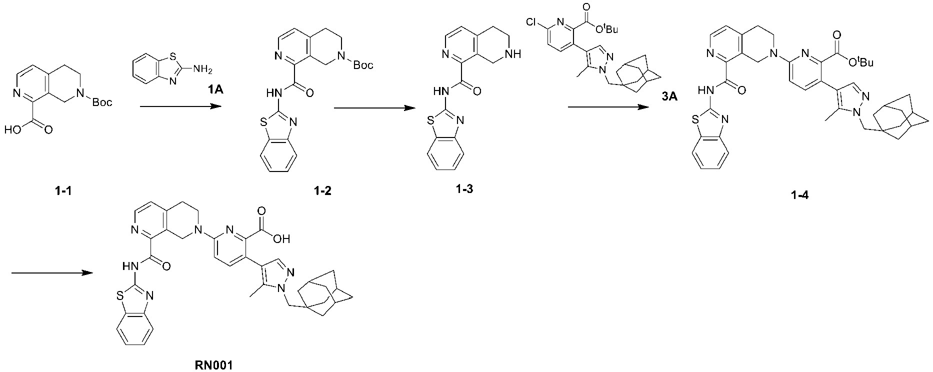

### Step 1: Synthesis of Compound 1-2

To a 25 mL single-necked flask was added acetonitrile (4.0 mL), and compound 1-1 (200 mg, 0.72 mmol) and CDI (144 mg, 0.89 mmol) were added sequentially under stirring. After the reaction solution was stirred at room temperature for 0.5 h, DBU (CAS No. 6674-22-2) (172 mg, 1.14 mmol) was added. After the reaction solution was stirred at room temperature for 0.5 h, 2-aminobenzothiazole (107 mg, 0.72 mmol) was added. Then the reaction solution was purged with nitrogen three times and stirred at 60 °C for 4 h. The reaction solution was diluted with water (50 mL) and extracted with ethyl acetate (50 mL × 3). The organic phases were washed with saturated brine (50 mL × 2), dried over anhydrous sodium sulfate, filtered, and concentrated under reduced pressure to afford a crude product containing the target compound, which was purified by column chromatography (mobile phase: ethyl acetate-petroleum ether, gradient: 0-50%) to afford white compound **1-2** (108 mg, 0.26 mmol, yield 36.6%).

MS (ESI) m/z = 411.2 [M+H]⁺.

**¹H NMR** (400 MHz, DMSO-*d₆*): *δ* 12.26 (s, 1H), 8.55 (d, *J*=4.8 Hz, 1H), 8.06 (d, *J*=7.6 Hz, 1H), 7.81 (d, *J*=8.0 Hz, 1H), 7.54 (d, *J*=5.2 Hz, 1H), 7.48 (t, *J*=7.2 Hz, 1H), 7.36 (t, *J*=7.2 Hz, 1H), 5.00 (s, 2H), 3.62 (t, *J*=5.6 Hz, 2H), 2.94 (t, *J=5.6* Hz, 2H), 1.43 (s, 9H).

### Step 2: Synthesis of Compound 1-3

To a 25 mL single-necked flask was added 1,4-dioxane (3 mL), and compound **1-2** (88 mg, 0.21 mmol) was added sequentially under stirring, followed by addition of 4 N hydrogen chloride in 1,4-dioxane (3 mL) in an ice bath. The reaction solution was stirred at room temperature for 3 h. The reaction solution was quenched with saturated sodium carbonate solution (30 mL), and extracted with methanol: dichloromethane (1/10, 50 mL). The organic phase was washed with saturated brine (30 mL × 2), dried over anhydrous sodium sulfate, filtered, and concentrated under reduced pressure to afford target compound **1-3** as a white solid (60 mg, 0.19 mmol, yield 90.1%). MS (ESI) m/z = 311.0 [M+H]⁺.

### Step 3: Synthesis of Compound 1-4

To a 10 mL microwave tube were added N,N-dimethylformamide (0.4 mL), compound **1-3** (20 mg, 0.06 mmol), compound **3A** (34.1 mg, 0.08 mmol), cesium carbonate (62.9 mg, 0.19 mmol) and bis(tri-tert-butylphosphine)palladium (4.62 mg, 0.01 mmol). Under nitrogen protection, the reaction solution was reacted under microwave at 120 °C for 5 h. Water (30 mL) was added to the reaction solution, followed by extraction with ethyl acetate (30 mL × 3). The organic phase was washed with saturated brine(15 mL × 2), dried over anhydrous sodium sulfate, filtered, and concentrated under reduced pressure, to afford a crude product containing the target compound, which was purified by column chromatography (mobile phase: methanol-dichloromethane, gradient: 0-10%) to afford yellow compound **1-4** (23 mg, 0.01 mmol, yield 18.4%).

MS (ESI) m/z = 716.4 [M+H]⁺.

### Step 4: Synthesis of Compound RN001

At 0 °C, to a 25 mL single-necked flask were added sequentially dichloromethane (1.0 mL), compound **1-4** (79 mg, 0.11 mmol) and trifluoroacetic acid (1.0 mL), and the mixture was stirred at 25 °C for 12 h. The reaction solution was concentrated, and purified by a reversed-phase HPLC to afford the brown target product **RN001** (1.25 mg, 18.9 mol, yield 1.8%).

MS (ESI) m/z = 660.4 [M+H]⁺.

**¹H NMR** (400 MHz, DMSO-*d₆*): *δ* 12.30 (br, 1H), 8.55 (d, *J*=3.6 Hz, 1H), 8.05 (d, *J*=7.6 Hz, 1H), 7.81 (d, *J*=8.0 Hz, 1H), 7.60-7.53 (m, 2H), 7.48 (t, *J*=7.6 Hz, 1H), 7.37 (t, *J*=7.6 Hz, 1H), 7.29 (s, 1H), 7.04 (d, *J*=8.8 Hz, 1H), 5.23 (s, 2H), 3.94 (t, *J=5.6* Hz, 2H), 3.71 (s, 2H), 3.05 (t, *J=5.6* Hz, 2H), 2.12 (s, 3H), 1.93 (s, 3H), 1.69-1.49 (m, 12H).

### Example 2. Synthesis of Compound RN003

The synthetic route is as follows:

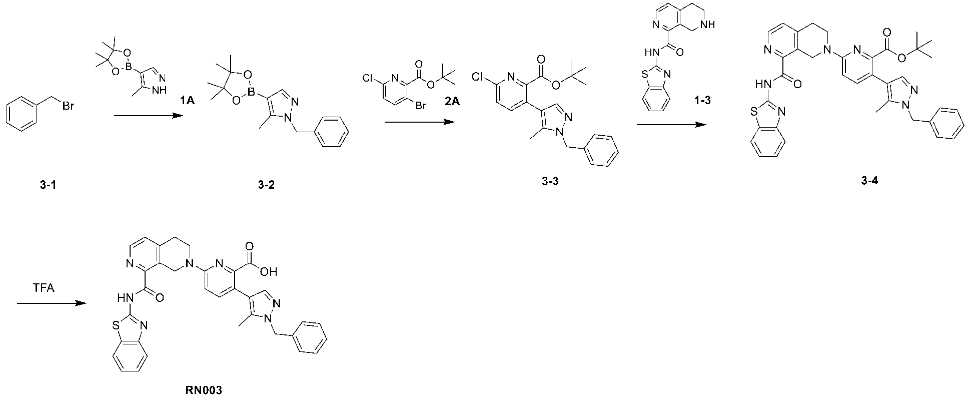

### Step 1: Synthesis of Compound 3-2

Compound **3-1** (2.99 mL, 25.0 mmol), compound 1A (4.00 g, 19.2 mmol), potassium carbonate (7.97 g, 57.7 mmol) and potassium iodide (1.28 mL, 11.5 mmol) were dissolved in N,N-dimethylformamide (40 mL) and reacted at 90 °C for 12 h. After completion of the reaction, the mixture was diluted with water (50 mL), extracted with ethyl acetate (20 mL × 3), and washed with saturated brine (20 mL), dried over anhydrous sodium sulfate, filtered, and concentrated under reduced pressure to afford a crude product containing the target compound, which was purified to afford the compound **3-2** as a brown solid (253 mg, 0.85 mmol, yield 4.41%).

MS (ESI) m/z =299.2 [M+H]⁺.

**¹H NMR** (400 MHz, CDCl₃): *δ* 7.76 (s, 1H), 7.33- 7.23 (m, 3H), 7.11 (d, *J =* 7.2 Hz, 2H), 5.31 (s, 2H), 2.38 (s, 3H), 1.30 (s, 12H).

### Step 2: Synthesis of Compound 3-3

Compound **2A** (298 mg, 1.02 mmol), compound **3-2** (253 mg, 0.85 mmol), Pd(dppf)Cl₂ (69.3 mg, 0.08 mmol) and potassium carbonate (351 mg, 2.55 mmol) were dissolved in 1,4-dioxane (2.5 mL) and H₂O (0.5 mL), and reacted at 90 °C for 2 h. After completion of the reaction, the mixture was concentrated under reduced pressure, diluted with water (10 mL), and extracted with ethyl acetate (2 mL × 3), and washed with saturated brine (10 mL), dried over anhydrous sodium sulfate, filtered, and concentrated under reduced pressure to give a crude compound, which was purified by column chromatography (mobile phase: ethyl acetate-petroleum ether, gradient: 0-5%) to afford compound **3-3** as a white solid (161 mg, 0.42 mmol, yield 49.4%).

MS (ESI) m/z =384.2 [M+H]⁺.

**¹H NMR** (400 MHz, CDCl₃): *δ* 7.57 - 7.51 (m, 2H), 7.40 (d, *J =* 8.0 Hz, 1H), 7.32 (dd, *J =* 12.0, 7.2 Hz, 3H), 7.22 (d, *J =* 6.8 Hz, 2H), 5.34 (s, 2H), 2.13 (s, 3H), 1.33 (s, 9H).

### Step 3: Synthesis of Compound 3-4

Compound **1-3** (80 mg, 0.26 mmol), compound **3-3** (98.4 mg, 0.26 mmol), Pd(t-Bu₃P)₂ (26.3 mg, 0.05 mmol) and Cs₂CO₃ (252 mg, 0.77 mmol) were dissolved in N,N-dimethylformamide (0.8 mL), and reacted at 130 °C for 2 h. The reaction solution was diluted with H₂O (8 mL), and extracted with ethyl acetate (2 mL × 4), and washed with saturated brine (5 mL), dried over anhydrous sodium sulfate, filtered, and concentrated under reduced pressure to obtain a crude compound, which was purified to afford compound **3-4** as a yellow solid (30 mg, 0.05 mmol, yield 17.8%).

MS (ESI) m/z =658.2 [M+H]⁺.

### Step 4: Synthesis of Compound RN003

Compound **3-4** (30 mg, 0.05 mmol) was dissolved in dichloromethane (5 mL), and TFA (0.1 mL, 1.34 mmol) was added. The reaction solution was stirred at 20 °C for 2 h. After completion of the reaction, the mixture was concentrated under reduced pressure to afford a crude product, which was purified by preparative HPLC to afford compound **RN003** as a yellow solid (5.9 mg, 0.01 mmol, yield 21.4%).

MS (ESI) m/z = 602.2 [M+H]⁺.

**¹H NMR** (400 MHz, DMSO-*d₆*): *δ* 12.30 (s, 1H), 8.55 (d, *J =* 4.8 Hz, 1H), 8.05 (d, *J =* 8.0 Hz, 1H), 7.81 (d, *J =* 8.0 Hz, 1H), 7.60 - 7.53 (m, 2H), 7.48 (t, *J =* 7.6 Hz, 1H), 7.37 - 7.25 (m, 5H), 7.12 (d, *J =* 7.2 Hz, 2H), 7.05 (d, *J =* 8.8 Hz, 1H), 5.33 (s, 2H), 5.23 (s, 2H), 3.94 (s, 2H), 3.05 (s, 2H), 2.10 (s, 3H).

### Example 3. Synthesis of Compound RN005

The synthetic route is as follows:

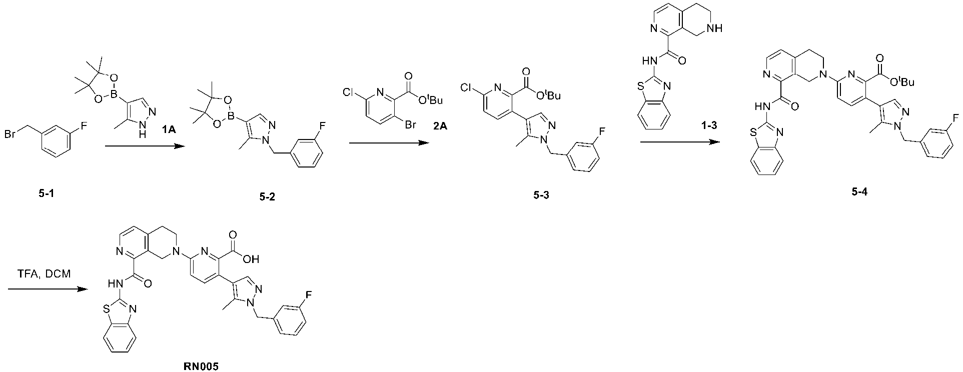

### Step 1: Synthesis of Compound 5-2

To a 100 mL single-necked flask was added N,N-dimethylformamide (30 mL). Under stirring, compound **5-1** (1.95 mL, 15.87 mmol), compound **1A** (3.30 g, 15.8 mmol), potassium carbonate (6.58 g, 47.61 mmol) and potassium iodide (2.63 g, 15.8 mmol) were added sequentially. The reaction solution was purged with nitrogen three times and stirred at 80 °C for 18 h. The reaction solution was diluted with water (100 mL) and extracted with ethyl acetate (100 mL × 3). The organic phase was washed with saturated brine (50 mL × 2), dried over anhydrous sodium sulfate, filtered, and concentrated under reduced pressure, and the residue was purified to afford white compound **5-2** (416 mg, 1.32 mmol, yield 8.29%).

MS (ESI) m/z = 317.2 [M+H]⁺.

### Step 2: Synthesis of Compound 5-3

To a single-necked flask was added dioxane (3.0 mL) and H₂O (0.6 mL). Under stirring, compound **5-2** (366 mg, 1.16 mmol), compound **2A** (336 mg, 1.16 mmol), potassium carbonate (479 mg, 3.47 mmol) and Pd(dppf)Cl₂ (84.70 mg, 0.12 mmol) were added sequentially. The reaction solution was purged with nitrogen three times and stirred at 90 °C for 3 h. The reaction solution was diluted with water (20 mL) and extracted with ethyl acetate (30 mL × 3). The organic phase was washed with saturated brine (20 mL × 2), dried over anhydrous sodium sulfate, filtered, and concentrated under reduced pressure to afford a crude product containing the target compound, which was purified to afford compound **5-3** as a yellow oil (246 mg, 0.62 mmol, yield 53.1%).

MS (ESI) m/z = 402.2 [M+H]⁺.

### Step 3: Synthesis of Compound 5-4

To 0.5 mL of dimethyl sulfoxide were added compound **5-3** (77.3 mg, 0.19 mmol), compound **1-3** (30 mg, 0.10 mmol), cesium carbonate (157 mg, 0.48 mmol) and potassium iodide (48.13 mg, 0.29 mmol). The reaction solution was purged with nitrogen three times and stirred under microwave at 150 °C for 4 h. The reaction solution was diluted with water (20 mL) and extracted with ethyl acetate (10 mL × 3). The organic phase was washed with saturated brine (20 mL × 2), dried over anhydrous sodium sulfate, filtered, and concentrated under reduced pressure to afford a crude product containing the target compound, which was purified by column chromatography (mobile phase: methanol-dichloromethane, gradient: 0-10%) to afford yellow compound **5-4** (10.0 mg, 0.01 mmol, yield 14.80%).

MS (ESI) m/z = 676.2 [M+H]⁺.

### Step 4: Synthesis of Compound RN005

At 0 °C, to a single-necked flask were added sequentially dichloromethane (0.5 mL), compound **5-4** (10 mg, 0.03 mmol) and TFA (0.5 mL), and the mixture was stirred at 25 °C for 2 h. The reaction solution was concentrated and purified by a reversed-phase HPLC to afford the brown target product **RN005** (2.39 mg, 3.86 µmol, yield 28.3%).

MS (ESI) m/z = 620.2 [M+H]⁺;
**¹H NMR** (400 MHz, DMSO-*d₆*): *δ* 12.9 (s, 1H), 12.3 (s, 1H), 8.56 (d, *J*=4.8 Hz, 1H), 8.06 (d, *J*=7.6 Hz, 1H), 7.82 (d, *J*=8.0 Hz, 1H), 7.63-7.55 (m, 2H), 7.50 (t, *J*=8.0 Hz, 1H), 7.43-7.33 (m, 3H), 7.11 (td, *J*=8.8, 2.4 Hz, 1H), 7.06 (d, *J*=8.8 Hz, 1H), 6.95 (d, *J*=7.6 Hz, 1H), 6.91 (d, *J*=9.6 Hz, 1H), 5.37 (s, 2H), 5.23 (s, 2H), 3.95 (t, *J=*5.6 Hz, 2H), 3.06 (t, *J=5.6* Hz, 2H), 2.11 (s, 3H).

### Example 4. Synthesis of Compound RN006

The synthetic route is as follows:

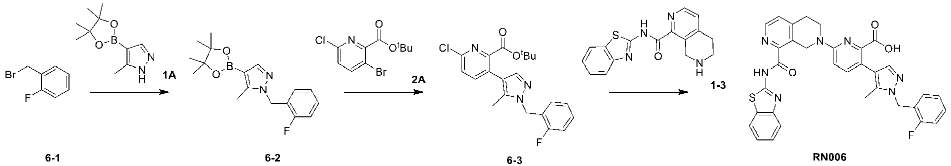

### Step 1: Synthesis of Compound 6-2

Into N,N-dimethylformamide (30 mL) were added compound **6-1** (3.00 g, 15.8 mmol), compound **1A** (3.30 g, 15.87 mmol), potassium iodide (2.63 g, 15.8 mmol) and potassium carbonate (6.58 g, 47.6 mmol). The mixture was stirred at 80 °C for 18 h. The reaction solution was diluted with water (100 mL) and extracted with ethyl acetate (50 mL × 2). The organic phase was washed with saturated brine (40 mL × 2), dried over anhydrous sodium sulfate, filtered, and concentrated under reduced pressure to afford a crude product containing the target compound, which was purified to afford compound **6-2** as a yellow liquid (450 mg, 1.42 mmol, yield 8.97%).

MS (ESI) m/z = 317.2 [M+H]⁺.

### Step 2: Synthesis of Compound 6-3

To a mixed solution of dioxane (5 mL) and water (1 mL) were added compound **6-2** (400 mg, 1.27 mmol), compound **2A** (444 mg, 1.52 mmol), potassium carbonate (524 mg, 3.80 mmol) and Pd(dppf)Cl₂ (92.5 mg, 0.13 mmol), and the mixture was stirred at 90 °C for 2 h. Water (15 mL) was added to the reaction solution, followed by extraction with ethyl acetate (20 mL × 2). The organic phase was washed with saturated brine (15 mL × 2), dried over anhydrous sodium sulfate, filtered, and concentrated under reduced pressure. The residue was purified to afford the target compound **6-3** as a white solid (102 mg, 0.25 mmol, yield 20.0%).

MS (ESI) m/z = 402.2 [M+H]⁺.

### Step 3: Synthesis of Compound RN006

To a 10 mL microwave tube were added N,N-dimethylformamide (0.7 mL), compound **6-3** (50 mg, 0.12 mmol), compound **1-3** (57.9 mg, 0.19 mmol), cesium carbonate (243 mg, 0.75 mmol), and potassium iodide (61.96 mg, 0.37 mmol). Under nitrogen protection, the reaction solution was reacted under microwave at 150 °C for 12 h. Water (10 mL) was added to the reaction solution, and extracted with dichloromethane (10 mL × 2). The organic phase was washed with saturated brine (10 mL), dried over anhydrous sodium sulfate, filtered, and concentrated under reduced pressure to afford a crude product containing the target compound, which was purified by a reversed-phase HPLC to afford the brown target product **RN006** (9 mg, 0.01 mmol, yield 10.7%).

MS (ESI) m/z = 620.2 [M+H]⁺.

**¹H NMR** (400 MHz, DMSO-*d₆*) *δ* 12.31 (s, 1H), 8.56 (d, *J =* 4.8 Hz, 1H), 8.06 (d, *J =* 7.6 Hz, 1H), 7.82 (d, *J =* 8.0 Hz, 1H), 7.62- 7.55 (m, 2H), 7.52- 7.45 (m, 1H), 7.40- 7.33 (m, 3H), 7.26-7.20 (m, 1H), 7.19- 7.13 (m, 1H), 7.06 (d, *J =* 8.8 Hz, 1H), 6.95- 6.89 (m, 1H), 5.36 (s, 2H), 5.23 (s, 2H), 3.99- 3.92 (m, 2H), 3.09- 3.01 (m, 2H), 2.14 (s, 3H).

### Example 5. Synthesis of Compound RN007

The synthetic route is as follows:

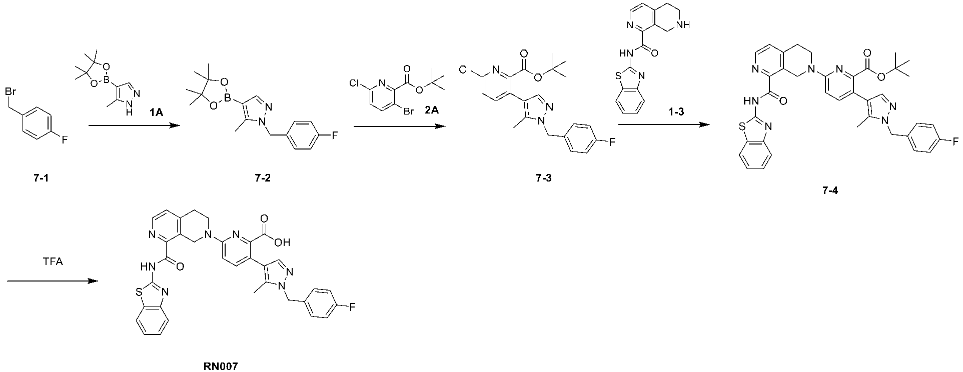

### Step 1: Synthesis of Compound 7-2

Compound **7-1** (4.00 g, 19.2 mmol), compound **1A** (2.39 mL, 19.2 mmol), potassium carbonate (7.97 g, 57.7 mmol) and potassium iodide (1.28 g, 7.69 mmol) were dissolved in N,N-dimethylformamide (40 mL) and reacted at 90 °C for 12 h. The reaction solution was diluted with H₂O (50 mL), extracted with ethyl acetate (20 mL × 3), washed with saturated brine (20 mL), dried over anhydrous sodium sulfate, filtered, and concentrated under reduced pressure to obtain a crude product containing the target compound, which was purified to afford compound **7-2** as a white solid (1.05 g, 3.32 mmol, yield 17.3%).

MS (ESI) m/z = 317.2 [M+H]⁺.

**¹H NMR** (400 MHz, CDCl₃): *δ* 7.75 (s, 1H), 7.09 (dd, *J*=8.4, 5.6 Hz, 2H), 6.99 (t, *J*=8.4 Hz, 2H), 5.26 (s, 2H),2.38 (s, 3H), 1.31 (s, 12H).

### Step 2: Synthesis of Compound 7-3

Compound **2A** (462 mg, 1.58 mmol), compound **7-2** (500 mg, 1.58 mmol), Pd(dppf)Cl₂ (116 mg, 0.16 mmol) and potassium carbonate (656 mg, 4.74 mmol) were dissolved in dioxane (5 mL) and H₂O (1 mL) and reacted at 100 °C for 12 h. The reaction solution was concentrated under reduced pressure, diluted with H₂O (15 mL), extracted with ethyl acetate (5 mL × 3), washed with saturated brine (5 mL), dried over anhydrous sodium sulfate, filtered, and concentrated under reduced pressure to obtain a crude compound, which was purified by column chromatography (mobile phase: ethyl acetate-petroleum ether, gradient: 0-8%) to afford compound **7-3** as a white solid (332 mg, 0.83 mmol, yield 52.2%).

MS (ESI) m/z = 402.2 [M+H]⁺.

**¹H NMR** (400 MHz, CDCl₃): *δ* 7.57-7.49 (m, 2H), 7.40 (d, *J*=8.4 Hz, 1H), 7.23-7.18 (m, 2H), 7.06-6.99 (m, 2H), 5.30 (s, 2H), 2.13 (s, 3H), 1.33 (s, 9H).

### Step 3: Synthesis of Compound 7-4

Compound **1-3** (92.68 mg, 0.30 mmol), compound **7-3** (100 mg, 0.25 mmol), potassium iodide (0.08 mL, 0.75 mmol) and cesium carbonate (486 mg, 1.49 mmol) were dissolved in dimethyl sulfoxide (1 mL) and reacted under microwave at 150 °C for 12 h. The reaction solution was filtered, concentrated and purified to afford compound **7-4** as a yellow solid (7 mg, 0.01 mmol, yield 4.14%).

MS (ESI) m/z = 676.2 [M+H]⁺.

### Step 4: Synthesis of Compound RN007

Compound **7-4** (30 mg, 0.05 mmol) was dissolved in dichloromethane (1 mL), and trifluoroacetic acid (1.5 mL, 20.1 mmol) was added. The reaction solution was stirred at 20 °C for 6 h. The reaction solution was concentrated to dryness directly to afford a crude product, which was purified by HPLC (mobile phase: acetonitrile-water (0.1% FA), gradient: 90-95%) to afford compound **RN007** as a yellow solid (3.3 mg, 0.01 mmol, yield 51.4%).

MS (ESI) m/z = 620.4 [M+H]⁺.

**¹H** NMR (400 MHz, DMSO-*d₆*): *δ* 12.30 (br, 1H), 8.56 (d, *J*=4.4 Hz, 1H), 8.13 (s, 1H), 8.05 (d, *J*=7.6 Hz, 1H), 7.81 (d, *J*=8.0 Hz, 1H), 7.59-7.54 (m, 2H), 7.49 (t, *J*=7.6 Hz, 1H), 7.40-7.34 (m, 2H), 7.21-7.14 (m, 4H), 7.05 (d, *J*=8.8 Hz, 1H), 5.32 (s, 2H), 5.23 (s, 2H), 3.94 (d, *J*=5.6 Hz, 2H), 3.05 (t, *J=5.6* Hz, 2H), 2.10 (s, 3H).

### Example 6. Synthesis of Compound RN008

The synthetic route is as follows:

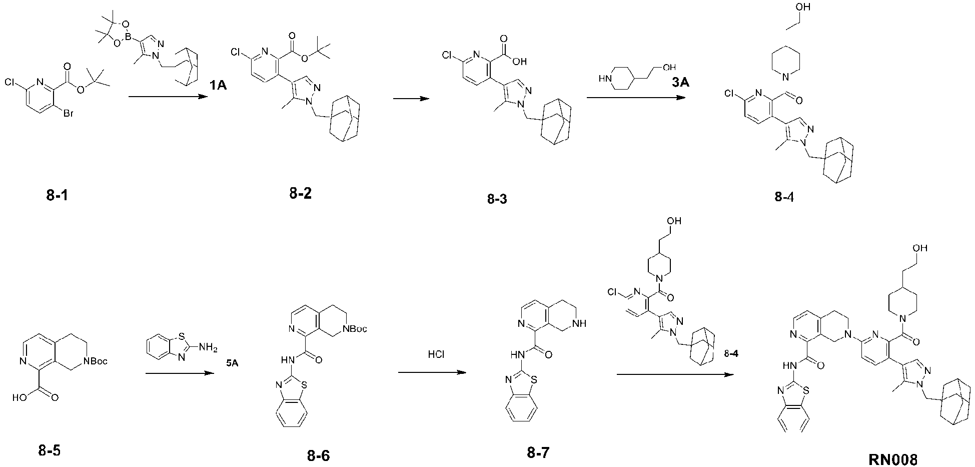

### Step 1: Synthesis of Compound 8-2

To a 250 mL three-necked flask were added dioxane (80 mL), water (80 mL), compound **8-1** (6.9 g, 23.6 mmol, 1.1 eq), cesium carbonate (20.8 g, 63.8 mmol, 3 eq), compound **1A** (7.6 g, 21.3 mmol, 1 eq), and [1,1'-bis(di-tert-butylphosphino)ferrocene]palladium dichloride (1.0 g, 1.53 mmol, 0.07 mmol). The mixture was purged with nitrogen and reacted at 90 °C for 14 h. After cooling, the reaction solution was concentrated under reduced pressure, 100 mL of water was added, and the mixture was extracted three times with 100 mL of ethyl acetate. The ethyl acetate layers were combined, washed twice with 50 mL of saturated sodium chloride solution, dried over anhydrous sodium sulfate, and concentrated under reduced pressure. Column chromatography was performed, and the pure product was eluted at dichloromethane: methanol = 5: 1, concentrated under reduced pressure and dried to afford compound **8-2** (3.6 g, yield 34.5%).

### Step 2: Synthesis of Compound 8-3

To a 100 mL three-necked flask were added compound **8-2** (2.8 g), acetonitrile (7 mL) and concentrated hydrochloric acid (7 mL), and the mixture was reacted at 40 °C for 4 h. TLC monitoring (dichloromethane: methanol = 10: 1) indicated the reaction was complete, and a large amount of white solid precipitated. 7 mL of acetonitrile was added, the mixture was filtered with suction, the filter cake was washed with 10 mL of acetonitrile, and dried to afford compound **8-3** (2.1 g, yield 85.9%).

### Step 3: Synthesis of Compound 8-4

To a 100 mL single-necked flask were added compound **8-3** (2.0 g, 5.19 mmol, 1.0 eq), 20 mL of N,N-dimethylformamide, N,N-diisopropylethylamine (2.68 g, 20.77 mmol, 4 eq), and TBTU (2-(1H-benzotriazol-1-yl)-1,1,3,3-tetramethyluronium tetrafluoroborate) (2.0 g, 6.23 mmol, 1.2 eq). The mixture was purged with nitrogen and stirred at room temperature for 30 min. 2-(Piperidin-4-yl)ethan-1-ol (0.74 g, 5.71 mmol, 1.1 eq) was added, and the mixture was reacted at 40 °C for 4 h. TLC monitoring (dichloromethane: methanol = 10: 1) indicated the reaction was complete. 100 mL of purified water was added to the reaction solution, and the resulting solution was washed three times with 100 mL of ethyl acetate. The ethyl acetate layers were combined, washed three times with 50 mL of saturated sodium chloride solution, and concentrated under reduced pressure to afford an oil, which was purified by column chromatography (dichloromethane: methanol = 15: 1) and concentrated under reduced pressure to afford compound **8-4** (2.22 g, yield 86.2%).

### Step 4: Synthesis of Compound 8-6

To a 25 mL single-necked flask were added compound **8-5** (250 mg, 0.906 mmol, 1 eq), 6 mL of N,N-dimethylformamide, diisopropylethylamine (350 mg, 2.70 mmol, 3 eq), and TBTU (2-(1H-benzotriazol-1-yl)-1,1,3,3-tetramethyluronium tetrafluoroborate) (347 mg, 1.08 mmol, 1.2 eq).

The mixture was purged with nitrogen and stirred magnetically for 30 min. 2-Aminobenzothiazole (147 mg, 0.99 mmol, 1.1 eq) was added, and the mixture was reacted at room temperature for 8 h. TLC monitoring indicated the starting material was consumed completely (dichloromethane: methanol = 20: 1), and the resulting mixture was concentrated under reduced pressure to afford a crude product of compound **8-6.**

### Step 5: Synthesis of Compound 8-7

To the crude solid compound **8-6** were added 4 mL of acetonitrile and 2 mL of concentrated hydrochloric acid, and the mixture was reacted at room temperature for 1.5 h. TLC monitoring indicated the reaction was complete. The mixture was poured into 30 mL of aqueous sodium bicarbonate solution, extracted with ethyl acetate, washed with saturated sodium chloride solution, concentrated under reduced pressure, and purified by column chromatography to afford compound **8-7** as a white solid (210 mg, total yield of two Steps 74.5%).

### Step 6: Synthesis of Compound RN008

To a 10 mL microwave tube were added compound **8-7** (150 mg, 0.483 mmol, 1 eq), compound **8-4** (240 mg, 0.483 mmol, 1 eq), cesium carbonate (394 mg, 1.61 mmol, 2.5 eq), and bis(tri-tert-butylphosphine)palladium (50 mg, 0.097 mmol, 0.2 eq). The mixture was purged with nitrogen, reacted under microwave at 130 °C for 3 h, and filtered with suction. The filter cake was washed with methanol, concentrated under reduced pressure, and separated by column chromatography to afford **RN008** as a white solid (14.8 mg, yield 3.9%).

**MS** (ESI) m/z = 771.4 [M+H]⁺.

**¹HNMR** (400 MHz, DMSO-*d₆*) *δ* 13.13 (s, 1H), 8.83 (s, 1H), 8.65 (s, 1H), 8.05 (d, *J =* 7.9 Hz, 1H), 7.81 (d, *J =* 8.0 Hz, 1H), 7.57 - 7.45 (m, 2H), 7.38 (t, *J* = 7.6 Hz, 1H), 7.21 (s, 1H), 6.96 (d, *J* = 8.9 Hz, 1H), 5.04 (s, 2H), 4.24 (d, *J =* 12.5 Hz, 1H), 3.88 (p, *J =* 6.6 Hz, 2H), 3.71 (d, *J* = 2.8 Hz, 2H), 3.27 (t, *J =* 6.4 Hz, 2H), 3.12 (d, *J =* 13.1 Hz, 2H), 3.07 - 3.00 (m, 2H), 2.74 - 2.59 (m, 2H), 2.15 (s, 3H), 1.92 (s, 3H), 1.72 - 1.30 (m, 16H), 1.27 - 1.09 (m, 3H).

### Example 7. Synthesis of Compound RN009

The synthetic route is as follows:

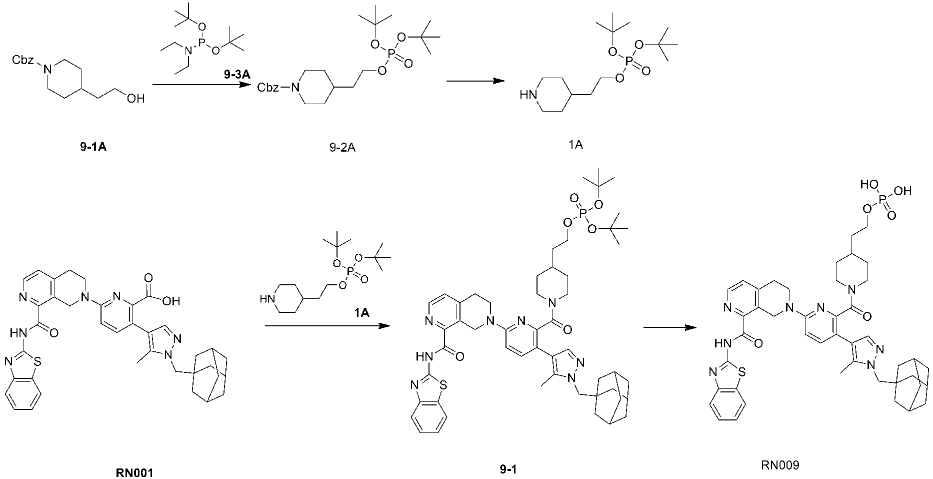

### Step 1 Synthesis of Compound 9-2A

At room temperature, to a 50 mL three-necked flask were added sequentially compound 9-1A (5 g, 18.99 mmol), DMA (50 mL), tetrazole (2.66 g, 37.98 mmol) and compound 9-3A (9.47 g, 37.98 mmol). The reaction system was stirred under nitrogen protection for 2 h, then cooled to - 10 °C, and 30% hydrogen peroxide (4.6 mL, 40 mmol) was added dropwise slowly. The mixture was stirred at room temperature for 2 h, then cooled to -10 °C, and the reaction was quenched with 0.5 M aqueous sodium thiosulfate solution. The reaction mixture was extracted with ethyl acetate (300 mL × 2), concentrated and purified to afford compound 9-2A (1 g, yield 57.8%).

### Step 2: Synthesis of Compound 1A

At room temperature, to a 100 mL pressure-resistant glass bottle were added sequentially compound 9-2A (2 g, 4.39 mmol), ethanol (20 mL) and palladium on carbon (palladium loading: 10%, 200 mg). The reaction system was stirred under hydrogen atmosphere (30 psi) for 2 h. The reaction solution was filtered, concentrated and purified to afford compound **1A** (1.2 g, yield 85.0%).

### Step 3: Synthesis of Compound 9-1

To a 5 mL brown bottle were added compound **RN001** (9 mg, 13.7 µmol), 0.5 mL of N,N-dimethylformamide, and N,N-diisopropylethylamine (2.6 mg, 20.5 µmol, 1.5 eq). The mixture was stirred at room temperature and purged with nitrogen. TBTU (2-(1H-benzotriazol-1-yl)-1,1,3,3-tetramethyluronium tetrafluoroborate) (6.6 mg, 20.5 µmol, 1.5 eq) was added, and the mixture was reacted at room temperature for 30 min. Compound **1A** (6.6 mg, 20.5 µmol, 1.5 eq) was added, and the mixture was reacted at room temperature for 2.5 h. TLC detection indicated the reaction was complete (dichloromethane: methanol = 20: 1). The reaction solution was poured

into 4 mL of ice water, and extracted twice with 2 mL of ethyl acetate. The ethyl acetate layers were combined, washed twice with 2 mL of saturated brine, dried over sodium sulfate, filtered with suction and concentrated under reduced pressure to afford compound **9-1** as a white solid (15 mg).

### Step 4: Synthesis of Compound RN009

The above solid compound **9-1** was added to a 5 mL brown bottle, dissolved with 1 mL of ethyl acetate. 0.2 mL of 2 M hydrogen chloride solution in ethyl acetate was added, and white solid precipitated gradually. The mixture was separated by preparative chromatography to afford a yellow compound **RN009** (0.73 mg).

**MS** (ESI) m/z = 850.9 [M+H]⁺.

**¹HNMR** (400 MHz, DMSO-*d₆*): δ 8.53 (s, 1H), 8.03 (d, *J* = 7.9 Hz, 1H), 7.79 (d, *J* = 7.9 Hz, 1H), 7.57 - 7.41 (m, 3H), 7.34 (s, 1H), 7.22 (s, 1H), 6.94 (d, *J =* 9.1 Hz, 1H), 5.18 (s, 2H), 4.26 (d, *J* = 12.2 Hz, 2H), 3.90 (s, 2H), 3.70 (s, 2H), 3.52 (s, 2H), 3.04 (s, 2H), 2.32 (s, 2H),2.15 (s, 3H), 1.91 (s, 3H), 1.69 - 1.41 (m, 14H), 1.23 (s, 5H).

### Example 8. Synthesis of Compound RN002

The synthetic route is as follows:

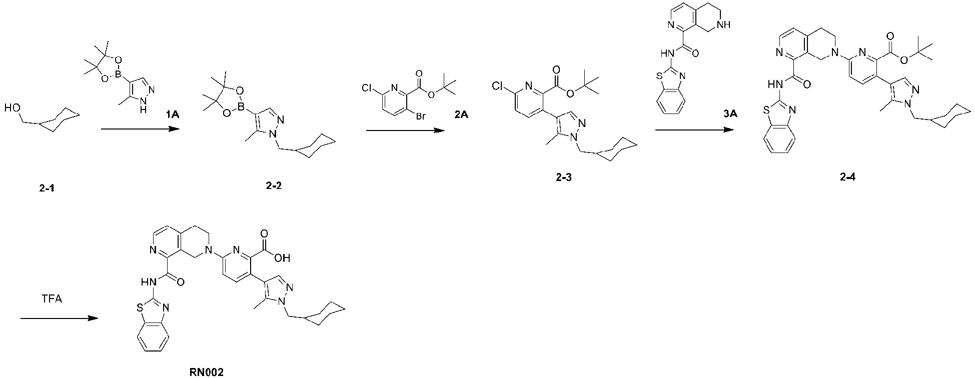

### Step 1: Synthesis of Compound 2-2

Compound **2-1** (3.00 g, 14.4 mmol), compound 1A (3.54 mL, 28.8 mmol), and CMBP (CAS: 157141-27-0) (4.87 g, 20.2 mmol) were dissolved in toluene (30 mL). After nitrogen replacement, the reaction solution was reacted at 90 °C for 12 h. After completion of the reaction, the mixture was diluted with water (20 mL), extracted with ethyl acetate (5 mL × 3), washed with saturated brine (10 mL), dried over anhydrous sodium sulfate, filtered, and concentrated under reduced pressure to afford a crude product, which was purified to afford compound **2-2** as a brown solid (316 mg, yield 7.20%).

MS (ESI) m/z = 305.2 [M+H]⁺.

**¹H NMR** (400 MHz, CDCl₃): *δ* 7.70 (s, 1H), 3.86 (d, *J =* 7.2 Hz, 2H), 2.42 (d, *J =* 6.4 Hz, 3H), 1.92 (dd, *J* = 7.2, 3.6 Hz, 1H), 1.75 - 1.57 (m, 6H), 1.29 (d, *J =* 13.6 Hz, 12H), 1.20 (d, *J =* 8.0 Hz, 2H), 0.99 (t, *J=* 12.0 Hz, 2H).

### Step 2: Synthesis of Compound 2-3

Compound **2-2** (365 mg, 1.25 mmol), tert-butyl 3-bromo-6-chloropyridine carboxylate (316 mg, 1.04 mmol), Pd(dppf)Cl₂ (84.8 mg, 0.10 mmol) and K₂CO₃ (431 mg, 3.12 mmol) were dissolved in dioxane (3 mL)/H₂O (0.6 mL) and reacted at 90 °C for 2 h. The reaction solution was concentrated under reduced pressure, diluted with H₂O (10 mL), extracted with ethyl acetate (5 mL × 3), washed with saturated brine (10 mL × 2), dried over anhydrous sodium sulfate, filtered, and concentrated under reduced pressure to obtain a crude product, which was purified by reversed-phase column chromatography (mobile phase: ethyl acetate-petroleum ether, gradient: 0-5%) to afford compound **2-3** as a white solid (361 mg, yield 89.1%).

MS (ESI) m/z = 390.2 [M+H]⁺.

**¹H NMR** (400 MHz, CDCl₃) *δ* 7.55 (d, *J =* 8.0 Hz, 1H), 7.46 (s, 1H), 7.41 (d, *J =* 8.0 Hz, 1H), 3.91 (d, *J =* 7.2 Hz, 2H), 2.18 (s, 3H), 1.93 (ddd, *J =* 11.2, 7.6, 3.6 Hz, 1H), 1.79- 1.64 (m, 6H), 1.40 (s, 9H), 1.20- 1.10 (m, 2H), 1.06- 0.98 (m, 2H).

### Step 3: Synthesis of Compound 2-4

Compound **3A** (80 mg, 0.26 mmol), compound **2-3** (167 mg, 0.43 mmol), Pd(t-Bu₃P)₂ (39.9 mg, 0.08 mmol) and Cs₂CO₃ (252 mg, 0.77 mmol) were dissolved in N,N-dimethylformamide (0.8 mL) and reacted at 130 °C for 2 h. The reaction solution was diluted with H₂O (8 mL), extracted with ethyl acetate (3 mL × 4), washed with saturated brine (5 mL), dried over anhydrous sodium sulfate, filtered, and concentrated under reduced pressure to obtain a crude compound, which was purified to afford compound **2-4** as a yellow solid (33.2 mg, yield 19.5%).

MS (ESI) m/z = 664.4 [M+H]⁺.

### Step 4: Synthesis of Compound RN002

Compound **2-4** (30 mg, 0.05 mmol) was dissolved in dichloromethane, and TFA (0.1 mL, 1.34 mmol) was added. The reaction solution was stirred at 20 °C for 2 h. The reaction solution was concentrated to dryness directly to afford a crude product, which was purified to afford compound **RN002** as a yellow solid (5.7 mg, yield 20.7%).

MS (ESI) m/z = 608.2 [M+H]⁺.

**¹H NMR** (400 MHz, DMSO-*d₆*) *δ* 8.55 (d, *J* = 4.4 Hz, 1H), 8.05 (d, *J =* 8.0 Hz, 1H), 7.81 (d, *J* = 8.0 Hz, 1H), 7.57- 7.45 (m, 3H), 7.40 - 7.30 (m, 2H), 6.99 (d, *J =* 8.8 Hz, 1H), 5.21 (t, 2H), 3.92 (t, *J =* 5.6 Hz, 2H), 3.85 (d, *J =* 7.2 Hz, 2H), 3.04 (t, 2H), 2.13 (s, 3H), 1.84 - 1.75 (m, 1H), 1.74 - 1.31 (m, 6H), 1.29 - 1.17 (m, 2H), 1.02 - 0.89 (m, 2H).

### Example 9. Synthesis of Compound RN004

The synthetic route is as follows:

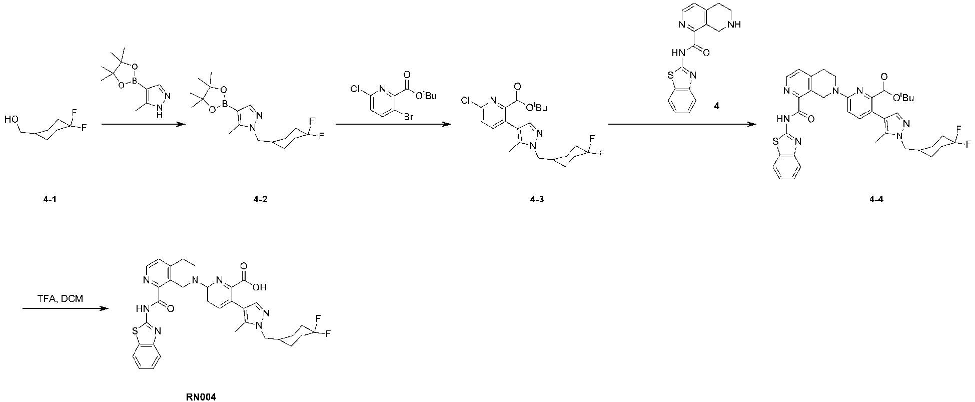

### Step 1: Synthesis of Compound 4-2

To a 100 mL single-necked flask were added toluene (40 mL) and H₂O (0.5 mL). Under stirring, compound **4-1** (5.02 mL, 38.4 mmol), 3-methylpyrazole-4-boronic acid pinacol ester (4.0 g, 19.2 mmol), and CMBP (7.06 mL, 26.9 mmol) were added sequentially. The reaction solution was purged with nitrogen three times and stirred at 90 °C for 18 h. The reaction solution was diluted with water (100 mL) and extracted with ethyl acetate (100 mL × 3). The organic phase was washed with saturated brine (50 mL × 2), dried over anhydrous sodium sulfate, filtered, and concentrated under reduced pressure. The residue was purified by reversed-phase chromatography to afford compound **4-2** as a white solid (418 mg, yield 6.39%).

MS (ESI) m/z = 341.2 [M+H]⁺.

**¹H NMR** (400 MHz, DMSO-*d₆*): *δ* 7.47 (s, 1H), 3.93 (d, *J =* 7.2 Hz, 2H), 2.37 (d, *J =* 4.4 Hz, 3H), 2.08- 1.46 (m, 9H), 1.24 (s, 12H).

### Step 2: Synthesis of Compound 4-3

To a 25 mL single-necked flask were added dioxane (4.0 mL) and H₂O (0.8 mL). Under stirring, compound **4-2** (368 mg, 1.08 mmol), tert-butyl 3-bromo-6-chloropyridine carboxylate (314 mg, 1.08 mmol), K₂CO₃ (448 mg, 3.24 mmol) and Pd(dppf)Cl₂ (79.1 mg, 0.11 mmol) were added sequentially. The reaction solution was purged with nitrogen three times and stirred at 90 °C for 2 h. The reaction solution was diluted with water (20 mL) and extracted with ethyl acetate (10 mL × 3). The organic phase was washed with saturated brine (10 mL × 2), dried over anhydrous sodium sulfate, filtered, and concentrated under reduced pressure to afford a crude product containing the target compound, which was purified to afford compound **4-3** as a yellow oil (169 mg, yield 27.2%).

MS (ESI) m/z = 426.2 [M+H]⁺.

### Step 3: Synthesis of Compound 4-4

To 0.5 mL of dimethyl sulfoxide were added compound **4-3** (80 mg, 0.19 mmol), compound **4** (58.5 mg, 0.19 mmol), Cs₂CO₃ (307 mg, 0.94 mmol) and KI (93.9 mg, 0.57 mmol). The reaction solution was purged with nitrogen three times and stirred under microwave at 150 °C for 2 h. The reaction solution was diluted with water (20 mL) and extracted with ethyl acetate (20 mL × 3). The organic phase was washed with saturated brine (20 mL × 2), dried over anhydrous sodium sulfate, filtered, and concentrated under reduced pressure to obtain a crude product containing the target compound, which was purified (mobile phase: methanol-dichloromethane, gradient: 0-10%) to afford yellow compound **4-4** (7.0 mg, yield 5.32%).

MS (ESI) m/z = 700.4 [M+H]⁺;

### Step 4: Synthesis of Compound RN004

At 0 °C, to a single-necked flask were added sequentially dichloromethane (0.5 mL), compound **4-4** (7 mg, 0.01 mmol) and TFA (0.5 mL). The mixture was stirred at 25 °C for 2 h. The reaction solution was concentrated, and purified by reversed-phase chromatography to afford the brown target product **RN004** (1.45 mg, yield 22.4%).

MS (ESI) m/z = 644.2[M+H]⁺.

**¹H NMR** (400 MHz, DMSO-*d₆*): *δ* 12.86 (s, 1H), 12.31 (s, 1H), 8.56 (d, *J =* 4.8 Hz, 1H), 8.06 (d, *J =* 8.1 Hz, 1H), 7.82 (d, *J =* 8.1 Hz, 1H), 7.59 - 7.52 (m, 2H), 7.49 (t, *J =* 7.7 Hz, 1H), 7.37 (t, *J* = 7.7 Hz, 1H), 7.31 (s, 1H), 7.05 (d, *J =* 8.8 Hz, 1H), 5.23 (s, 2H), 3.98 - 3.94 (m, 4H), 3.06 (t, *J* = 5.5 Hz, 2H), 2.14 (s, 3H), 2.04 - 1.95 (m, 3H), 1.87 - 1.68 (m, 2H), 1.66 - 1.58 (m, 2H), 1.32 - 1.19 (m, 2H).

The following example compounds in Table 1 were prepared according to the same method as the above examples, using commercially available compounds or by reference to the preparation methods of the intermediates shown.

**Table 1**

| Compound ID | Starting Material | Structure | MS |
|---|---|---|---|
| **RN011** | 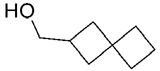 | 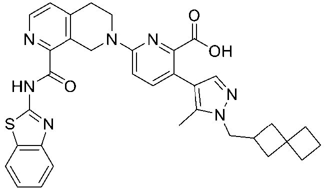 | MS (ESI) m/z = 620.5 [M+H]⁺; |
| **RN012** | 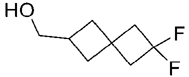 | 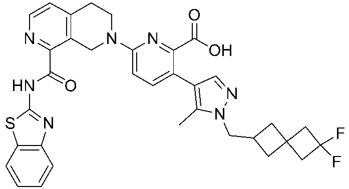 | MS (ESI) m/z = 656.5 [M+H]⁺; |
| **RN087** | 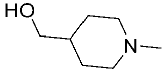 | 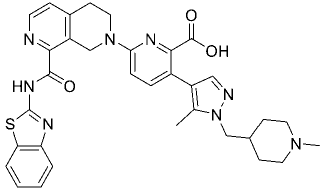 | MS (ESI) m/z = 623.6 [M+H]⁺; |
| **RN088** | 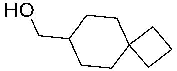 | 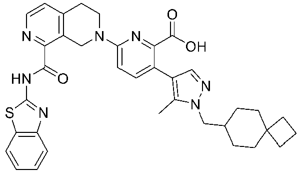 | MS (ESI) m/z = 648.5 [M+H]⁺; |
| **RN015** | 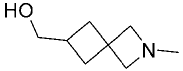 | 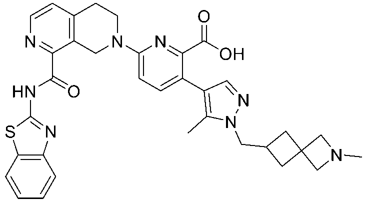 | MS (ESI) m/z = 635.7 [M+H]⁺; |
| **RN016** | 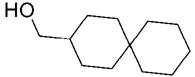 | 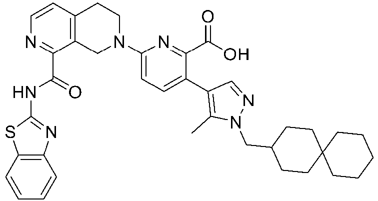 | MS (ESI) m/z = 676.5 [M+H]⁺; |
| **RN017** | 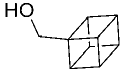 | 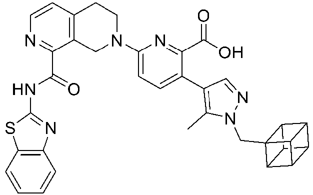 | MS (ESI) m/z = 628.3 [M+H]⁺; |
| **RN086** | 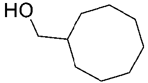 | 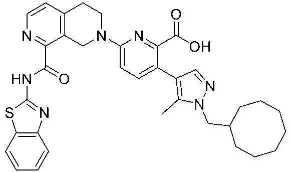 | MS (ESI) m/z = 636.7 [M+H]⁺; |
| **RN080** | 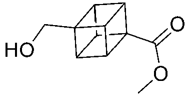 | 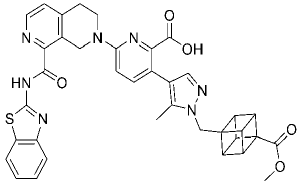 | MS (ESI) m/z = 686.7 [M+H]⁺; |
| **RN081** | 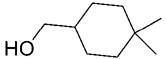 | 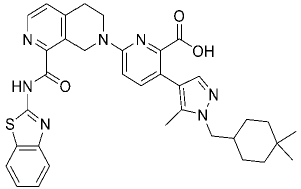 | MS (ESI) m/z = 636.8 [M+H]⁺; |
| **RN082** | 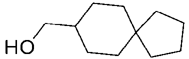 | 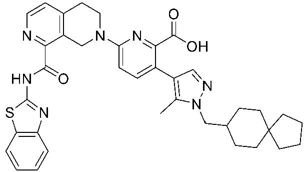 | MS (ESI) m/z = 662.8 [M+H]⁺; |
| **RN083** | 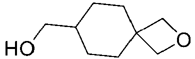 | 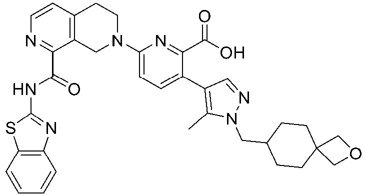 | MS (ESI) m/z = 650.7 [M+H]⁺; |
| **RN084** | 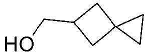 | 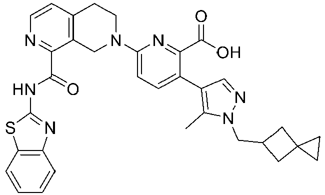 | MS (ESI) m/z = 606.7 [M+H]⁺; |
| **RN085** | 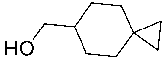 | 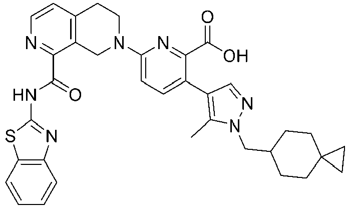 | MS (ESI) m/z = 634.7 [M+H]⁺; |

### Example 10. Synthesis of Compound RN018

The synthetic route is as follows:

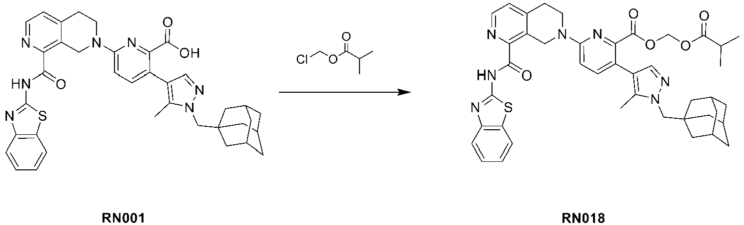

### Step 1: Synthesis of Compound RN018

To a 5 mL brown bottle were added compound **RN001** (9 mg, 13.7 µmol), N,N-dimethylformamide (1 mL), potassium carbonate (10 mg), and chloromethyl isobutyrate (3 mg). The mixture was purged with nitrogen and reacted at room temperature for 8 h. TLC monitoring indicated the reaction was complete (dichloromethane: methanol = 20: 1). The reaction solution was poured into 4 mL of ice water, and extracted twice with 2 mL of ethyl acetate. The ethyl acetate layers were combined, washed twice with 2 mL of saturated brine, dried over sodium sulfate, filtered and concentrated under reduced pressure to afford an oil, which was separated by preparative liquid chromatography to afford compound **RN018** as a white solid (1.5 mg, yield 14.5%).

MS (ESI) m/z = 760.4 [M+H]⁺.

**¹H NMR** (400 MHz, DMSO-*d₆*): *δ* 12.32 (s, 1H), 8.56 (d, *J =* 4.9 Hz, 1H), 8.05 (d, *J =* 7.2 Hz, 1H), 7.82 (d, *J =* 8.0 Hz, 1H), 7.61 (d, *J* = 8.8 Hz, 1H), 7.57 (d, *J* = 4.9 Hz, 1H), 7.53 - 7.44 (m, 1H), 7.41 - 7.33 (m, 1H), 7.24 (s, 1H), 7.13 (d, *J =* 8.9 Hz, 1H), 5.74 (s, 2H), 5.22 (s, 2H), 3.95 (t, *J* = 5.9 Hz, 2H), 3.72 (s, 2H), 3.05 (t, *J =* 5.8 Hz, 2H), 2.11 (s, 3H), 2.00 (q, *J =* 7.0 Hz, 1H), 1.93 (s, 3H), 1.72 - 1.39 (m, 12H), 1.05 (dd, *J=* 12.7, 6.9 Hz, 6H).

The following example compounds in Table 2 were prepared according to the same method as the above examples, using commercially available compounds or by reference to the preparation methods of the intermediates shown.

**Table 2**

| Compound ID | Starting Material | Structure | MS |
|---|---|---|---|
| 2D | 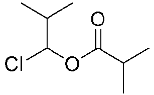 | 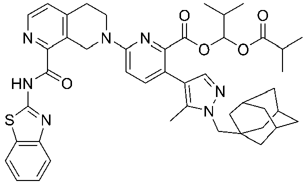 | MS (ESI) m/z = 802.7 [M+H]⁺; |
| 3D | 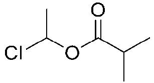 | 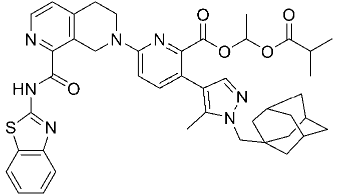 | MS (ESI) m/z = 774.6 [M+H]⁺; |
| 4D | 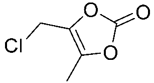 | 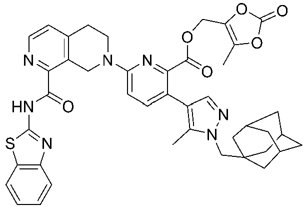 | MS (ESI) m/z = 772.6 [M+H]⁺; |
| 5D | 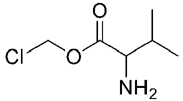 | 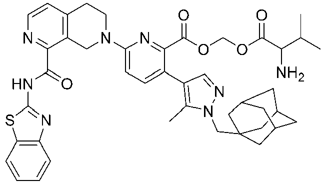 | MS (ESI) m/z = 788.7 [M+H]⁺; |

### Example 11. Synthesis of Compound RN019

The synthetic route is as follows:

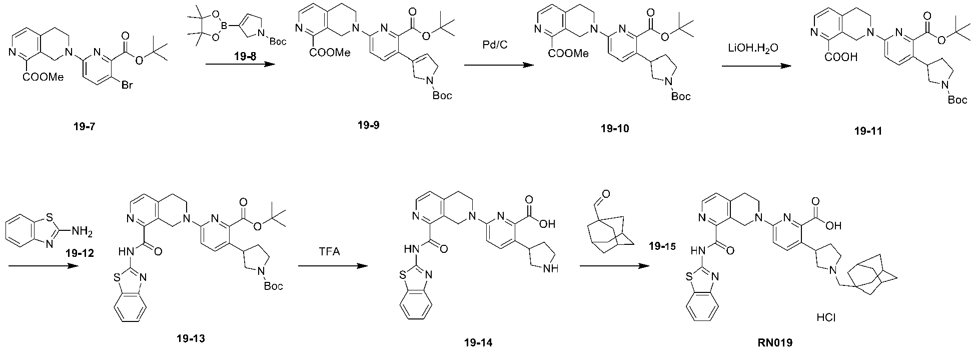

### Step 1: Synthesis of Compound 19-9

Compound **19-7** (200 mg, 446.12 µmol, 1 eq) and compound **19-8** (263.37 mg, 892.24 µmol, 2 eq) were dissolved in 1 mL of dioxane and 0.2 mL of water, then 1,1'-bis(diphenylphosphino)ferrocene palladium dichloride (16.32 mg, 22.31 µmol, 0.05 eq) and potassium carbonate (184.97 mg, 1.34 mmol, 3 eq) were added. The reaction solution was stirred at 90 °C for 16 h under nitrogen protection. 5 mL of water was added to the reaction solution, then extracted twice with 5 mL of ethyl acetate. The organic phase was washed with 5 mL of saturated brine, dried over anhydrous magnesium sulfate, filtered and concentrated to afford a crude product, which was purified by column chromatography to afford compound **19-9** as a white solid (200 mg, yield 83.54%).

**¹H NMR:** *δ* 8.57 (br d, *J* = 4.1 Hz, 1H), 7.51 - 7.41 (m, 1H), 7.34 (br d, *J =* 4.6 Hz, 1H), 6.84 (br d, *J* = 8.6 Hz, 1H), 5.77 (br d, *J =* 17.6 Hz, 1H), 5.09 (s, 2H), 4.46 - 4.21 (m, 4H), 4.06 (s, 5H), 3.07 (br s, 2H), 1.60 (s, 9H), 1.51 (d, *J =* 4.3 Hz, 9H)

### Step 2: Synthesis of Compound 19-10

Compound **19-9** (150 mg, 279.53 µmol, 1 eq) was dissolved in 10 mL of methanol, then wet palladium on carbon (150.00 mg, 140.95 µmol, 10%, 5.04e-1 eq) was added. The mixture was purged with argon three times. The reaction solution was stirred at 25 °C under 15 psi pressure for 16 h. LCMS monitoring indicated the starting material was completely reacted and the product was formed. The reaction solution was filtered, and the filtrate was concentrated under reduced pressure to afford compound **19-10** as a yellow solid (150 mg, crude).

### Step 3: Synthesis of Compound 19-11

Compound **19-10** (150 mg, 278.48 µmol, 1 eq) was dissolved in 2 mL of methanol and 1 mL of water, then lithium hydroxide monohydrate (23.37 mg, 556.96 µmol, 2 eq) was added. The reaction solution was stirred at 25 °C for 1 h. LCMS monitoring indicated the starting material was completely reacted and the product was formed. The reaction solution was adjusted to pH 6 with 1 mol/L hydrochloric acid solution. Then it was concentrated under reduced pressure to afford compound **19-11** as a yellow solid (146 mg, 278.30 µmol, crude).

### Step 4: Synthesis of Compound 19-13

Compound **19-11** (136 mg, 259.24 µmol, 1 eq) and 1,3-benzothiazol-2-amine (101.24 mg, 674.03 µmol, 2.6 eq) were dissolved in 2 mL of N,N-dimethylformamide, then N,N-diisopropylethylamine (167.53 mg, 1.30 mmol, 225.78 µL, 5 eq) and 1-[bis(dimethylamino)methylene]-1H-1,2,3-triazolo[4,5-b]pyridinium 1-oxide hexafluorophosphate (197.14 mg, 518.48 µmol, 2 eq) were added. The reaction solution was stirred at 25 °C for 1 h. LCMS monitoring indicated the starting material was completely reacted and the product was formed. The reaction solution was filtered, and the filtrate was purified by preparative liquid chromatography to afford compound **19-13** as a white solid (28 mg, yield 16.44%).

### Step 5: Synthesis of Compound 19-14

Compound **19-13** (28 mg, 42.63 µmol, 1 eq) was dissolved in 2 mL of dichloromethane, then trifluoroacetic acid (145.83 mg, 1.28 mmol, 95.00 µL, 30 eq) was added. The reaction solution

was stirred at 25 °C for 1 h. Monitoring indicated the starting material was completely reacted and the product was formed. The reaction solution was concentrated under reduced pressure to afford compound **19-14** as a brown solid (26 mg, crude).

### Step 6: Synthesis of Compound RN019

Compound **19-14** (20 mg, 39.95 µmol, 1 eq) and 1-adamantane formaldehyde (19.69 mg, 119.86 µmol, 3 eq) were dissolved in 2 mL of methanol and 2 mL of N,N-dimethylformamide, then triethylamine (12.13 mg, 119.86 µmol, 16.68 µL, 3 eq) and sodium cyanoborohydride (5.02 mg, 79.91 µmol, 2 eq) were added sequentially. The reaction solution was stirred at 25 °C for 16 h. The reaction was quenched by addition of 1 mL of water, then filtered. The filtrate was purified by preparative liquid chromatography to afford **RN019** hydrochloride as a yellow solid (3.27 mg, yield 9.18%).

**¹H NMR:** (400 MHz, DMSO-*d₆*): *δ* 12.49 - 12.11 (m, 1H), 10.30 - 10.12 (m, 1H), 9.62 - 9.46 (m, 1H), 8.56 (d, *J* = 4.6 Hz, 1H), 8.13 - 8.03 (m, 1H), 7.89 (d, *J =* 9.0 Hz, 1H), 7.83 (d, *J=* 8.1 Hz, 1H), 7.59 - 7.53 (m, 1H), 7.50 (t, *J =* 7.7 Hz, 1H), 7.43 - 7.30 (m, 1H), 7.24 - 7.09 (m, 1H), 5.24 (s, 2H), 4.09 - 3.98 (m, 1H), 3.97 - 3.88 (m, 2H), 3.87 - 3.74 (m, 2H), 3.28 - 3.10 (m, 2H), 3.09 - 2.99 (m, 4H), 2.30 - 2.02 (m, 2H), 1.97 (br d, *J*= 2.9 Hz, 3H), 1.75 - 1.57 (m, 12H).

### Example 12. Synthesis of Compounds RN013 and RN014

The synthetic route is as follows:

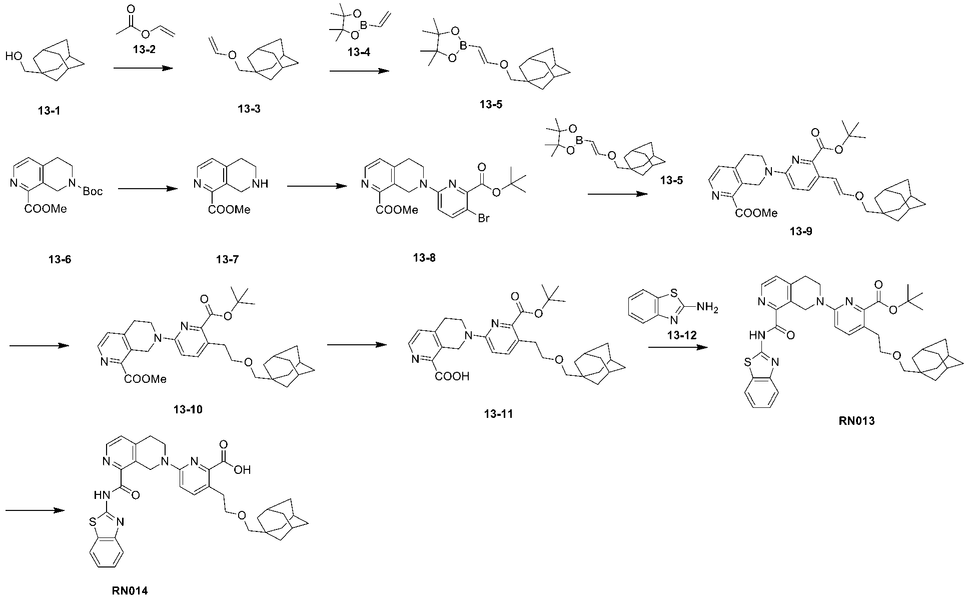

### Step 1: Synthesis of Compound 13-3

[(3R,5S,7s)-Adamantan-1-yl]methanol (10 g, 60.15 mmol, 1 eq) was dissolved in 100 mL of toluene, vinyl acetate (15.53 g, 180.44 mmol, 16.70 mL, 3 eq), potassium carbonate (3.82 g, 36.09 mmol, 0.6 eq) and 1,5-cyclooctadiene iridium chloride dimer (404.01 mg, 601.47 µmol, 0.01 eq) were added. The reaction solution was stirred at 110 °C for 16 h. TLC indicated a new spot was formed and the starting material was completely reacted. The reaction solution was quenched with 200 mL of saturated sodium carbonate solution, then extracted twice with 200 mL of ethyl acetate. The combined organic phase was washed with 200 mL of saturated brine, dried over anhydrous magnesium sulfate, filtered and concentrated. The residue was purified by column chromatography to afford compound **13-3** (7.61 g, yield 65.80%).

**¹H NMR:** (400 MHz, CDCl₃): *δ* 6.41 (dd, *J =* 6.8, 14.4 Hz, 1H), 4.06 (dd, *J =* 1.8, 14.3 Hz, 1H), 3.85 (dd, *J =* 1.8, 6.8 Hz, 1H), 3.16 (s, 2H), 1.91 (br s, 3H), 1.70 - 1.64 (m, 3H), 1.61 - 1.56 (m, 3H), 1.49 (d, *J =* 2.4 Hz, 6H)

### Step 2: Synthesis of Compound 13-5

Compound **13-3** (3 g, 15.60 mmol, 1 eq) was dissolved in 30 mL of toluene, 2-vinyl-4,4,5,5-tetramethyl-1,3,2-dioxaborolane (2.40 g, 15.60 mmol, 2.65 mL, 1 eq) and Grubbs second-generation catalyst (1.32 g, 1.56 mmol, 0.1 eq) were added. The reaction solution was stirred at 110 °C for 16 h. TLC indicated a new spot was formed. The reaction solution was post-treated and purified by column chromatography to afford compound **13-5** as a yellow oil (2.13 g, yield 42.90%).

**¹H NMR** (400 MHz, CDCl₃): *δ* 7.06 (d, *J =* 14.4 Hz, 1H), 4.41 (d, *J =* 14.3 Hz, 1H), 3.33 (s, 2H), 1.98 (br s, 3H), 1.75 - 1.70 (m, 3H), 1.68 - 1.64 (m, 3H), 1.55 (d, *J =* 2.4 Hz, 6H), 1.25 (s, 12H)

### Step 3: Synthesis of Compound 13-7

Compound **13-6** (1.95 g, 6.67 mmol, 1 eq) was dissolved in 20 mL of dichloromethane, trifluoroacetic acid (15.35 g, 134.62 mmol, 10 mL, 20.18 eq) was added. The reaction solution was stirred at 25 °C for 1 h. The reaction solution was concentrated to obtain a crude product, which was dissolved in 30 mL of dichloromethane, and was adjusted to pH 9 with saturated aqueous sodium bicarbonate solution, and extracted five times with a mixed solution of dichloromethane: methanol = 1: 1 (50 mL). The combined organic phase was dried over anhydrous magnesium sulfate, filtered and concentrated to afford compound **13-7** as a yellow oil (1.06 g, yield 82.67%).

### Step 4: Synthesis of Compound 13-8

Compound **13-7** (1.06 g, 5.51 mmol, 1 eq) was dissolved in 10 mL of dimethyl sulfoxide. Potassium fluoride (1.60 g, 27.57 mmol, 5 eq) and tert-butyl 3-bromo-6-chloropyridine-2-carboxylate (3.23 g, 11.03 mmol, 2 eq) were added. The reaction solution was stirred at 100 °C for 16 hours. The reaction solution was poured into 30 mL of water and extracted twice with 30 mL of ethyl acetate. The combined organic phase was washed with 50 mL of saturated brine, dried over anhydrous magnesium sulfate, filtered and concentrated. The residue was purified by column chromatography to afford compound **13-8** as a yellow oil (1.51 g, yield 61.08%).

### Step 5: Synthesis of Compound 13-9

Compound **13-8** (300 mg, 669.18 µmol, 1 eq) was dissolved in 3 mL of dioxane and 0.6 mL of water, and 4,4,5,5-tetramethyl-2-[(1E)-2-{[(3R,5S,7s)-adamantan-1-yl]methoxy}vinyl]-1,3,2-dioxaborolane (563 mg, 1.77 mmol, 2.64 eq), potassium carbonate (277.45 mg, 2.01 mmol, 3 eq) and 1,1'-bis(diphenylphosphino)ferrocene palladium dichloride (48.96 mg, 66.92 µmol, 0.1 eq) were added. The reaction solution was stirred at 100 °C for 2 hours under nitrogen atmosphere. The reaction solution was poured into 30 mL of water and extracted twice with 30 mL of ethyl acetate. The combined organic phase was washed with 50 mL of saturated brine, dried over anhydrous magnesium sulfate, filtered and concentrated. The residue was purified by column chromatography to afford compound **13-9** as a yellow oil (302 mg, yield 80.63%).

### Step 6: Synthesis of Compound 13-10

Compound **13-9** (200 mg, 357.34 µmol, 1 eq) was dissolved in 5 mL of tetrahydrofuran, and wet palladium on carbon (100.00 mg, 10% palladium content) was added. The reaction solution was stirred at 25 °C under 15 psi hydrogen atmosphere for 16 hours. The reaction solution was filtered and concentrated and the residue was purified by column chromatography to afford yellow compound **13-10** (141 mg, yield 70.25%).

### Step 7: Synthesis of Compound 13-11

Compound **13-10** (141 mg, 251.02 µmol, 1 eq) was dissolved in a mixed solution of 1 mL of tetrahydrofuran, 1 mL of methanol and 1 mL of water, and lithium hydroxide monohydrate (21.07 mg, 502.04 µmol, 2 eq) was added. The reaction solution was stirred at 25 °C for 1 hour under nitrogen atmosphere. The reaction solution was adjusted to pH 7 with 0.5 mol/L hydrochloric acid, and then lyophilized to afford compound **13-11** as a yellow solid (121 mg, crude).

### Step 8: Synthesis of Compound RN013

Compound **13-11** (60 mg, 109.55 µmol, 1 eq) was dissolved in 1 mL of N,N-dimethylformamide. N,N-diisopropylethylamine (70.79 mg, 547.76 µmol, 95.41 µL, 5 eq) and 1,3-benzothiazol-2-amine (32.91 mg, 219.10 µmol, 2 eq) were added, followed by addition of HATU (124.97 mg, 328.66 µmol, 3 eq). The reaction solution was stirred at 25 °C for 2 hours. LCMS indicated the

starting material was completely consumed. The reaction solution was poured into 10 mL of water and extracted twice with 10 mL of ethyl acetate. The combined organic phase was washed with 10 mL of saturated brine, dried over anhydrous magnesium sulfate, filtered and concentrated. The residue was purified by column chromatography to give **RN013** as a yellow oil (20 mg, yield 26.85%).

### Step 9: Synthesis of Compound RN014

Compound **RN013** (48 mg, 70.60 µmol, 1 eq) was dissolved in 1 mL of dichloromethane, and trifluoroacetic acid (1.54 g, 13.46 mmol, 1 mL, 190.68 eq) was added. The reaction solution was stirred at 25 °C for 2 hours. 3 mL of N,N-dimethylformamide was added to the reaction solution, and the resulting solution was adjusted to pH 8 with DIEA. Then the solution was purified by preparative liquid chromatography to afford compound **RN014** as an off-white solid (24.11 mg, yield 54.75%).

**¹H NMR:** (400 MHz, DMSO-*d₆*): δ 8.53 (d, *J =* 4.8 Hz, 1H), 8.05 (d, *J =* 7.9 Hz, 1H), 7.81 (d, *J* = 8.0 Hz, 1H), 7.52 (d, *J = 4.8* Hz, 1H), 7.50 - 7.42 (m, 2H), 7.39 - 7.33 (m, 1H), 6.83 (d, *J = 8.6* Hz, 1H), 5.14 (s, 2H), 3.88 (br t, *J =* 5.7 Hz, 2H), 3.47 (br s, 2H), 3.01 (br t, *J =* 5.5 Hz, 2H), 2.93 (s, 2H), 2.80 (br t, *J =* 6.9 Hz, 2H), 1.89 (br s, 3H), 1.70 - 1.61 (m, 3H), 1.61 - 1.54 (m, 3H), 1.45 (d, *J =* 1.6 Hz, 6H)

### Example 13. Synthesis of Compound RN020

The synthetic route is as follows:

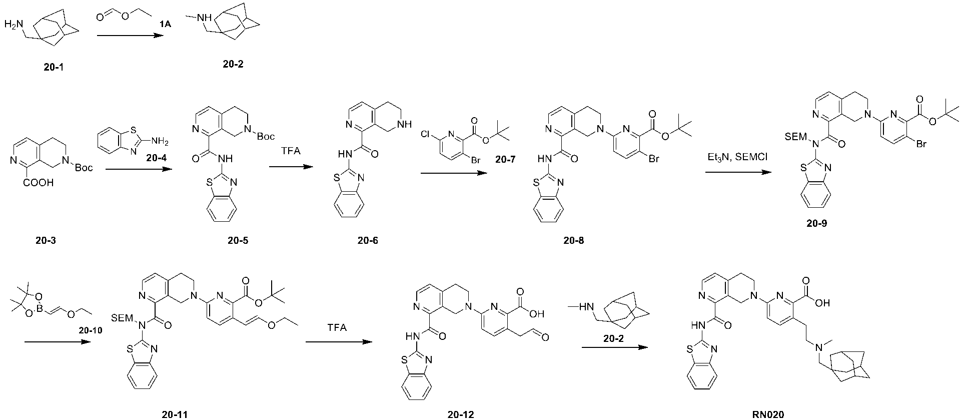

### Step 1: Synthesis of Compound 20-2

Compound **20-1** (200 mg, 1.21 mmol, 214.36 µL, 1 eq) was dissolved in ethyl formate (1.84 g, 24.87 mmol, 2 mL, 20.55 eq). The reaction solution was stirred at 60 °C for 16 h under nitrogen atmosphere, then the solvent was removed by rotary evaporation to dryness. The residue was dissolved in 4 mL of tetrahydrofuran and cooled to 0 °C. Lithium aluminum hydride (2.5 M, 1.45 mL, 3 eq) was added dropwise at 0 °C, then the reaction solution was slowly warmed to room temperature. The reaction solution was stirred at 80 °C for 16 h under nitrogen atmosphere. TLC (petroleum ether: ethyl acetate = 4: 1) indicated a new spot was formed. The reaction solution was quenched with 20 mL of ice water at 0 °C, then 20 mL of 2N aqueous sodium hydroxide solution was added, and the mixture was extracted twice with 30 mL of ethyl acetate. The combined organic phase was washed with 50 mL of saturated brine, dried over anhydrous magnesium sulfate, filtered and concentrated to afford compound **20-2** as a yellow oil (91 mg, yield 41.94%).

**¹H NMR:** *δ* 2.42 (s, 3H), 2.22 (s, 2H), 1.97 (br s, 3H), 1.76 - 1.69 (m, 3H), 1.68 - 1.61 (m, 3H), 1.53 (br s, 6H).

### Step 2: Synthesis of Compound 20-5

Compound **20-3** (2.7 g, 9.70 mmol, 1 eq) was dissolved in 40 mL of N,N-dimethylformamide, followed by addition of N,N-diisopropylethylamine (3.76 g, 29.10 mmol, 5.07 mL, 3 eq) and compound **20-3** (1.75 g, 11.64 mmol, 1.2 eq). Then HATU (5.53 g, 14.55 mmol, 1.5 eq) was added in batches at 0 - 5 °C. The reaction solution was stirred at 25 °C for 2 h. LCMS monitoring indicated 75% of product was formed. The reaction solution was poured into 50 mL of water and filtered. The residue was washed twice with 30 mL of ethyl acetate, then compound **20-5** as a yellow solid (2.19 g, yield 54.99%) was obtained by rotary evaporation to dryness.

### Step 3: Synthesis of Compound 20-6

Compound **20-5** (1.82 g, 4.43 mmol, 1 eq) was dissolved in 10 mL of dichloromethane, and trifluoroacetic acid (15.35 g, 134.62 mmol, 10 mL, 30.36 eq) was added. The reaction solution was stirred at 25 °C for 1 h. LCMS monitoring indicated 99% of product was formed. The reaction solution was concentrated to give a crude product, and 40 mL of saturated aqueous sodium bicarbonate solution was poured into the crude product and filtered, and the filter cake was concentrated to afford compound **20-6** as a yellow solid (1.01 g, yield 73.40%).

### Step 4: Synthesis of Compound 20-8

Compound **20-6** (N-(1,3-benzothiazol-2-yl)-5,6,7,8-tetrahydro-2,7-naphthyridine-1-carboxamide) (1.01 g, 3.25 mmol, 1 eq) was dissolved in 15 mL of dimethyl sulfoxide, and cesium carbonate (4.24 g, 13.02 mmol, 4 eq), compound **20-7** tert-butyl 3-bromo-6-chloropyridine-2-carboxylate (2.86 g, 9.76 mmol, 3 eq) were added. The reaction solution was stirred at 95 °C for 16 h. LCMS monitoring indicated 36% of product is formed. The reaction

solution was poured into 30 mL of water, adjusted to pH 7 with 1N hydrochloric acid, filtered, and the filtrate was extracted twice with 30 mL of ethyl acetate. The combined organic phase was washed with 30 mL of saturated brine, dried over anhydrous magnesium sulfate, filtered and concentrated. The residue was purified by column chromatography to afford compound **20-8** as a yellow solid (1.02 g, yield 55.33%).

### Step 5: Synthesis of Compound 20-9

Compound **20-8** (1 g, 1.77 mmol, 1 eq) was dissolved in 10 mL of tetrahydrofuran, and triethylamine (357.26 mg, 3.53 mmol, 491.42 µL, 2 eq) and 2-(trimethylsilyl)ethoxymethyl chloride (441.47 mg, 2.65 mmol, 468.66 µL, 1.5 eq) were added. The reaction solution was stirred at 25 °C for 1 h. The reaction solution was poured into 50 mL of water, and extracted twice with 50 mL of dichloromethane. The combined organic phase was washed with 100 mL of saturated brine, dried over anhydrous magnesium sulfate, filtered and concentrated. The residue was purified by column chromatography to afford compound **20-9** as a yellow oil (0.97 g, yield 78.86%).

### Step 6: Synthesis of Compound 20-11

Compound **20-9** (150.00 mg, 215.29 µmol, 1 eq) was dissolved in 2 mL of dioxane and 0.5 mL of water, and compound **20-10** (2-[(1E)-2-ethoxyvinyl]-4,4,5,5-tetramethyl-1,3,2-dioxaborolane) (127.93 mg, 645.88 µmol, 3 eq), potassium carbonate (89.27 mg, 645.88 µmol, 3 eq) and 1,1-bis(diphenylphosphino)ferrocene palladium dichloride (15.75 mg, 21.53 µmol, 0.1 eq) were added. The reaction solution was stirred at 95 °C for 2 h under nitrogen atmosphere. The reaction solution was poured into 10 mL of water, extracted twice with 10 mL of ethyl acetate. The combined organic phase was washed with 10 mL of saturated brine, dried over anhydrous magnesium sulfate, filtered and concentrated. The residue was purified by column chromatography to afford compound **20-11** as a yellow oil (128 mg, yield 86.43%).

### Step 7: Synthesis of Compound 20-12

Compound **20-11** (128.00 mg, 186.07 µmol, 1 eq) was dissolved in 2 mL of dichloromethane, and trifluoroacetic acid (1.54 g, 13.46 mmol, 1 mL, 72.35 eq) was added. The reaction solution was stirred at 25 °C for 16 h. The reaction solution was concentrated to afford compound **20-12** as a brown oil (88.1 mg, crude).

### Step 8: Synthesis of Compound RN020

Compound **20-12** (88.10 mg, 186.06 µmol, 1 eq) was dissolved in 2 mL of dichloromethane, and triethylamine (94.14 mg, 930.30 µmol, 129.49 µL, 5 eq) and compound **20-2** (40.03 mg, 223.27 µmol, 1.2 eq) were added. The reaction solution was stirred at 25 °C for 1 h. Sodium triacetoxyborohydride (78.87 mg, 372.12 µmol, 2 eq) was added, and the reaction solution was stirred at 25 °C for 1 h under nitrogen atmosphere. 3 mL of N,N-dimethylformamide was added into the reaction solution, filtered, and the filtrate was purified by preparative liquid chromatography to afford compound **RN020** as a yellow solid (18.11 mg, yield 14.73%).

MS (ESI) m/z = 637.2 [M+H]⁺.

**¹H NMR** (400 MHz, DMSO-*d₆*): *δ* 8.54 (d, *J =* 4.8 Hz, 1H), 8.05 (d, *J =* 7.6 Hz, 1H), 7.81 (d, *J* = 8.1 Hz, 1H), 7.58 - 7.45 (m, 3H), 7.41 - 7.32 (m, 1H), 6.94 (d, *J =* 8.8 Hz, 1H), 5.16 (s, 2H), 3.89 (br t, *J =* 5.7 Hz, 2H), 3.01 (br t, *J =* 5.6 Hz, 2H), 2.80 - 2.75 (m, 2H), 2.69 - 2.65 (m, 2H), 2.36 (s, 3H), 2.16 (s, 2H), 1.75 (br s, 3H), 1.54 - 1.48 (m, 3H), 1.47 - 1.41 (m, 3H), 1.30 (br s, 6H).

### Example 14: Synthesis of Compound RN021

The synthetic route is as follows:

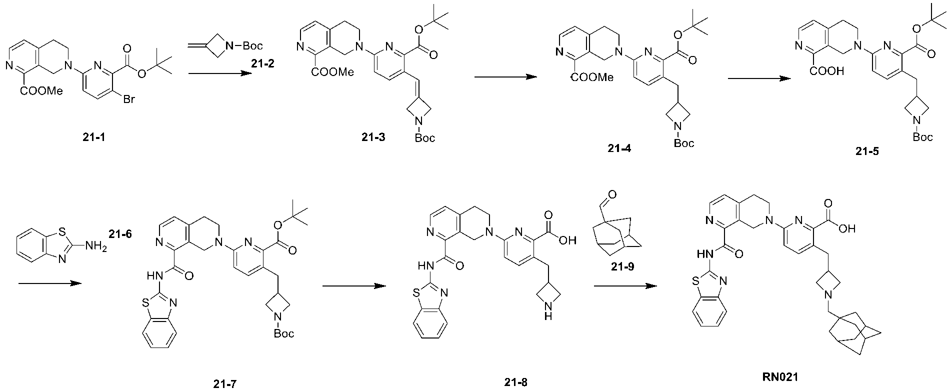

### Step 1: Synthesis of Compound 21-3

Compound **21-1** (300 mg, 669.18 µmol, 1 eq) and compound **21-2** (339.72 mg, 2.01 mmol, 3 eq) were dissolved in 3 mL of N,N-dimethylformamide. Then, cesium carbonate (654.10 mg, 2.01 mmol, 3 eq) and 1,1-bis(diphenylphosphino)ferrocene palladium dichloride (97.93 mg, 133.84 µmol, 0.2 eq) were added. The reaction solution was stirred at 100 °C for 16 h under nitrogen protection. The starting material was completely consumed and product was formed. 10 mL of water was added to the reaction solution, and then extracted twice with 10 mL of ethyl acetate. The organic phase was washed with 10 mL of saturated brine, dried over anhydrous magnesium sulfate, filtered and concentrated to afford a crude product. The crude product was purified by column chromatography to afford compound **21-3** as a yellow solid (230 mg, yield 64.05%).

### Step 2: Synthesis of Compound 21-4

Compound **21-3** (230 mg, 428.61 µmol, 1 eq) was dissolved in 5 mL of methanol. Wet palladium on carbon (115 mg, 108.06 µmol, 10% purity) was then added, and the mixture was purged with argon three times. The reaction solution was stirred at 25 °C under 15 psi for 16 h. The reaction solution was filtered, and the filtrate was concentrated under reduced pressure to afford a crude product. The crude product was purified by column chromatography to afford compound **21-4** as a yellow solid (175 mg, yield 75.80%).

### Step 3: Synthesis of Compound 21-5

Compound **21-4** (175 mg, 324.90 µmol, 1 eq) was dissolved in 1 mL of methanol and 1 mL of water, and lithium hydroxide monohydrate (27.27 mg, 649.79 µmol, 2 eq) was added. The reaction solution was stirred at 25 °C for 1 h. The reaction solution was adjusted to pH 8 with 1 mol/L hydrochloric acid and concentrated under reduced pressure to afford compound **21-5** as a yellow solid (170 mg, crude).

### Step 4: Synthesis of Compound 21-7

Compound **21-5** (150 mg, 285.93 µmol, 1 eq) and 1,3-benzothiazol-2-amine (51.54 mg, 343.11 µmol, 1.2 eq) were dissolved in 2 mL of tetrahydrofuran. Then, N,N-diisopropylethylamine (184.77 mg, 1.43 mmol, 249.02 µL, 5 eq) and 2-chloro-1-methylpyridinium iodide (219.15 mg, 857.78 µmol, 3 eq) were added. The reaction solution was stirred at 60 °C for 16 h. 10 mL of water was added to the reaction solution and extracted twice with 10 mL of ethyl acetate. The organic phase was washed with 10 mL of saturated brine, dried over anhydrous magnesium sulfate, filtered and concentrated to afford crude product. The crude product was purified by column chromatography to afford yellow compound **21-7** (110 mg, 167.48 µmol, yield 58.57%).

### Step 5: Synthesis of Compound 21-8

Compound **21-7** (100 mg, 152.26 µmol, 1 eq) was dissolved in 2 mL of dichloromethane, and trifluoroacetic acid (3.07 g, 26.92 mmol, 2 mL, 176.84 eq) was then added. The reaction solution was stirred at 25 °C for 2 h. LCMS monitoring indicated starting material was completely consumed and product was formed. The reaction solution was concentrated under reduced pressure to afford compound **21-8** as a brown solid (76 mg, crude).

### Step 6: Synthesis of Compound RN021

Compound **21-8** (76 mg, 151.83 µmol, 1 eq) and 1-adamantaneformaldehyde (62.34 mg, 379.57 µmol, 2.5 eq) were dissolved in 2 mL of N,N-dimethylformamide, then triethylamine (46.09 mg, 455.48 µmol, 63.40 µL, 3 eq) and sodium cyanoborohydride (19.08 mg, 303.65 µmol, 2 eq) were added sequentially. The reaction solution was stirred at 25 °C for 1 h. The reaction solution was quenched by adding 0.5 mL of water, then filtered. The filtrate was purified by preparative liquid chromatography to afford **RN021** as a yellow solid (2.51 mg, yield 2.47%).

### Example 15. Synthesis of Compound RN022

The synthetic route is as follows:

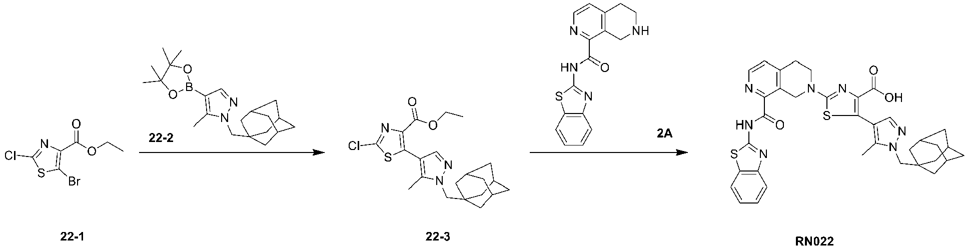

### Step 1: Synthesis of Compound 22-3

To a 25 mL single-necked flask were added dioxane (5 mL) and water (0.5 mL). Under stirring, compound **22-1** (300 mg, 1.11 mmol), compound **22-2** (395 mg, 1.11 mmol), Pd(dppf)Cl₂ (71.6 mg, 0.11 mmol) and cesium carbonate (1.08 g, 3.33 mmol) were added sequentially. The reaction solution was purged with nitrogen three times and stirred at 70 °C for 18 h. After completion of the reaction, the reaction mixture was diluted with water (50 mL), extracted with ethyl acetate (50 mL × 3). The organic phase was washed with saturated brine, dried over anhydrous sodium sulfate, filtered, and concentrated under reduced pressure to afford a crude product containing the target compound, which was purified to afford compound **22-3** as a colorless oil (59 mg, yield 12.6%).

MS (ESI) m/z = 420.2 [M+H]⁺.

### Step 2: Synthesis of Compound RN022

To a 25 mL three-necked flask was added dimethyl sulfoxide (1 mL). Under stirring, compound **22-3** (50 mg, 0.12 mmol), compound **2A** (N-(benzo[d][1,3]thiazol-2-yl)-5,6,7,8-tetrahydropyrido[3,4-c]pyridine-1-carboxamide) (36.9 mg, 0.12 mmol), cesium carbonate (193 mg, 0.60 mmol) and potassium iodide (59.2 mg, 0.36 mmol) were added. The reaction solution was purged with nitrogen three times and stirred at 130 °C for 18 h. The reaction solution was filtered, and the filtrate was purified by reversed-phase preparative chromatography to afford **RN022** as a white solid (2.22 mg, yield 2.8%).

MS (ESI) m/z = 666.2 [M+H]⁺.

**¹H NMR** (400MHz, DMSO-*d₆*): *δ* = 12.30 (br, 1H), 8.60 (d, *J =* 4.8 Hz, 1H), 8.05 (d, *J =* 7.6 Hz, 1H), 7.82 (d, *J =* 8.0 Hz, 1H), 7.61 (d, *J =* 4.8 Hz, 1H), 7.49 (t, *J =* 7.6 Hz, 1H), 7.45 (s, 1H), 7.37 (t, *J =* 7.6 Hz, 1H), 5.15 (s, 2H), 3.81 (t, *J =* 4.8 Hz, 2H), 3.73 (s, 2H), 3.12 (t, *J =* 5.6 Hz, 2H), 2.16 (s, 3H), 1.93 (s, 3H), 1.70-1.61 (m, 3H), 1.60-1.49 (m, 9H).

### Example 16. Synthesis of Compounds RN023 and RN024

The synthetic route is as follows:

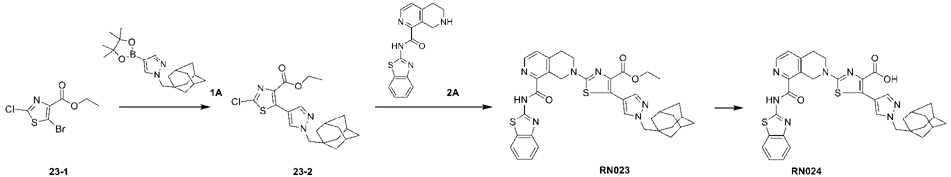

### Step 1: Synthesis of Compound 23-2

Compound **23-1** (1.00 g, 3.70 mmol) and compound **1A** (1.27 g, 3.70 mmol) were dissolved in dioxane (10 mL) and water (1 mL). Cesium carbonate (3.61 g, 11.0 mmol) and Pd(dtbpf)Cl₂ (0.24 g, 0.37 mmol) were added to the reaction solution, and the reaction solution was reacted at 70 °C for 18 h. After completion of the reaction, the reaction solution was concentrated under reduced pressure, diluted with water (30 mL), extracted with ethyl acetate (30 mL × 3), washed with saturated brine (10 mL), dried and concentrated to afford a crude product, which was purified (mobile phase: ethyl acetate-petroleum ether, gradient: 0 - 20%) to afford compound **23-2** as a yellow solid (424 mg, 1.04 mmol, yield 28.2%).

MS (ESI) m/z = 406.2 [M+H]⁺.

### Step 2: Synthesis of Compound RN023

Compound **23-2** (50 mg, 0.12 mmol), compound **2A** (57.3 mg, 0.18 mmol), cesium carbonate (200 mg, 0.62 mmol) and potassium iodide (61.3 mg, 0.37 mmol) were dissolved in dimethyl sulfoxide (1.5 mL) and reacted at 100 °C for 16 h. The reaction solution was diluted with water (20 mL), extracted with ethyl acetate (20 mL × 2), washed with saturated brine (20 mL), dried over anhydrous sodium sulfate, and concentrated under reduced pressure to afford a crude product, which was purified by Prep-TLC (dichloromethane: methanol = 10: 1) to afford **RN023** as a white solid (20 mg, yield 23.8%).

MS (ESI) m/z = 680.2 [M+H]⁺.

### Step 3: Synthesis of Compound RN024

Compound **RN023** (20 mg, 0.03 mmol) and lithium hydroxide monohydrate (6.17 mg, 0.15 mmol) were dissolved in tetrahydrofuran (0.3 mL), methanol (0.05 mL) and water (0.1 mL). The reaction solution was reacted at 25 °C for 3 h. The resulting solution was concentrated and purified by reversed-phase preparative chromatography to afford **RN024** as a white solid (1.02 mg, yield 5.32%).

MS (ESI) m/z = 652.2 [M+H]⁺.

**¹H NMR** (400 MHz, DMSO-*d₆*): *δ* 12.31 (s, 1H), 8.60 (d, *J =* 4.8 Hz, 1H), 8.06 (t, *J =* 3.6 Hz, 2H), 7.82 (d, *J* = 8.0 Hz, 1H), 7.73 (s, 1H), 7.61 (d, *J =* 4.8 Hz, 1H), 7.49(d, *J =* 7.8 Hz, 1H), 7.37 (t, *J* = 7.8 Hz, 1H), 5.15 (s, 2H), 3.84 (t, *J =* 6.0 Hz, 2H), 3.80 (s, 2H), 3.12 (t, *J =* 5.2 Hz, 2H), 1.93 (s,3H), 1.64 (d, *J =* 11.6 Hz, 3H), 1.54 (d, *J =* 10.8 Hz, 3H), 1.47 (s, 6H).

### Example 17. Synthesis of Compounds RN025 and RN026

The synthetic route is as follows:

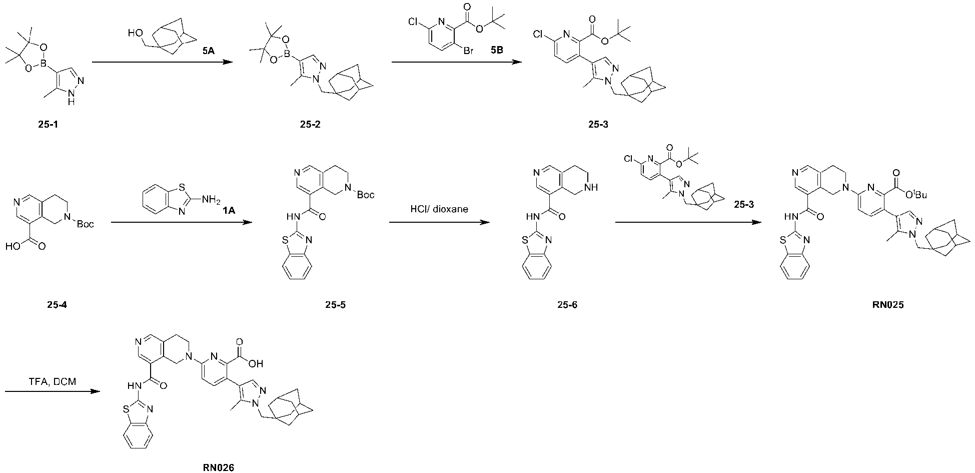

### Step 1: Synthesis of Compound 25-2

At 0 °C, under nitrogen protection, to a 500 mL single-necked flask were added anhydrous toluene (300 mL), 3-methylpyrazole-4-boronic acid pinacol ester (30 g, 144 mmol), 1-adamantanemethanol (33.5 g, 201 mmol). The reaction solution was purged with nitrogen three times and stirred at 100 °C for 12 h. The reaction solution was diluted with water (200 mL), extracted with ethyl acetate (200 mL × 2). The organic phase was washed with saturated brine (100 mL × 2), dried over anhydrous sodium sulfate, filtered, and concentrated under reduced pressure to afford a crude product containing the target compound, which was purified to afford white compound **25-2** (2.5 g, yield 4.87%).

MS (ESI) m/z = 357.2 [M+H]⁺.

**¹H NMR** (400 MHz, CDCl₃): *δ* 7.69 (s, 1H), 3.71 (s, 2H), 2.42 (s, 3H), 1.97 (s, 3H), 1.74-1.55 (m, 12H), 1.31 (s, 12H).

### Step 2: Synthesis of Compound 25-3

To a 100 mL single-necked flask were added 1,4-dioxane (20 mL), water (2.0 mL), tert-butyl 3-bromo-6-chloropyridine carboxylate (1.72 g, 5.87 mmol), compound **25-2** (1.9 g, 5.33 mmol), Pd(dppf)Cl₂ (0.34 g, 0.53 mmol), cesium carbonate (5.21 g, 16.0 mmol). The reaction solution was purged with nitrogen three times and stirred at 90 °C for 12 h. The reaction solution was diluted with water (100 mL), extracted with ethyl acetate (50 mL × 3). The organic phase was washed with saturated brine (50 mL × 2), dried over anhydrous sodium sulfate, filtered, and concentrated under reduced pressure to give a crude product containing the target compound, which was purified to afford yellow compound **25-3** (858 mg, yield 36.4%).

MS (ESI) m/z = 442.2 [M+H]⁺.

**¹H NMR** (400 MHz, DMSO-*d₆*): *δ* 7.85 (d, *J =* 8.0 Hz, 1H), 7.66 (d, *J =* 8.0 Hz, 1H), 7.36 (s, 1H), 3.77 (s, 2H), 2.17 (s, 3H), 1.93 (s, 3H), 1.72-1.53 (m, 12H), 1.36 (s, 9H).

### Step 3: Synthesis of Compound 25-5

To a 25 mL single-necked flask was added N,N-dimethylformamide (3.0 mL). Under stirring, compound **25-4** (300 mg, 1.08 mmol), N,N-diisopropylethylamine (0.54 mL, 3.23 mmol) and HATU (614 mg, 1.62 mmol) were added sequentially. The reaction solution was stirred at room temperature for 0.5 h, followed by addition of 2-aminobenzothiazole (194 mg, 1.29 mmol). The reaction solution was purged with nitrogen three times and stirred at 40 °C for 5 h. The reaction solution was diluted with water (15 mL), extracted with ethyl acetate (10 mL × 3). The organic phase was washed with saturated brine (10 mL × 2), dried over anhydrous sodium sulfate, filtered, and concentrated under reduced pressure to afford a crude product containing the target compound, which was slurried in ethyl acetate (15 mL) and purified to afford white compound **25-5** (350 mg, yield 79.1%).

MS (ESI) m/z = 411.1 [M+H]⁺.

**¹H NMR** (400 MHz, DMSO-*d₆*): *δ* 13.09 (s, 1H), 8.78 (s, 1H), 8.57 (s, 1H), 8.05 (d, *J*=7.6 Hz, 1H), 7.80 (d, *J*= 7.6 Hz, 1H), 7.49 (d, *J*=7.2 Hz, 1H), 7.37 (t, *J*=7.6 Hz, 1H), 4.78 (s, 2H), 3.62 (s, 2H), 2.89 (t, *J*=4.0 Hz, 2H), 1.41 (s, 9H).

### Step 4: Synthesis of Compound 25-6

To a 25 mL single-necked flask was added 1,4-dioxane (5 mL). Under stirring, compound **25-5** (260 mg, 0.63 mmol) was added sequentially, and 4 N hydrogen chloride in 1,4-dioxane (10 mL) was added under ice bath. The reaction solution was stirred at room temperature for 3 h, quenched with saturated sodium carbonate solution (10 mL), extracted with n-butanol (20 mL × 3). The organic phase was washed with saturated brine (10 mL), dried over anhydrous sodium sulfate, filtered, and concentrated under reduced pressure to afford the target compound **25-6** as a white solid (130 mg, yield 66.1%).

MS (ESI) m/z = 311.0 [M+H]⁺.

**¹H NMR** (400 MHz, DMSO-*d₆*): *δ* 13.09 (s, 1H), 8.78 (s, 1H), 8.57 (s, 1H), 8.05 (d, *J*=7.6 Hz, 1H), 7.80 (d, *J*=7.6 Hz, 1H), 7.49 (d, *J*=7.2 Hz, 1H), 7.37 (t, *J*=7.6 Hz, 1H), 4.78 (s, 2H), 3.62 (s, 2H), 2.89 (t, *J*=4.0 Hz, 2H).

### Step 5: Synthesis of Compound RN025

To a 10 mL microwave tube were added N,N-dimethylformamide (3.0 mL), compound **25-6** (84.2 mg, 0.27 mmol), compound **25-3** (80 mg, 0.18 mmol), cesium carbonate (206 mg, 0.63 mmol), and bis(tri-tert-butylphosphine)palladium (18.5 mg, 0.04 mmol). Under nitrogen protection, the reaction solution was reacted under microwave at 120 °C for 2 h. Water (15 mL) was added to the reaction solution, extracted with ethyl acetate (10 mL × 3). The organic phase was washed with saturated brine (10 mL), dried over anhydrous sodium sulfate, filtered, and concentrated under reduced pressure to afford a crude product containing the target compound, which was purified (mobile phase: methanol/dichloromethane, gradient: 0 - 8%) to afford yellow compound **RN025** (106 mg, yield 69.5%).

MS (ESI) m/z = 716.3 [M+H]⁺.

### Step 6: Synthesis of Compound RN026

At 0 °C, to a 25 mL single-necked flask were added dichloromethane (2.0 mL), compound **RN025** (79 mg, 0.11 mmol) and trifluoroacetic acid (2.0 mL), and the reaction solution was stirred at 25 °C for 4 h. The reaction solution was concentrated, and the white target product **RN026** (6.35 mg, yield 8.72%) was obtained by reversed-phase preparative chromatography.

MS (ESI) m/z = 660.4 [M+H]⁺.

**¹H NMR** (400 MHz, DMSO-*d₆*): *δ* 13.09 (br, 1H), 12.83 (br, 1H),8.80 (s, 1H), 8.60 (s, 1H), 8.05 (d, *J*=7.6 Hz, 1H), 7.81 (d, *J*=8.4 Hz, 1H), 7.54 (d, *J*=8.8 Hz, 1H), 7.49 (t, *J*=7.9 Hz, 1H), 7.37 (t, *J*=7.2 Hz, 1H), 7.28 (s, 1H), 7.03 (d, *J*=8.8 Hz, 1H), 5.02 (s, 2H), 3.94 (t, *J*=5.6 Hz, 2H), 3.71 (s, 2H), 3.01 (t, *J*=5.6 Hz, 2H), 2.11 (s, 3H), 1.93 (s, 3H), 1.68-1.61 (m, 3H), 1.60-1.50 (m, 9H).

### Example 18. Synthesis of Compound RN027

The synthetic route is as follows:

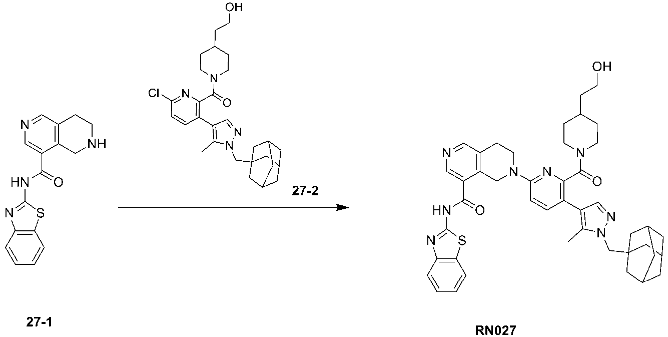

### Step 1: Synthesis of Compound RN027

To a 10 mL microwave tube were added compound **27-1** (200 mg, 0.644 mmol, 1 eq), compound **27-2** (320 mg, 0.644 mmol, 1 eq), cesium carbonate (525 mg, 1.61 mmol, 2.5 eq), bis(tri-tert-butylphosphine)palladium (66 mg, 0.129 mmol, 0.2 eq). The reaction solution was purged with nitrogen, reacted under microwave at 130 °C for 3 h, and filtered with suction. The filter cake was washed with methanol, concentrated under reduced pressure, and separated by column chromatography to afford **RN027** as a white solid (5.75 mg).

MS (ESI) m/z = 771.4 [M+H]⁺.

**¹H NMR** (400 MHz, DMSO-*d₆*): *δ* 13.13 (s, 1H), 8.83 (s, 1H), 8.65 (s, 1H), 8.05 (d, *J =* 7.9 Hz, 1H), 7.81 (d, *J =* 8.0 Hz, 1H), 7.57 - 7.45 (m, 2H), 7.38 (t, *J =* 7.6 Hz, 1H), 7.21 (s, 1H), 6.96 (d, *J* = 8.9 Hz, 1H), 5.04 (s, 2H), 4.24 (d, *J =* 12.5 Hz, 1H), 3.88 (p, *J =* 6.6 Hz, 2H), 3.71 (d, *J =* 2.8 Hz, 2H), 3.27 (t, *J* = 6.4 Hz, 2H), 3.12 (d, *J =* 13.1 Hz, 2H), 3.07 - 3.00 (m, 2H), 2.74 - 2.59 (m, 2H), 2.15 (s, 3H), 1.92 (s, 3H), 1.72 - 1.30 (m, 16H), 1.27 - 1.09 (m, 3H).

### Example 19. Synthesis of Compound RN030

The synthetic route is as follows:

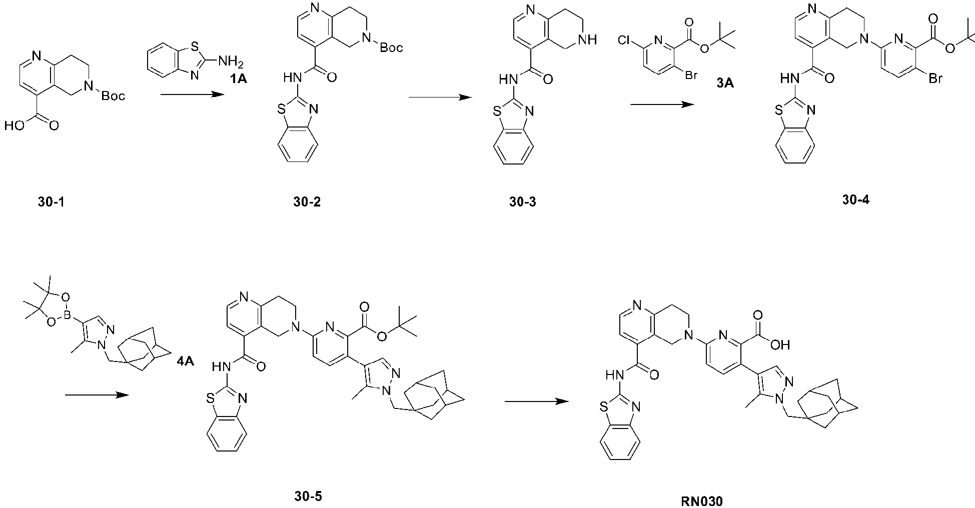

### Step 1: Synthesis of Compound 30-2

Compound **30-1** (440 mg, 1.58 mmol) was dissolved in acetonitrile (5 mL), and CDI (0.28 mL, 2.21 mmol) was added and stirred for 0.5 h. Thereafter, DBU (0.38 mL, 2.53 mmol) was added to the reaction solution and stirred for 0.5 h, then compound **1A** (332 mg, 2.21 mmol) was added and the temperature was raised to 60 °C and stirred for 12 h. The reaction solution was concentrated under reduced pressure to remove acetonitrile, and extracted with a mixed solution of dichloromethane: methanol = 10: 1 (20 mL). The organic phase was washed with saturated brine (5 mL), dried over anhydrous sodium sulfate, and concentrated to afford a crude product, which was purified (mobile phase: dichloromethane-methanol, gradient: 0 - 10%) to afford compound **30-2** as a yellow oil (576 mg, yield 88.8%).

MS (ESI) m/z = 411.1 [M+H)]⁺.

### Step 2: Synthesis of Compound 30-3

Compound **30-2** (520 mg, 1.27 mmol) was dissolved in dioxane (1 mL), and 4N HCl/dioxane (1 mL, 4.00 mmol) was added. The reaction solution was reacted at 20 °C for 2 h. The reaction solution was concentrated under reduced pressure to afford compound **30-3** as a yellow solid (crude 467 mg hydrochloride) which is used directly in the next step.

MS (ESI) m/z = 311.1 [M+H]⁺.

### Step 3: Synthesis of Compound 30-4

Compound **30-3** (467 mg, 1.50 mmol) and compound **3A** (880 mg, 3.01 mmol) were dissolved in dimethyl sulfoxide (10 mL), and cesium carbonate (2450 mg, 7.52 mmol) and potassium iodide (749 mg, 4.51 mmol) were added. The reaction solution was stirred at 100 °C for 48 h. The reaction solution was diluted with water (100 mL), extracted with ethyl acetate (25 mL × 4), washed with brine (50 mL), dried over anhydrous sodium sulfate, and concentrated to afford a crude product compound **30-4** (198 mg, yield 23.2%).

MS (ESI) m/z = 566.0 [M+H]⁺.

### Step 4: Synthesis of Compound 30-5

Compound **30-4** (90.0 mg, 0.16 mmol) and compound **4A** (62.3 mg, 0.17 mmol) were dissolved in water (0.3 mL) and dioxane (1.2 mL), and Pd₂(dba)₃ (14.5 mg, 0.02 mmol), meCgPPh (CAS: 97739-46-3) (9.00 mg, 0.03 mmol) and sodium bicarbonate (53.4 mg, 0.64 mmol) were added. The reaction solution was stirred at 120 °C for 12 h. The reaction solution was filtered through a funnel, and the organic phase was concentrated to afford a crude product, which was purified to afford compound **30-5** as a yellow solid (25.0 mg, yield 22.0%).

MS (ESI) m/z = 716.0 [M+H]⁺.

### Step 5: Synthesis of Compound RN030

Compound 5 (20.0 mg, 0.03 mmol) was dissolved in DCM (0.5 mL), and TFA (0.25 mL, 3.35 mmol) was added. The reaction solution was stirred at 20 °C for 6 h. The reaction solution was concentrated to afford a crude product, which was purified (mobile phase: acetonitrile - water (0.1% FA), gradient: 92 - 98%) to afford product **RN030** as a yellow solid (5.70 mg, yield 30.9%). MS (ESI) m/z = 660.0 [M+H]⁺.

**¹H NMR** (400 MHz, DMSO-*d₆*): *δ* 8.60 (d, J=4.8 Hz, 1H), 8.05 (d,J=8.0 Hz, 1H), 7.81 (d, J= 7.7Hz, 1H), 7.59 (d, J=4.9 Hz, 1H), 7.50 (dd, J= 16.0, 8.2 Hz, 2H), 7.37 (t, J=7.7 Hz, 1H), 7.27(s, 1H), 7.04 (d, J= 8.5 Hz, 1H), 4.97 (s, 2H), 4.01 (s, 2H), 3.70 (s, 2H), 3.11 (s, 2H), 2.10 (s, 3H), 1.93 (s, 3H), 1.59 (dd, J= 40.8, 16.0 Hz, 12H).

### Example 20. Synthesis of Compound RN031

The synthetic route is as follows:

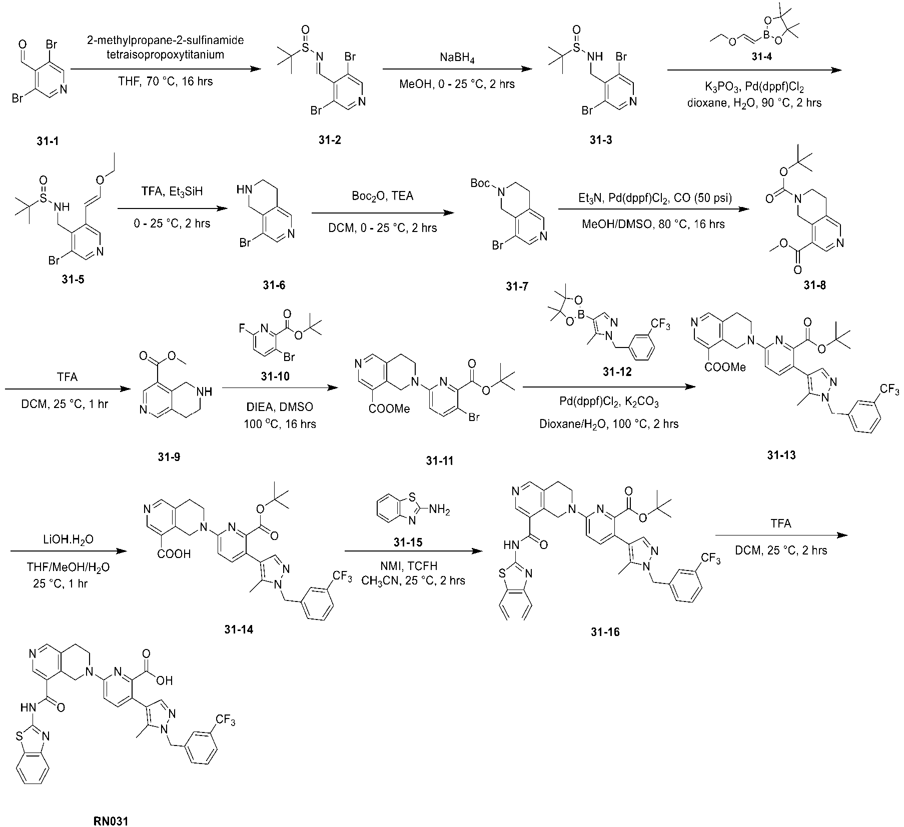

### Step 1: Synthesis of Compound 31-2

3,5-Dibromopyridine-4-formaldehyde (23.42 g, 88.41 mmol, 1 eq) was dissolved in 250 mL of tetrahydrofuran, and 2-methylpropane-2-sulfinamide (12.86 g, 106.09 mmol, 1.2 eq) and titanium tetraisopropoxide (22.61 g, 79.57 mmol, 23.48 mL, 0.9 eq) were added. The reaction solution was stirred at 70 °C for 16 h, and was concentrated to afford compound **31-2** as a yellow solid (32.54 g, crude).

MS (ESI) m/z = 368.7 [M+H]⁺.

### Step 2: Synthesis of Compound 31-3

Compound **31-2** (32.54 g, 88.40 mmol, 1 eq) was dissolved in 350 mL of methanol, and sodium borohydride (1.17 g, 30.94 mmol, 0.35 eq) was added in batches at 0 - 5 °C under nitrogen protection. The reaction solution was stirred at 25 °C for 2 h under nitrogen protection. The reaction solution was quenched with 100 mL of methanol at 0 - 5 °C, then filtered. The filter cake was washed twice with 100 mL of methanol, and the filtrate was concentrated to afford a crude product. The crude product was purified by column chromatography to afford compound **31-3** as a pale yellow solid (26.48 g, yield 80.93%).

### Step 3: Synthesis of Compound 31-5

Compound **31-3** (27.78 g, 75.06 mmol, 1 eq) was dissolved in 300 mL of dioxane and 60 mL of water. Compound **31-4** (15.61 g, 78.81 mmol, 1.05 eq) and potassium phosphate (31.87 g, 150.12 mmol, 2 eq) were added, then the reaction solution was purged with nitrogen three times and 1,1-bis(diphenylphosphino)ferrocene palladium dichloride (5.49 g, 7.51 mmol, 0.1 eq) was added. The reaction solution was stirred at 90 °C for 2 h under nitrogen protection. The reaction solution was poured into 500 mL of water, extracted twice with 500 mL of ethyl acetate. The combined organic phase was washed with 500 mL of saturated brine, dried over anhydrous magnesium sulfate, filtered and concentrated. The residue was purified by column chromatography to afford compound **31-5** as an orange oil (21.64 g, yield 79.80%).

MS (ESI) m/z = 363.0 [M+H] ⁺.

### Step 4: Synthesis of Compound 31-6

Compound **31-5** (21.64 g, 59.90 mmol, 1 eq) was dissolved in 150 mL of trifluoroacetic acid, and triethylsilane (109.20 g, 939.14 mmol, 150 mL, 15.68 eq) was added dropwise at 0 - 5 °C under nitrogen protection. The reaction solution was stirred at 25 °C for 16 h. The reaction solution was concentrated to afford compound **31-6** as a brown oil (12.76 g, crude).

MS (ESI) m/z = 215.0 [M+H]⁺.

### Step 5: Synthesis of Compound 31-7

Compound **31-6** (12.76 g, 59.89 mmol, 1 eq) was dissolved in 150 mL of dichloromethane. Triethylamine (24.24 g, 239.54 mmol, 33.34 mL, 4 eq) was added, then di-tert-butyl dicarbonate (26.14 g, 119.77 mmol, 27.52 mL, 2 eq) was added in batches slowly at 0 °C under nitrogen protection. The reaction solution was stirred at 25 °C for 2 h under nitrogen protection. The reaction solution was poured into 100 mL of water, extracted twice with 100 mL of dichloromethane. The combined organic phase was washed with 100 mL of saturated brine, dried over anhydrous magnesium sulfate, filtered and concentrated. The residue was purified by column chromatography to afford compound **31-7** as a yellow oil (3.32 g, yield 17.70%).

MS (ESI) m/z = 314.9 [M+H] ⁺.

### Step 6: Synthesis of Compound 31-8

Compound **31-7** (3.32 g, 10.60 mmol, 1 eq) was dissolved in 30 mL of methanol and 30 mL of dimethyl sulfoxide, and triethylamine (3.22 g, 31.80 mmol, 4.43 mL, 3 eq) and 1,1-bis(diphenylphosphino)ferrocene palladium dichloride (1.16 g, 1.59 mmol, 0.15 eq) were added. The reaction solution was stirred at 80 °C under 50 psi of carbon dioxide for 16 h. The reaction solution was poured into 300 mL of water and filtered. The filtrate was extracted twice with 100 mL of ethyl acetate. The combined organic phase was dried over anhydrous magnesium sulfate, filtered and concentrated. The residue was purified by column chromatography to afford compound **31-8** as a yellow oil (2.21 g, yield 71.32%).

MS (ESI) m/z = 293.2 [M+H]⁺.

### Step 7: Synthesis of Compound 31-9

Compound **31-8** was dissolved in 10 mL of dichloromethane, and trifluoroacetic acid (15.35 g, 134.62 mmol, 10 mL, 39.35 eq) was added. The reaction solution was stirred at 25 °C for 1 h. The reaction solution was concentrated to afford a crude product. The crude product was dissolved in 50 mL of dichloromethane, then 10 mL of water was added, and pH was adjusted to 9 with sodium carbonate. The solution was extracted five times with a mixed solution of chloroform: isopropanol = 10: 1 (30 mL). The combined organic phase was dried over anhydrous magnesium sulfate, filtered and concentrated to afford compound **31-9** as a yellow oil (651 mg, crude).

MS (ESI) m/z = 193.1 [M+H] ⁺.

### Step 8: Synthesis of Compound 31-11

Compound **31-9** (651 mg, 3.39 mmol, 1 eq) was dissolved in 10 mL of dimethyl sulfoxide, and N,N-diisopropylethylamine (1.31 g, 10.16 mmol, 1.77 mL, 3 eq) and compound **31-10** (3.23 g, 11.03 mmol, 2 eq) were added. The reaction solution was stirred at 100 °C for 16 h. The reaction solution was poured into 30 mL of water, extracted three times with 30 mL of ethyl acetate. The combined organic phase was dried over anhydrous magnesium sulfate, filtered and concentrated. The residue was purified by column chromatography to afford compound **31-11** as an orange oil (1.36 g, yield 89.57%).

### Step 9: Synthesis of Compound 31-13

Compound **31-11** (200 mg, 446.12 µmol, 1 eq) was dissolved in 4 mL of dioxane and 0.4 mL of water, and compound **31-12** (442.12 mg, 892.24 µmol, 2 eq), potassium carbonate (184.97 mg, 1.34 mmol, 3 eq) and 1,1-bis(diphenylphosphino)ferrocene palladium dichloride (32.64 mg, 44.61 µmol, 0.1 eq) were added. The reaction solution was stirred at 100 °C for 2 h under nitrogen protection. The reaction solution was poured into 10 mL of water, extracted twice with 10 mL of ethyl acetate. The combined organic phase was washed with 10 mL of saturated brine, dried over anhydrous magnesium sulfate, filtered and concentrated. The residue was purified by column chromatography to afford compound **31-13** as a yellow oil (261 mg, yield 96.28%).

MS (ESI) m/z = 608.2 [M+H]⁺.

### Step 10: Synthesis of Compound 31-14

Compound **31-13** (261 mg, 429.54 µmol, 1 eq) was dissolved in a mixed solution of 1 mL of tetrahydrofuran, 1 mL of methanol and 1 mL of water, and lithium hydroxide monohydrate (36.05 mg, 859.09 µmol, 2 eq) was added. The reaction solution was stirred at 25 °C for 1 h under nitrogen protection. The reaction solution was adjusted to pH 7 with 1M hydrochloric acid, then compound **31-14** (275 mg, crude) was obtained by rotary evaporation to dryness.

MS (ESI) m/z = 594.2 [M+H] ⁺.

### Step 11: Synthesis of Compound 31-16

Compound **31-14** (76.54 mg, 509.61 µmol, 1.1 eq) was dissolved in 5 mL of acetonitrile, and N-methylimidazole (190.18 mg, 2.32 mmol, 184.64 µL, 5 eq) and 1,3-benzothiazol-2-amine (275 mg, 463.28 µmol, 1 eq) were added. The reaction solution was stirred at 25 °C for 10 min, then N,N,N,N-tetramethylchloroformamidinium hexafluorophosphate (389.96 mg, 1.39 mmol, 3 eq) was added. The reaction solution was stirred at 25 °C for 2 h. The reaction solution was poured into 10 mL of water, extracted twice with 10 mL of ethyl acetate. The combined organic phase was washed with 10 mL of saturated brine, dried over anhydrous magnesium sulfate, filtered and concentrated. The residue was purified by column chromatography to afford compound **31-16** (35 mg, yield 9.96%).

MS (ESI) m/z = 726.3 [M+H]⁺.

### Step 11: Synthesis of RN031

Compound **31-16** (32 mg, 42.19 µmol, 1 eq) was dissolved in 1 mL of dichloromethane, and trifluoroacetic acid (1.54 g, 13.46 mmol, 1 mL, 319.06 eq) was added. The reaction solution was stirred at 25 °C for 2 h. The reaction solution was concentrated to afford a crude product, which was purified by preparative liquid chromatography to afford compound **RN031** as a yellow solid (21.97 mg, yield 76.83%).

MS (ESI) m/z = 670.2 [M+H]⁺.

**¹H NMR** (400 MHz, DMSO-*d₆*): *δ* = 8.80 (s, 1H), 8.58 (s, 1H), 8.02 (d, *J =* 7.6 Hz, 1H), 7.78 (d, *J =* 8.0 Hz, 1H), 7.68 - 7.62 (m, 1H), 7.61 - 7.50 (m, 3H), 7.50 - 7.44 (m, 1H), 7.40 (s, 1H), 7.38 - 7.26 (m, 2H), 7.01 (d, *J =* 8.8 Hz, 1H), 5.44 (s, 2H), 5.04 (s, 2H), 3.94 (br t, *J =* 5.8 Hz, 2H), 3.00 (br t, *J =* 5.6 Hz, 2H), 2.11 (s, 3H).

### Example 21. Synthesis of Compound RN032

The synthetic route is as follows:

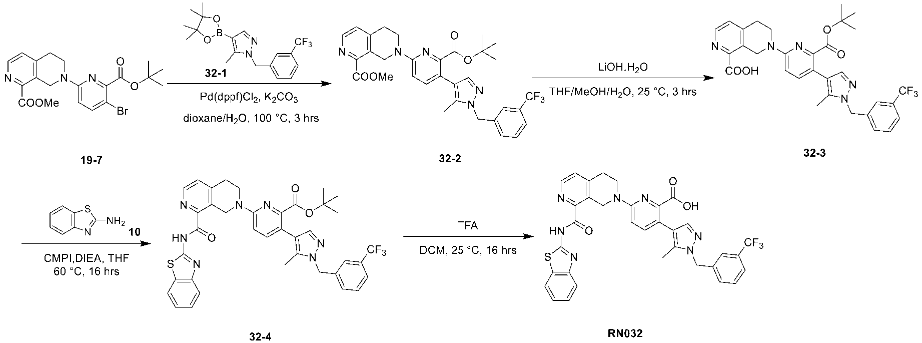

### Step 1: Synthesis of Compound 32-2

Compound **19-7** (3.00 g, 6.69 mmol, 1.00 eq) and compound **32-1** (4.41 g, 12.1 mmol, 1.80 eq) were dissolved in 60 mL of dioxane and 12 mL of water, and 1,1-bis(diphenylphosphino)ferrocene palladium dichloride (979 mg, 1.34 mmol, 0.2 eq) and cesium carbonate (4.36 g, 13.4 mmol, 2.00 eq) were added. The reaction solution was stirred at 100 °C for 3 h under nitrogen protection. The reaction solution was poured into 40 mL of water, extracted twice with 40 mL of ethyl acetate. The combined organic phase was washed with 40 mL of saturated brine, dried over anhydrous magnesium sulfate, filtered and concentrated to afford a crude product. The crude product was purified by column chromatography to afford compound **32-2** as a yellow oil (3.0 g, yield 64.8%).

MS (ESI) m/z = 608.1 [M+H]⁺.

### Step 2: Synthesis of Compound 32-3

Compound **32-2** (2.20 g, 3.62 mmol, 1.00 eq) was dissolved in 10 mL of methanol, 10 mL of tetrahydrofuran and 10 mL of water, and lithium hydroxide monohydrate (456 mg, 10.9 mmol, 3.00 eq) was added. The reaction solution was stirred at 25 °C for 3 h. The reaction solution was adjusted to pH 6~7 with 1M hydrochloric acid. The reaction solution was dried by rotary evaporatation to afford compound **32-3** as a black solid (2.15 g, crude).

MS (ESI) m/z = 594.0 [M+H] ⁺.

### Step 3: Synthesis of Compound 32-4

Compound **32-3** (2.15 g, 3.62 mmol, 1.00 eq) and 1,3-benzothiazol-2-amine (544 mg, 3.62 mmol, 1.00 eq) were dissolved in 20 mL of tetrahydrofuran, and N,N-diisopropylethylamine (2.34 g, 18.1 mmol, 5.00 eq) and 2-chloro-1-methylpyridinium iodide (4.63 g, 18.1 mmol, 5.00 eq) were added. The reaction solution was stirred at 60 °C for 16 h. The reaction solution was poured into 20 mL of water, extracted twice with 20 mL of ethyl acetate. The combined organic phase was washed with 20 mL of saturated brine, dried over anhydrous magnesium sulfate, filtered and concentrated to afford a crude product. The crude product was purified by column chromatography to afford compound **32-4** as a brown solid (2.0 g, yield 65.6%).

MS (ESI) m/z = 726.0 [M+H]⁺.

**¹H NMR** (400 MHz, DMSO-*d₆*): *δ* 12.34 (s, 1H), 8.55 (d, *J =* 4.9 Hz, 1H), 8.05 (d, *J =* 7.5 Hz, 1H), 7.81 (d, *J =* 8.0 Hz, 1H), 7.70 - 7.65 (m, 3H), 7.57 - 7.49 (m, 3H), 7.40 - 7.35 (m, 3H), 7.04 (d, *J =* 8.9 Hz, 1H), 5.44 (s, 2H), 5.21 (s, 2H), 3.93 (br t, *J =* 5.8 Hz, 2H), 3.05 (br t, *J =* 5.8 Hz, 2H), 2.09 (s, 3H), 1.35 (s, 9H).

### Step 4: Synthesis of Compound RN032

Compound **32-4** (2.0 g, 2.76 mmol, 1.00 eq) was dissolved in 10 mL of dichloromethane. Trifluoroacetic acid (23.0 g, 202 mmol, 15 mL, 73.3 eq) was added. The reaction solution was stirred at 25 °C for 16 h. The reaction solution was blown dry with nitrogen to remove dichloromethane and trifluoroacetic acid. The crude product was diluted with 5 mL of dimethyl formamide, adjusted to pH 7-8 with N,N-diisopropylethylamine, and purified by preparative liquid chromatography to afford compound **RN032** as an off-white solid (318 mg, yield 16.82%). MS (ESI) m/z = 670.0 [M+H]⁺.

**¹H NMR** (400 MHz, DMSO-*d₆*): *δ* 8.55 (d, *J* = 4.9 Hz, 1H), 8.05 (d, *J =* 7.9 Hz, 1H), 7.81 (d, *J* = 7.9 Hz, 1H), 7.68 - 7.63 (m, 1H), 7.61 - 7.53 (m, 3H), 7.51 - 7.45 (m, 3H), 7.39 - 7.33 (m, 2H), 6.91 (br d, *J =* 8.8 Hz, 1H), 5.43 (s, 2H), 5.19 (s, 2H), 3.93 (br t, *J =* 5.8 Hz, 2H), 3.05 - 3.02 (m, 2H), 2.14 (s, 3H).

The following example compounds in Table 3 were prepared according to the same method as the above examples, using commercially available compounds or by reference to the preparation methods of the intermediates shown.

**Table 3**

| Compound ID | Starting material | Structure | ¹H-NMR |
|---|---|---|---|
| **RN033** | 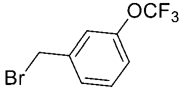 | 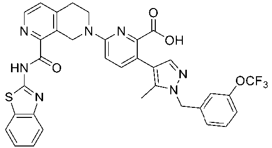 | **¹H NMR:** (400 MHz, DMSO-*d₆*): *δ* = 8.77 - 8.38 (m, 1H), 8.05 (d, *J* = 8.0 Hz, 1H), 7.82 (br d, *J =* 7.8 Hz, 1H), 7.71 - 7.51 (m, 2H), 7.51 - 7.45 (m, 2H), 7.41 (s, 1H), 7.39 - 7.33 (m, 1H), 7.28 (br d, *J* = 7.9 Hz, 1H), 7.16 - 7.07 (m, 2H), 7.03 (br d, *J =* 8.9 Hz, 1H), 5.40 (s, 2H), 5.23 (br s, 2H), 3.94 (br t, *J =* 5.4 Hz, 2H), 3.10 - 2.99 (m, 2H), 2.11 (s, 3H). |
| **RN034** | 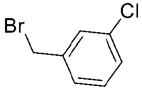 | 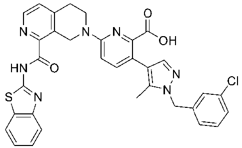 | ¹H NMR: (400 MHz, DMSO-*d₆*): δ = 8.55 (d, *J =* 4.9 Hz, 1H), 8.05 (d, *J* = 7.4 Hz, 1H), 7.81 (d, *J =* 8.0 Hz, 1H), 7.56 (d, *J =* 4.8 Hz, 1H), 7.52 - 7.43 (m, 3H), 7.42 - 7.30 (m, 3H), 7.22 (s, 1H), 7.07 (d, *J =* 6.9 Hz, 1H), 6.92 (br d, *J* = 8.5 Hz, 1H), 5.34 (s, 2H), 5.20 (s, 2H), 3.93 (br t, *J =* 6.0 Hz, 2H), 3.05 (br t, *J =* 5.8 Hz, 2H), 2.13 (s, 3H). |
| **RN035** | 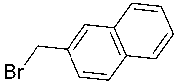 | 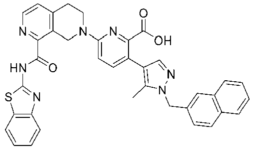 | **¹H NMR:** (400 MHz, DMSO-*d₆*): *δ* = 12.43 - 12.20 (m, 1H), 8.56 (d, *J =* 4.9 Hz, 1H), 8.05 (d, *J* = 7.9 Hz, 1H), 7.91 - 7.84 (m, 3H), 7.81 (d, *J =* 7.9 Hz, 1H), 7.61 - 7.55 (m, 3H), 7.53 - 7.30 (m, 7H), 7.06 (d, *J* = 8.8 Hz, 1H), 5.51 (s, 2H), 5.23 (s, 2H), 3.95 (br t, *J =* 5.8 Hz, 2H), 3.06 (br t, *J* = 5.7 Hz, 2H), 2.14 (s, 3H). |
| **RN036** | 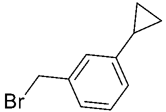 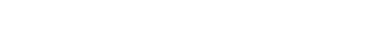 | 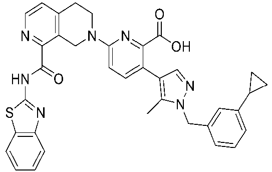 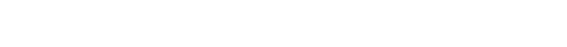 | **¹H NMR:** (400 MHz, DMSO-*d₆*): *δ* = 8.55 (d, *J =* 4.8 Hz, 1H), 8.05 (d, *J* = 7.8 Hz, 1H), 7.81 (d, *J =* 7.9 Hz, 1H), 7.60 - 7.44 (m, 3H), 7.41 - 7.31 (m, 2H), 7.19 (t, *J =* 7.6 Hz, 1H), 7.04 - 6.97 (m, 1H), 6.95 (d, *J* = 7.9 Hz, 1H), 6.88 (s, 1H), 6.84 (d, *J* = 7.5 Hz, 1H), 5.27 (s, 2H), 5.22 (s, 2H), 3.94 (br t, *J =* 5.7 Hz, 2H), 3.05 (br t, *J* = 5.7 Hz, 2H), 2.11 (s, 3H), 1.92 - 1.83 (m, 1H), 0.97 - 0.88 (m, 2H), 0.66 - 0.58 (m, 2H). |
| **RN037** | 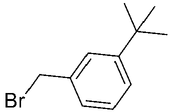 | 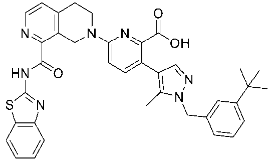 | **¹H NMR:** (400 MHz, DMSO-*d₆*): *δ* = 8.55 (d, *J* = 4.9 Hz, 1H), 8.05 (d, *J =* 7.6 Hz, 1H), 7.81 (d, *J = 8.1* Hz, 1H), 7.56 (d, *J =* 4.9 Hz, 1H), 7.54 - 7.45 (m, 2H), 7.42 - 7.34 (m, 2H), 7.31 - 7.19 (m, 3H), 7.02 - 6.95 (m, 1H), 6.85 (d, *J =* 7.5 Hz, 1H), 5.32 (s, 2H), 5.21 (s, 2H), 3.94 (br t, *J =* 5.8 Hz, 2H), 3.05 (br t, *J =* 5.1 Hz, 2H), 2.12 (s, 3H), 1.25 (s, 9H). |
| **RN038** | 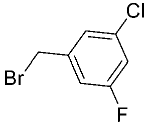 | 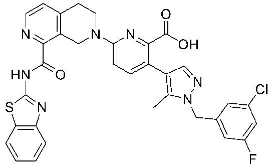 | **¹H** NMR: (400 MHz, DMSO-*d₆*): δ = 8.55 (br d, *J =* 4.5 Hz, 1H), 8.05 (d, *J* = 8.1 Hz, 1H), 7.82 (d, *J* = 8.1 Hz, 1H), 7.56 (br d, *J* = 4.3 Hz, 1H), 7.53 - 7.43 (m, 3H), 7.42 - 7.30 (m, 2H), 7.08 (s, 1H), 6.91 (br dd, *J =* 9.1, 12.9 Hz, 2H), 5.36 (s, 2H), 5.21 (s, 2H), 3.94 (br t, *J* = 5.8 Hz, 2H), 3.05 (br t, *J* = 5.5 Hz, 2H), 2.14 (s, 3H). |
| **RN039** | 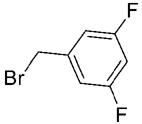 | 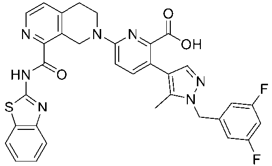 | **¹H NMR:** (400 MHz, DMSO-*d₆*): δ = 8.55 (d, *J* = 4.9 Hz, 1H), 8.05 (d, *J =* 7.9 Hz, 1H), 7.81 (d,*J =* 8.0 Hz, 1H), 7.59 - 7.52 (m, 2H), 7.51 - 7.41 (m, 2H), 7.40 - 7.32 (m, 1H), 7.21 - 7.09 (m, 1H), 6.99 (br d, *J* = 9.1 Hz, 1H), 6.79 (br d, *J =* 6.5 Hz, 2H), 5.37 (s, 2H), 5.22 (s, 2H), 3.94 (br t, *J =* 5.7 Hz, 2H), 3.05 (br t, *J =* 5.3 Hz, 2H), 2.12 (s, 3H). |
| **RN040** | 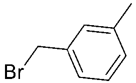 | 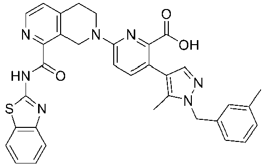 | **¹H NMR:** (400 MHz, DMSO-*d₆*): δ = 8.56 (d, *J* = 4.8 Hz, 1H), 8.06 (d, *J =* 7.4 Hz, 1H), 7.82 (d, *J* = 8.0 Hz, 1H), 7.57 (d, *J =* 5.0 Hz, 1H), 7.54 - 7.45 (m, 2H), 7.41 (s, 1H), 7.39 - 7.31 (m, 1H), 7.27 - 7.18 (m, 1H), 7.08 (d, *J =* 7.5 Hz, 1H), 7.02 - 6.94 (m, 2H), 6.91 (d, *J =* 7.6 Hz, 1H), 5.28 (s, 2H), 5.21 (s, 2H), 3.94 (br t, *J=* 5.8 Hz, 2H), 3.06 (br t, *J* = 5.6 Hz, 2H), 2.28 (s, 3H), 2.12 (s, 3H). |
| **RN041** | 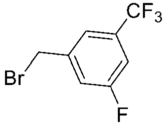 | 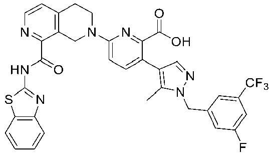 | **¹H NMR:** (400 MHz, DMSO-*d₆*): δ = 8.54 (d, *J* = 4.8 Hz, 1H), 8.05 (d, *J =* 7.9 Hz, 1H), 7.81 (d, *J* = 8.1 Hz, 1H), 7.61 (br d, *J* = 8.9 Hz, 1H), 7.55 (d, *J* = 4.9 Hz, 1H), 7.52 - 7.45 (m, 3H), 7.43 (s, 1H), 7.39 - 7.32 (m, 1H), 7.18 (br d, *J* = 10.0 Hz, 1H), 6.89 (d, *J =* 8.4 Hz, 1H), 5.45 (s, 2H), 5.19 (s, 2H), 3.93 (br t, *J =* 5.7 Hz, 2H), 3.09 - 2.98 (m, 2H), 2.15 (s, 3H). |

### Example 22. Synthesis of Compound RN042

The synthetic route is as follows:

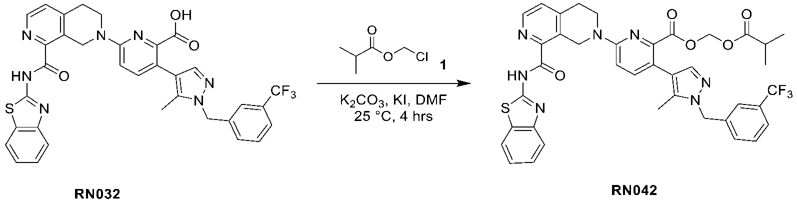

### Step 1: Synthesis of Compound RN042

Compound **RN032** (25.0 mg, 37.3 µmol, 1.00 eq) was dissolved in 1 mL of dimethylformamide, and potassium iodide (6.20 mg, 37.3 µmol, 1.00 eq) and potassium carbonate (10.3 mg, 74.7 µmol, 2.00 eq) were added. The reaction solution was stirred at 25 °C for 10 minutes. Then chloromethyl 2-methylpropanoate (5.10 mg, 37.3 µmol, 1.00 eq) was added, and the reaction solution was stirred at 25 °C for 4 h. The reaction solution was filtered, then the filtrate was purified by preparative liquid chromatography to afford compound **RN042** as an off-white solid (3.5 mg, yield 11.9%).

MS (ESI) m/z = 770.0 [M+H]⁺.

**¹H NMR** (400 MHz, DMSO-*d₆*): *δ* 12.29 (br s, 1H), 8.55 (d, *J* = 4.8 Hz, 1H), 8.07 - 8.00 (m, 1H), 7.81 (d, *J* = 8.1 Hz, 1H), 7.68 - 7.45 (m, 6H), 7.40 - 7.31 (m, 3H), 7.13 (d, *J* = 8.9 Hz, 1H), 5.74 (s, 2H), 5.45 (s, 2H), 5.22 (s, 2H), 3.94 (br t, *J =* 5.7 Hz, 2H), 3.04 (br t, *J =* 5.6 Hz, 2H), 2.48 - 2.40 (m, 1H), 2.09 (s, 3H), 0.99 (d, *J* = 7.0 Hz, 6H).

### Example 22. Synthesis of Compound RN043

The structure of compound **RN043** is as follows:

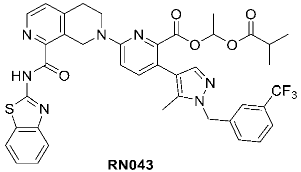

Compound **RN043** was prepared as an off-white solid (7 mg, yield 16.8%) according to the same synthetic method as for Example **RN042**.

MS (ESI) m/z = 784.0 [M+H] ⁺. **¹H NMR** (400 MHz, DMSO-*d₆*): *δ* 12.30 (br s, 1H), 8.54 (d, *J =* 4.8 Hz, 1H), 8.04 (d, *J* = 7.9 Hz, 1H), 7.81 (d, *J =* 8.0 Hz, 1H), 7.68 - 7.63 (m, 1H), 7.61 - 7.53 (m, 4H), 7.48 (t, *J =* 7.6 Hz, 1H), 7.42 (br d, *J =* 7.6 Hz, 1H), 7.39 - 7.31 (m, 2H), 7.14 (d, *J = 8.9* Hz, 1H), 6.73 (q, *J =* 5.4 Hz, 1H), 5.45 (s, 2H), 5.31 - 5.17 (m, 2H), 3.94 (br t, *J =* 5.8 Hz, 2H), 3.04 (br t, *J =* 5.5 Hz, 2H), 2.39 (td, *J =* 7.0, 13.9 Hz, 1H), 2.09 (s, 3H), 1.21 (d, *J =* 5.4 Hz, 3H), 0.96 (dd, *J =* 3.3, 6.9 Hz, 6H).

### Example 23. Synthesis of Compound RN044

The synthetic route is as follows:

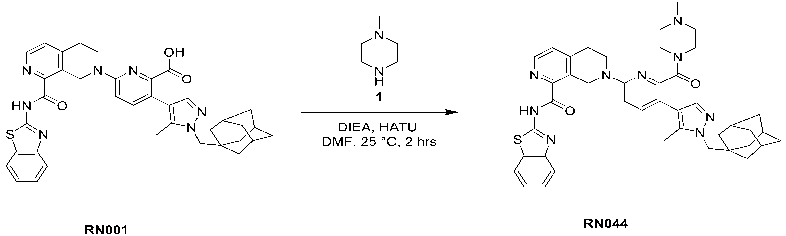

### Step: Synthesis of Compound RN044

Compound **RN001** (30 mg, 45.47 µmol, 1 eq) was dissolved in 1 mL of N,N-dimethylformamide, and N,N-diisopropylethylamine (17.63 mg, 136.41 µmol, 23.76 µL, 3 eq) and 1-methylpiperazine (6.83 mg, 68.20 µmol, 7.57 µL, 1.5 eq) were added. The reaction solution was stirred at 25 °C for 10 minutes, then HATU (25.93 mg, 68.20 µmol, 1.5 eq) was added, and the reaction solution was stirred at 25 °C for 2 h. The reaction solution was purified by preparative liquid chromatography to afford compound **RN044** as a yellow solid (9.5 mg, yield 27.91%).

MS (ESI) m/z = 742.4 [M+H]⁺.

**¹H NMR** (400 MHz, DMSO-*d₆*): *δ* = 12.42 - 12.20 (m, 1H), 8.55 (d, *J =* 4.9 Hz, 1H), 8.04 (d, *J* = 7.5 Hz, 1H), 7.81 (d, *J* = 7.9 Hz, 1H), 7.58 - 7.53 (m, 2H), 7.52 - 7.44 (m, 1H), 7.41 - 7.32 (m, 1H), 7.22 (s, 1H), 6.97 (d, *J =* 8.9 Hz, 1H), 5.21 (s, 2H), 3.90 (br t, *J =* 5.8 Hz, 2H), 3.72 (s, 2H), 3.41 (br s, 2H), 3.05 (br t, *J =* 5.6 Hz, 2H), 2.94 (br s, 2H), 2.19 - 2.09 (m, 5H), 2.01 (s, 3H), 1.93 (br s, 5H), 1.70 - 1.62 (m, 3H), 1.59 - 1.51 (m, 9H).

The following example compounds in Table 4 were prepared according to the same method as the above examples, using commercially available compounds or by reference to the preparation methods of the intermediates shown. 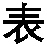 **4**

| Compound ID | Starting material | Structure | ¹H-NMR |
|---|---|---|---|
| **RN045** | 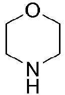 | 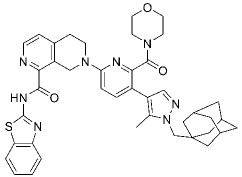 | **¹H NMR:** (400 MHz, DMSO-*d₆*): *δ* = 12.42 - 12.21 (m, 1H), 8.56 (d, *J =* 4.9 Hz, 1H), 8.05 (d, *J* = 7.8 Hz, 1H), 7.81 (d, *J =* 7.9 Hz, 1H), 7.63 - 7.53 (m, 2H), 7.52 - 7.42 (m, 1H), 7.41 - 7.31 (m, 1H), 7.25 (s, 1H), 6.99 (d, *J* = 8.9 Hz, 1H), 5.20 (s, 2H), 3.91 (t, *J* = 5.9 Hz, 2H), 3.74 (s, 2H), 3.43 (br d, *J =* 3.3 Hz, 2H), 3.40 (br d, *J =* 4.0 Hz, 2H), 3.16 (br s, 2H), 3.06 (br t, *J* = 5.8 Hz, 2H), 2.96 (br d, *J =* 3.6 Hz, 2H), 2.17 (s, 3H), 1.93 (br s, 3H), 1.69 - 1.60 (m, 3H), 1.59 - 1.49 (m, 9H). |
| RN-046 | 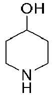 | 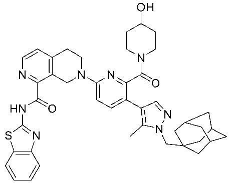 | **¹H NMR:** (400 MHz, DMSO-*d₆*): *δ* = 12.29 (br d, *J =* 1.6 Hz, 1H), 8.55 (d, *J* = 4.9 Hz, 1H), 8.05 (d, *J* = 7.8 Hz, 1H), 7.81 (d, *J* = 8.0 Hz, 1H), 7.59 - 7.52 (m, 2H), 7.52 - 7.43 (m, 1H), 7.40 - 7.29 (m, 1H), 7.23 (s, 1H), 6.95 (d, *J* = 8.9 Hz, 1H), 5.19 (q, *J =* 18.3 Hz, 2H), 4.66 (br d, *J =* 2.9 Hz, 1H), 3.91 (br t, *J* = 5.9 Hz, 2H), 3.75 - 3.63 (m, 3H), 3.55 (br d, *J =* 3.3 Hz, 1H), 3.23 - 3.08 (m, 2H), 3.05 (br t, *J* = 5.6 Hz, 2H), 2.84 - 2.73 (m, 1H), 2.16 (s, 3H), 1.92 (br s, 3H), 1.69 - 1.60 (m, 3H), 1.59 - 1.44 (m, 11H), 1.39 - 1.29 (m, 1H), 1.08 - 0.95 (m, 1H). |
| RN-047 | 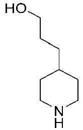 | 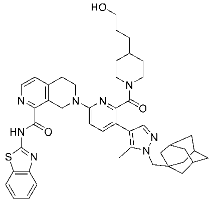 | **¹H NMR:** (400 MHz, DMSO-*d₆*): *δ* = 12.41 - 12.19 (m, 1H), 8.55 (d, *J* = 4.9 Hz, 1H), 8.04 (d, *J* = 7.8 Hz, 1H), 7.81 (d, *J =* 8.1 Hz, 1H), 7.61 - 7.52 (m, 2H), 7.51 - 7.43 (m, 1H), 7.41 - 7.32 (m, 1H), 7.22 (s, 1H), 6.96 (d, *J =* 8.9 Hz, 1H), 5.21 (s, 2H), 4.35 - 4.24 (m, 2H), 3.99 - 3.82 (m, 2H), 3.78 - 3.65 (m, 2H), 3.29 - 3.23 (m, 2H), 3.13 (br d, *J* = 12.9 Hz, 1H), 3.05 (br t, *J =* 5.6 Hz, 2H), 2.75 - 2.63 (m, 1H), 2.15 (s, 3H), 1.92 (br s, 3H), 1.68 - 1.61 (m, 3H), 1.60 - 1.45 (m, 11H), 1.39 - 1.23 (m, 4H), 1.09 - 0.99 (m, 2H), 0.85 - 0.69 (m, 1H), 0.65 - 0.45 (m, 1H). |
| RN-048 | 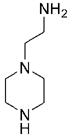 | 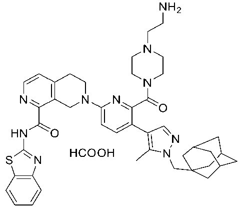 | **¹H NMR:** (400 MHz, DMSO-*d₆*): *δ* = 8.51 (d, *J =* 4.9 Hz, 1H), 8.41 (s, 1H), 7.99 (d, *J =* 7.5 Hz, 1H), 7.76 (d, *J = 7.9* Hz, 1H), 7.54 (d, *J =* 8.8 Hz, 1H), 7.50 (d, *J =* 4.9 Hz, 1H), 7.47 - 7.40 (m, 1H), 7.36 - 7.28 (m, 1H), 7.21 (s, 1H), 6.97 (d, *J* = 8.9 Hz, 1H), 5.17 (s, 2H), 3.90 (br t, *J* = 5.7 Hz, 2H), 3.71 (s, 2H), 3.41 (br s, 4H), 3.03 (br t, *J =* 5.7 Hz, 2H), 2.93 (br s, 2H), 2.67 (br t, *J* = 6.3 Hz, 2H), 2.27 - 2.17 (m, 4H), 2.14 (s, 3H), 2.00 (br s, 2H), 1.92 (br s, 3H), 1.68 - 1.61 (m, 3H), 1.59 - 1.49 (m, 9H). |
| **RN049** | 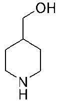 | 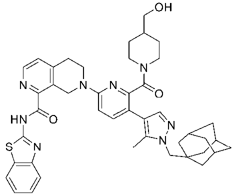 | **¹H NMR:** (400 MHz, DMSO-*d₆*): δ = 12.33 (s, 1H), 8.56 (d, *J =* 4.8 Hz, 1H), 8.06 (d, *J =* 7.5 Hz, 1H), 7.82 (d, *J* = 7.9 Hz, 1H), 7.62 - 7.52 (m, 2H), 7.52 - 7.45 (m, 1H), 7.42 - 7.33 (m, 1H), 7.23 (s, 1H), 6.96 (d, *J =* 8.9 Hz, 1H), 5.27 - 5.17 (m, 2H), 4.32 (br d, *J* = 13.0 Hz, 1H), 3.97 - 3.87 (m, 2H), 3.78 - 3.66 (m, 2H), 3.21 - 3.04 (m, 5H), 2.76 - 2.70 (m, 1H) , 2.16 (s, 3H), 1.94 (br s, 3H), 1.71 - 1.62 (m, 4H), 1.60 - 1.47 (m, 10H), 1.42 (br d, *J= 10.1* Hz, 2H), 0.90 - 0.74 (m, 1H), 0.70 - 0.50 (m, 1H). |
| RN050 | 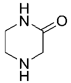 | 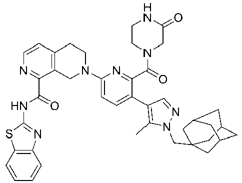 | **¹H NMR:** (400 MHz, DMSO-*d₆*): *δ* = 12.28 (br d, *J* = 2.9 Hz, 1H), 8.56 (d, *J* = 4.5 Hz, 1H), 8.11 - 7.86 (m, 2H), 7.81 (d, *J =* 8.0 Hz, 1H), 7.62 - 7.53 (m, 2H), 7.52 - 7.43 (m, 1H), 7.36 (t, *J =* 7.6 Hz, 1H), 7.28 - 7.13 (m, 1H), 7.01 (dd, *J* = 4.0, 8.6 Hz, 1H), 5.20 (s, 2H), 3.96 - 3.86 (m, 3H), 3.71 (br d, *J* = 5.0 Hz, 2H), 3.62 - 3.56 (m, 1H), 3.23 - 3.17 (m, 2H), 3.07 - 2.95 (m, 4H), 2.13 (d, *J* = 11.0 Hz, 3H), 1.92 (br s, 3H), 1.68 - 1.60 (m, 3H), 1.59 - 1.48 (m, 9H). |
| **RN051** | 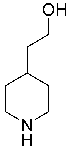 | 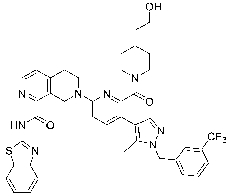 | **¹H NMR:** (400 MHz, DMSO-*d₆*): *δ* 12.25 (br s, 1H), 8.54 (d, *J* = 4.8 Hz, 1H), 8.03 (d, *J=* 7.9 Hz, 1H), 7.80 (d, *J= 8.0* Hz, 1H), 7.68 - 7.62 (m, 1H), 7.60 - 7.52 (m, 4H), 7.48 (t, *J =* 7.7 Hz, 1H), 7.42 - 7.33 (m, 2H), 7.32 (s, 1H), 6.97 (d, *J =* 8.9 Hz, 1H), 5.43 (s, 2H), 5.30 - 5.13 (m, 2H), 4.36 - 4.24 (m, 2H), 3.97 - 3.80 (m, 2H), 3.29 - 3.23 (m, 2H), 3.15 (br d, *J =* 13.3 Hz, 1H), 3.04 (br s, 2H), 2.68 (br t, *J* = 11.7 Hz, 1H), 2.48 (br s, 1H), 2.15 (s, 3H), 1.55 (br d, *J* = 11.5 Hz, 1H), 1.48 - 1.37 (m, 1H), 1.32 (br d, *J* = 11.3 Hz, 1H), 1.12 (q, *J* = 6.3 Hz, 2H), 0.83 - 0.69 (m, 1H), 0.58 (br d,*J* = 9.9 Hz, 1H). |

### Example 24. Synthesis of Compound RN052

The synthetic route is as follows:

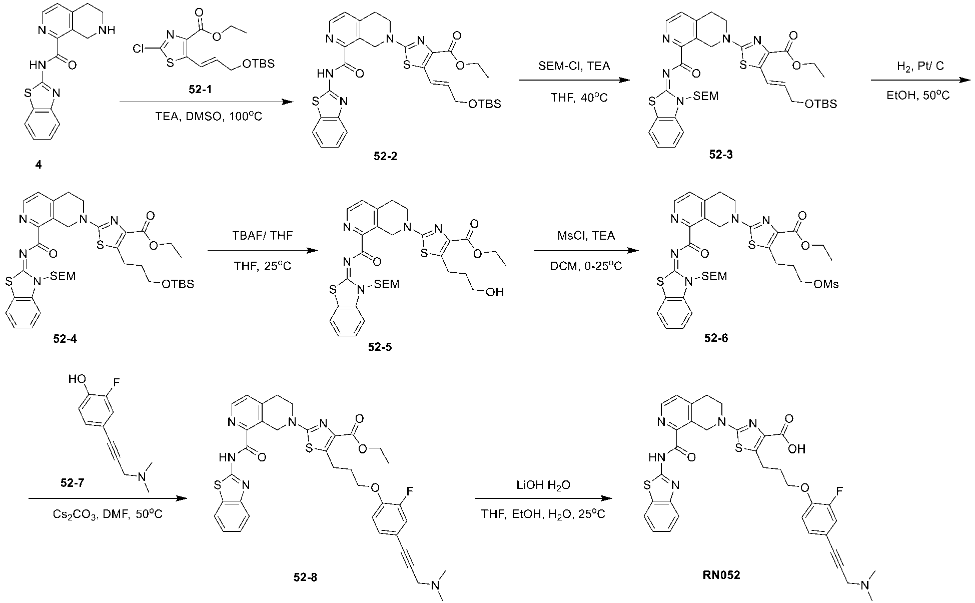

### Step 1: Synthesis of Compound 52-2

Compound 4 (400 mg, 1.29 mmol) was dissolved in DMSO (3 mL), and compound 52-1 (699 mg, 1.93 mmol) and triethylamine (0.89 mL, 6.44 mmol) were added. The reaction solution was stirred at 100 °C for 18 h under N₂ protection. The reaction solution was extracted with water (20 mL) and DCM (15 mL × 2). The organic phase was washed with saturated brine (20 mL), dried over anhydrous sodium sulfate, filtered and concentrated. The residue was purified by column chromatography to afford compound **52-2** as a brown oil (598 mg, yield 58.3%).

MS (ESI) m/z = 636.2 [M+H]⁺.

### Step 2: Synthesis of Compound 52-3

Compound **52-2** (598 mg, 0.75 mmol) was dissolved in THF (5 mL), and triethylamine (0.31 mL, 2.26 mmol) and SEM-Cl (0.20 mL, 1.13 mmol) were added. The reaction solution was stirred at 40 °C for 1 h. The reaction solution was extracted with water (30 mL) and dichloromethane (10 mL × 3). The organic phase was separated, washed with saturated brine (20 mL), dried over sodium sulfate, filtered and concentrated to afford a residue. The residue was purified by preparative thin layer chromatography to afford compound **52-3** as a brown solid (682 mg, yield 70.9%).

MS (ESI) m/z = 766.4 [M+H]⁺.

### Step 3: Synthesis of Compound 52-4

Compound **52-3** (650 mg, 0.51 mmol) was dissolved in ethanol (10 mL), and platinum on carbon (99.3 mg) was added under nitrogen protection. The suspension was degassed and purged with hydrogen three times. The mixture was stirred at 50 °C under hydrogen (15 Psi) for 18 h. LCMS indicated the reaction was complete. The suspension was filtered through Celite, the filtrate was concentrated, and the residue was purified by preparative thin layer chromatography to afford compound **52-4** as a brown oil (195 mg, yield 33.4%).

MS (ESI) m/z = 768.4 [M+H]⁺.

### Step 4: Synthesis of Compound 52-5

Compound **52-4** (195 mg, 0.17 mmol) was dissolved in THF (1 mL), and TBAF·THF (0.34 mL, 0.34 mmol, 1 M) was added. The reaction solution was stirred at 25 °C for 1 h under N₂ protection. LCMS indicated the reaction was complete. The reaction mixture was extracted with water (10 mL) and DCM (5 mL × 3). The organic phase was washed with saturated brine (10 mL), dried over anhydrous sodium sulfate, filtered, and concentrated under reduced pressure to afford a residue, thereby to afford compound **52-5** as a brown oil (102 mg, yield 59.6%).

MS (ESI) m/z = 654.3 [M+H]⁺.

### Step 5: Synthesis of Compound 52-6

Compound **52-5** (100 mg, 0.10 mmol) and triethylamine (0.04 mL, 0.30 mmol) were dissolved in DCM (1 mL), and MsCl (14.8 mg, 0.13 mmol) was added at 0 °C under nitrogen protection. The reaction solution was stirred at 25 °C for 1 h. LCMS indicated the reaction was complete. The reaction mixture was extracted with water (10 mL) and DCM (5 mL × 2). The organic phase was washed with saturated brine (10 mL), dried over anhydrous sodium sulfate, filtered, and concentrated to afford a residue. The residue was purified by preparative thin layer chromatography to afford compound **52-6** as a brown oil (43 mg, yield 38.4%).

MS (ESI) m/z = 732.2 [M+H]⁺.

### Step 5: Synthesis of Compound 52-8

Compound **52-6** (38 mg, 0.05 mmol), compound **52-7** (15.0 mg, 0.08 mmol), and cesium carbonate (50.7 mg, 0.16 mmol) were mixed with DMF (0.6 mL). The mixture was degassed and purged with nitrogen three times. The mixture was stirred at 50 °C for 1 h under nitrogen protection. LCMS indicated the reaction was complete. The reaction mixture was diluted with water (5 mL), extracted with ethyl acetate (5 mL × 3), washed with saturated brine (5 mL), and dried over anhydrous sodium sulfate. The residue was purified by preparative thin layer chromatography to afford compound **52-8** as a yellow solid (8.00 mg, yield 22.0%).

### Step 6: Synthesis of Compound RN052

Compound **52-8** (8.00 mg, 0.01 mmol) was dissolved in a mixed solvent of ethanol (0.3 mL)/water (0.3 mL), and lithium hydroxide monohydrate (1.44 mg, 0.03 mmol) was added. The mixture was stirred at 25 °C for 2 h. LCMS indicated the reaction was complete. The mixture was concentrated. The crude product was purified by Prep-HPLC to afford compound **RN052** as a white solid (0.96 mg, yield 12.5%).

MS (ESI) m/z = 671.4 [M+H]⁺.

**¹H NMR** (400 MHz, DMSO-*d₆*): δ 8.56 (s, 1H), 8.33 (s, 1H), 8.03 (d, J = 7.6 Hz, 1H), 7.80 (d, J = 8.0 Hz, 1H), 7.57 (s, 1H), 7.47 (t, J = 7.6 Hz, 1H), 7.35 (t, J = 7.6 Hz, 1H), 7.25-7.28 (m, 1H), 7.19 (d, J = 6.8 Hz, 1H), 7.11-7.13 (m, 1H), 5.06 (s, 2H), 4.09 (s, 2H), 3.76 (s, 2H), 3.19 (s, 2H), 3.08 (s, 2H), 2.21 (s, 6H), 1.97-2.04 (m, 4H).

### Example 25. Synthesis of Compound RN053

The synthetic route is as follows:

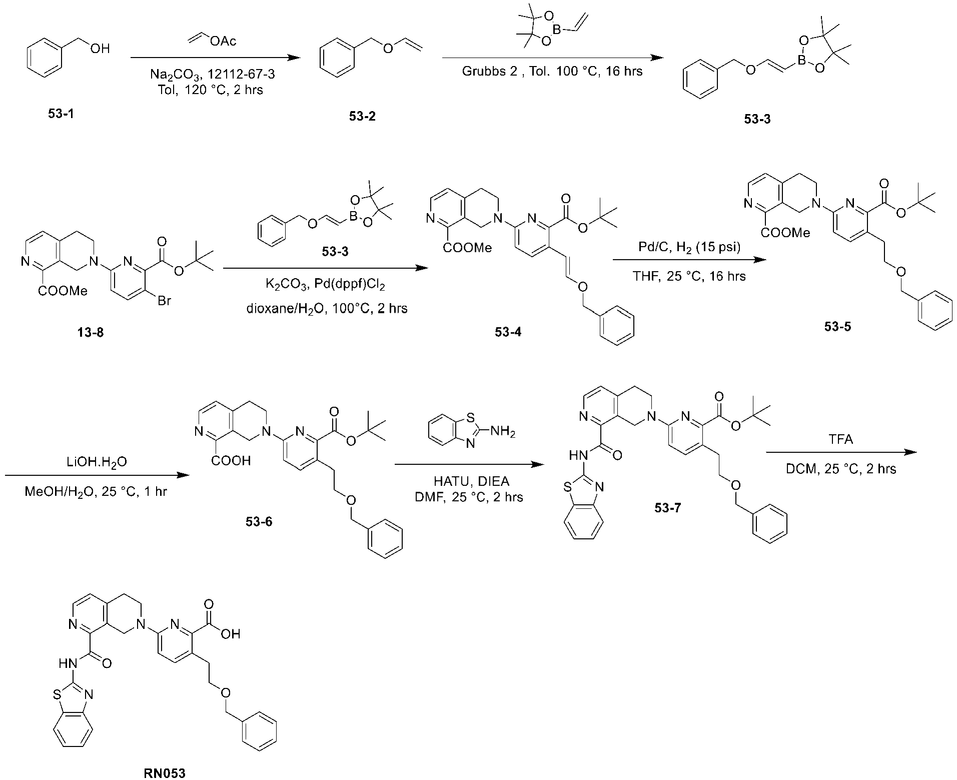

### Step 1: Synthesis of Compound 53-2

Benzyl alcohol (5 g, 46.24 mmol, 4.79 mL, 1 eq) and vinyl acetate (11.94 g, 138.71 mmol, 12.84 mL, 3 eq) were dissolved in 50 mL of toluene, and potassium carbonate (2.94 g, 27.74 mmol, 0.6 eq) and chloro(1,5-cyclooctadiene)iridium dimer (310.58 mg, 462.37 µmol, 0.01 eq) were added. The reaction solution was stirred at 120 °C for 2 h. 5mL of Water was added to the reaction solution and extracted twice with 5 mL of ethyl acetate. The combined organic phase was washed with saturated brine (10 mL), dried over anhydrous magnesium sulfate, filtered and concentrated. The crude product was purified by column chromatography to afford compound **53-2** as a yellow oil (3.9 g, yield 62.86%).

**¹H NMR** (400 MHz, CDCl₃): δ 7.34 - 7.16 (m, 5H), 6.49 (dd, *J =* 6.8, 14.3 Hz, 1H), 4.68 (s, 2H), 4.23 (dd, *J =* 2.1, 14.3 Hz, 1H), 4.00 (dd, *J =* 2.1, 6.8 Hz, 1H).

### Step 2: Synthesis of Compound 53-3

Compound **53-2** (1 g, 7.45 mmol, 1 eq) and 2-vinyl-4,4,5,5-tetramethyl-1,3,2-dioxaborolane (1.15 g, 7.45 mmol, 1.26 mL, 1 eq) were dissolved in 10 mL of toluene, and Grubbs second-generation catalyst (632.74 mg, 745.30 µmol, 0.1 eq) was added. The reaction solution was stirred at 100 °C for 16 h under nitrogen atmosphere. The reaction solution was directly purified by column chromatography to afford compound **53-3** as a yellow oil (531 mg, yield 27.39%) without subjected to treatment.

MS (ESI) m/z = 281.1 [M+H]⁺.

### Step 3: Synthesis of Compound 53-4

Compound **13-8** (400 mg, 892.24 µmol, 1 eq) was dissolved in 2 mL of 1,4-dioxane and 0.5 mL of water, and compound **53-3** (464.21 mg, 1.78 mmol, 2 eq), potassium carbonate (369.95 mg, 2.68 mmol, 3 eq) and 1,1-bis(diphenylphosphino)ferrocene palladium dichloride (65.29 mg, 89.22 µmol, 0.1 eq) were added. The reaction solution was stirred at 100 °C for 2 h under nitrogen protection. The reaction solution was poured into 30 mL of water and extracted with ethyl acetate (30 mL × 2). The combined organic phase was washed with saturated brine (30 mL), dried over anhydrous magnesium sulfate, filtered and concentrated. The residue was purified by column chromatography to afford compound **53-4** as a yellow oil (286 mg, yield 63.91%).

MS (ESI) m/z = 502.5 [M+H] ⁺.

### Step 4: Synthesis of Compound 53-5

Compound **53-4** (286 mg, 570.21 µmol, 1 eq) was dissolved in 5 mL of tetrahydrofuran. Wet palladium on carbon (100 mg, 93.97 µmol, 10% purity, 1.65 e-1 eq) was added. The reaction solution was stirred at 25 °C under 15 psi hydrogen atmosphere for 16 h. The reaction solution was filtered and concentrated. The residue was purified by column chromatography to afford compound 53-5 as a yellow oil (242 mg, yield 84.28%).

MS (ESI) m/z = 504.1 [M+H]⁺.

### Step 5: Synthesis of Compound 53-6

Compound **53-5** (242 mg, 480.55 µmol, 1 eq) was dissolved in a mixed solution of 2 mL of methanol and 2 mL of water, and lithium hydroxide monohydrate (40.33 mg, 961.10 µmol, 2 eq) was added. The reaction solution was stirred at 25 °C for 1 h. The reaction solution was adjusted to pH 1 with 0.5 M hydrochloric acid, then extracted with dichloromethane (20 mL × 3). The combined organic phase was dried over anhydrous magnesium sulfate, filtered, and the filtrate was concentrated to afford compound **53-6** as a yellow solid (219 mg, yield 93.09%).

MS (ESI) m/z = 490.1 [M+H]⁺.

### Step 6: Synthesis of Compound 53-7

Compound tert-butyl 1,3-benzothiazole-2-amine (128.25 mg, 853.83 µmol, 2 eq) was dissolved in 4 mL of N,N-dimethylformamide. N,N-diisopropylethylamine (275.87 mg, 2.13 mmol, 371.79 µL, 5 eq) and compound **53-6** (209 mg, 426.91 µmol, 1 eq) were added, followed by HATU (486.97 mg, 1.28 mmol, 3 eq). The reaction solution was stirred at 25 °C for 2 h. The reaction solution was poured into 10 mL of water and extracted with ethyl acetate (10 mL × 2). The combined organic phase was washed with saturated brine (10 mL), dried over anhydrous magnesium sulfate, filtered and concentrated. The residue was purified by column chromatography to afford compound **53-7** as a yellow oil (149 mg, yield 56.13%).

MS (ESI) m/z = 622.3 [M+H]⁺.

### Step 7: Synthesis of Compound RN053

Compound **53-7** (139 mg, 223.56 µmol, 1 eq) was dissolved in 1.5 mL of dichloromethane, and trifluoroacetic acid (2.30 g, 20.19 mmol, 1.5 mL, 90.33 eq) was added. The reaction solution was stirred at 25 °C for 2 h. The reaction solution was concentrated to afford a crude product. The crude product was purified by preparative liquid chromatography to afford compound **RN053** as an off-white solid (10.16 mg, yield 7.64%).

MS (ESI) m/z = 566.1 [M+H]⁺.

**¹H NMR** (400 MHz, DMSO-*d₆*): *δ* = 8.53 (d, *J =* 4.8 Hz, 1H), 8.05 (d, *J =* 7.6 Hz, 1H), 7.81 (d, *J* = 8.0 Hz, 1H), 7.55 - 7.45 (m, 3H), 7.40 - 7.21 (m, 6H), 6.84 (d, *J =* 8.6 Hz, 1H), 5.14 (s, 2H), 4.44 (s, 2H), 3.89 (br t, *J =* 5.7 Hz, 2H), 3.59 - 3.56 (m, 2H), 3.01 (br *t, J =* 5.5 Hz, 2H), 2.88 (br t, *J =* 7.0 Hz, 2H).

### Example 26. Synthesis of Compound RN054

The synthetic route is as follows:

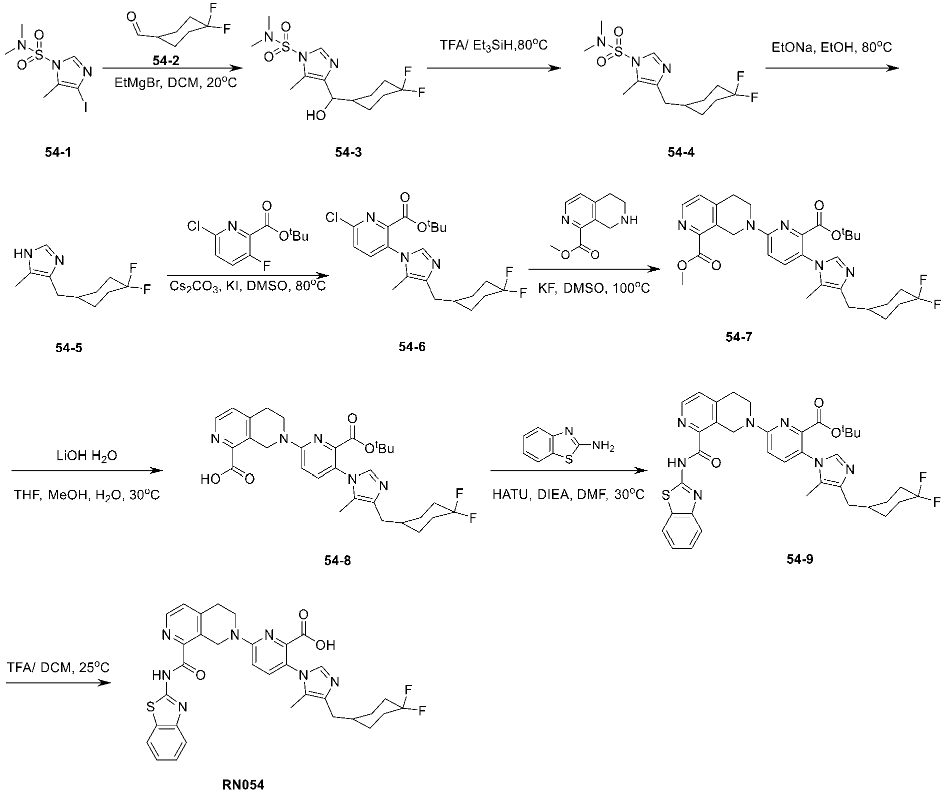

### Step 1: Synthesis of Compound 54-3

To a solution of compound **54-1** (3.1 g, 9.84 mmol) in DCM (62 mL) was added dropwise EtMgBr (5.4 mL, 2 M) at 20 °C under N₂ protection. After 30 minutes, compound **54-2** (1.8 g, 11.80 mmol) was added, and the reaction solution was stirred at 20 °C for 18 h. After completion of the reaction, the reaction was quenched with saturated NH₄Cl (30 mL) and extracted with DCM (30 mL × 3). The organic layer was separated, washed with saturated sodium chloride solution, and concentrated to afford a crude product, which was purified (mobile phase: methanol (0.1% ammonia water)-dichloromethane, gradient: 0-6%) to afford compound **54-3** as a pale yellow oil (1.9 g, yield 46.0%).

MS (ESI) m/z = 338.2 [M+H]⁺.

### Step 2: Synthesis of Compound 54-4

Compound **54-3** (2.1 g, 6.22 mmol) was dissolved in TFA (15 mL) at room temperature. Then Et₃SiH (15 mL) was added, and the reaction solution was stirred at 80 °C for 18 h under N₂ protection. After completion of the reaction, the reaction was quenched with saturated NaHCO₃ to pH ~ 8 and extracted with DCM (30 mL × 3). The organic layer was separated, washed with saturated sodium chloride solution, and concentrated to afford a crude product, which was purified (mobile phase: methanol (0.1% ammonia water)-dichloromethane, gradient: 0-10%) to afford compound **54-4** as a white solid (1.72 g, yield 68.8%).

MS (ESI) m/z = 322.2 [M+H]⁺.

### Step 3: Synthesis of Compound 54-5

Compound **54-4** (1.5 g, 4.67 mmol) was dissolved in EtOH (15 mL) at room temperature. Then EtONa (3.8 g, 56.01 mmol) was added, and the reaction solution was stirred at 80 °C for 18 h under N₂ protection. After completion of the reaction, the reaction was quenched with saturated NaHCO₃ to pH ~ 8 and extracted with EtOAc (100 mL × 3). The organic layer was separated, washed with saturated sodium chloride solution, and concentrated to afford a crude product, which was purified (mobile phase: methanol (0.1% ammonia water)-dichloromethane, gradient: 0-10%) to afford compound **54-5** as a yellow solid (664 mg, yield 66.4%).

MS (ESI) m/z = 215.2 [M+H]⁺.

### Step 4: Synthesis of Compound 54-6

Compound **54-5** (664 mg, 3.10 mmol) was dissolved in DMSO (8 mL) at room temperature, and tert-Butyl 6-chloro-3-fluoropyridine-2-carboxylate (1.1 g, 4.65 mmol), KI (1.2 g, 6.20 mmol) and Cs₂CO₃ (3.0 g, 9.30 mmol) were added. The reaction solution was stirred at 80 °C for 2 h under N₂ protection. After completion of the reaction, the reaction solution was diluted with water (50 mL) and extracted with EtOAc (50 mL × 3). The organic layer was separated, washed with saturated sodium chloride solution, and concentrated to afford a crude product which was slurried with EtOAc (5 mL) at 25 °C to afford compound **54-6** as a white solid (350 mg, yield 26.6%).

MS (ESI) m/z = 426.0 [M+H]⁺.

### Step 5: Synthesis of Compound 54-7

Compound **54-6** (330 mg, 0.78 mmol) was dissolved in DMSO (3.5 mL) at room temperature, and methyl 5,6,7,8-tetrahydropyrido[3,4-c]pyridine-1-carboxylate (179.6 mg, 0.93 mmol) and KF (226.1 mg, 3.89 mmol) were added. The reaction solution was stirred at 100 °C for 24 hours under N₂ protection. After completion of the reaction, the reaction solution was diluted with water (50 mL) and extracted with EtOAc (50 mL × 3). The organic layer was separated, washed with saturated sodium chloride solution, and concentrated to afford a crude product, which was purified by preparative TLC (DCM: MeOH = 10: 1) to afford compound **54-7** as a white solid (160 mg, yield 35.5%).

MS (ESI) m/z = 582.4 [M+H]⁺.

### Step 6: Synthesis of Compound 54-8

Compound **54-7** (160 mg, 0.28 mmol) was dissolved in THF (0.7 mL), H₂O (0.7 mL) and MeOH (0.7 mL) at room temperature, and LiOH·H₂O (23.1 mg, 0.55 mmol) was added. The reaction solution was stirred at 30 °C for 5 h under N₂ protection. The reaction solution was concentrated to afford compound **54-8** as a yellow solid (169 mg, 0.30 mmol, crude), which was used directly in the next step without further purification.

MS (ESI) m/z = 568.4 [M+H]⁺.

### Step 7: Synthesis of Compound 54-9

Compound **54-8** (149 mg, 0.26 mmol) was dissolved in DMF (3 mL), and DIEA (204.3 mg, 1.58 mmol), HATU (500.8 mg, 1.32 mmol) and benzo[2,1-d]thiazol-2-amine (59.3 mg, 0.40 mmol) were added. The reaction solution was stirred at 30 °C for 16 h under N₂ protection. The reaction solution was diluted with water (20 mL), adjusted to pH 3 with 0.5 N HCl, and extracted with EtOAc (30 mL × 3). The organic layer was separated, washed with brine, and concentrated. The crude product was purified by preparative TLC (DCM: MeOH = 10: 1) to afford compound **54-9** as a yellow solid (80 mg, yield 21.8%).

MS (ESI) m/z = 700.4 [M+H]⁺.

### Step 8: Synthesis of Compound RN054

To a solution of compound **54-9** (70 mg, 0.10 mmol) in DCM (1 mL) was added TFA (1 mL). The reaction solution was stirred at 25 °C for 4 h under N₂ protection. After completion of the reaction, the reaction solution was concentrated. The crude product was purified by Prep-HPLC to afford **RN054** as a white solid (19.59 mg, yield 30.3%).

MS (ESI) m/z = 644.4 [M+H]⁺.

**¹H NMR** (400 MHz, DMSO-*d₆*): *δ* 12.30 (s, 1H), 8.57 (d, *J* = 4.8 Hz, 1H), 8.15 (s, 0.06H, HCOOH), 8.05 (d, *J =* 7.8 Hz, 1H), 7.81 (d, *J =* 8.0 Hz, 1H), 7.64 (d, *J* = 9.0 Hz, 1H), 7.58 (d, *J* = 4.9 Hz, 1H), 7.48 (t, *J =* 7.7 Hz, 1H), 7.44 (s, 1H), 7.36 (t, *J =* 7.6 Hz, 1H), 7.08 (d, *J =* 9.0 Hz, 1H), 5.27 (s, 2H), 3.98 (t, *J =* 5.7 Hz, 2H), 3.07 (t, *J =* 5.5 Hz, 2H), 2.36 (d, *J =* 6.5 Hz, 2H), 2.04 - 1.93 (m, 2H), 1.90 (s, 3H), 1.84 - 1.67 (m, 5H), 1.29 - 1.15 (m, 2H).

The following example compounds in Table 5 were prepared according to the same method as the above examples, using commercially available compounds or by reference to the preparation methods of the intermediates shown.

**Table 5**

| Compou nd ID | Starting Material | Structure | ¹H-NMR |
|---|---|---|---|
| **RN055** | 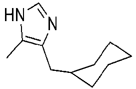 | 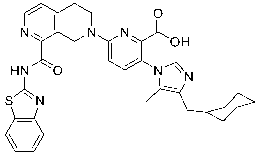 | **¹H NMR** (400MHz, DMSO-*d₆*): δ 12.29 (br s, 1H), 8.57 (d, J = 4.8 Hz, 1H), 8.05 (d, J = 8.0 Hz, 1H), 7.81 (d, J = 8.0 Hz, 1H), 7.65 (d, J = 8.8 Hz, 1H), 7.58 (d, J = 4.8 Hz, 1H), 7.44-7.51 (m, 2H), 7.36 (t, J = 7.6 Hz, 1H), 7.09 (d, J = 9.2 Hz, 1H), 5.27 (s, 2H), 3.98 (t, J = 6.0 Hz, 2H), 3.07 (t, J = 5.6 Hz, 2H), 2.29 (d, J = 6.8 Hz, 2H), 1.88 (s, 3H), 1.52-1.66 (m, 6H), 1.12-1.17 (m, 3H), 0.89-0.94 (m, 2H). |
| **RN056** | 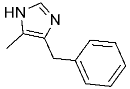 | 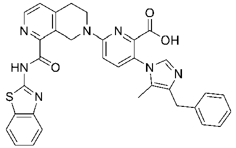 | **¹H NMR** (400MHz, DMSO-*d₆*): δ 12.34 (s, 1H), 9.17 (s, 1H), 8.59 (d, J = 4.8 Hz, 1H), 8.06 (dd, J = 1.2, 8.0 Hz, 1H), 7.94 (d, J = 8.8 Hz, 1H), 7.85 - 7.78 (m, 1H), 7.60 (d, J = 4.8 Hz, 1H), 7.49 (m, 1H), 7.40 - 7.31 (m, 3H), 7.30 - 7.20 (m, 4H), 5.36 (s, 2H), 4.11 (s, 2H), 4.06 (t, J = 6.0 Hz, 2H), 3.09 (t, J = 6.0 Hz, 2H), 2.05 (s, 3H). |
| **RN057** | 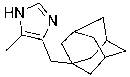 | 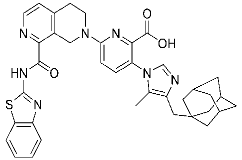 | **¹H NMR** (400MHz, DMSO-*d₆*): δ 8.57 (d, J = 4.8 Hz, 1H), 8.05 (d, J = 7.6 Hz, 1H), 7.81 (d, J = 8.4 Hz, 1H), 7.58 (d, J = 4.8 Hz, 2H), 7.48 (t, J = 7.6 Hz, 1H), 7.34-7.39 (m, 2H), 7.02 (d, J = 9.2 Hz, 1H), 5.26 (s, 2H), 3.97 (s, 2H), 3.07 (s, 2H), 2.17 (s, 2H), 1.89 (s, 6H), 1.61-1.64 (m, 3H), 1.50-1.57 (m, 9H). |
| **RN058** | 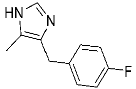 | 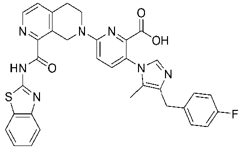 | **¹H NMR** (400MHz, DMSO-*d₆*): δ 12.31 (br s, 1H), 8.57 (d, J = 4.8 Hz, 1H), 8.05 (d, J = 7.6 Hz, 1H), 7.81 (d, J = 8.4 Hz, 1H), 7.67 (d, J = 9.2 Hz, 1H), 7.58 (d, J = 4.8 Hz, 1H), 7.48 (t, J = 7.6 Hz, 1H), 7.44 (s, 1H), 7.36 (t, J = 7.6 Hz, 1H), 7.22-7.26 (m, 2H), 7.04-7.11 (m, 3H), 5.28 (s, 2H), 3.98 (t, J = 5.6 Hz, 2H), 3.79 (s, 2H), 3.07 (t, J = 5.6 Hz, 2H), 1.94 (s, 3H). |
| **RN059** | 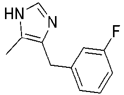 | 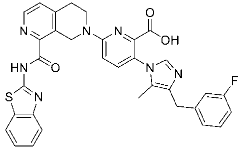 | **¹H NMR** (400MHz, DMSO-*d₆*): δ 12.30 (s, 1H), 8.57 (d, J = 4.4 Hz, 1H), 8.05 (d, J = 8.0 Hz, 1H), 7.81 (d, J = 8.0 Hz, 1H), 7.68 (d, J = 9.2 Hz, 1H), 7.58 (d, J = 4.8 Hz, 1H), 7.45-7.50 (m, 2H), 7.36 (t, J = 7.6 Hz, 1H), 7.29 (dd, J = 8.0, 14.0 Hz, 1H), 6.95-7.11 (m, 4H), 5.28 (s, 2H), 3.99 (t, J = 5.6 Hz, 2H), 3.83 (s, 2H), 3.08 (d, J = 5.2 Hz, 2H), 1.94 (s, 3H). |
| **RN060** | 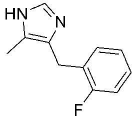 | 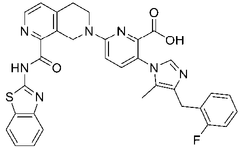 | **¹HNMR** (400MHz, DMSO-*d₆*): δ 12.33 (s, 1H), 8.58 (d, J = 4.8 Hz, 1H), 8.21 (s, 1H), 8.06 (d, J = 8.0 Hz, 1H), 7.81 (t, J = 7.2 Hz, 2H), 7.59 (d, J = 4.8 Hz, 1H), 7.49 (t, J = 7.6 Hz, 1H), 7.37 (t, J = 7.6 Hz, 1H), 7.27 (t, J = 7.2 Hz, 1H), 7.23 - 7.09 (m, 4H), 5.32 (s, 2H), 4.02 (t, J = 6.0 Hz, 2H), 3.95 (s, 2H), 3.09 (t, J = 6.0 Hz, 2H), 1.98 (s, 3H). |

### Example 27. Synthesis of Compound RN061

The synthetic route is as follows:

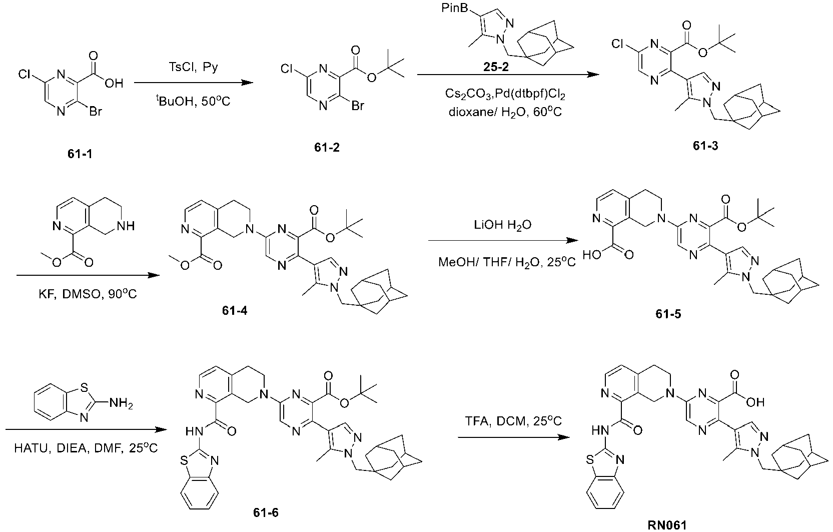

### Step 1: Synthesis of Compound 61-2

Compound **61-1** (2.00 g, 8.42 mmol) was dissolved in tert-butanol (40 mL). Pyridine (4.76 mL, 58.9 mmol) was added, and p-toluenesulfonyl chloride (3.85 mL, 20.2 mmol) was added dropwise at 20 °C. The reaction solution was reacted at 50 °C for 6 h under N₂ protection. Water (50 mL) was added to the reaction solution, and extracted with ethyl acetate (50 mL × 3). The organic layer was washed with saturated brine, dried over anhydrous sodium sulfate and concentrated. The residue was purified by column chromatography to afford compound **61-2** as a colorless oil (1.50 g, yield 60.6%).

MS (ESI) m/z = 293.0 [M+H]⁺.

### Step 2: Synthesis of Compound 61-3

Compound **61-2** (690 mg, 2.35 mmol) was dissolved in dioxane (10 mL), and water (1 mL), compound **25-2** (600 mg, 1.68 mmol), Pd(dtbpf)Cl₂ (108 mg, 0.17 mmol) and cesium carbonate (2.74 g, 8.42 mmol) were added. The reaction solution was reacted at 60 °C for 12 h under N₂ protection. The reaction solution was diluted with water (30 mL) and extracted with ethyl acetate (30 mL × 3). The organic layer was washed with saturated brine (10 mL), dried over anhydrous sodium sulfate and concentrated. The residue was purified by column chromatography to afford compound **61-3** as a brown solid (493 mg, yield 66.0%).

MS (ESI) m/z = 443.2 [M+H]⁺.

### Step 3: Synthesis of Compound 61-4

Methyl 5,6,7,8-tetrahydropyrido[3,4-c]pyridine-1-carboxylate (120 mg, 0.62 mmol) was dissolved in DMSO (2.5 mL) and compound **61-3** (276 mg, 0.62 mmol) and potassium fluoride (108 mg, 1.87 mmol) were added. The reaction solution was reacted at 90 °C for 4 h under N₂ protection. The reaction solution was poured into ice-cold water (8 mL), and the precipitate was filtered and dried under vacuum. The crude product was purified by preparative thin layer chromatography (dichloromethane: methanol = 20: 1) to afford compound **61-4** as a pale yellow gum (51 mg, yield 13.6%).

MS (ESI) m/z = 599.4 [M+H]⁺.

### Step 4: Synthesis of Compound 61-5

Compound **61-4** (51 mg, 0.09 mmol) was dissolved in a mixed solvent of methanol (0.3 mL), tetrahydrofuran (0.3 mL) and water (0.3 mL), and lithium hydroxide monohydrate (10.7 mg, 0.26 mmol) was added. The reaction solution was reacted at 25 °C for 2 h. The reaction solution was concentrated to afford compound **61-5** as a pale yellow solid (49.8 mg, crude).

MS (ESI) m/z = 585.4 [M+H]⁺.

### Step 5: Synthesis of Compound 61-6

Compound **61-5** (40 mg, 0.07 mmol) was dissolved in DMF (0.8 mL), and benzo[d][1,3]thiazol-2-amine (11.3 mg, 0.08 mmol), DIEA (26.5 mg, 0.21 mmol) and HATU (53.2 mg, 0.14 mmol) were added. The reaction solution was stirred at 25 °C for 4 h under N₂ protection. The reaction solution was diluted with water (10 mL) and extracted with ethyl acetate (5 mL × 2). The organic layer was washed with water (3 mL) and saturated brine (3 mL), dried over anhydrous sodium sulfate and concentrated to afford a residue. The residue was purified to afford compound **61-6** as a yellow oil (29 mg, yield 59.1%).

MS (ESI) m/z = 359 [M/2+H]⁺.

### Step 6: Synthesis of Compound RN061

Compound **61-6** (29 mg, 0.04 mmol) was dissolved in dichloromethane (0.3 mL), and trifluoroacetic acid (0.3 mL) was added. The reaction solution was stirred at 25 °C for 2 h and concentrated to afford a residue. The residue was purified by preparative high performance liquid chromatography to afford compound **RN061** as a pale yellow solid (2.75 mg, yield 10.2%).

MS (ESI) m/z = 661.4 [M+H]⁺.

**¹H NMR** (400MHz, DMSO-*d₆*): δ 8.56 (s, 1H), 8.43 (s, 1H), 8.15 (s, 1H), 8.05 (d, J = 5.6 Hz, 1H), 7.81 (d, J = 8.4 Hz, 1H), 7.46-7.56 (m, 3H), 7.37 (t, J = 7.2 Hz, 1H), 5.27 (s, 2H), 3.95 (s, 2H), 3.73 (s, 2H), 3.08 (s, 2H), 2.32 (s, 3H), 1.93 (s, 3H), 1.53-1.66 (m, 12H).

### Example 28. Synthesis of Compound RN062

The synthetic route is as follows:

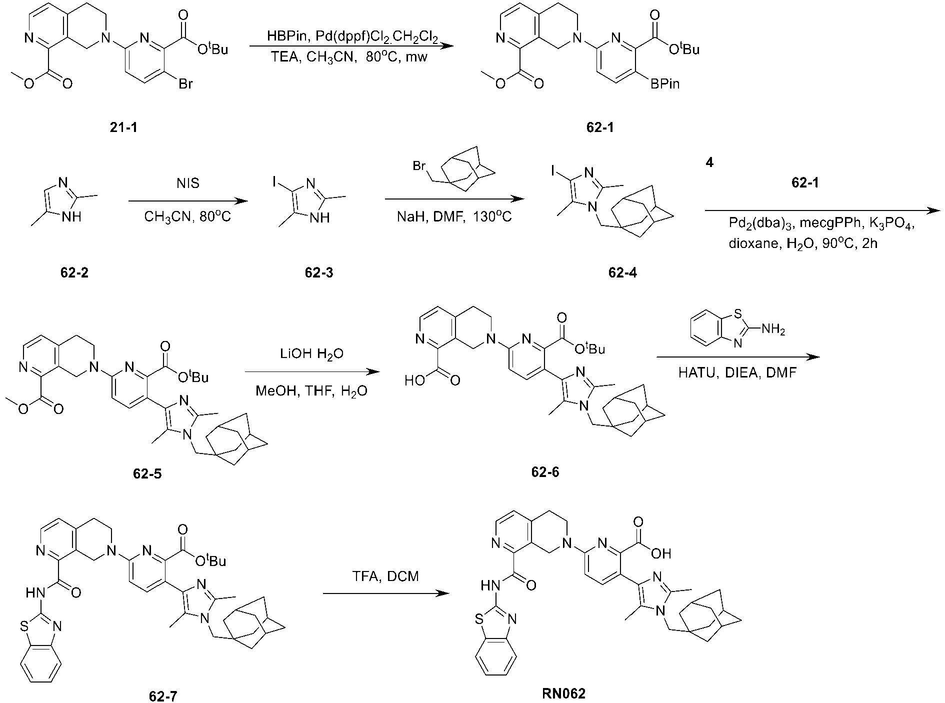

### Step 1: Synthesis of Compound 62-1

Compound **21-1** (976 mg, 2.19 mmol) was dissolved in CH₃CN (10 mL), and Pd(dppf)Cl₂·CH₂Cl₂ (178.6 mg, 0.22 mmol), TEA (0.97 mL, 7.00 mmol) and HBPin (4.8 mL, 32.80 mmol) were added. The reaction solution was stirred at 80 °C for 40 min. MeOH (5 mL) and water (30 mL) were added to the reaction solution, and extracted with EtOAc (30 mL × 3). The organic layer was separated, washed with saturated NaCl solution and concentrated. The residue was purified by silica gel column chromatography to afford compound 62-1 as a yellow oil (1.03 g, 1.67 mmol, yield 76.37%).

MS (ESI) m/z = 496.2 [M+H]⁺.

### Step 2: Synthesis of Compound 62-3

Compound **62-2** (10 g, 104.03 mmol) was dissolved in CH₃CN (100 mL), and NIS (28.1 g, 124 mmol) was added. The reaction solution was stirred at 80 °C for 12 h under N₂ protection. The reaction solution was concentrated to remove CH₃CN, diluted with water (100 mL) and extracted with EtOAc (200 mL × 3). The organic layer was separated, further washed with saturated Na₂CO₃ aqueous solution and concentrated. The residue was slurried with EtOAc (100 mL) at 25 °C and filtered to afford compound **62-3** as a yellow solid (12.0 g, yield 51.9%).

MS (ESI) m/z = 222.8 [M+H]⁺.

### Step 3: Synthesis of Compound 62-4

Compound **62-3** (2.5 g, 11.26 mmol) was dissolved in DMF (25 mL). NaH (0.90 g, 22.52 mmol) was added at 0 °C, and the reaction solution was stirred at room temperature for 30 min. Then 1-(bromomethyl)tricyclo[3.3.1.13,7]decane (3.1 g, 13.51 mmol) was added to the above reaction solution. The reaction solution was stirred at 130 °C for 18 h under N₂ protection. Saturated NH₄Cl (100 mL) was added to the reaction solution, and extracted with EtOAc (100 mL × 3). The organic layer was separated, washed with saturated NaCl solution, concentrated, slurried with EtOAc (20 mL) at 25 °C, and filtered to afford compound **62-4** as a yellow solid (1.11 g, yield 23.2%).

MS (ESI) m/z = 371.0 [M+H]⁺.

### Step 4: Synthesis of Compound 62-5

Compound **62-1** (1.34 g, 2.70 mmol) was dissolved in dioxane (10 mL) and H₂O (2.5 mL). Then, compound **62-4** (1.0 g, 2.70 mmol), Pd₂(dba)₃ (0.25 g, 0.27 mmol), meCgPPh (0.32 g, 1.08 mmol) and K₃PO₄ (1.72 g, 8.11 mmol) were added. The reaction solution was stirred at 90 °C for 2 h under N₂ protection. The reaction solution was diluted with water (50 mL) and extracted with EtOAc (50 mL × 3). The organic layer was separated, washed with saturated brine (50 mL) and concentrated to afford a residue, which was purified by Prep-HPLC and concentrated to afford compound 62-5 as a yellow solid (300 mg, yield 16.9%).

MS (ESI) m/z = 612.4 [M+H]⁺.

### Step 5: Synthesis of Compound 62-6

Compound **62-5** (300 mg, 0.49 mmol) was dissolved in THF (2 mL), MeOH (2 mL) and water (2 mL), And LiOH·H₂O (41.1 mg, 0.98 mmol) was added. The reaction solution was stirred at 30 °C for 4 h under N₂ protection. After concentration, compound **62-6** was obtained as a yellow solid (314 mg, crude), which was used directly in the next step without further purification.

MS (ESI) m/z = 598.4 [M+H]⁺.

### Step 6: Synthesis of Compound 62-7

Compound **62-6** (314 mg, 0.53 mmol) was dissolved in DMF (3 mL), and DIEA (340.6 mg, 2.64 mmol), HATU (400.8 mg, 1.05 mmol) and benzo[d][1,3]thiazol-2-amine (79.2 mg, 0.53 mmol) were added. The reaction solution was stirred at 25 °C for 1 h under N₂ protection. The reaction solution was diluted with water (20 mL) and extracted with EtOAc (3 mL × 3). The organic layer was separated, washed with 0.5 N HCl solution. The organic layer was separated, then washed with saturated brine and concentrated. The residue was purified by column chromatography to afford compound **62-7** as a yellow solid (137 mg, yield 35.7%).

MS (ESI) m/z = 730.2 [M+H]⁺.

### Step 7: Synthesis of Compound RN062

Compound **62-7** (137 mg, 0.19 mmol) was dissolved in DCM (2 mL), and TFA (2 mL) was added. The reaction solution was stirred at 25 °C for 5 h under N₂ protection. The reaction solution was concentrated and purified by prep-HPLC to afford compound **RN062** as a white solid (3.28 mg, yield 2.6%).

MS (ESI) m/z = 674.2 [M+H]⁺.

**¹H NMR** (400 MHz, DMSO-*d₆*): δ 12.29 (s, 1H), 8.56 (d, *J =* 4.8 Hz, 1H), 8.15 (s, 1H), 8.06 (d, *J* = 7.6 Hz, 1H), 7.82 (d, *J* = 8.0 Hz, 1H), 7.68 (d, *J =* 8.7 Hz, 1H), 7.57 (d, *J =* 4.9 Hz, 1H), 7.49 (t, *J =* 7.7 Hz, 1H), 7.37 (t ,*J* = 7.6 Hz, 1H), 7.02 (d, *J =* 8.8 Hz, 1H), 5.22 (s, 2H), 3.95 (t, *J* = 5.6 Hz, 2H), 3.57 (s, 2H), 3.06 (t, *J =* 5.8 Hz, 2H), 2.31 (s, 3H), 2.08 (s, 3H), 1.96 (s, 3H), 1.69 - 1.49 (m, 12H).

### Example 29. Synthesis of Compound RN063

The synthetic route is as follows:

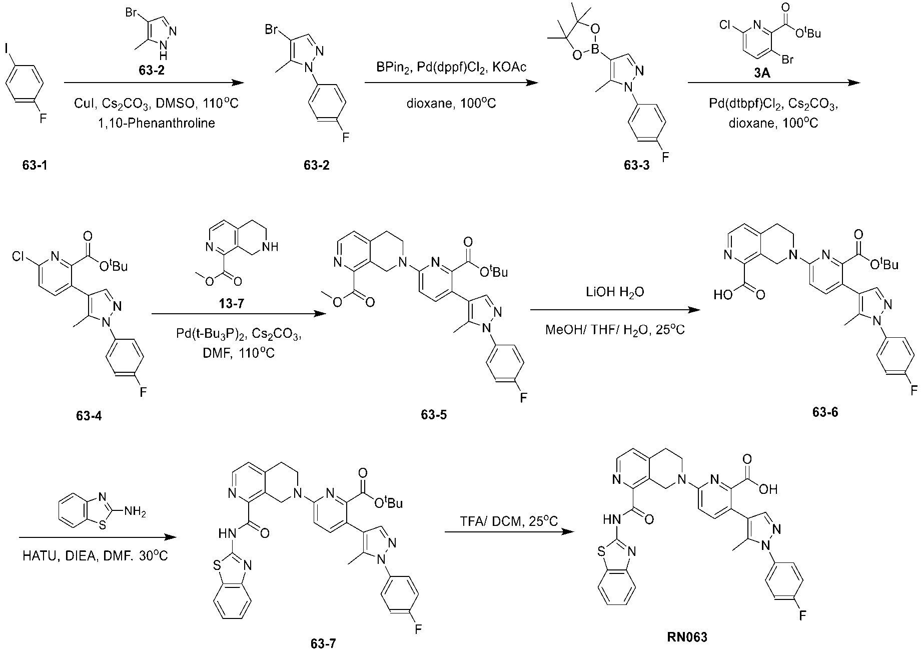

### Step 1: Synthesis of Compound 63-2

Compound **63-1** (5.00 g, 31.0 mmol) was dissolved in dimethyl sulfoxide (100 mL), and compound **63-2** (10.3 g, 46.5 mmol) and copper(I) iodide (1.18 g, 6.21 mmol) were added. After thorough nitrogen purging, cesium carbonate (30.3 g, 93.1 mmol) and 1,10-phenanthroline (5.60 g, 31.0 mmol) were added. The reaction solution was stirred at 110 °C for 16 h. After completion of the reaction, water (100 mL) was added to quench the reaction, extracted with dichloromethane (100 mL × 2), washed with water (200 mL) and saturated brine (100 mL), and dried over anhydrous sodium sulfate. The mother liquor was concentrated in vacuo and purified by silica gel column chromatography to afford compound **63-2** as a white solid (1.20 g, yield 15.2%).

### Step 2: Synthesis of Compound 63-3

Compound **63-2** (1.20 g, 4.70 mmol), Pd(dppf)Cl₂ (385 mg, 0.47 mmol), bis(pinacolato)diboron (1.55 g, 6.12 mmol) and potassium acetate (1.02 g, 10.3 mmol) were dissolved in 1,4-dioxane (12 mL). After nitrogen purging, the reaction solution was stirred at 100 °C for 16 h. The reaction solution was concentrated in vacuo, extracted with dichloromethane (30 mL × 2), washed with water (50 mL × 2) and saturated brine (30 mL), and dried over anhydrous sodium sulfate, followed by purification by silica gel column chromatography to afford compound **63-3** as a yellow oil (680 mg, yield 47.8%).

MS (ESI) m/z = 303.2 [M+H]⁺.

### Step 3: Synthesis of Compound 63-4

Under N₂ protection, compound **63-3** (680 mg, 2.25 mmol), compound **3A** (790 mg, 2.70 mmol), Pd(dtbpf)Cl₂ (145 mg, 0.23 mmol) and cesium carbonate (2.20 g, 6.75 mmol) were dissolved in dioxane (7 mL) and water (0.7 mL). The reaction solution was reacted at 100 °C for 18 h. After completion of the reaction, water (30 mL) was added, and the reaction solution was extracted with ethyl acetate (30 mL × 2). The organic phase was washed with saturated brine (20 mL), dried over anhydrous sodium sulfate, concentrated in vacuo and purified by silica gel column chromatography to afford compound **63-4** as a white solid (410 mg, yield 47.2%).

MS (ESI) m/z = 388.2 [M+H]⁺.

### Step 4: Synthesis of Compound 63-5

Under nitrogen protection, compound **63-4** (350 mg, 2.91 mmol), compound **13-7** (211 mg, 1.09 mmol), Pd(t-Bu₃P)₂ (46.4 mg, 0.09 mmol) and cesium carbonate (886 mg, 2.72 mmol) were dissolved in N,N-dimethylformamide (4 mL). The reaction solution was stirred at 110 °C for 3 h. After completion of the reaction, the reaction solution was diluted with water (30 mL) and extracted with dichloromethane (20 mL × 2). The organic phase was washed with saturated brine (10 mL), dried over anhydrous sodium sulfate, concentrated in vacuo and purified by silica gel column chromatography to afford compound **63-5** as a white solid (220 mg, yield 44.8%).

MS (ESI) m/z = 544.2 [M+H]⁺.

### Step 5: Synthesis of Compound 63-6

Compound **63-5** (200 mg, 0.37 mmol) and lithium hydroxide monohydrate (30.99 mg, 0.74 mmol) was dissolved in methanol (2 mL), tetrahydrofuran (2 mL) and water (2 mL). The reaction solution was reacted at 25 °C for 3 h. The reaction solution was concentrated in vacuo to afford a crude white compound **63-6** (220 mg).

MS (ESI) m/z = 530.2 [M+H]⁺.

### Step 6: Synthesis of Compound 63-7

Compound **63-6** (180 mg, 0.34 mmol), 2-aminobenzothiazole (56.4 mg, 0.38 mmol), HATU (285 mg, 0.75 mmol) and DIEA (132 mg, 1.02 mmol) were dissolved in N,N-dimethylformamide (2 mL). The reaction solution was reacted at 30 °C for 2 h. After completion of the reaction, the reaction solution was diluted with water (20 mL) and extracted with ethyl acetate (20 mL × 2). The organic phase was washed with saturated brine (10 mL), dried over anhydrous sodium sulfate, concentrated in vacuo and purified by silica gel column chromatography to afford compound **63-7** as a yellow oil (60 mg, yield 26.7%).

MS (ESI) m/z = 662.2 [M+H]⁺.

### Step 7: Synthesis of Compound RN063

Compound **63-7** (40 mg, 0.06 mmol) was dissolved in dichloromethane (1 mL), and trifluoroacetic acid (1 mL) was added. The reaction solution was stirred at 25 °C for 2 h. After completion of the reaction, the reaction solution was concentrated. The crude product was purified by Prep-HPLC to afford **RN063** as a white solid (6.3 mg, yield 17.2%).

MS (ESI) m/z = 606.2 [M+H]⁺.

**¹H NMR** (400MHz, DMSO-*d₆*): δ 8.56 (s, 1H), 8.06 (d, *J =* 7.9 Hz, 1H), 7.82 (d, *J =* 8.1 Hz, 1H), 7.64 (s, 1H), 7.61 - 7.51 (m, 4H), 7.50 - 7.41 (m, 1H), 7.37 (tt, *J =* 8.3, 2.1 Hz, 3H), 7.00 (d, *J =* 8.8Hz, 1H), 5.23 (s, 2H), 3.95 (t, *J =* 5.8 Hz, 2H), 3.06 (t, *J =* 5.4 Hz, 2H), 2.23 (s, 3H).

The following example compounds in Table 6 were prepared according to the same method as the above examples, using commercially available compounds or by reference to the preparation methods of the intermediates shown.

**Table 6**

| Compound ID | Starting Material | Structure | ¹H-NMR |
|---|---|---|---|
| **RN064** | 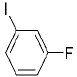 | 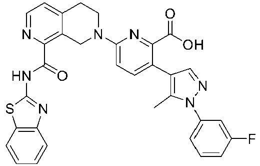 | **¹H NMR** (400 MHz, DMSO-*d₆*): *δ* 12.97 (s, 1H), 12.31 (s, 1H), 8.56 (d, J = 4.8 Hz, 1H), 8.06 (d, J = 7.7 Hz, 1H), 7.82 (d, J = 8.0 Hz, 1H), 7.68 - 7.55 (m, 4H), 7.52 - 7.41 (m, 3H), 7.37 (t, J = 7.5 Hz, 1H), 7.33 - 7.26 (m, 1H), 7.11 (d, J = 8.9 Hz, 1H), 5.26 (s, 2H), 3.97 (t, J = 5.8 Hz, 2H), 3.07 (t, J = 5.5 Hz, 2H), 2.27 (s, 3H). |
| **RN065** | 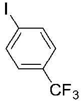 | 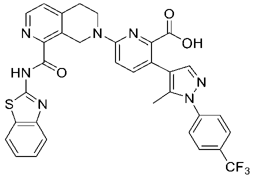 | **¹H NMR** (400 MHz, DMSO-*d₆*): *δ* 8.56 (d, J = 4.8 Hz, 1H), 8.06 (d, J = 7.7 Hz, 1H), 7.82 (d, J = 8.0 Hz, 1H), 7.68 - 7.55 (m, 4H), 7.52 - 7.41 (m, 3H), 7.37 (t, J = 7.5 Hz, 1H), 7.33 - 7.26 (m, 1H), 7.03 (d, J = 8.9 Hz, 1H), 5.26 (s, 2H), 3.96 (t, J = 5.8 Hz, 2H), 3.07 (t, J = 5.5 Hz, 2H), 2.31(s, 3H). |
| **RN066** | 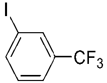 | 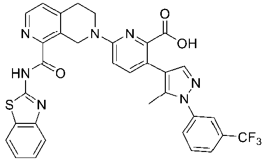 | **1H NMR** (400MHz, DMSO-*d₆*): δ 13.00 (s, 1H), 12.31 (s, 1H), 8.57 (d, J = 4.8 Hz, 1H), 8.06 (d, J = 7.8 Hz, 1H), 7.95 - 7.88 (m, 2H), 7.86 - 7.77 (m, 3H), 7.66 (m, 2H), 7.58 (d, J = 4.8 Hz, 1H), 7.52 - 7.46 (m, 1H), 7.37 (t, J = 7.6 Hz, 1H), 7.13 (d, J = 8.9 Hz, 1H), 5.26 (s, 2H), 3.98 (t, J = 5.8 Hz, 2H), 3.07 (t, J = 5.6 Hz, 2H), 2.29 (s, 3H). |
| **RN067** | 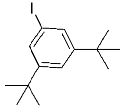 | 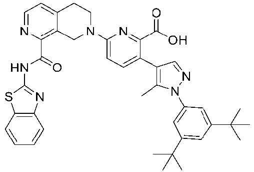 | **¹H NMR** (400 MHz, DMSO-*d₆*): δ 12.98 (s, 1H), 12.32 (s, 1H), 8.57 (d, J = 4.3 Hz, 1H), 8.06 (d, J = 7.6 Hz, 1H), 7.82 (d, J = 7.5 Hz, 1H), 7.68 (d, J = 8.7 Hz, 1H), 7.63 - 7.54 (m, 2H), 7.53 - 7.43 (m, 2H),7.37 (t, J = 7.7 Hz, 1H), 7.30 (s, 2H), 7.11 (d, J = 8.7 Hz, 1H), 5.26 (s, 2H), 3.97 (t, J = 5.6 Hz, 2H), 3.08 (t, J = 5.1 Hz, 2H), 2.22 (s, 3H), 1.34 (s, 18H). |
| **RN068** | 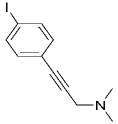 | 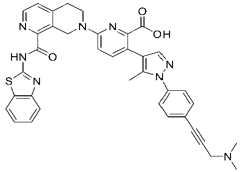 | **¹H NMR:** (400 MHz, DMSO-*d₆*): *δ* 12.72 - 11.80 (m, 1H), 8.56 (d, *J=* 4.9 Hz, 1H), 8.06 (d, *J=* 7.8 Hz, 1H), 7.82 (d, *J =* 8.0 Hz, 1H), 7.66 - 7.54 (m, 7H), 7.51 - 7.46 (m, 1H), 7.40 - 7.34 (m, 1H), 7.10 (d, *J* = 8.9 Hz, 1H), 5.25 (s, 2H), 3.97 (br t, *J =* 5.8 Hz, 2H), 3.49 (s, 2H), 3.07 (br t, *J=* 5.6 Hz, 2H), 2.28 - 2.22 (m, 9H). |
| **RN069** | 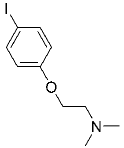 | 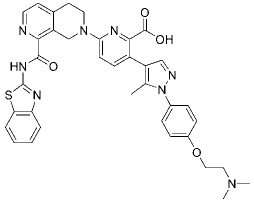 | **¹H NMR:** (400 MHz, DMSO-*d₆*): *δ* 8.56 (d, *J =* 4.8 Hz, 1H), 8.06 (d, *J* = 7.8 Hz, 1H), 7.82 (d, *J =* 8.0 Hz, 1H), 7.65 - 7.53 (m, 3H), 7.49 (t, *J* = 7.7 Hz, 1H), 7.44 - 7.33 (m, 3H), 7.11 - 7.02 (m, 3H), 5.24 (s, 2H), 4.12 (t, *J* = 5.7 Hz, 2H), 3.96 (br t, *J* = 5.6 Hz, 2H), 3.07 (br t, *J =* 5.6 Hz, 2H), 2.71 (t, *J* = 5.6 Hz, 2H), 2.27 (s, 6H), 2.18 (s, 3H). |
| **RN070** | 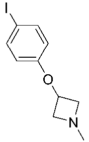 | 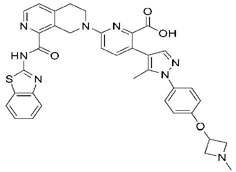 | **¹H NMR:** (400 MHz, DMSO-*d₆*): *δ* 8.55 (br d, *J* = 4.6 Hz, 1H), 8.05 (d, *J =* 7.8 Hz, 1H), 7.82 (d, *J* = 8.1 Hz, 1H), 7.64 - 7.54 (m, 3H), 7.48 (t, *J* = 7.4 Hz, 1H), 7.42 - 7.33 (m, 3H), 7.01 (d, *J =* 8.8 Hz, 1H), 6.95 (d, *J* = 8.9 Hz, 2H), 5.23 (s, 2H), 4.86 (br t, *J =* 5.8 Hz, 1H), 3.95 (br t, *J =* 6.1 Hz, 4H), 3.28 (br d, *J =* 7.4 Hz, 2H), 3.06 (br t, *J =* 5.4 Hz, 2H), 2.44 (s, 3H), 2.18 (s, 3H). |

### Example 30. Synthesis of Compound RN071

The synthetic route is as follows:

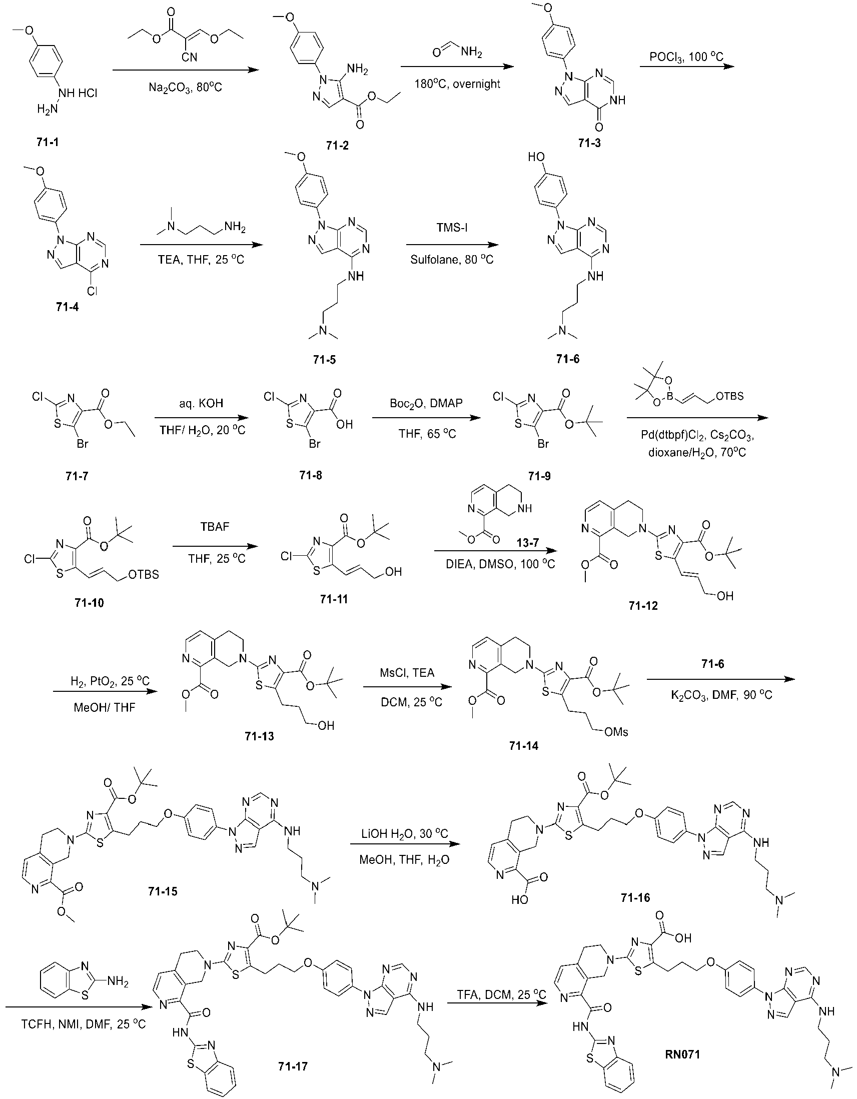

### Step 1: Synthesis of Compound 71-2

Compound **71-1** (3.10 g, 17.7 mmol), ethyl (E)-2-cyano-3-ethoxyacrylate (3.00 g, 17.7 mmol) and sodium carbonate (1.88 g, 17.7 mmol) were dissolved in ethanol (30 mL). The reaction solution was stirred and heated to 80 °C under N₂ protection for 5 h, then cooled to 25 °C and stood for 12 h. The reaction solution was concentrated under reduced pressure to remove the solvent. The crude product was purified by column chromatography to afford compound **71-2** as a yellow solid (3.80 g, yield 82.0%).

MS (ESI) m/z = 262.2 [M+H]⁺.

**¹H NMR** (400 MHz, DMSO-*d₆*): δ 7.65 (s, 1H), 7.45 - 7.38 (m, 2H), 7.11 - 7.02 (m, 2H), 6.17 (s, 2H), 4.21 (q, *J =* 7.1 Hz, 2H), 3.81 (s, 3H), 1.26 (t, *J =* 7.1 Hz, 3H).

### Step 2: Synthesis of Compound 71-3

Compound **71-2** (3.8 g, 14.5 mmol) and formamide (38 mL) were mixed. The mixture was stirred and heated to 180 °C under N₂ protection for 18 h. LCMS indicated the reaction was complete. The suspension was filtered. The crude product was recrystallized from ethanol/water = 10/1 (22 mL) at 25 °C to afford compound **71-3** (2.23 g, yield 63.3%).

MS (ESI) m/z = 243.0 [M+H]⁺.

**¹H NMR** (400 MHz, DMSO-*d₆*): δ 12.37 (s, 1H), 8.28 (s, 1H), 8.16 (s, 1H), 7.94 - 7.84 (m, 2H), 7.16 - 7.08 (m, 2H), 3.82 (s, 3H).

### Step 3: Synthesis of Compound 71-4

Compound **71-3** (1.00 g, 4.13 mmol) was dissolved in phosphorus trichloride (10 mL). The mixture was stirred at 100 °C for 2 hours. LCMS indicated the reaction was complete. The reaction mixture was extracted with ice water (100 mL) and dichloromethane (100 mL). The organic phase was washed with saturated aqueous sodium chloride solution (80 mL), dried over anhydrous sodium sulfate, filtered, and concentrated under reduced pressure to afford compound **71-4** as a pale yellowish-white solid (700 mg, yield 65.1%).

MS (ESI) m/z = 261.0 [M+H]⁺.

### Step 4: Synthesis of Compound 71-5

Compound **71-4** (700 mg, 2.69 mmol), 3-(dimethylamino)propylamine (0.41 mL, 3.22 mmol) and triethylamine (0.82 mL, 5.91 mmol) were dissolved in tetrahydrofuran (10 mL). The reaction solution was stirred at 25 °C for 18 hours under N₂ atmosphere. LCMS indicated the reaction was complete. The reaction mixture was extracted with water (20 mL) and ethyl acetate (20 mL). The organic phase was washed with saturated aqueous sodium chloride solution (20 mL), dried over anhydrous sodium sulfate, filtered, and concentrated under reduced pressure to afford compound **71-5** as a white solid (790 mg, 2.42 mmol, yield 90.1%).

MS (ESI) m/z = 327.2 [M+H]⁺.

### Step 5: Synthesis of Compound 71-6

Compound **71-5** (400 mg, 1.23 mmol) was dissolved in sulfolane (5 mL), and trimethylsilyl iodide (1.75 mL, 12.2 mmol) was added. The mixture was stirred at 80 °C for 5 h. LCMS indicated the reaction was complete. The reaction mixture was concentrated under reduced pressure to remove the solvent. The crude product was purified by reversed-phase high performance liquid chromatography to afford compound **71-6** as a pale yellowish-white solid (157 mg, yield 41.0%).

MS (ESI) m/z = 313.2 [M+H]⁺.

**¹H NMR** (400 MHz, DMSO-*d₆*): δ 8.45 (t, *J =* 5.7 Hz, 1H), 8.31 (d, *J =* 10.3 Hz, 2H), 8.17 (s, 1H), 7.88 - 7.82 (m, 2H), 6.93 - 6.87 (m, 2H), 3.56 (q, *J =* 6.6 Hz, 2H), 2.63 (t, *J =* 7.4 Hz, 2H), 2.40 (s, 6H).

### Step 6: Synthesis of Compound 71-8

Compound **71-7** (3.50 g, 12.9 mmol) was dissolved in tetrahydrofuran (24 mL), and a mixture of KOH (1.09 g, 19.4 mmol)/H₂O (24 mL) was added dropwise at 0 °C. The mixture was stirred at 20 °C for 2 h. LCMS indicated the reaction was complete. The mixture was diluted with water (50 mL), then extracted with MTBE (10 mL × 3). The aqueous phase was acidified to pH = 1 with 6N hydrochloric acid. A pale yellow solid precipitated, which was filtered and washed with water (5 mL). The crude product was slurried with 15 mL water for 30 minutes, filtered and dried under vacuum to afford compound **71-8** as a pale yellow oil (2.80 g, yield 89.2%).

MS (ESI) m/z = 239.9 [M-H]⁻

### Step 7: Synthesis of Compound 71-9

Compound **71-8** (3.50 g, 14.4 mmol) was dissolved in tetrahydrofuran (70 mL), and Boc₂O (7.64 mL, 33.2 mmol) and DMAP (0.35 g, 2.89 mmol) were added. The mixture was stirred at 65 °C for 16 h. The reaction solution was diluted with water (30 mL), and the organic layer was separated and the aqueous phase was extracted with ethyl acetate (15 mL). The organic phases were combined, washed with water (10 mL) and saturated brine (10 mL × 2), and dried over anhydrous sodium sulfate. The crude product was isolated and purified to afford compound **71-9** as a white solid (3.70 g, yield 85.8%).

### Step 8: Synthesis of Compound 71-10

Compound **71-9** (1.00 g, 3.35 mmol) was dissolved in dioxane (12 mL)/water (1.2 mL), and (6E)-2,2,3,3-tetramethyl-7-(4,4,5,5-tetramethyl-1,3,2-dioxaborolan-2-yl)-4-oxo-3-silahept-6-ene (1.50 g, 5.02 mmol), cesium carbonate (2.73 g, 8.37 mmol) and Pd(dtbpf)Cl₂ (0.22 g, 0.33 mmol) were added. The mixture was stirred at 70 °C for 1.5 h under nitrogen protection. LCMS indicated the reaction was complete. The mixture was concentrated, diluted with water (15 mL), extracted with ethyl acetate (10 mL × 3), washed with water (5 mL) and brine (5 mL), dried over anhydrous sodium sulfate, followed by concentration and purification to afford compound **71-10** as a pale yellow oil (522 mg, yield 39.9%).

MS (ESI) m/z = 412.0 [M+Na]⁺_{∘}

**¹H NMR** (400 MHz, CDCl₃): δ 7.46 (dt, *J =* 2.0, 16.0 Hz, 1H), 6.16 (dt, *J =* 4.8, 16.0 Hz, 1H), 4.33 (dd, *J =* 1.6, 4.8 Hz, 1H), 1.60 (s, 9H), 0.93 (s, 9H), 0.10 (s, 6H).

### Step 9: Synthesis of Compound 71-11

Compound **71-10** (520 mg, 1.33 mmol) was dissolved in tetrahydrofuran solution (7 mL), and TBAF (2.67 mL, 2.67 mmol, 1 M in tetrahydrofuran) was added. The mixture was stirred at 25 °C for 2 h under N₂ protection. LCMS indicated the reaction was complete. The mixture was concentrated, diluted with water (15 mL), extracted with ethyl acetate (10 mL × 3), washed with water (5 mL) and brine (5 mL), and dried over anhydrous sodium sulfate. The crude product was concentrated and purified to afford compound **71-11** as a yellow oil (264 mg, yield 71.9%).

MS (ESI) m/z = 220.0 [M-tBu+H]⁺.

### Step 10: Synthesis of Compound 71-12

Compound **71-11** (264 mg, 0.96 mmol) was dissolved in DMSO solution (4 mL), and compound **13-7** (220 mg, 1.15 mmol) and DIEA (0.50 mL, 2.87 mmol) were added. The reaction solution was reacted at 100 °C for 16 h under N₂ protection. LCMS indicated the reaction was complete. The reaction solution was diluted with H₂O (25 mL), extracted with ethyl acetate (10 mL × 3), washed with H₂O (5 mL) and saturated brine (5 mL), dried over anhydrous sodium sulfate, and concentrated. The crude product was purified to afford compound **71-12** as a yellow solid (309 mg, yield 74.8%).

MS (ESI) m/z = 432.2 [M+H]⁺.

### Step 11: Synthesis of Compound 71-13

Compound **71-12** (309 mg, 0.72 mmol) was dissolved in a mixed solvent of methanol (10 mL)/tetrahydrofuran (2 mL), and platinum dioxide (56.9 mg, 0.25 mmol) was added. The reaction solution was stirred at 25 °C for 12 h, while hydrogen gas (15 psi) was introduced. LCMS indicated the reaction was complete. The reaction mixture was filtered and concentrated to afford compound **71-13** as a yellow solid (262 mg, yield 84.4%). The crude product was used directly in the next step without further purification.

MS (ESI) m/z = 434.2 [M+H]⁺.

### Step 12: Synthesis of Compound 71-14

To a solution of compound **71-13** (260 mg, 0.60 mmol) in dichloromethane (5 mL) were added triethylamine (0.25 mL, 1.80 mmol) and MsCl (0.07 mL, 0.90 mmol) at 0 °C. The reaction solution was stirred at 25 °C for 2 h under nitrogen protection. LCMS indicated the reaction was complete. The reaction solution was diluted with water (10 mL), extracted with dichloromethane (10 mL × 3), washed with water (5 mL) and saturated brine (5 mL), dried over anhydrous sodium sulfate. The crude product was concentrated and purified by column chromatography to afford compound **71-14** as a yellow solid (256 mg, yield 83.4%).

MS (ESI) m/z = 512.2 [M+H]⁺.

### Step 13: Synthesis of Compound 71-15

Compound **71-14** (256 mg, 0.50 mmol) was dissolved in DMF solution (4 mL), and potassium carbonate (207 mg, 1.50 mmol) and compound **71-6** (390 mg, 1.25 mmol) were added. The reaction solution was stirred at 90 °C for 12 h under N₂ protection. LCMS indicated the reaction was complete. The reaction solution was added dropwise to ice-cold H₂O (15 mL) with stirring, stirred for 10 minutes, then filtered. The crude product was purified by preparative column chromatography to afford compound **71-15** as a yellow solid (216 mg, yield 59.3%).

MS (ESI) m/z = 728.4 [M+H]⁺.

### Step 14: Synthesis of Compound 71-16

Compound **71-15** (60 mg, 0.08 mmol) was dissolved in a mixed solution of methanol (0.2 mL)/tetrahydrofuran (0.2 mL)/H₂O (0.2 mL), and lithium hydroxide monohydrate (6.92 mg, 0.16 mmol) was added. The reaction solution was stirred at 30 °C for 2 h under N₂ protection. LCMS indicated the reaction was complete. The reaction solution was concentrated to afford compound **71-16** (58.8 mg, crude), which was used directly in the next step without further purification.

MS (ESI) m/z = 714.4 [M+H]⁺.

### Step 15: Synthesis of Compound 71-17

Compound **71-16** (58.8 mg, 0.08 mmol) was dissolved in DMF solution (1.5 mL), and 2-benzothiazolamine (13.6 mg, 0.09 mmol), NMI (20.2 mg, 0.25 mmol) and TCFH (115 mg, 0.41 mmol) were added. The mixture was stirred at 25 °C for 2 h under N₂ protection. LCMS indicated the reaction was complete. The reaction solution was diluted with water (10 mL), extracted with ethyl acetate (5 mL × 3), washed with water (3 mL) and saturated brine (5 mL), and dried over anhydrous sodium sulfate. The crude product was purified by column chromatography to afford compound **71-17** as a yellow oil (26 mg, yield 37.3%).

MS (ESI) m/z = 846.4 [M+H]⁺.

### Step 16: Synthesis of Compound RN-071

Compound **71-17** (16 mg, 0.02 mmol) was dissolved in dichloromethane (1 mL). Trifluoroacetic acid (1 mL) was added. The reaction solution was stirred at 25 °C for 3 h under nitrogen protection. LCMS indicated the reaction was complete. The reaction solution was concentrated. The crude product was purified by preparative high performance liquid chromatography to afford compound **RN071** as a pale yellow solid (3.03 mg, yield 20.2%).

MS (ESI) m/z = 790.2 [M+H]⁺.

**¹H NMR** (400 MHz, DMSO-*d₆*): δ 8.55 (s, 1H), 8.47 (s, 1H), 8.26-8.31 (m, 3H), 7.98 (d, *J =* 8.8 Hz, 2H), 7.79 (d, *J =* 7.2 Hz, 1H), 7.56 (s, 1H), 7.47 (s, 1H), 7.35 (s, 1H), 7.06 (d, *J =* 7.2 Hz, 2H), 5.07 (s, 2H), 4.04 (s, 2H), 3.77 (s, 2H), 3.54 (s, 2H), 3.23 (s, 2H), 3.07 (s, 2H), 2.42 (s, 2H), 2.23 (s, 6H), 2.05 (s, 2H), 1.79 (s, 2H).

### Example 31. Synthesis of Compound RN072

Structure of Compound **RN072** :

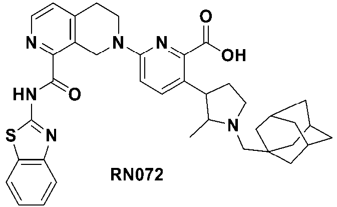

Compound **RN072** was prepared as a yellow solid (2.16 mg, yield 2.00%) using the same method as Example **RN019.**

MS (ESI) m/z = 663.4 [M+H]⁺.

**¹H NMR** (400 MHz, DMSO-*d₆*): δ = 8.54 (d, *J =* 4.8 Hz, 1H), 8.05 (d, *J =* 8.0 Hz, 1H), 7.81 (d, *J= 8.0* Hz, 1H), 7.70 (d, *J =* 8.8 Hz, 1H), 7.55 (d, *J =* 5.0 Hz, 1H), 7.51 - 7.44 (m, 1H), 7.41 - 7.29 (m, 1H), 6.95 (br d, *J =* 9.0 Hz, 1H), 5.17 (br d, *J =* 5.0 Hz, 2H), 3.94 - 3.82 (m, 2H), 3.59 - 3.49 (m, 1H), 3.03 (br t, *J =* 5.3 Hz, 2H), 2.85 - 2.70 (m, 1H), 2.42 - 2.35 (m, 4H), 2.22 - 1.99 (m, 2H), 1.94 (br s, 3H), 1.72 - 1.41 (m, 12H), 0.66 (d, *J =* 6.3 Hz, 3H).

### Example 32. Synthesis of Compound RN073

The synthetic route is as follows:

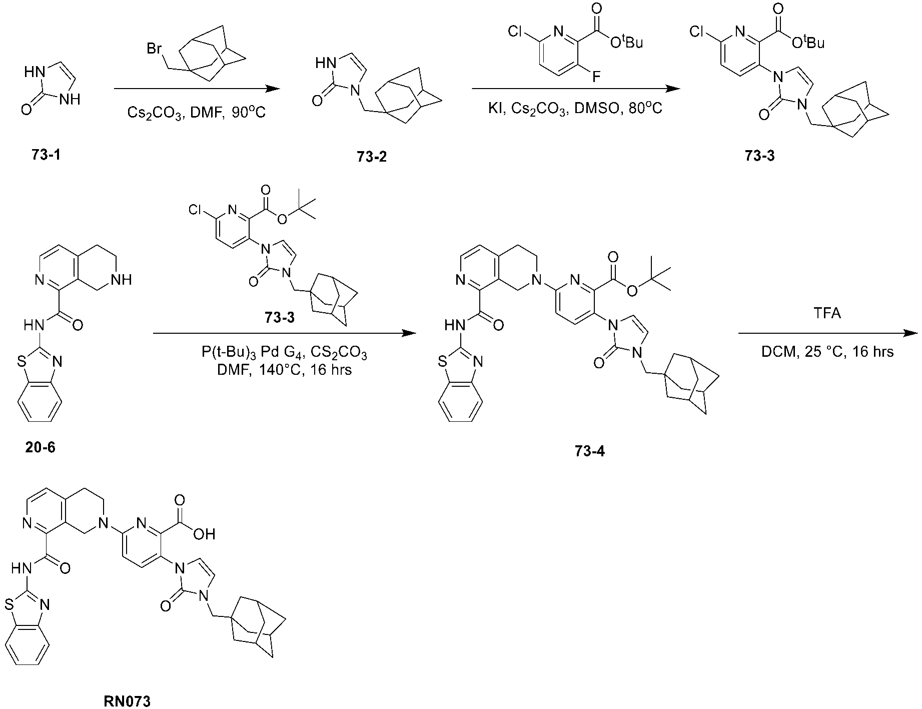

### Step 1: Synthesis of Compound 73-2

Compound **73-1** (5.00 g, 59.4 mmol) was dissolved in DMSO (150 mL), and 1-bromomethyladamantane (8.02 mL, 47.5 mmol) and cesium carbonate (48.4 g, 148 mmol) were added. The reaction solution was stirred at 90 °C for 16 h. After completion of the reaction, the reaction solution was diluted with water (1.5 L) and extracted with ethyl acetate (150 mL × 3). The organic phase was concentrated to afford crude product as a white solid (488 mg, 1.26 mmol, yield 2.12%), which was used directly in the next step.

MS (ESI) m/z = 233.2 [M+H]⁺.

### Step 2: Synthesis of Compound 73-3

Compound **73-2** (200 mg, 0.86 mmol) was dissolved in DMSO (2 mL), and tert-Butyl 6-chloro-3-fluoropyridine-2-carboxylate (197 mg, 0.86 mmol), cesium carbonate (841 mg, 2.58 mmol) and potassium iodide (142 mg, 0.86 mmol) were added. The reaction solution was stirred at 80 °C for 2 h. The reaction solution was diluted with water (20 mL), extracted with ethyl acetate (10 mL × 2), washed with saturated brine (5 mL), dried and concentrated. The crude product was purified by prep-HPLC (0.1% TFA, ACN: H₂O = 70% to 93%) to afford compound **73-3** as a white solid (45 mg, yield 13.4%).

MS (ESI) m/z = 388.2 [M-^{t}Bu+H]⁺.

### Step 3: Synthesis of Compound 73-4

Compound **20-6** (80.0 mg, 0.26 mmol) was dissolved in DMF (3 mL) and compound **73-3** (136 mg, 0.31 mmol), cesium carbonate (251 mg, 0.77 mmol) and Pd(t-Bu₃P)₂ (13.1 mg, 0.03 mmol) were added. The reaction solution was stirred at 130 °C for 6 h under microwave conditions. After completion of the reaction, the reaction solution was diluted with water (10 mL), extracted with ethyl acetate (5 mL × 2), washed with water (3 mL) and saturated brine (3 mL), and dried over anhydrous sodium sulfate. The crude product was purified by preparative chromatography to afford compound **73-4** as a yellow oil (44.0 mg, yield 23.8%).

MS (ESI) m/z = 716.4 [M+H]⁺.

### Step 4: Synthesis of Compound RN073

Compound **73-4** (34.0 mg, 0.05 mmol) was dissolved in dichloromethane (0.8 mL), and trifluoroacetic acid (0.8 mL) was added. The reaction solution was stirred at 25 °C for 2 h. After completion of the reaction, the reaction solution was concentrated. The crude product was purified by Prep-HPLC to afford **RN073** as a white solid (11.5 mg, yield 37.1%).

MS (ESI) m/z = 660.4 [M+H]⁺.

**¹H NMR** (400 MHz, DMSO-*d₆*): δ 8.57 (d, *J =* 4.8 Hz, 1H), 8.05 (d, *J =* 7.6 Hz, 1H), 7.81 (d, *J* = 8.4 Hz, 1H), 7.58 (d, *J* = 4.8 Hz, 2H), 7.48 (t, *J =* 7.6 Hz, 1H), 7.34-7.39 (m, 2H), 7.02 (d, *J =* 9.2 Hz, 1H), 5.26 (s, 2H), 3.97 (s, 2H), 3.07 (s, 2H), 2.17 (s, 2H), 1.89 (s, 6H), 1.61-1.64 (m, 3H), 1.50-1.57 (m, 9H).

### Example 33. Synthesis of Compound RN074

The synthetic route is as follows:

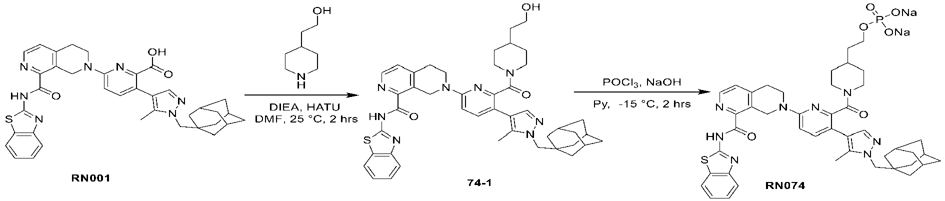

### Step 1: Synthesis of Compound 74-1

Compound **RN001** (50 mg, 75.78 µmol, 1 eq) was dissolved in 1 mL N,N-dimethylformamide, and N,N-diisopropylethylamine DIEA (29.38 mg, 227.34 µmol, 39.60 µL, 3 eq) and 2-(piperidin-4-yl)ethan-1-ol (14.69 mg, 113.67 µmol, 1.5 eq) were added. The reaction solution was stirred at 25 °C for 10 minutes, then HTAU (43.22 mg, 113.67 µmol, 1.5 eq) was added. The reaction solution was stirred at 25 °C for 2 h. The reaction solution was poured into 10 mL water and extracted twice with 10 mL ethyl acetate. The combined organic phases were washed with 10 mL saturated brine, dried over anhydrous magnesium sulfate, filtered and concentrated to afford a crude product. The crude product was purified by column chromatography to afford compound **74-1** as a yellow solid (51 mg, yield 87.29%).

MS (ESI) m/z = 771.2 [M+H]⁺.

### Step 2: Synthesis of Compound RN074

A solution of compound **74-1** (40 mg, 51.88 µmol, 1 eq) in 0.5 mL pyridine was added dropwise to a solution of phosphorus oxychloride (12.73 mg, 83.01 µmol, 7.74 µL, 1.6 eq) in 0.5 mL pyridine at -15 °C under nitrogen protection. The reaction mixture was stirred at -15 °C for 2 hours. The reaction was carefully quenched with 0.5 mL water at -5 °C, then the mixture was stirred at -5 °C for 1 h. The reaction mixture was basified to pH = 10 with 4 M NaOH, then concentrated to afford a crude product. 3 mL dimethyl sulfoxide and 1 mL pyridine were added to the crude product, and the mixture was filtered. The filter cake was washed twice with 5 mL water and lyophilized to afford compound **RN074** as a yellow solid (1.12 mg, yield 2.35%).

MS (ESI) m/z = 851.1 [M-2Na+H] ⁺.

**¹H NMR** (400 MHz, DMSO-*d₆*): *δ* = 8.32 (br d, *J =* 3.8 Hz, 1H), 7.72 (br s, 1H), 7.57 - 7.42 (m, 2H), 7.29 - 7.16 (m, 3H), 7.12 - 7.01 (m, 1H), 6.79 (br d, *J =* 8.0 Hz, 1H), 4.90 (br s, 2H), 4.29 - 4.20 (m, 1H), 3.91 - 3.82 (m, 2H), 3.69 (br s, 2H), 3.50 (br s, 2H), 3.11 - 3.06 (m, 2H), 2.95 (br *d, J* = 5.6 Hz, 2H), 2.13 (br s, 3H), 1.91 (br d, *J =* 2.4 Hz, 3H), 1.67 - 1.62 (m, 3H), 1.57 - 1.48 (m, 11H), 1.40 - 1.33 (m, 2H), 1.25 - 1.19 (m, 2H), 0.73 (ddd, *J =* 2.7, 6.0, 10.3 Hz, 1H), 0.62 - 0.35 (m, 1H).

### Example 34. Synthesis of Compound RN075

Structure of Compound **RN075** is as follows.

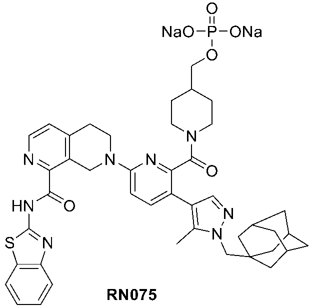

Compound **RN075** was prepared as a yellow solid (2.33 mg, yield 2.12%) using the same method as Example 33.

MS (ESI) m/z = 837.2 [M-2Na+H] ⁺.

**¹H NMR** (400 MHz, DMSO-*d₆*): *δ* = 8.31 (br d, *J =* 4.8 Hz, 1H), 7.69 (br d, *J =* 7.5 Hz, 1H), 7.48 (br d, *J=* 8.8 Hz, 2H), 7.25 - 7.07 (m, 3H), 7.03 (br t, *J* = 7.4 Hz, 1H), 6.86 (br d, *J* = 8.6 Hz, 1H), 5.17 - 4.94 (m, 1H), 4.84 (br d, *J =* 17.4 Hz, 1H), 4.19 (br d, *J =* 11.6 Hz, 1H), 3.99 (br dd, *J=* 6.1, 7.9 Hz, 1H), 3.86 - 3.75 (m, 1H), 3.74 - 3.63 (m, 2H), 3.12 - 2.78 (m, 5H), 2.66 - 2.55 (m, 1H), 2.12 (s, 3H), 1.91 (br s, 3H), 1.68 - 1.59 (m, 3H), 1.58 - 1.30 (m, 13H), 0.80 - 0.67 (m, 1H), 0.41 (br dd, *J* = 1.5, 11.5 Hz, 1H).

### Example 35. Synthesis of Compound RN076

The synthetic route is as follows:

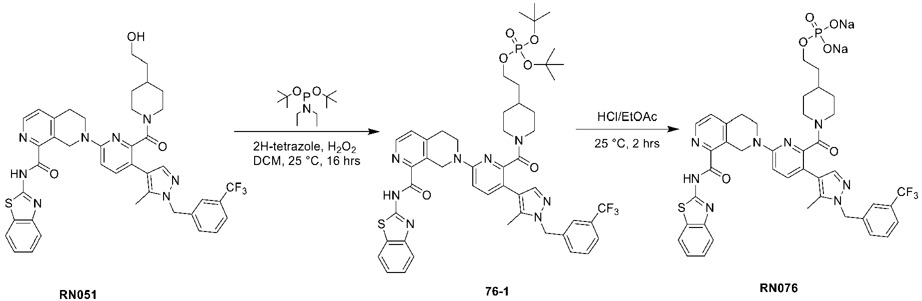

### Step 1: Synthesis of Compound 76-1

Compound **RN051** (40 mg, 51.23 µmol, 1 eq) was dissolved in 2 mL dichloromethane and [bis(tert-butoxy)phosphino]diethylamine (63.86 mg, 256.13 µmol, 5 eq) and tetrazole (14.35 mg, 204.90 µmol, 18.17 µL, 4 eq) were added. The reaction solution was stirred at 25 °C for 1 h, then hydrogen peroxide (0.2 g, 1.76 mmol, 169.49 µL, 30% purity, 34.44 eq) was added dropwise to the above mixture. The reaction solution was stirred at 25 °C for 16 h. The reaction solution was poured into 10 mL water and extracted twice with 10 mL dichloromethane. The combined organic phases were dried over anhydrous magnesium sulfate, filtered and concentrated to afford a crude product. The crude product was purified by column chromatography to afford compound 76-1 as a yellow oil (46 mg, yield 92.29%).

### Step 2: Synthesis of Compound RN076

Compound **76-1** (43 mg, 44.19 µmol, 1 eq) was dissolved in HCl-ethyl acetate solution (2 M, 3 mL, 135.77 eq). The reaction solution was stirred at 25 °C for 2 h. The reaction solution was concentrated to afford a crude. The crude was purified by preparative liquid chromatography to afford a crude product. The crude product was then dissolved in 1 mL methanol, and was adjusted to pH 7 - 8 with 0.5 M sodium hydroxide solution, then lyophilized to afford compound **RN076** as a yellow solid (2.44 mg, yield 5.60%).

MS (ESI) m/z = 861.1 [M-2Na+H] ⁺.

**¹H NMR** (400 MHz, DMSO-*d₆*): δ = 8.40 (d, *J =* 4.8 Hz, 1H), 7.84 (br d, *J* = 7.8 Hz, 1H), 7.69 - 7.55 (m, 4H), 7.52 (d, *J* = 8.8 Hz, 1H), 7.40 - 7.26 (m, 4H), 7.23 - 7.12 (m, 1H), 6.86 (d, *J =* 8.8 Hz, 1H), 5.53 - 5.38 (m, 2H), 5.03 (br s, 2H), 4.25 (br d, *J =* 12.3 Hz, 1H), 3.93 - 3.81 (m, 2H), 3.52 (br s, 1H), 3.14 - 3.06 (m, 2H), 2.98 (br t, *J =* 5.3 Hz, 2H), 2.65 - 2.58 (m, 1H), 2.45 - 2.38 (m, 1H), 2.14 (s, 3H), 1.52 (br d, *J =* 11.1 Hz, 1H), 1.46 - 1.36 (m, 1H), 1.32 (br d, *J =* 12.0 Hz, 1H), 1.16 (td, *J =* 6.1, 12.4 Hz, 2H), 0.77 - 0.62 (m, 1H), 0.56 - 0.37 (m, 1H).

### Example 36. Synthesis of Compound RN077

Structure of Compound **RN077** is as follows:

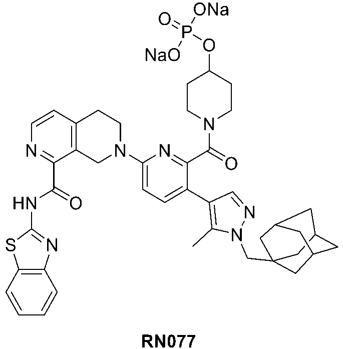

Compound **RN077** was prepared as a yellow solid (6.92 mg, yield 17.97%) using the same method as Example **RN076.**

MS (ESI) m/z = 823.4 [M-2Na+H]⁺. **¹H NMR** (400 MHz, DMSO-*d₆*): *δ* = 8.55 (d, *J =* 4.8 Hz, 1H), 8.06 (d, *J =* 7.9 Hz, 1H), 7.81 (d, *J =* 8.0 Hz, 1H), 7.59 - 7.53 (m, 2H), 7.52 - 7.44 (m, 1H), 7.42 - 7.30 (m, 1H), 7.24 (s, 1H), 6.96 (d, *J =* 8.9 Hz, 1H), 5.28 - 5.12 (m, 2H), 4.31 - 4.24 (m, 1H), 3.93 - 3.88 (m, 2H), 3.71 (br s, 2H), 3.19 - 3.09 (m, 3H), 3.05 (br t, *J =* 5.6 Hz, 2H), 2.97 - 2.91 (m, 1H), 2.16 (s, 3H), 1.93 (br s, 3H), 1.73 - 1.41 (m, 15H), 1.35 - 1.25 (m, 1H).

### Example 37. Synthesis of Compound RN078

Structure of Compound **RN078** is as follows:

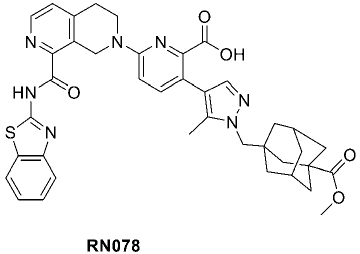

Compound **RN078** was prepared as a white solid (24 mg, yield 42.60%) using the same method as Example **RN001.**

MS (ESI) m/z = 718.3 [M+H]⁺.

**¹H NMR** (400 MHz, DMSO-*d₆*): *δ* = 8.56 (br d, *J =* 4.6 Hz, 1H), 8.05 (d, *J =* 7.6 Hz, 1H), 7.81 (d, *J =* 8.0 Hz, 1H), 7.61 - 7.44 (m, 3H), 7.41 - 7.29 (m, 2H), 6.99 (d, *J =* 8.8 Hz, 1H), 5.21 (s, 2H), 3.94 (br t, *J =* 5.8 Hz, 2H), 3.77 (s, 2H), 3.58 (s, 3H), 3.06 (br t, *J =* 5.6 Hz, 2H), 2.13 (s, 3H), 2.04 (br s, 2H), 1.79 - 1.65 (m, 6H), 1.62 - 1.41 (m, 6H).

### Example 38. Synthesis of Compound RN079

The synthetic route is as follows:

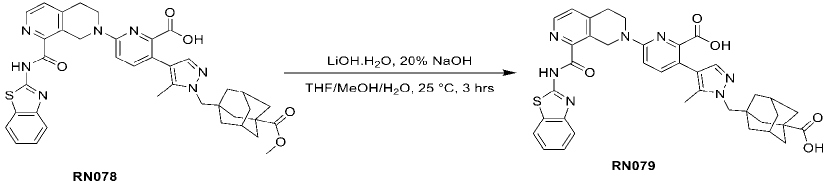

### Step 1: Synthesis of Compound RN079

Compound **RN078** (16 mg, 22.29 µmol, 1 eq) was dissolved in a mixed solution of 1 mL methanol, 1 mL tetrahydrofuran and 1 mL water, and lithium hydroxide monohydrate (2.81 mg, 66.87 µmol, 3 eq) and aqueous sodium hydroxide solution (0.3 mL, 20% purity) were added. The reaction solution was stirred at 25 °C for 3 hours. The reaction solution was concentrated to afford a crude product. The crude product was purified by preparative liquid chromatography to afford compound **RN079** as a yellow solid (6.62 mg, yield 41.30%).

MS (ESI) m/z = 704.3 [M+H]⁺.

**¹H NMR** (400 MHz, DMSO-*d₆*): δ = 8.55 (d, *J =* 4.8 Hz, 1H), 8.05 (d, *J =* 7.8 Hz, 1H), 7.81 (d, *J* = 8.0 Hz, 1H), 7.55 (d, *J =* 4.8 Hz, 1H), 7.52 - 7.42 (m, 2H), 7.41 - 7.29 (m, 2H), 6.90 (d, *J =* 8.8 Hz, 1H), 5.19 (s, 2H), 3.92 (br t, *J =* 5.6 Hz, 2H), 3.76 (s, 2H), 3.05 (br t, *J =* 5.7 Hz, 2H), 2.14 (s, 3H), 2.03 (br s, 2H), 1.75 - 1.61 (m, 6H), 1.58 - 1.41 (m, 6H).

### Test Example 1. Senescent Cell Activity Data

This test example adopted the human non-small cell lung cancer cell line (A549 cells). Senescent cells above were obtained by induction with etoposide (10 µM). The effect of the compound of interest on the viability of senescent cells was detected, so as to evaluate the specific killing effect of the compound on senescent cells.

Briefly, senescent cells were selected and plated (96-well plate). After adding the compound (at a concentration of 1 µM) and incubating for 72 h, they were added to the CellTiter-Glo^{®} Luminescent Cell Viability Assay kit (Promega) to determine cell viability. The results are shown in **Table 7. Table 7** summarizes the killing activity (IC₅₀) of the test compounds against senescent cells.

**Table 7**

| Compound ID | Structure | IC₅₀ (µM, senescent cells) |
|---|---|---|
| **RN026** | 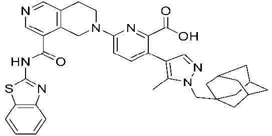 | B |
| **RN001** | 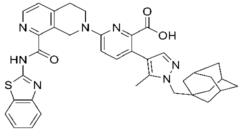 | A |
| **RN027** | 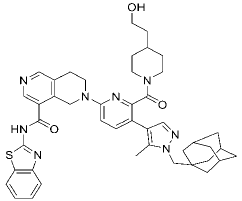 | B |
| **RN008** | 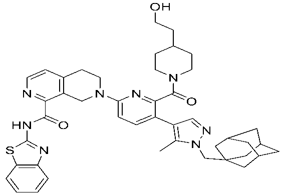 | A |
| **RN002** | 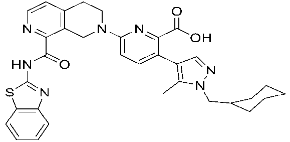 | A |
| **RN003** | 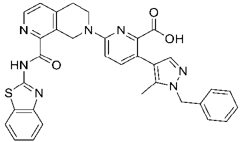 | A |
| **RN004** | 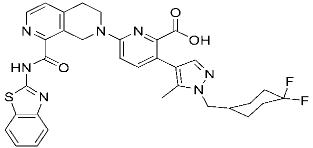 | A |
| **RN005** | 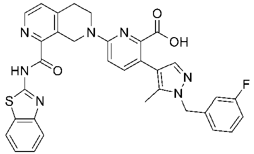 | A |
| **RN009** | 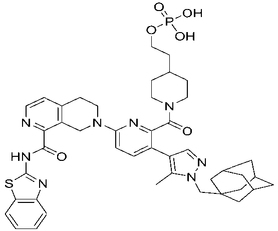 | A |
| **RN018** | 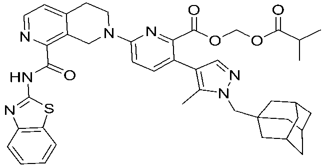 | A |
| **RN030** | 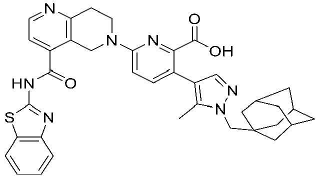 | C |
| **RN006** | 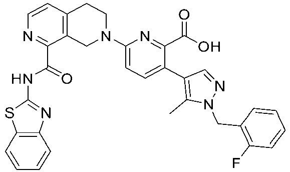 | B |
| **RN007** | 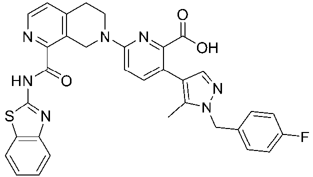 | A |
| **RN011** | 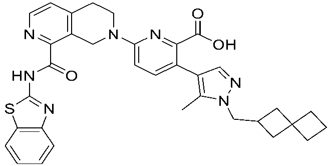 | B |
| **RN012** | 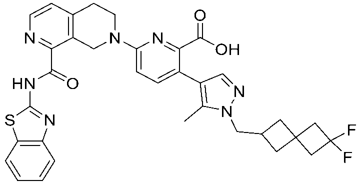 | B |
| **RN014** | 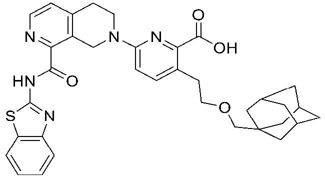 | C |
| **RN017** | 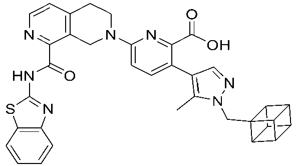 | B |
| **RN019** | 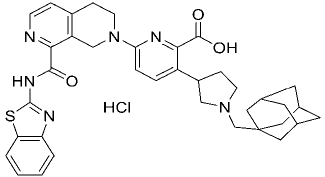 | C |
| **RN020** | 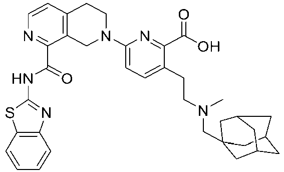 | C |
| **RN021** | 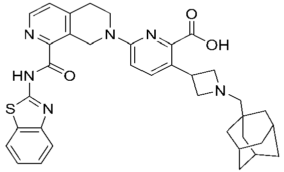 | C |
| RN022 | 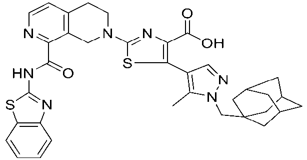 | C |
| RN024 | 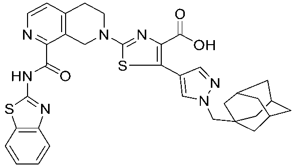 | C |
| RN031 | 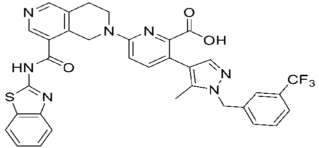 | C |
| **RN032** | 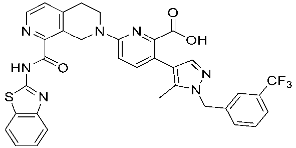 | A |
| **RN033** | 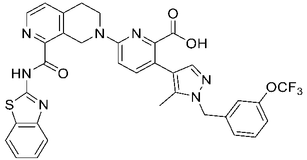 | A |
| **RN034** | 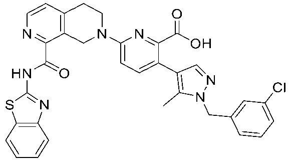 | A |
| **RN035** | 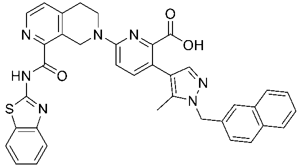 | B |
| **RN036** | 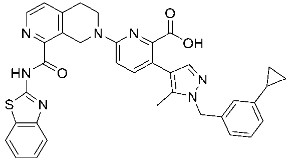 | B |
| **RN037** | 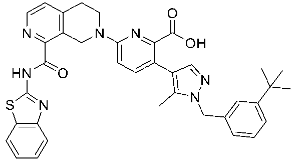 | B |
| **RN038** | 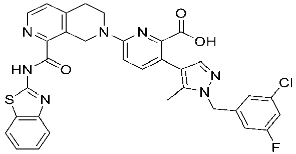 | A |
| **RN039** | 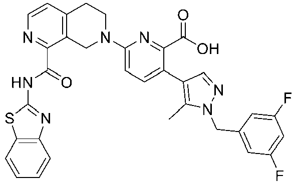 | A |
| **RN040** | 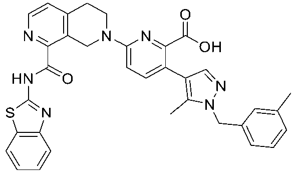 | A |
| **RN041** | 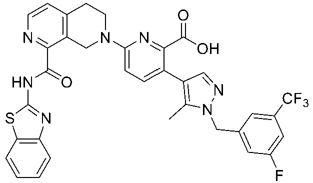 | A |
| **RN044** | 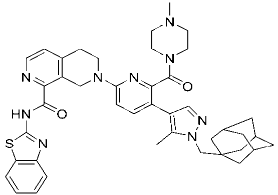 | B |
| **RN048** | 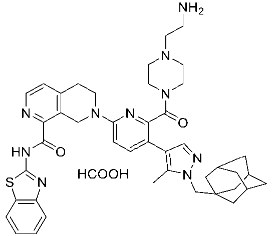 | B |
| **RN049** | 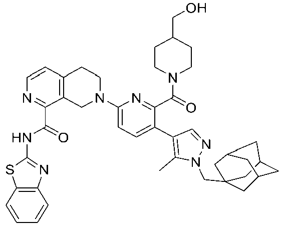 | A |
| **RN050** | 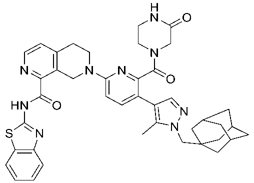 | A |
| **RN051** | 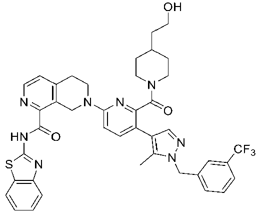 | B |
| **RN052** | 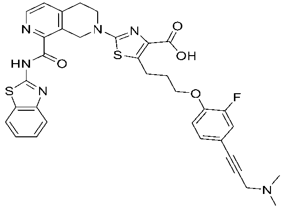 | A |
| **RN053** | 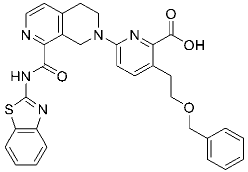 | C |
| **RN054** | 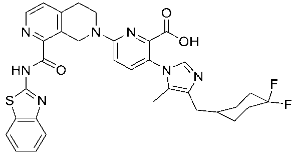 | B |
| **RN055** | 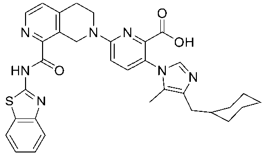 | B |
| **RN056** | 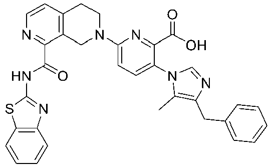 | C |
| **RN057** | 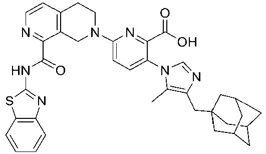 | A |
| **RN061** | 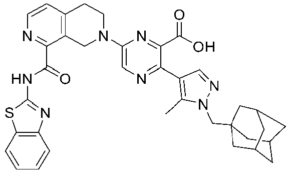 | A |
| **RN062** | 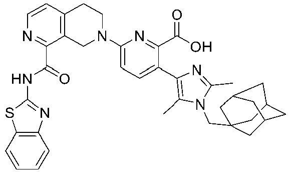 | B |
| **RN072** | 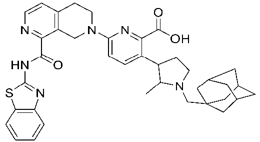 | C |
| **RN073** | 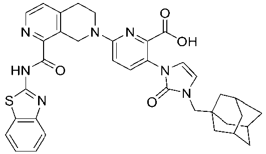 | C |
| **RN074** | 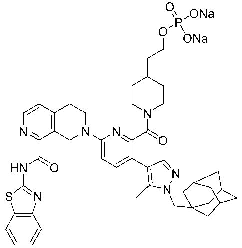 | A |
| **RN075** | 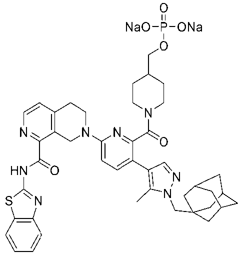 | A |
| **RN076** | 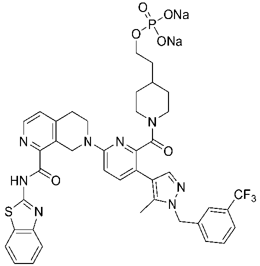 | A |
| **RN077** | 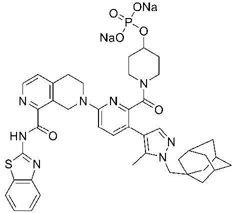 | A |
| **RN078** | 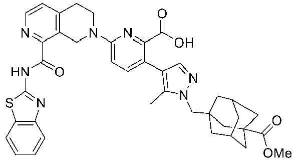 | A |

| | | |
|---|---|---|
| Note: In the table, A stands for less than 0.2 µM; B stands for: 0.2-1.0 µM; C stands for: 1-10 µM. | | |

### Test Example 2. SPR data for the target proteins BCL-2 and BCL-XL

To investigate the influence of the nitrogen heterocyclic compound of Formula I of the present application on the affinity and selectivity toward target proteins, SPR experiments were performed.

SPR measurements were carried out on a Biacore 8K instrument (GE Healthcare). At 25 °C, immobilization of the target proteins was performed on an NTA chip (Catalog BR100532). The immobilization buffer contained 50 mM Tris-HCl, 150 mM NaCl, pH 8.0, 0.005% Tween-20, and 1 mM TCEP. Target proteins (BCL-2 at 10 µg/mL, BCL-XL at 20 µg/mL) were thawed and diluted in the immobilization buffer. Parameters were set in the "Ligand" with contact time = 200 s and flow rate = 2 µL/min; BCL-2 and BCL-XL proteins were covalently coupled to a density of 2000-4000 RU (response units). All SPR kinetic assays were performed at 25 °C. The test buffer contained 50 mM Tris-HCl, 150 mM NaCl, pH 8.0, 0.005% Tween-20, 1 mM TCEP, and 5% DMSO. Click "1. Method", set parameters in "Startup", select "Wash 1", and use the assay buffer as the solution. Parameters were set in "Single Cycle Kinetics"; the analyte was selected with contact time = 70 s, dissociation time = 1800 s, and flow rate = 30 µL/min. Ligand immobilization channels were selected, and the gradient concentrations of analytes as well as plate positions were defined. Prior to data analysis using Biacore 8K evaluation software, background from reference channels and blanks were subtracted from the raw data. A 1: 1 interaction model was used to fit the sensorgrams recorded at different compound concentrations in the single-cycle experiments.

The test results are shown in Table **8.** It can be seen that, compared with compound **A-1331852** (CAS No. 1430844-80-6), the inhibitors described in the present application exhibit higher affinity and better selectivity toward the target protein BCL-XL.

**Table 8**

| Compound ID | KD (M) | |
|---|---|---|
| | Bcl-xL | Bcl-2 |
| A-1331852 | 1.05E-10 | 2.99E-08 |
| **RN001** | 1.77E-11 | 2.63E-08 |

### Test Example 3. Kinetic solubility data

15 µL of 10 mM compound stock solution was added to 485 µL of 100 mM phosphate-buffered saline (PBS). The mixture was shaken at 1100 rpm for 1 hour at room temperature. The solution was filtered, followed by determination of the concentration of the compound in the resulting filtrate/supernatant by LC-MS/MS. Compound concentrations were calculated by comparison with a standard calibration curve.

The test results, as shown in Table **9,** show that the kinetic solubility of the inhibitors described in the present application is significantly improved (i.e., the values are elevated) compared to compound **A-1331852.**

**Table 9**

| **Compound ID** | **Kinetic solubility (µM)** |
|---|---|
| Progesterone | 14.00 |
| Diclofenac | 299.71 |
| **RN026** | 2.07 |
| **RN002** | 2.57 |
| **RN003** | 7.81 |
| **RN074** | 30.00 |
| **A-1331852** | 0.021 |

### Test Example 4. Efficacy in an animal model of hyperoxia-induced retinopathy

The effect of compound **RN001** was investigated in an oxygen-induced retinopathy (OIR) mouse model, which serves as an *in vivo* model indicative of retinopathy of prematurity (ROP), diabetic retinopathy, and wet age-related macular degeneration to a certain extent.

From postnatal day 7 (P7) to postnatal day 12 (P12), C57Bl/6 mouse pups and their CD1 foster dams were exposed to a hyperoxic environment (75% O₂). On P12, animals were intravitreally injected with 1 µL of test composition (compound concentration: 200 µM) formulated in 1% DMSO, 10% Tween 80, and 20% PEG-400, and then returned to ambient air until P17. Eyeballs were enucleated on P17, and retinas were dissected for vascular staining. To quantify the avascular area and neovascular area, retinas were flattened and stained with isolectin B4 (IB4).

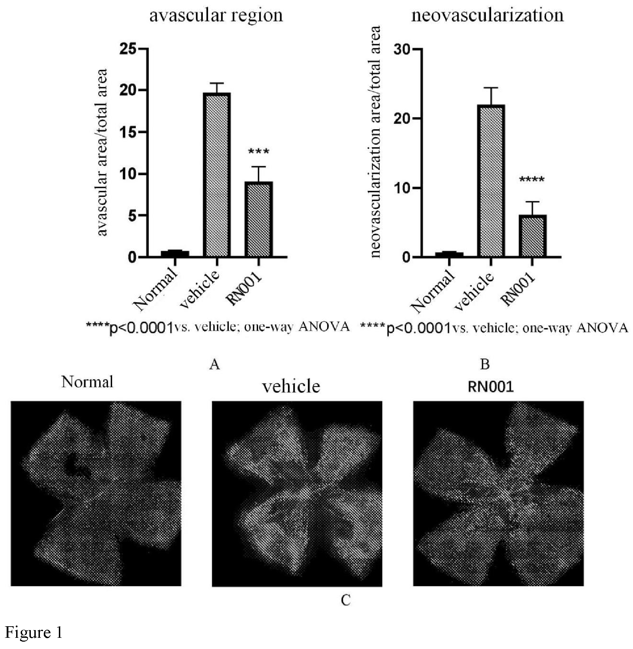

Figure 1 shows that intravitreal (IVT) administration of **RN001** leads to a statistically significant improvement in avascular area and neovascularization.

### Test Example 5. Efficacy in an animal model of topical imiquimod-induced psoriasis

The efficacy of compound **RN001** was investigated in an imiquimod-induced psoriasis mouse model, which is indicative of aging-related skin diseases to a certain extent.

Healthy male Balb/c mice were taken and depilated at the middle of the back over an area of 2 × 3 cm². On the next day, animals without skin damage after depilation were selected for grouping and model establishment. The normal control group received no special treatment. Except for the normal control group, all other animals were modeled by topical application of 5% imiquimod (Sichuan Mingxin Pharmaceutical Co., Ltd.) with a brush for 6 consecutive days. In addition to imiquimod-induced modeling, the drug administration group was further treated with daily topical application of compound **RN001** at a dose of 1 mg/kg. On the seventh day, after photography and erythema and scaling scoring, materials were collected, fixed, paraffin-embedded, and stained with HE. The success of the model and the improvement after administration were evaluated by comparison with the normal control group.

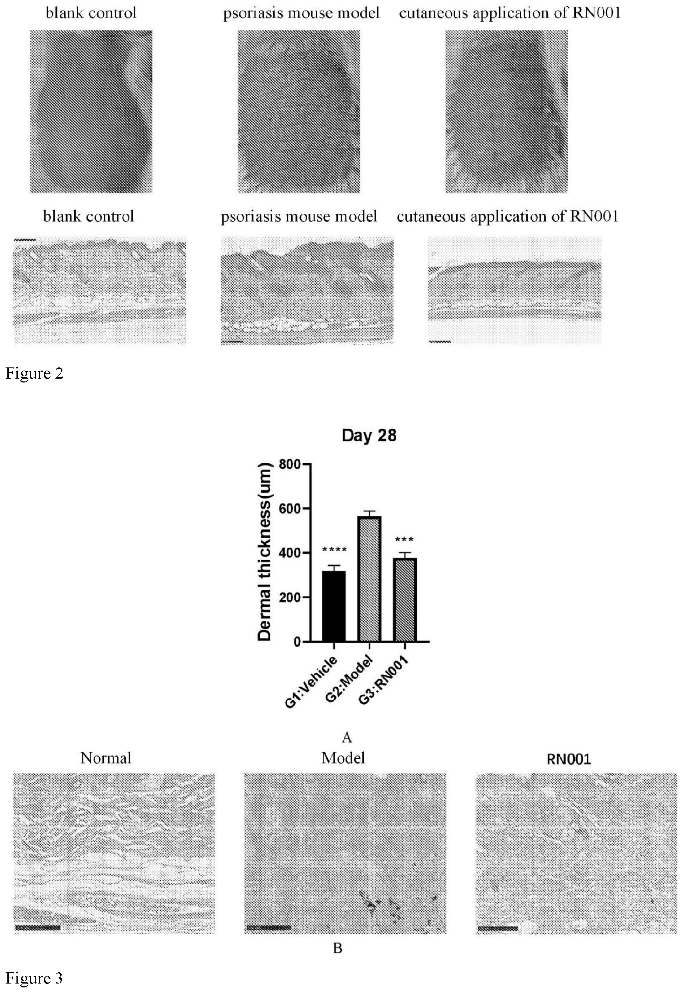

Figure 2 shows that cutaneous application of **RN001** reduced erythema and scaling scores and decreased epidermal thickness in HE staining.

### Test Example 6. Efficacy in an animal model of bleomycin-induced scleroderma

The effect of compound **RN001** was investigated in a bleomycin-induced scleroderma model, wherein the scleroderma is localized scleroderma or diffuse scleroderma.

Male C57BL/6J mice were taken and depilated on the middle of the back. On the next day, animals without skin damage after depilation were selected for grouping and model establishment. The normal control group received no special treatment. Except for the normal control group, all other animals were subcutaneously injected with 0.075 mg bleomycin on the back for 4 consecutive weeks. In addition to subcutaneous injection of bleomycin for modeling, the drug administration group was further treated with daily topical application of compound **RN001** at a dose of 1 mg/kg. Four weeks after the initiation of modeling, skins were collected, fixed, paraffin-embedded, and subjected to HE and Masson staining. The stained sections were scanned and analyzed for changes in dermal thickness and collagen fiber content of skin tissue.

Figure 3 shows that cutaneous application of **RN001** significantly improved dermal thickness and alleviated collagen fiber deposition in the skin as indicated by Masson staining.

### Test Example 7. Efficacy in an Animal Model of Idiopathic Pulmonary Fibrosis

In this example, an animal model of idiopathic pulmonary fibrosis (IPF) was adopted to test the therapeutic effect of the compound of the present disclosure on idiopathic pulmonary fibrosis.

Briefly, 6-week-old anesthetized SD rats were fixed on a foam board with medical tape. The skin over the trachea was incised with surgical scissors to fully expose the trachea. A 100 µL microsyringe loaded with a certain volume of bleomycin (BLM, 5 mg/kg) solution according to the rat body weight was inserted into the trachea via the oral cavity for administration. Immediately after BLM administration, the foam board was rotated and shaken to distribute the BLM solution evenly in the lung tissues, and then the skin over the trachea was sutured with surgical needle and thread. Starting from day 7 after modeling, the solution of the compound of the present disclosure was administered by intraperitoneal (IP) injection (5 mg/kg) according to the rat body weight for 3 consecutive weeks. On day 28, the rats were euthanized with carbon dioxide. Lung tissues were harvested; the left alveolar lavage fluid was collected for white blood cell (WBC) count. Part of the right lung tissue was subjected to hydroxyproline (HYP) detection, and the remaining lung tissue was used for pathological H&E staining and Masson staining.

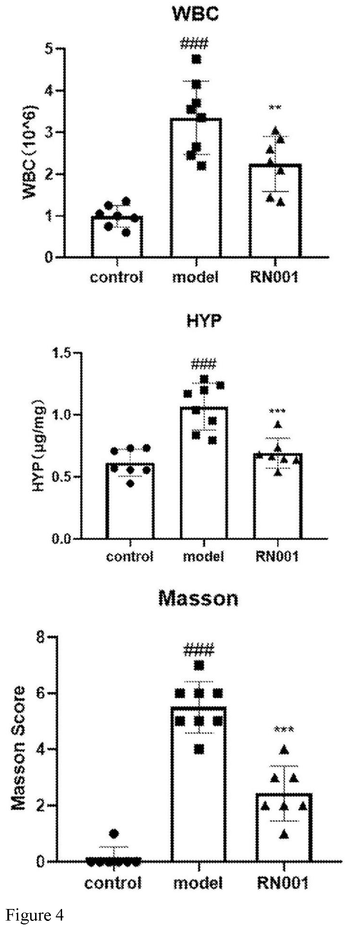

Figure 4 shows that intraperitoneal injection (IP) administration of **RN001** resulted in statistically significant improvements in WBC, HYP, and pathology scores on the IPF model.

### Test Example 8. Efficacy in an animal model of osteoarthritis

In this example, an animal model of osteoarthritis was adopted to test the effect of the compound of the present disclosure on osteoarthritis.

The osteoarthritis animal model was established by the following steps. Mice were anesthetized with isoflurane and fixed. The hair on the right hind limb of mice was completely shaved, the skin was disinfected with alcohol, and the skin at the joint site was cut open. Under an optical microscope, the medial side of the flexed right knee joint was longitudinally cut with a blade without severing the patellar ligament. The patellar ligament was pulled aside, and sterile cotton swabs were continuously used for compression hemostasis to fully expose the articular cavity. Normal saline was added in a timely manner to prevent drying of the articular cavity. Excess muscle tissue was gently separated with blunt forceps to expose the meniscal ligament, and the anterior cruciate ligament was located deep in the articular cavity and cut with microsurgical scissors. The wound surface was disinfected with penicillin solution, muscle tissue was sutured with sterilized needle and thread, and skin tissue was sutured finally. Drug efficacy was evaluated by intra-articular injection administration. At the experimental endpoint, the plantar mechanical pain threshold was measured, and joint tissue was collected for Safranin-O/fast green staining.

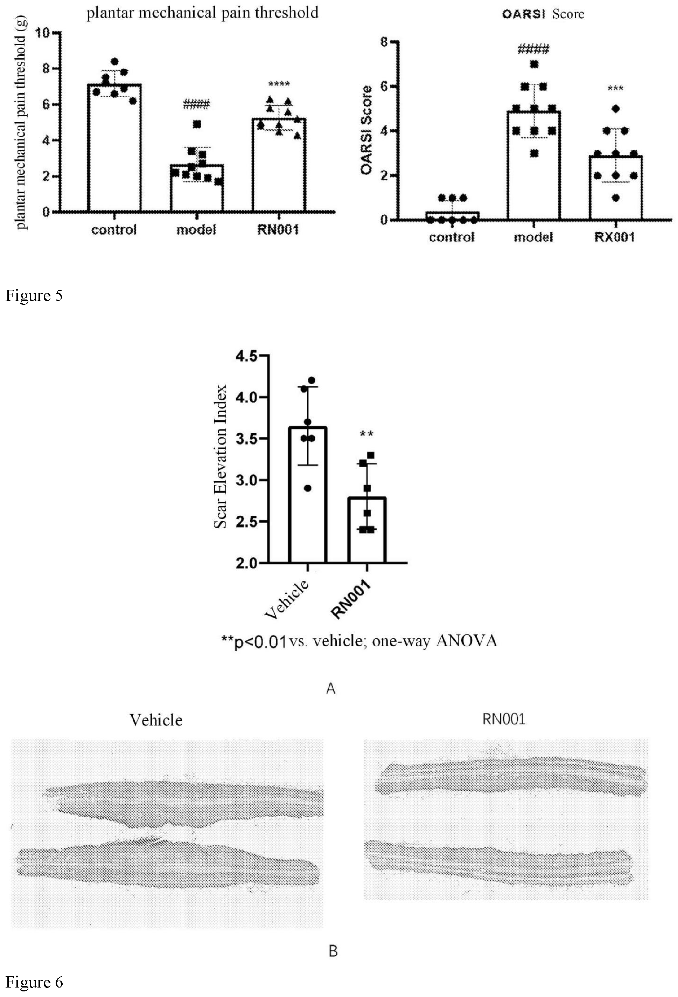

Figure 5 shows that intra-articular injection of **RN001** resulted in statistically significant improvements in plantar mechanical pain threshold and pathological score of Safranin O/Fast Green staining in the osteoarthritis (OA) model.

### Test Example 9. Efficacy in rabbit ear hypertrophic scar model

In this example, a rabbit ear hypertrophic scar animal model was used to test the effect of the compound of the present disclosure on hypertrophic scars. Briefly, six 3-month-old male New Zealand white rabbits were selected. After the New Zealand rabbits were anesthetized with pentobarbital sodium, four circular full-thickness wounds with a diameter of 10 mm were made on the ventral surface of each ear by removing the epidermis, dermis and perichondrium until the cartilage was exposed. The rabbits were randomly divided into a control group and an RN001 experimental group. On day 14 after surgery, after the wounds were completely re-epithelialized, a compound solution (150 µM, 100 µL, 10% DMSO + 90% normal saline) or a negative control solution (100 µL, 10% DMSO + 90% normal saline) was taken and injected from the wound margin into the center of each lesion. The injection was performed once a week for four times in total. The improvement effect of the drug on scar formation was evaluated by SEI (Scar Elevation Index).

Figure 6 shows that administration of **RN001** resulted in a statistically significant improvement in the Scar Elevation Index in the rabbit ear hypertrophic scar model.

## Claims

1. A nitrogen heterocyclic compound, or a pharmaceutically acceptable salt, a solvate, a hydrate, a polymorph, a co-crystal, a tautomer, a stereoisomer, or an isotopic compound thereof, wherein the nitrogen heterocyclic compound has the structure shown by Formula I: 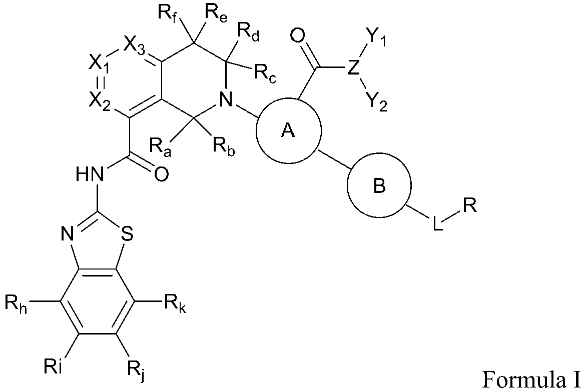 wherein:
X₁, X₂ and X₃ are each independently selected from CR_{g} and N, and X₁, X₂ and X₃ are not CR_{g} at the same time;
Rₐ, R_{b}, R_{c}, R_{d}, Rₑ, R_{f}, R_{g}, Rₕ, Rᵢ, Rⱼ and Rₖ are each independently selected from hydrogen, deuterium, halogen-substituted or unsubstituted C1-C8 alkyl, halogen-substituted or unsubstituted C3-C8 cycloalkyl, halogen-substituted or unsubstituted C2-C8 alkenyl, halogen-substituted or unsubstituted C2-C8 alkynyl, halogen atom, hydroxy, amino, nitro, cyano, carboxy, acyl, and halogen-substituted or unsubstituted C2-C8 alkoxy;
Z is selected from O and N;
when Z is O, Y₂ is absent, and Y₁ is independently selected from hydrogen, deuterium, substituted or unsubstituted C1-C10 alkyl, substituted or unsubstituted C3-C10 cycloalkyl, -R₁-R₂-R₃, -R₂-R₃ and -R₁-R₃, wherein R₁ is selected from substituted or unsubstituted C1-C10 alkylene, R₂ is selected from -O-C(O)- and -O-C(O)-O-, and R₃ is selected from substituted or unsubstituted C1-C10 alkyl, substituted or unsubstituted C3-C20 cycloalkyl, substituted or unsubstituted C2-C20 alkenyl, substituted or unsubstituted C2-C20 alkynyl, substituted or unsubstituted C6-C20 aryl, substituted or unsubstituted C1-C20 heteroaryl, and substituted or unsubstituted C2-C20 heteroalicyclic group;
when Z is N, Y₁ and Y₂ are each independently selected from hydrogen, deuterium, C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl and C2-C8 alkynyl may be replaced by one or two or more groups selected from -O-, -S-, -SO₂-, - C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are optionally substituted by one or two or more substituents selected from halogen atom, cyano, nitro, C6-C20 aryl, C1-C20 heteroaryl, C1-C10 alkoxy, C6-C20 aryloxy, C2-C20 heteroalicyclic group, amino, hydroxy, mercapto, 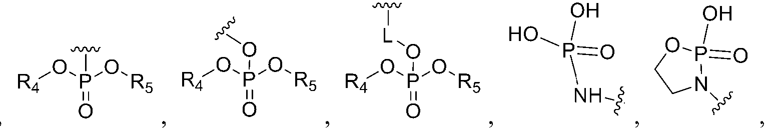 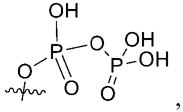 and -NR₄R₅; or
Y₁ and Y₂ together with the N atoms to which they are attached form a heteroalicyclic group, preferably a C2-C20 heteroalicyclic group, wherein the heteroalicyclic group is optionally substituted with one or two or more substituents of halogen atom, cyano, nitro, C6-C20 aryl, C1-C20 heteroaryl, C1-C10 chain alkoxy, C6-C20 aryloxy, C2-C20 heteroalicyclic group, amino, hydroxy, mercapto, 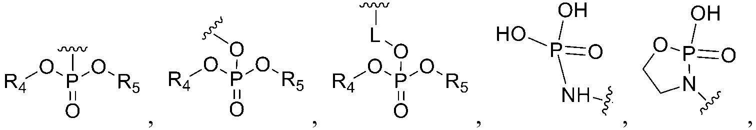 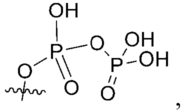 and -NR₄R₅; or
Y₁ is hydrogen or deuterium, and Y₂ is selected from C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl and C2-C8 alkynyl may be replaced by one or two or more groups selected from -O-, -S-, -SO₂-, -C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C2-C8 alkenyl, or C2-C8 alkynyl, and wherein the substituents of at least two positions together form an aliphatic ring, a heteroalicyclic ring, an aromatic ring or a heteroaromatic ring;
R₄ and R₅ are independently selected from hydrogen, deuterium, aryl, heteroaryl, C1-C8 alkyl, C3-C8 cycloalkyl, C2-C8 alkenyl, and C2-C8 alkynyl, wherein the aryl and heteroaryl are optionally substituted with halogen atom, hydroxy, mercapto, amino, nitro, cyano, carboxy, acyl, alkoxy, aryl, heteroaryl, heteroalicyclic group, C1-C10 alkyl, C3-C8 cycloalkyl, C2-C8 alkenyl, or C2-C8 alkynyl, and wherein optionally, the substituents of at least two positions together form an aliphatic ring, a heteroalicyclic ring, an aromatic ring or a heteroaromatic ring;
ring A is independently selected from substituted or unsubstituted aromatic ring or heteroaromatic ring;
ring B is absent or independently selected from substituted or unsubstituted aromatic ring or heteroaromatic ring, C3-C8 cycloalkyl, C3-C8 heterocycloalkyl, C6-C10 spiro-ring, C6-C10 spiro-heterocycle, C6-C10 fused cycle, and C6-C10 fused heterocycle;
L is absent or selected from C1-C6 alkylene, -C1-C6 alkylene-O-, and -C1-C3 alkylene-O-Cl-C3 alkylene-, wherein the alkylene is optionally substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C3-C8 cycloalkyl, C2-C8 alkenyl or C2-C8 alkynyl; and
R is independently selected from aromatic ring, heteroaromatic ring, aromatic ring-fused heteroaromatic ring, C3-C8 cycloalkyl, C3-C8 heterocycloalkyl, C6-C14 spiro-ring, and C6-C14 fused cycloalkyl; wherein the R is optionally substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C3-C8 cycloalkyl, C2-C8 alkenyl or C2-C8 alkynyl.

2. The compound according to claim 1, **characterized in that**,
X₃ is CR_{g};
or, X₃ is N, and X₁ and X₂ are CR_{g};
or, X₁ is N, and X₂ and X₃ are CR_{g};
or, X₂ is N, and X₁ and X₃ are CR_{g}.

3. The compound according to claim 1 or 2, **characterized in that**, Rₐ, R_{b}, R_{c}, R_{d}, Rₑ, R_{f}, R_{g}, Rₕ, Rᵢ, Rⱼ and Rₖ are each independently selected from hydrogen, deuterium, halogen-substituted or unsubstituted C1-C6 alkyl, halogen-substituted or unsubstituted C3-C6 cycloalkyl, halogen-substituted or unsubstituted C2-C6 alkenyl, halogen-substituted or unsubstituted C2-C6 alkynyl, halogen atom, hydroxy, amino, nitro, cyano, carboxy, and halogen-substituted or unsubstituted C2-C6 alkoxy;
preferably, Rₐ, R_{b}, R_{c}, R_{d}, R_{f}, R_{g}, Rₕ, Rᵢ, Rⱼ and Rₖ are each independently selected from hydrogen, deuterium, halogen-substituted or unsubstituted C1-C6 alkyl, halogen-substituted or unsubstituted C3-C6 cycloalkyl, halogen atom, and C2-C6 alkoxy;
preferably, Rₐ, R_{b}, R_{c}, R_{d}, Rₑ, R_{f}, R_{g}, Rₕ, Rᵢ, Rⱼ, and Rₖ are hydrogen.

4. The compound according to any one of claims 1 to 3, **characterized in that**, Z is O, Y₂ is absent, and Y₁ is selected from hydrogen, deuterium, substituted or unsubstituted C1-C10 alkyl, substituted or unsubstituted C3-C10 cycloalkyl, -R₁-R₂-R₃, -R₂-R₃ and -R₁-R₃, wherein R₁ is selected from substituted or unsubstituted C1-C10 alkylene, R₂ is selected from -O-C(O)- and - O-C(O)-O-, and R₃ is selected from substituted or unsubstituted C1-C10 alkyl, substituted or unsubstituted C3-C20 cycloalkyl, substituted or unsubstituted C2-C20 alkenyl, substituted or unsubstituted C2-C20 alkynyl, substituted or unsubstituted C6-C20 aryl, substituted or unsubstituted C1-C20 heteroaryl, and substituted or unsubstituted C2-C20 heteroalicyclic group.

5. The compound according to any one of claims 1 to 4, **characterized in that**, Z is O, Y₂ is absent, Y₁ is selected from hydrogen and deuterium; or
Z is O, Y₂ is absent, and Y₁ is selected from substituted or unsubstituted C1-C6 alkyl and substituted or unsubstituted C3-C10 cycloalkyl; or
Z is O, Y₂ is absent, and Y₁ is selected from -R₁-R₂-R₃, -R₂-R₃, and -R₁-R₃.

6. The compound according to any one of claims 1 to 5, **characterized in that**, R₁ is selected from substituted or unsubstituted C1-C6 alkylene, preferably substituted or unsubstituted C1-C3 alkylene, and more preferably methylene;
R₃ is selected from substituted or unsubstituted C1-C8 alkyl, substituted or unsubstituted C3-C10 cycloalkyl, substituted or unsubstituted C2-C8 alkenyl, substituted or unsubstituted C2-C8 chain alkynyl, substituted or unsubstituted C6-C12 aryl, substituted or unsubstituted C2-C12 heteroaryl, and substituted or unsubstituted C1-C12 heteroalicyclic group; preferably, R₃ is selected from substituted or unsubstituted C1-C6 alkyl, substituted or unsubstituted C6-C12 aryl, substituted or unsubstituted C1-C10 heteroaryl, substituted or unsubstituted C3-C8 cycloalkyl, and substituted or unsubstituted C1-C10 heteroalicyclic group;
preferably, R₃ is selected from the following groups:

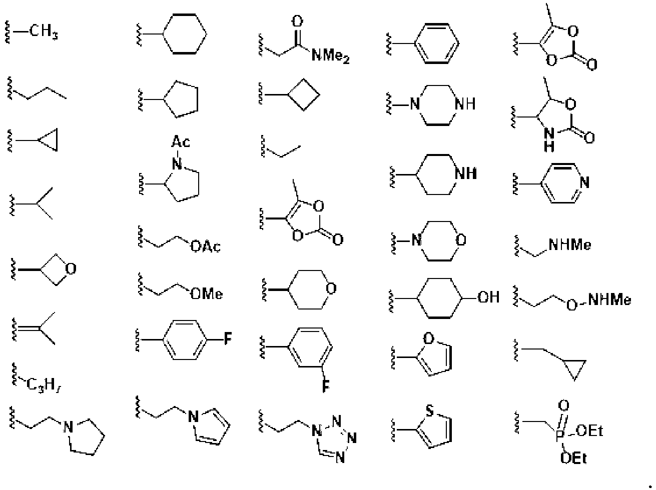

7. The compound according to any one of claims 1 to 6, **characterized in that**, Z is N,
Y₁ and Y₂ are independently selected from C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl, and C2-C8 alkynyl may be replaced by one or two or more groups selected from -O-, -S-, -SO₂-, -C(O)-, and -NR₃-,
the C1-C10 alkyl, C3-C8 cycloalkyl, C2-C8 alkenyl, C2-C8 alkynyl, C6-C20 aryl, C1-C20 heteroaryl, and C2-C20 heteroalicyclic group are optionally substituted with one or two or more substituents of halogen atom, cyano, nitro, C6-C20 aryl, C1-C20 heteroaryl, C1-C10 chain alkoxy, C6-C20 aryloxy, C2-C20 heteroalicyclic group, amino, hydroxy, mercapto, 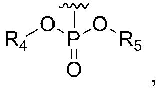 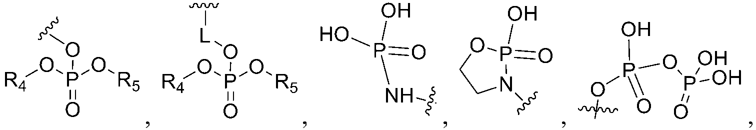 and -NR₄R₅; or
Y₁ and Y₂ together with the N atoms to which they are attached form a heteroalicyclic group, preferably a C2-C20 heteroalicyclic group; or
Y₁ is hydrogen, and Y₂ is selected from C1-C10 alkyl, C3-C8 cycloalkyl, C2-C8 alkenyl, C2-C8 alkynyl, C6-C20 aryl, C1-C20 heteroaryl, and C2-C20 heteroalicyclic group, wherein CH₂ in the C1-C10 alkyl, C3-C8 cycloalkyl, C2-C8 alkenyl and C2-C8 alkynyl may be replaced by one or two or more groups selected from -O-, -S-, -SO₂-, -C(O)- and -NR₃-, and wherein the C1-C10 alkyl, C3-C8 cycloalkyl, C2-C8 alkenyl, C2-C8 alkynyl, C6-C20 aryl, C1-C20 heteroaryl and C2-C20 heteroalicyclic group are substituted with halogen atom, hydroxy, mercapto, amino, nitro, cyano, carboxy, acyl, C1-C10 alkoxy, C6-C20 aryl, C1-C20 heteroaryl, C2-C20 heteroalicyclic group, C1-C10 alkyl, C2-C8 alkenyl, or C2-C8 alkynyl, and wherein the substituents of at least two positions together form an aliphatic ring, a heteroalicyclic ring, an aromatic ring or a heteroaromatic ring.

8. The compound according to any one of claims 1 to 7, **characterized in that**,
Y₁ and Y₂ together with the N atoms to which they are attached form a C2-C20 heteroalicyclic group such as a C2-C10 heteroalicyclic group, wherein the ring of the C2-C20 heteroalicyclic group optionally contains 1 or 2 additional heteroatoms selected from N and O,
the C2-C20 heteroalicyclic group are optionally substituted with one or two or more substituents of halogen atom, cyano, nitro, C6-C10 aryl, C1-C10 heteroaryl, C1-C6 chain alkoxy, C6-C10 aryloxy, C2-C10 heteroalicyclic group, amino, hydroxy, mercapto, carbonyl, carboxy, acyl, 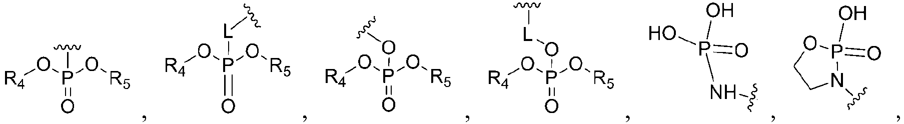 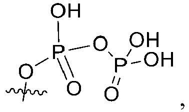 and -NR₄R₅, R₄ and R₅ are independently selected from hydrogen, C6-C10 aryl, C1-C10 heteroaryl, C1-C8 alkyl, C3-C8 cycloalkyl, C2-C8 alkenyl, and C2-C8 alkynyl, wherein the aryl and heteroaryl are optionally substituted with halogen atom, hydroxy, mercapto, amino, nitro, cyano, carboxy, acyl, C1-C8 alkoxy, C6-C10 aryl, C1-C10 heteroaryl, C2-C10 heteroalicyclic group, C1-C8 alkyl, C3-C8 cycloalkyl, C2-C6 alkenyl, or C2-C8 alkynyl, and optionally, wherein the substituents of at least two positions together form a C3-C10 aliphatic ring, a C2-C10 heteroalicyclic ring, a C6-C10 aromatic ring or a C1-C10 heteroaromatic ring;
for example, the C2-C20 heteroalicyclic group are optionally substituted with a substituent selected from halogen atom, hydroxy, mercapto, amino, nitro, cyano, C1-C10 alkoxy, C1-C10 alkyl, C3-C8 cycloalkyl, C2-C8 alkenyl, C2-C8 alkynyl, 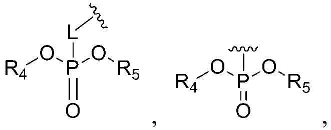 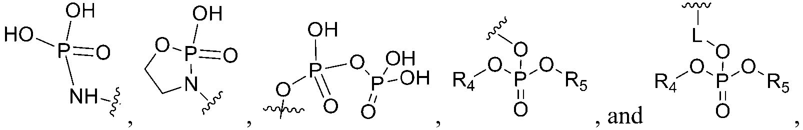 wherein R₄ and R₅ are independently selected from hydrogen and C1-C6 alkyl;
for example, the C2-C8 heteroalicyclic group is optionally substituted with a substituent selected from halogen, -NH₂, -OH, -NO₂, carbonyl, -CH₂OH, carboxy, methyl, ethyl, propyl, isopropyl, methoxy, ethoxy, propoxy, and isopropoxy.

9. The compound according to any one of claims 1 to 8, **characterized in that**,
Y₁ and Y₂ together with the N atoms to which they are attached form a C4-C20 heteroalicyclic group, selected from the groups shown below: 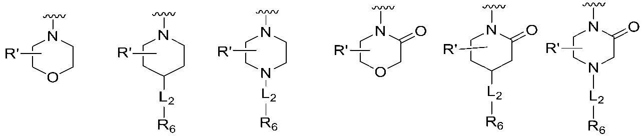 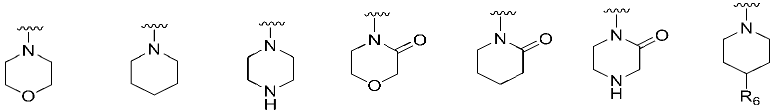 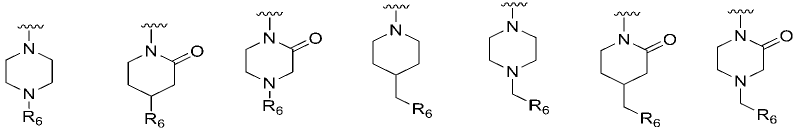 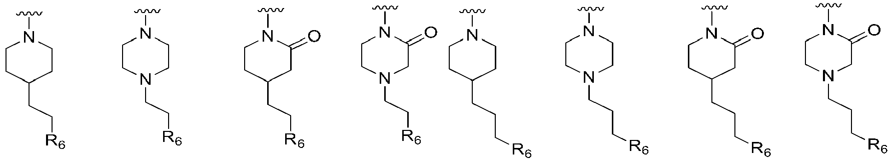
R' each independently represents no substituent, a single substituent or a plurality of substituents, each of the substituents being independently selected from deuterium, hydroxy, halogen, NH₂, carboxy (-COOH), 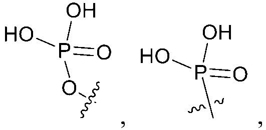 C1-C6 alkyl, halogen-substituted C1-C6 alkyl, hydroxy substituted C1-C6 alkyl, amino-substituted C1-C6 alkyl, morpholino-substituted C1-C6 alkyl, -COO-C1-C6 alkyl, cyano, C1-C6 alkoxy, C3-C6 cycloalkyl, halogen-substituted C3-C6 cycloalkyl, hydroxy-substituted C3-C6 cycloalkyl, phenyl, and benzyl;
L₂ is absent or C1-C6 alkylene, halogen, hydroxy, C1-C6 alkoxy-substituted C1-C6 alkylene, preferably methylene, ethylene, or propylene;
R₆ is H, deuterium, halogen, hydroxy, NH₂, carboxy (-COOH), -CONH₂, sulfo (-SO₃H), -SO₂-C1-C6 alkyl, 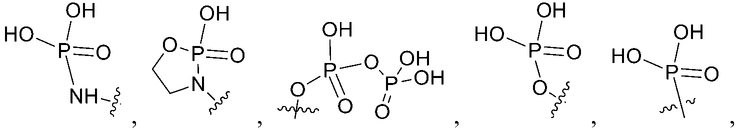 C1-C6 alkyl, halogen-substituted C1-C6 alkyl, morpholino-substituted C1-C6 alkyl, -COO-C1-C6 alkyl, cyano, C1-C6 alkoxy, hydroxy-substituted C1-C6 alkyl, amino-substituted C1-C6 alkyl, C3-C6 cycloalkyl, halogen-substituted C3-C6 cycloalkyl, hydroxy-substituted C3-C6 cycloalkyl, phenyl or benzyl.

10. The compound according to any one of claims 1 to 9, **characterized in that**, ring A is selected from pyridine, pyrimidine, pyridazine, quinoline, thiazole, imidazole, pyrrole, pyrazole, thiophene, thienofuran, thienothiazole, carbazolopyrrole, pyridopyrazole, pyridopyrrole, indole, azaindole, isoquinoline, anthracene, phenanthrene, benzofuran, benzothiophene, and indazole; preferably, ring A is a pyridine ring, a pyrimidine ring, or a pyridazine ring.

11. The compound according to any one of claims 1 to 10, **characterized in that**, the compound has a structure as shown by Formula IA, or as shown by any one of Formulas I-1 to I-9: 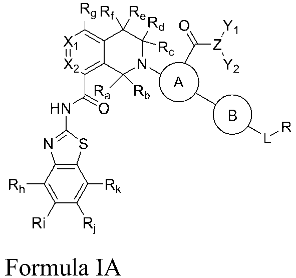 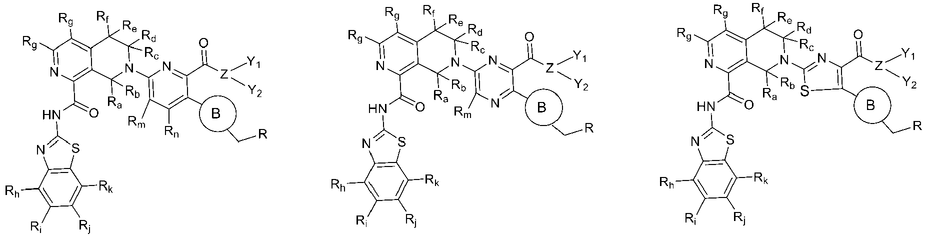 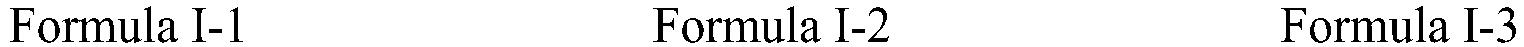 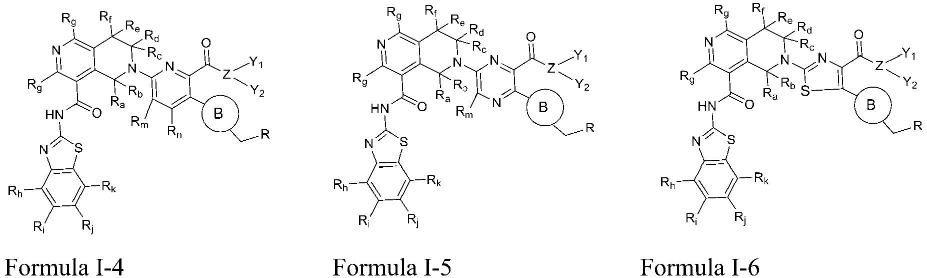 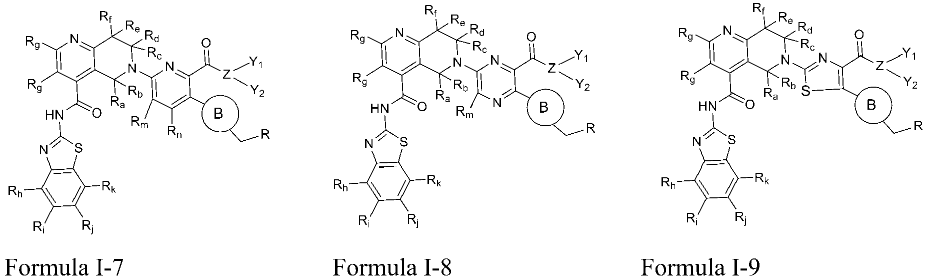 in each of the above formulas, each symbol is as defined in any one of claims 1-11.

12. The compound according to any one of claims 1 to 11, **characterized in that**, ring B is selected from: pyrazole, pyrrole, imidazole, pyridine, pyrimidine, indole, indazole, tetrahydropyrrole, piperidine, azetidine, cubane, pyridazine, quinoline, thiazole, imidazole, pyrrole, pyrazole, thiophene, thienofuran, thienothiazole, carbazolopyrrole, pyridopyrazole, pyridopyrrole, indole, azaindole, isoquinoline, anthracene, phenanthrene, benzofuran, benzothiophene, and indazole; preferably, ring B is selected from the following groups: 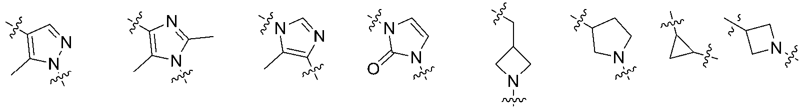 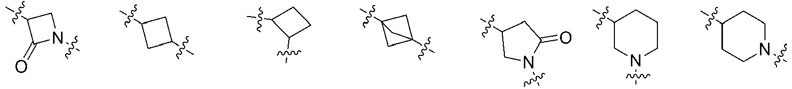 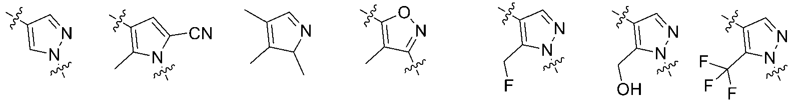 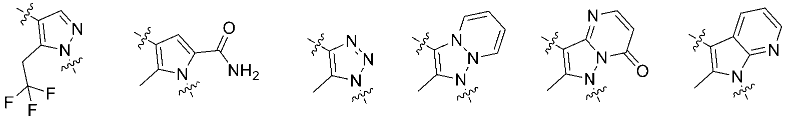 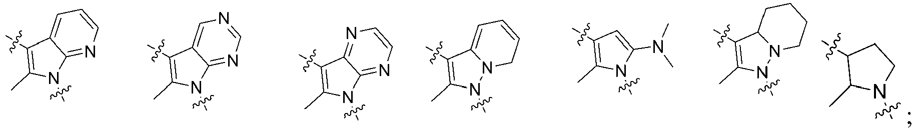 R is selected from phenyl, pyridyl, pyrimidinyl, pyrazinyl, cycloalkyl, quinolinyl, isoquinolinyl, tetrahydroquinolinyl, tetrahydroisoquinolinyl, indolyl, indazolyl, cyclohexyl, cyclopentyl, cycloheptyl, oxaspiro[3.3]heptanyl, spiro[2.5]octanyl, and adamantyl;
preferably, R is selected from the following groups:

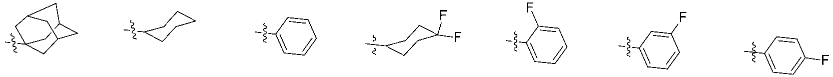

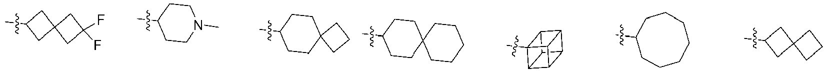

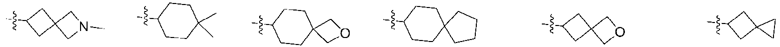

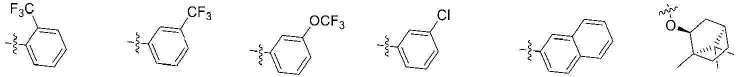

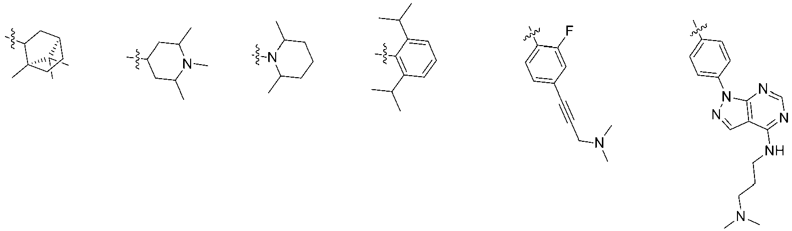

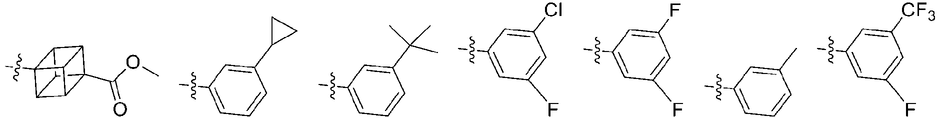

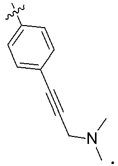

13. The compound according to claim 1, **characterized in that**, the compound or a pharmaceutically acceptable salt thereof is selected from:

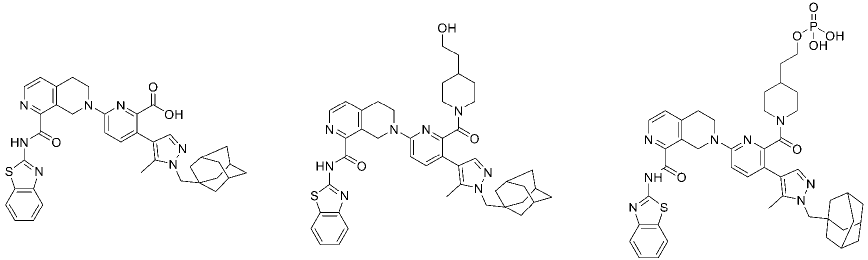

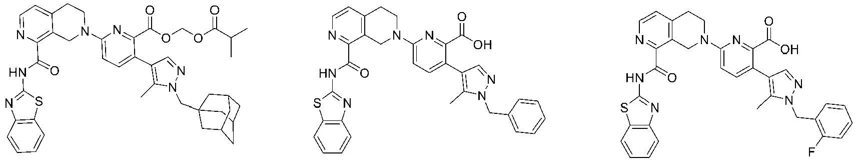

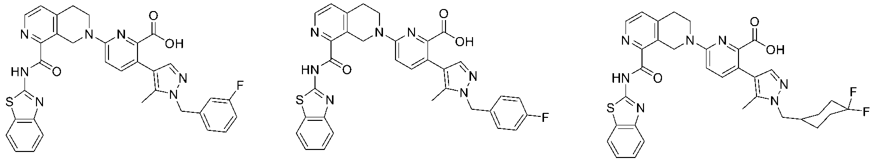

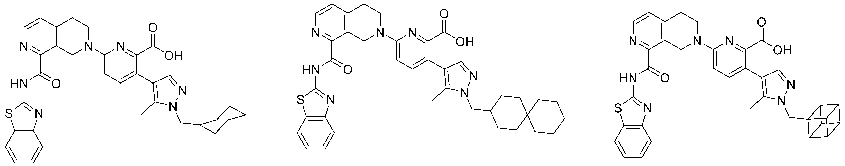

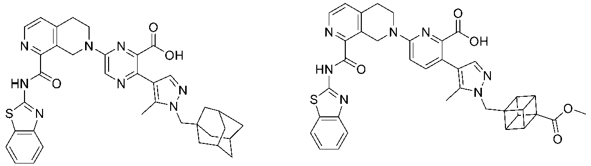

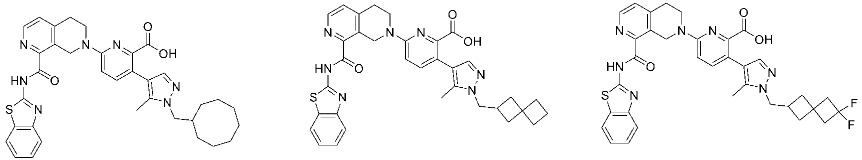

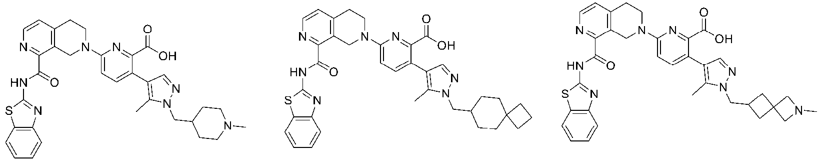

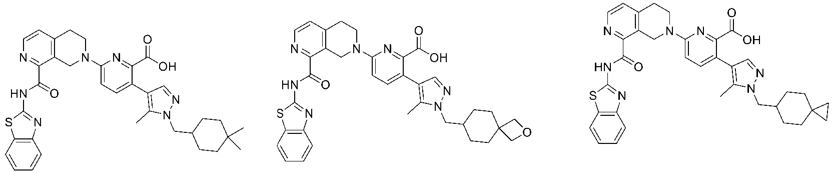

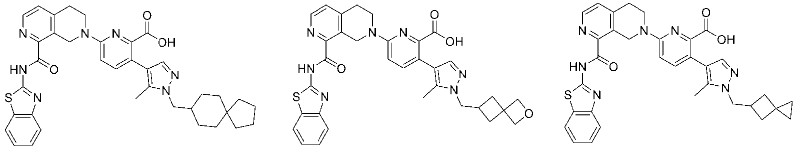

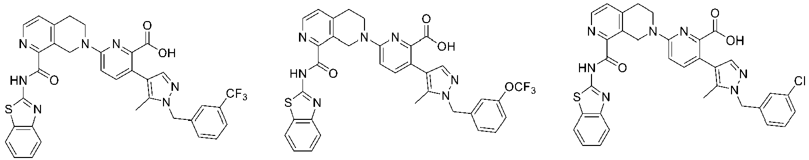

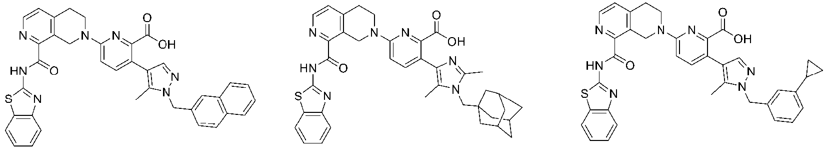

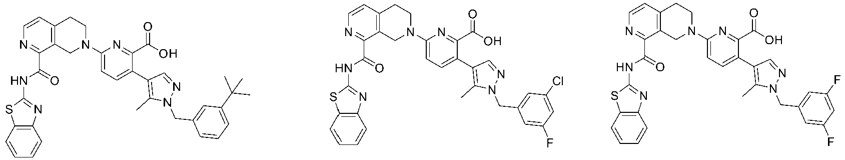

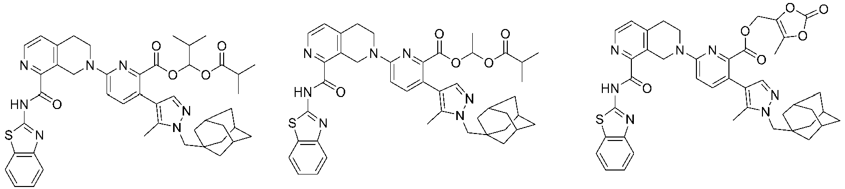

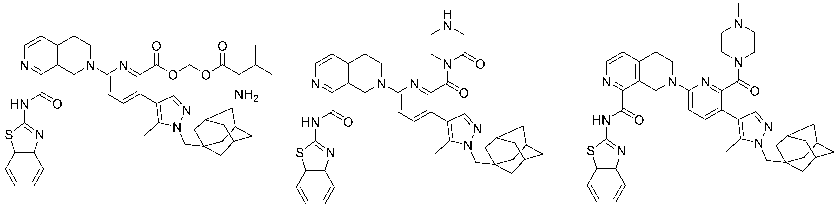

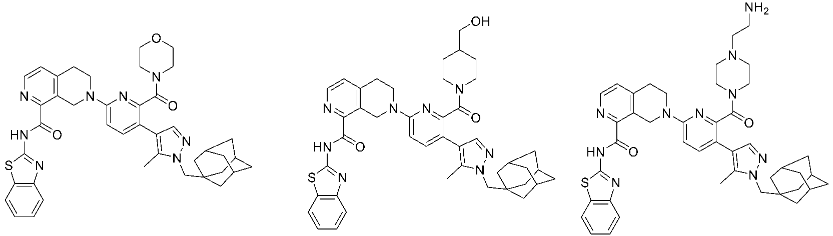

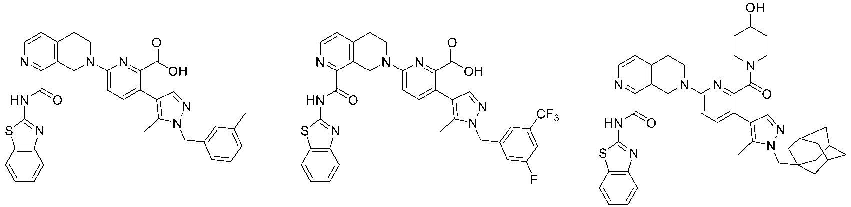

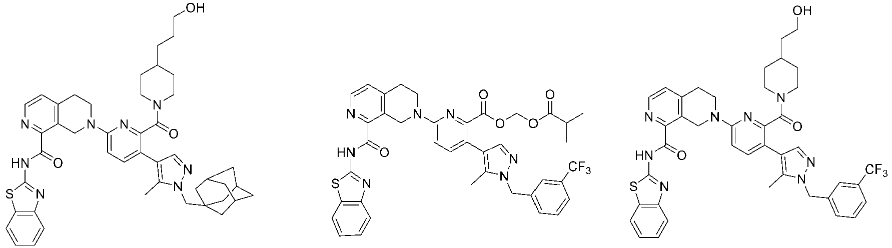

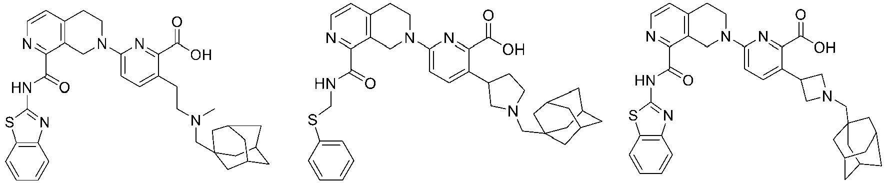

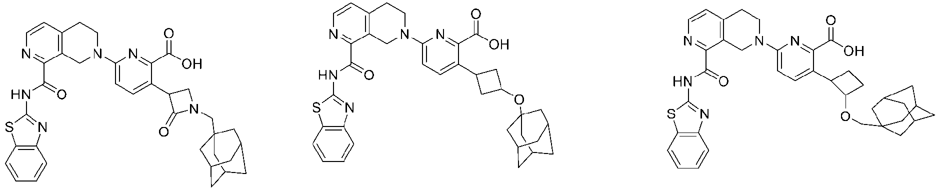

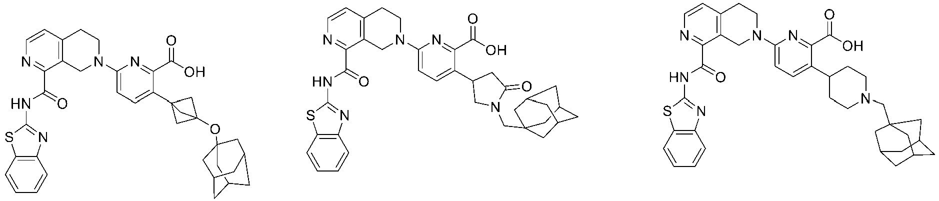

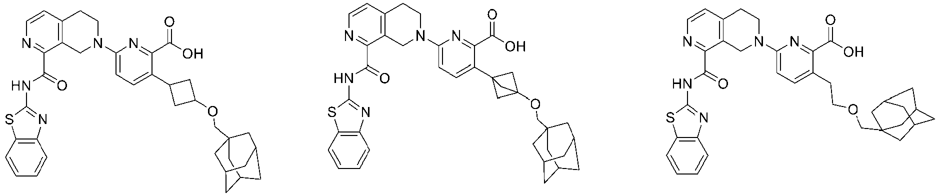

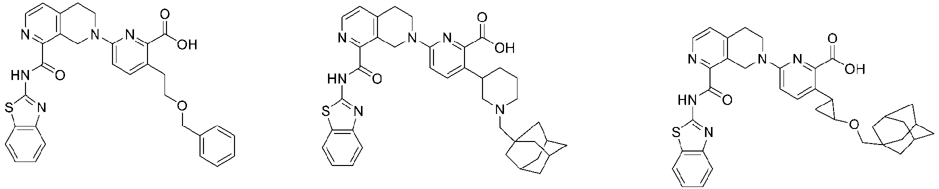

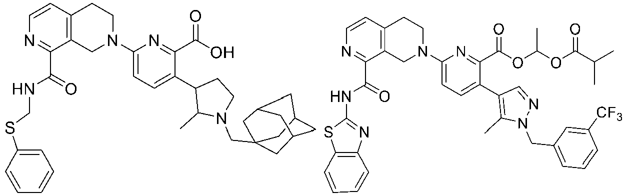

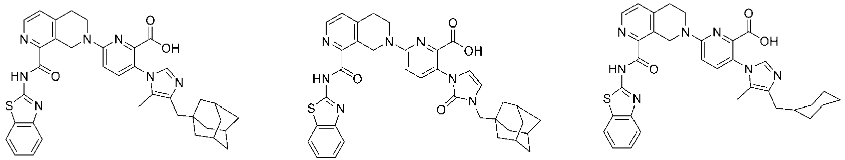

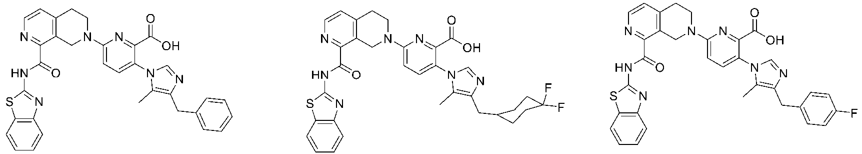

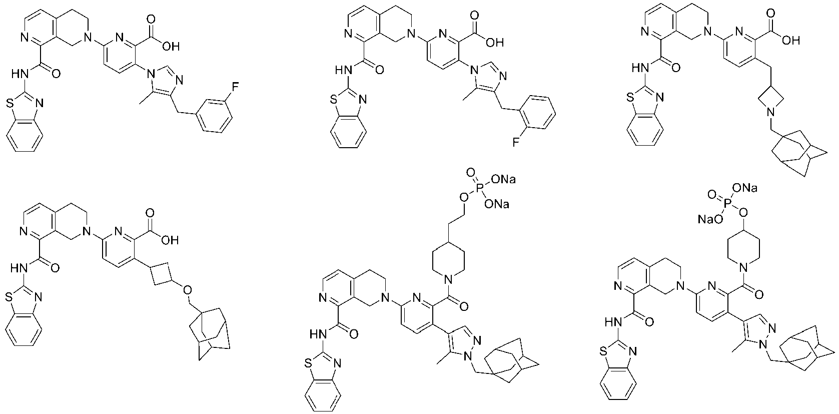

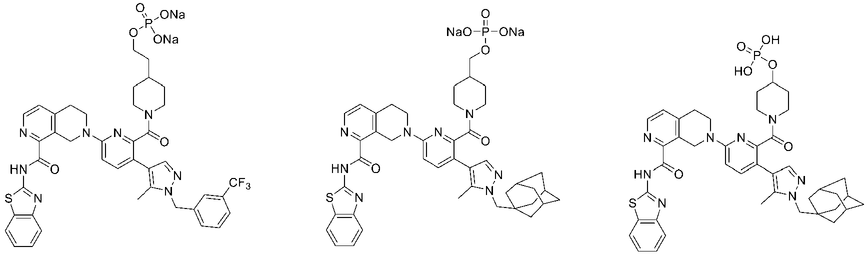

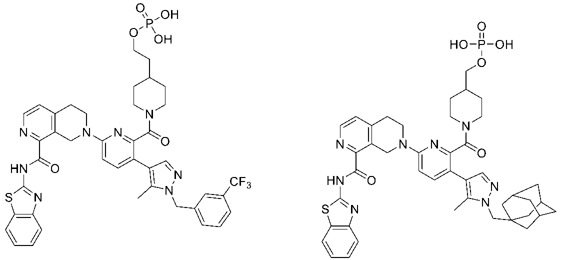

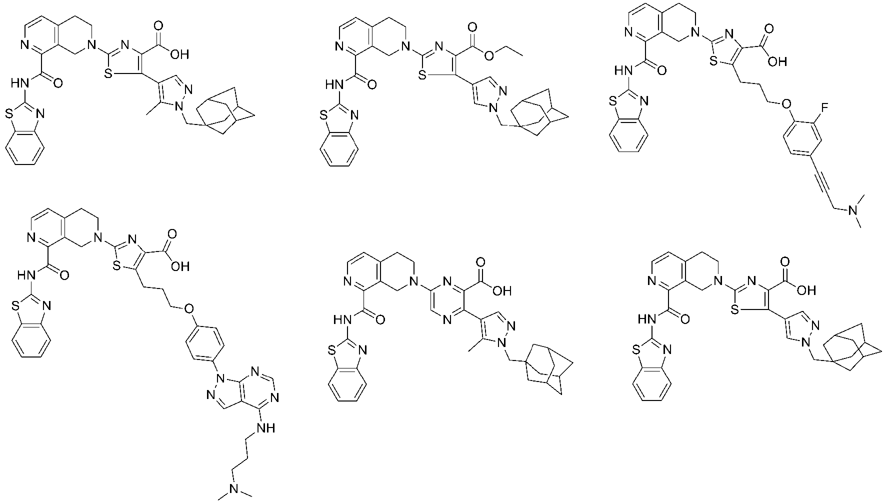

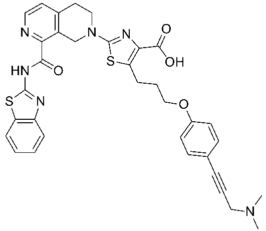

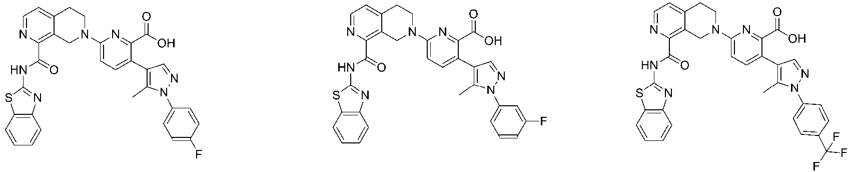

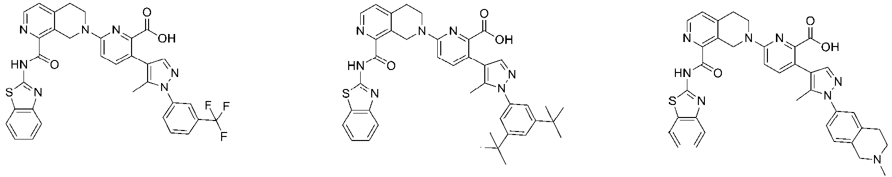

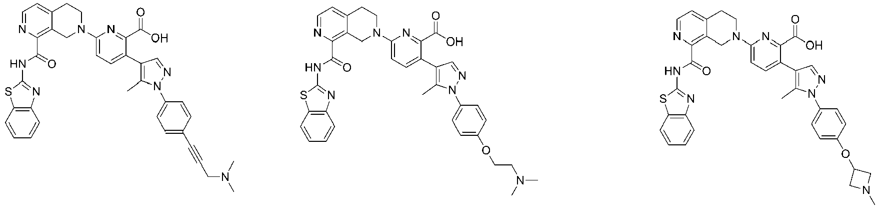

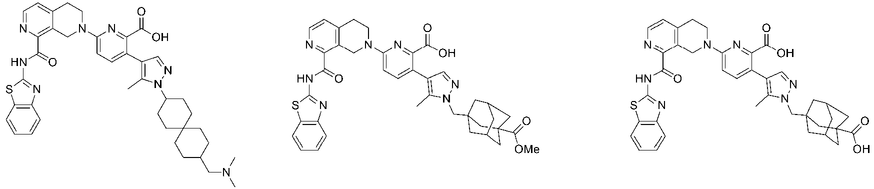

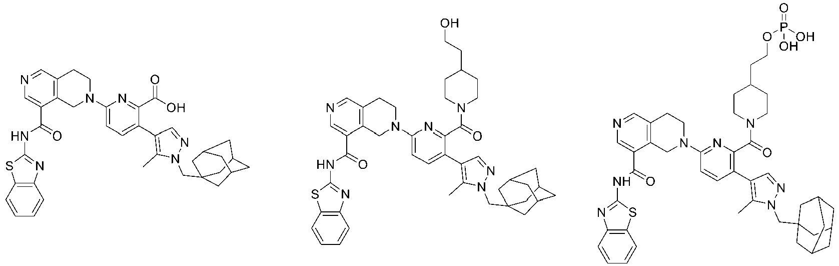

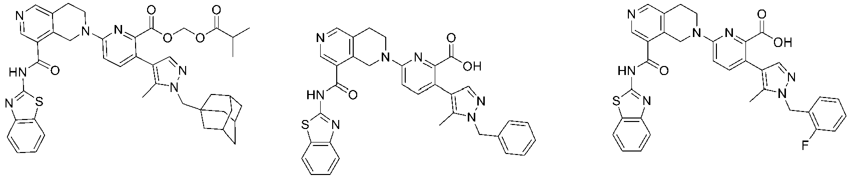

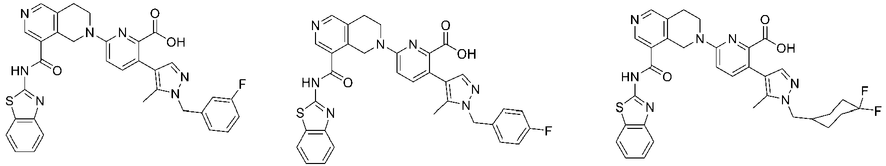

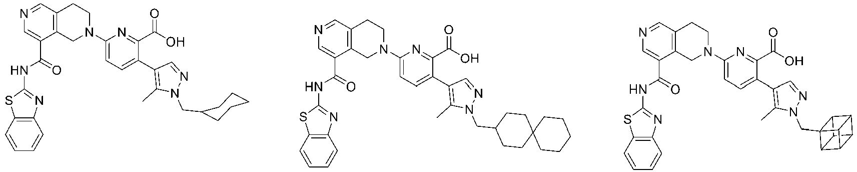

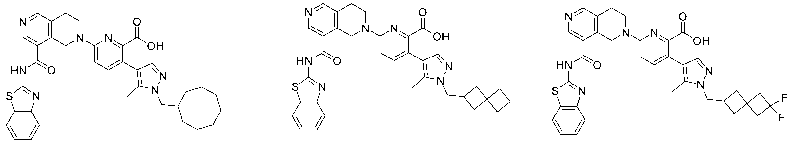

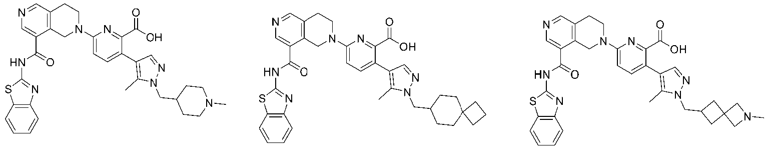

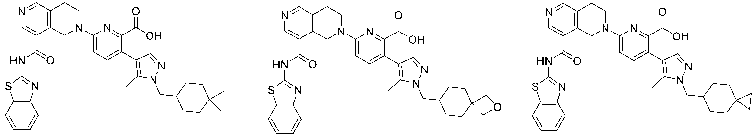

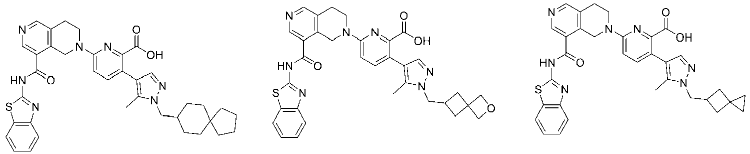

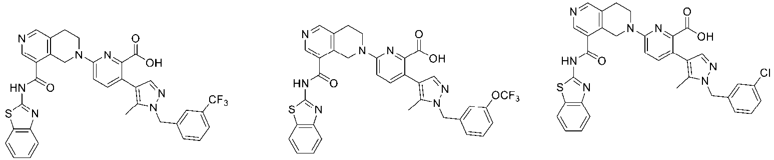

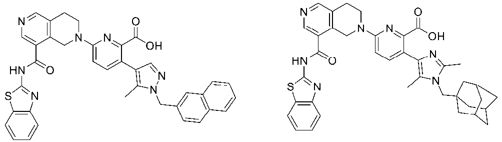

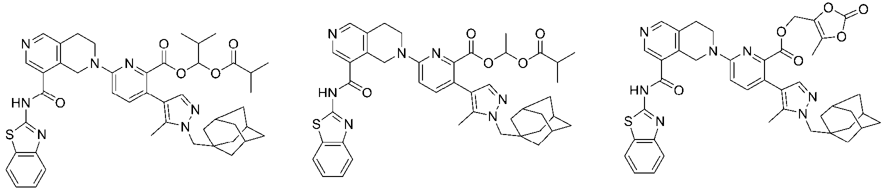

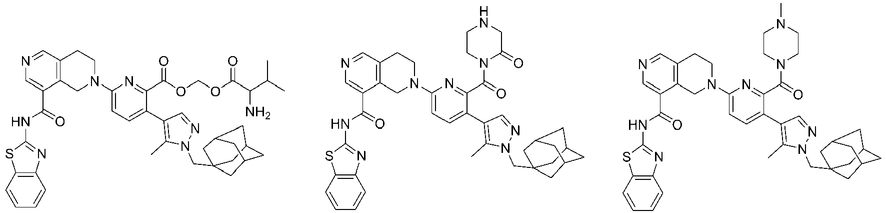

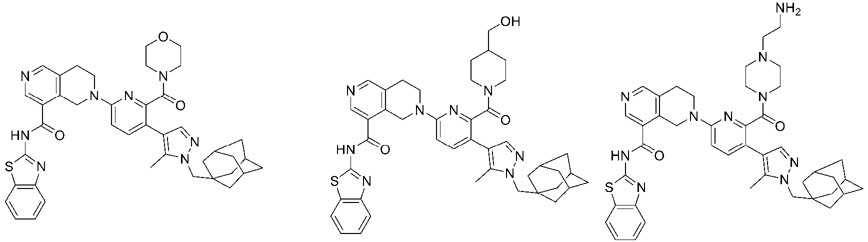

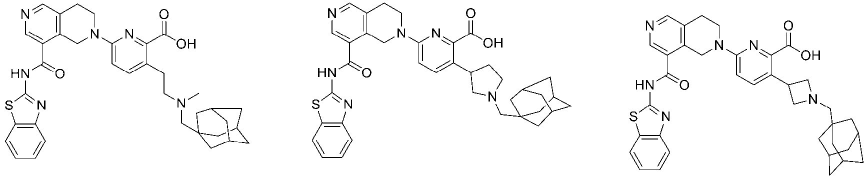

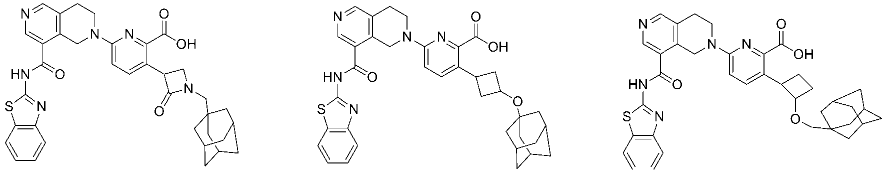

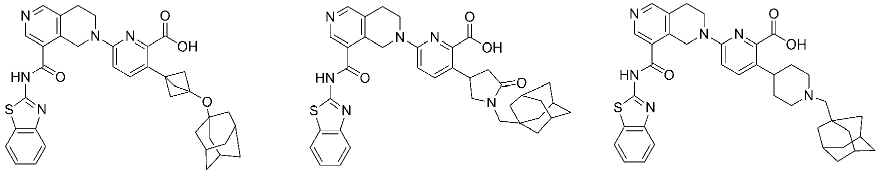

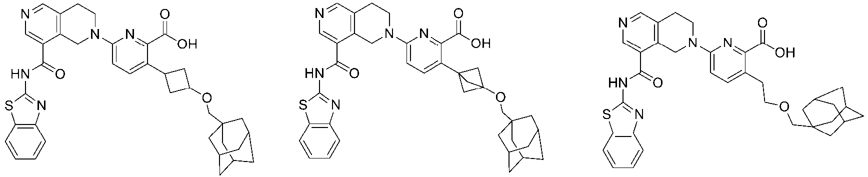

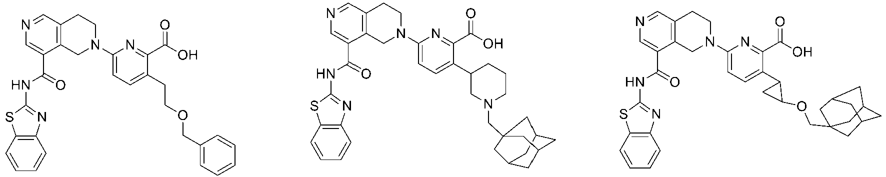

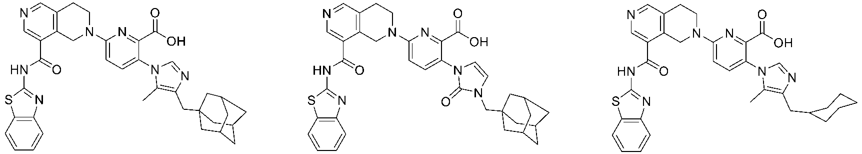

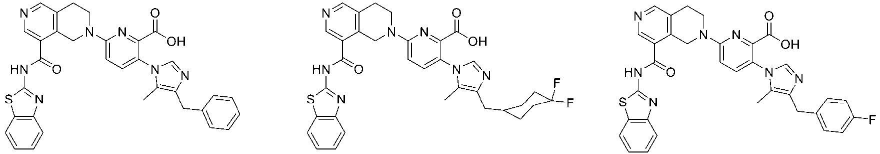

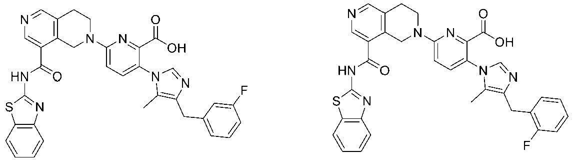

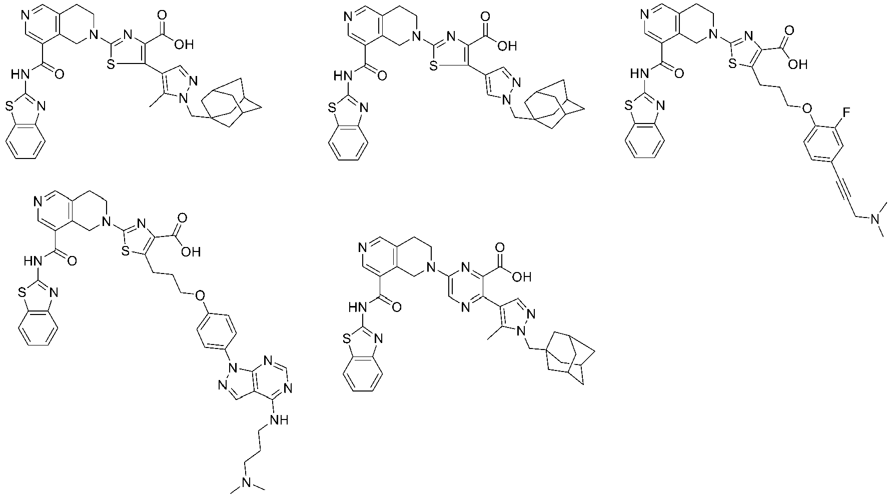

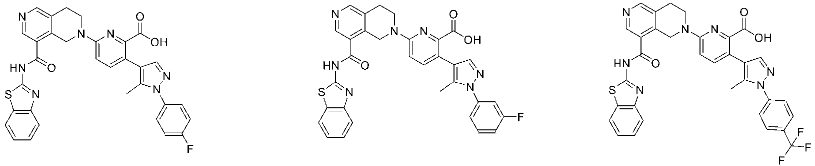

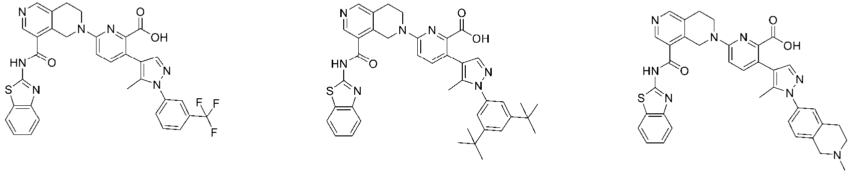

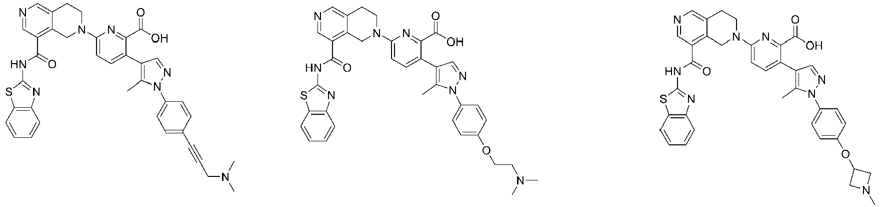

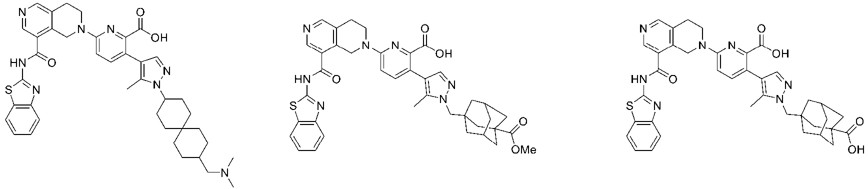

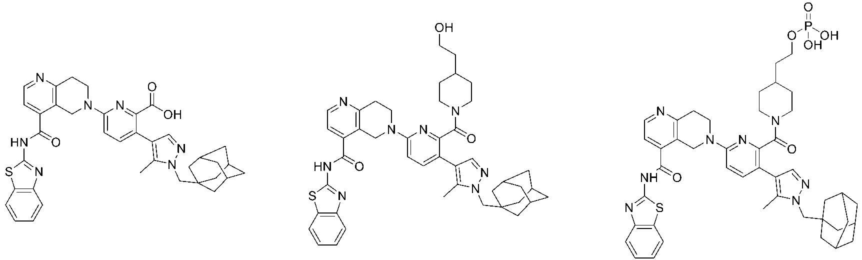

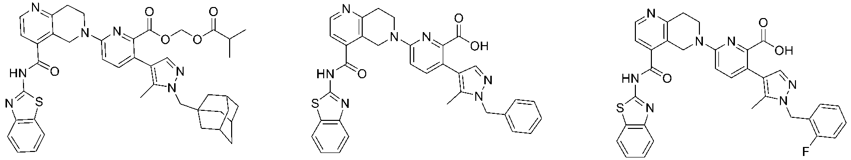

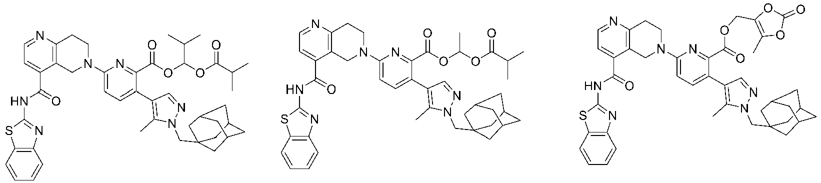

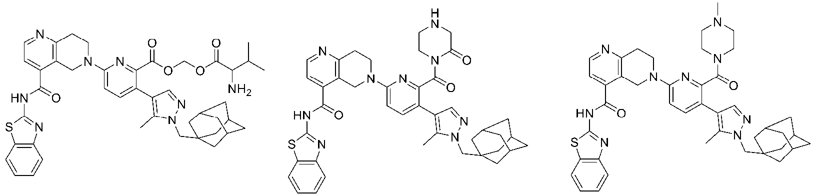

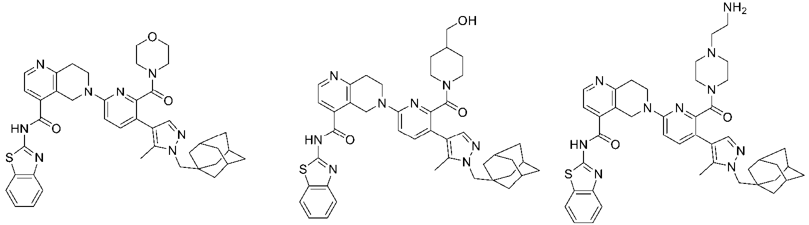

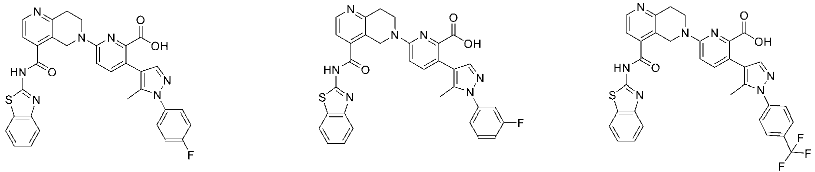

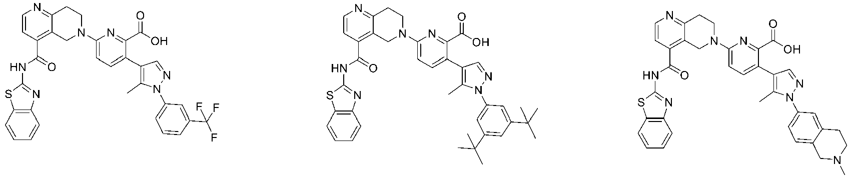

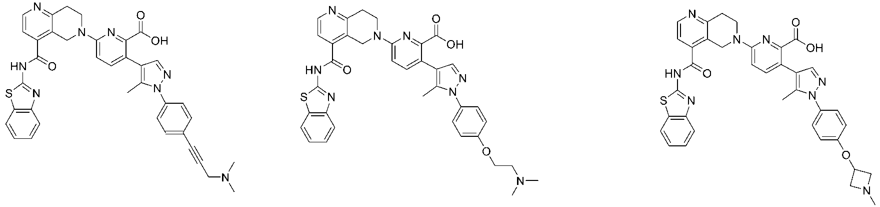

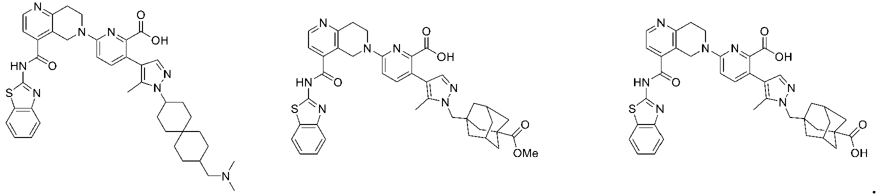

14. The compound, or a pharmaceutically acceptable salt, a solvate, a hydrate, a polymorph, a co-crystal, a tautomer, a stereoisomer, or an isotopic compound thereof according to any one of claims 1 to 13, **characterized in that**, the salt of the compound is an alkali metal salt, preferably a sodium salt.

15. A pharmaceutical composition, comprising the compound, or a pharmaceutically acceptable salt, a solvate, a hydrate, a polymorph, a co-crystal, a tautomer, a stereoisomer, or an isotopic compound thereof of any one of claims 1 to 14, and a pharmaceutically acceptable excipient.

16. A method for preventing or treating aging-related diseases, comprising administering to a subject in need thereof a therapeutically effective amount of the compound or a pharmaceutically acceptable salt, solvate, hydrate, polymorph, co-crystal, tautomer, stereoisomer or isotopic compound thereof of any one of claims 1 to 14, or the pharmaceutical composition of claim 15.

17. The method according to claim 16, **characterized in that**, the disease is selected from diseases associated with the accumulation of senescent cells, and the disease is preferably selected from one or more of idiopathic pulmonary fibrosis, pulmonary fibrosis, liver fibrosis, renal fibrosis, inflammation and tissue fibrosis and atrophy of the upper respiratory tract and lungs caused by viruses, cystic fibrosis, myelofibrosis, myocardial fibrosis, skin fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritic neuropathy, keratitis sicca, Fuchs corneal dystrophy, presbyopia, cataract, degenerative vitreous disorders including vitreomacular traction syndrome, macular hole, retinal tear, retinal detachment, proliferative vitreoretinopathy, osteoarthritis, intervertebral disc herniation, osteoporosis, Alzheimer's disease, Parkinson's disease, atherosclerosis, chronic obstructive pulmonary disease, diabetes, diabetic nephropathy, scar, superficial scar or flat scar, linear scar or contracture scar, webbed scar, depressed scar, atrophic scar, bridged scar and pedunculated scar, hypertrophic scar, keloid, scar carcinoma, scleroderma, morphea, linear scleroderma, guttate scleroderma, acroscleroderma, diffuse scleroderma, CREST syndrome, acute coronary syndrome, myocardial infarction, stroke, hypertension, obesity, adipose dysfunction, coronary artery disease, cerebrovascular disease, periodontal disease, cancer treatment-related disabilities such as atrophy and fibrosis in various tissues, myelodysplastic syndrome associated with brain and heart injury treatment, promyelocytic syndrome, ataxia-telangiectasia, Fanconi anemia, Friedreich's ataxia, dyskeratosis congenita, aplastic anemia, aneurysm, inflammatory bowel disease, lipoatrophy, renal transplant failure, sarcopenia, wound healing, alopecia, cardiomyocyte hypertrophy, glomerulosclerosis, and cancer.

18. Use of the compound or a pharmaceutically acceptable salt, solvate, hydrate, polymorph, co-crystal, tautomer, stereoisomer or isotopic compound thereof of any one of claims 1 to 14, or the pharmaceutical composition of claim 15 in the manufacture of a medicament for the prevention or treatment of aging-related diseases.

19. The use according to claim 16, **characterized in that**, the disease is selected from diseases associated with the accumulation of senescent cells, and the disease is preferably selected from one or more of idiopathic pulmonary fibrosis, pulmonary fibrosis, liver fibrosis, renal fibrosis, inflammation and tissue fibrosis and atrophy of the upper respiratory tract and lungs caused by viruses, cystic fibrosis, myelofibrosis, myocardial fibrosis, skin fibrosis, interstitial lung disease, fibrotic pancreatitis, retinopathy of prematurity, macular degeneration, diabetic macular edema, diabetic retinopathy, age-related macular degeneration, wet age-related macular degeneration, dry age-related macular degeneration, glaucoma, sickle cell retinopathy, ischemic arteritic neuropathy, keratitis sicca, Fuchs corneal dystrophy, presbyopia, cataract, degenerative vitreous disorders including vitreomacular traction syndrome, macular hole, retinal tear, retinal detachment, proliferative vitreoretinopathy, osteoarthritis, intervertebral disc herniation, osteoporosis, Alzheimer's disease, Parkinson's disease, atherosclerosis, chronic obstructive pulmonary disease, diabetes, diabetic nephropathy, scar, superficial scar or flat scar, linear scar or contracture scar, webbed scar, depressed scar, atrophic scar, bridged scar and pedunculated scar, hypertrophic scar, keloid, scar carcinoma, scleroderma, morphea, linear scleroderma, guttate scleroderma, acroscleroderma, diffuse scleroderma, CREST syndrome, acute coronary syndrome, myocardial infarction, stroke, hypertension, obesity, adipose dysfunction, coronary artery disease, cerebrovascular disease, periodontal disease, cancer treatment-related disabilities such as atrophy and fibrosis in various tissues, myelodysplastic syndrome associated with brain and heart injury treatment, promyelocytic syndrome, ataxia-telangiectasia, Fanconi anemia, Friedreich's ataxia, dyskeratosis congenita, aplastic anemia, aneurysm, inflammatory bowel disease, lipoatrophy, renal transplant failure, sarcopenia, wound healing, alopecia, cardiomyocyte hypertrophy, glomerulosclerosis, and cancer.
